# EUROPEAN PATENT APPLICATION

(11) **EP 1 792 913 A1**
(43) Date of publication of application: **06.06.2007**
(21) Application number: 06023488.7
(22) Date of filing: 28.05.1999
(51) Int. Cl.: C07K 14/47, C07K 14/705

(54) **Novel secreted and membrane-associated proteins and uses therefor**

(30) Priority: 29.05.1998 US 86892; 29.05.1998 US 87121
(62) Divisional of application: 99924537.6
(71) Applicant: MILLENNIUM PHARMACEUTICALS, INC., Cambridge, MA 02139 (US)
(72) Inventor: McCarthy, Sean A., San Diego CA 92106 (US)
(74) Representative: Lock, Graham James

(57) **Abstract**

Novel secreted and membrane-associated polypeptides, proteins, and nucleic acid molecules are disclosed (e.g., BDSF and STMST molecules). In addition to isolated, full-length proteins, the invention further provides isolated fusion proteins, antigenic peptides and antibodies. The invention also provides nucleic acid molecules, recombinant expression vectors containing a nucleic acid molecule of the invention, host cells into which the expression vectors have been introduced and non-human transgenic animals in which a BDSF or STMST gene has been introduced or disrupted. Diagnostic, screening and therapeutic methods utilizing compositions of the invention are also provided.

### Annex to the application documents - subsequently filed sequences listing

SEQUENCE LISTING

<110> Millennium Biotherapeutics, Inc.

<120> NOVEL SECRETED AND MEMBRANE-ASSOCIATED PROTEINS AND
      USES THEREFOR

<130> MEI-017CPPC

<140> EP06023488.7
<141> 1999-05-28

<150> USSN 09/086,892
<151> 1998-05-29

<150> USSN 09/087,121
<151> 1998-05-29

<160> 30

<170> PatentIn Ver. 2.0

<210> 1
<211> 1119
<212> DNA
<213> Homo sapiens

<220>
<221> CDS
<222> (140)..(871)

<400> 1
gtcgacccac gcgtccggca ggatgtttgc agtgtcgcgc ccagggctct gagactgagc 60

ctgccatcca ctcgcacgcc tttctttcag ggcttttcgg ctgttggcta cactgatgtg 120

acccccctcc ctttttgga atg atg ggg atc ttt ttg gtg tat gtt gga ttt 172
                    Met Met Gly Ile Phe Leu Val Tyr Val Gly Phe
                     1               5                  10

gtt ttc ttt tcc gtt tta tat gta caa caa ggg ctt tct tct caa gca 220
Val Phe Phe Ser Val Leu Tyr Val Gln Gln Gly Leu Ser Ser Gln Ala
            15                  20                  25

aaa ttt acc gag ttt ccg cgg aac gtg acg gcg acc gag ggg cag aat 268
Lys Phe Thr Glu Phe Pro Arg Asn Val Thr Ala Thr Glu Gly Gln Asn
        30                  35                  40

gtg gag atg tcc tgc gcc ttc cag agc ggc tcc gcc tcg gtg tat ctg 316
Val Glu Met Ser Cys Ala Phe Gln Ser Gly Ser Ala Ser Val Tyr Leu
    45                  50                  55

gag atc caa tgg tgg ttc ctg cgg ggg ccg gag gac ctg gat ccc ggg 364
Glu Ile Gln Trp Trp Phe Leu Arg Gly Pro Glu Asp Leu Asp Pro Gly
60                  65                  70                  75

gcc gag ggg gcc ggc gcg cag gtg gag ctc ttg ccc gac aga gac ccg     412
Ala Glu Gly Ala Gly Ala Gln Val Glu Leu Leu Pro Asp Arg Asp Pro
                80                  85                  90

gac agc gac ggg acc aag atc agc aca gtg aaa gtc caa ggc aat gac     460
Asp Ser Asp Gly Thr Lys Ile Ser Thr Val Lys Val Gln Gly Asn Asp
            95                  100                 105

atc tcc cac aag ctt cag att tcc aaa gtg agg aaa aag gat gaa ggc     508
Ile Ser His Lys Leu Gln Ile Ser Lys Val Arg Lys Lys Asp Glu Gly
        110                 115                 120

tta tat gag tgc agg gtg act gat gcc aac tac ggg gag ctt cag gaa     556
Leu Tyr Glu Cys Arg Val Thr Asp Ala Asn Tyr Gly Glu Leu Gln Glu
    125                 130                 135

cac aag gcc cag gcc tat ctg aaa gtc aat gcc aac agc cat gcc cgc     604
His Lys Ala Gln Ala Tyr Leu Lys Val Asn Ala Asn Ser His Ala Arg
140                 145                 150                 155

aga atg cag gcc ttc gaa gcc tcg ccc atg tgg ctg cag gat atg aag     652
Arg Met Gln Ala Phe Glu Ala Ser Pro Met Trp Leu Gln Asp Met Lys
                160                 165                 170

ccc cgc aag aac gtc tcc gca gcc atc ccc agc agc atc cat ggc tct     700
Pro Arg Lys Asn Val Ser Ala Ala Ile Pro Ser Ser Ile His Gly Ser
            175                 180                 185

gcc aac caa cga acg cac tcc acc tcc agc cct caa gtg gta gcc aaa     748
Ala Asn Gln Arg Thr His Ser Thr Ser Ser Pro Gln Val Val Ala Lys
        190                 195                 200

atc ccc aaa caa agt cca caa tca ggt atg gaa acc cat ttc gag cct     796
Ile Pro Lys Gln Ser Pro Gln Ser Gly Met Glu Thr His Phe Glu Pro
    205                 210                 215

ttt att tta cca ctc aca aac gct cca cag aaa ggt cag tcg tat aga     844
Phe Ile Leu Pro Leu Thr Asn Ala Pro Gln Lys Gly Gln Ser Tyr Arg
220                 225                 230                 235

gta gac aga ttt atg aat ggt gat ttt taaaatcgga gacctagttc           891
Val Asp Arg Phe Met Asn Gly Asp Phe
                240

agtgcaagtg attatgagag gtgagcactg agcctgcacc aattcactca gagctcaaag 951

catgtgggtg caccccgtca gtcccctagt ggtgcttcat ttccagggca tctgagagct 1011

ggactctggt tttatccttt tctgtattta cacattataa gaacaataaa tcatgtaatg 1071

ttggttacat tacaaaaaaa aaaaaaaaaa aaaaaaaagg gcggccgc              1119

<210> 2
<211> 244
<212> PRT
<213> Homo sapiens

<400> 2
Met Met Gly Ile Phe Leu Val Tyr Val Gly Phe Val Phe Phe Ser Val
1               5                   10                  15

Leu Tyr Val Gln Gln Gly Leu Ser Ser Gln Ala Lys Phe Thr Glu Phe
            20                  25                  30

Pro Arg Asn Val Thr Ala Thr Glu Gly Gln Asn Val Glu Met Ser Cys
        35                  40                  45

Ala Phe Gln Ser Gly Ser Ala Ser Val Tyr Leu Glu Ile Gln Trp Trp
    50                  55                  60

Phe Leu Arg Gly Pro Glu Asp Leu Asp Pro Gly Ala Glu Gly Ala Gly
65                  70                  75                  80

Ala Gln Val Glu Leu Leu Pro Asp Arg Asp Pro Asp Ser Asp Gly Thr
                85                  90                  95

Lys Ile Ser Thr Val Lys Val Gln Gly Asn Asp Ile Ser His Lys Leu
            100                 105                 110

Gln Ile Ser Lys Val Arg Lys Lys Asp Glu Gly Leu Tyr Glu Cys Arg
        115                 120                 125

Val Thr Asp Ala Asn Tyr Gly Glu Leu Gln Glu His Lys Ala Gln Ala
    130                 135                 140

Tyr Leu Lys Val Asn Ala Asn Ser His Ala Arg Arg Met Gln Ala Phe
145                 150                 155                 160

Glu Ala Ser Pro Met Trp Leu Gln Asp Met Lys Pro Arg Lys Asn Val
                165                 170                 175

Ser Ala Ala Ile Pro Ser Ser Ile His Gly Ser Ala Asn Gln Arg Thr
            180                 185                 190

His Ser Thr Ser Ser Pro Gln Val Val Ala Lys Ile Pro Lys Gln Ser
        195                 200                 205

Pro Gln Ser Gly Met Glu Thr His Phe Glu Pro Phe Ile Leu Pro Leu
    210                 215                 220

Thr Asn Ala Pro Gln Lys Gly Gln Ser Tyr Arg Val Asp Arg Phe Met
225                 230                 235                 240

Asn Gly Asp Phe

<210> 3
<211> 732
<212> DNA
<213> Homo sapiens

<220>
<221> CDS
<222> (1)..(732)

<400> 3
atg atg ggg atc ttt ttg gtg tat gtt gga ttt gtt ttc ttt tcc gtt     48
Met Met Gly Ile Phe Leu Val Tyr Val Gly Phe Val Phe Phe Ser Val
1               5                   10                  15

tta tat gta caa caa ggg ctt tct tct caa gca aaa ttt acc gag ttt     96
Leu Tyr Val Gln Gln Gly Leu Ser Ser Gln Ala Lys Phe Thr Glu Phe
            20                  25                  30

ccg cgg aac gtg acg gcg acc gag ggg cag aat gtg gag atg tcc tgc     144
Pro Arg Asn Val Thr Ala Thr Glu Gly Gln Asn Val Glu Met Ser Cys
        35                  40                  45

gcc ttc cag agc ggc tcc gcc tcg gtg tat ctg gag atc caa tgg tgg     192
Ala Phe Gln Ser Gly Ser Ala Ser Val Tyr Leu Glu Ile Gln Trp Trp
    50                  55                  60

ttc ctg cgg ggg ccg gag gac ctg gat ccc ggg gcc gag ggg gcc ggc     240
Phe Leu Arg Gly Pro Glu Asp Leu Asp Pro Gly Ala Glu Gly Ala Gly
65                  70                  75                  80

gcg cag gtg gag ctc ttg ccc gac aga gac ccg gac agc gac ggg acc     288
Ala Gln Val Glu Leu Leu Pro Asp Arg Asp Pro Asp Ser Asp Gly Thr
                85                  90                  95

aag atc agc aca gtg aaa gtc caa ggc aat gac atc tcc cac aag ctt     336
Lys Ile Ser Thr Val Lys Val Gln Gly Asn Asp Ile Ser His Lys Leu
            100                 105                 110

cag att tcc aaa gtg agg aaa aag gat gaa ggc tta tat gag tgc agg     384
Gln Ile Ser Lys Val Arg Lys Lys Asp Glu Gly Leu Tyr Glu Cys Arg
        115                 120                 125

gtg act gat gcc aac tac ggg gag ctt cag gaa cac aag gcc cag gcc     432
Val Thr Asp Ala Asn Tyr Gly Glu Leu Gln Glu His Lys Ala Gln Ala
    130                 135                 140

tat ctg aaa gtc aat gcc aac agc cat gcc cgc aga atg cag gcc ttc     480
Tyr Leu Lys Val Asn Ala Asn Ser His Ala Arg Arg Met Gln Ala Phe
145                 150                 155                 160

gaa gcc tcg ccc atg tgg ctg cag gat atg aag ccc cgc aag aac gtc     528
Glu Ala Ser Pro Met Trp Leu Gln Asp Met Lys Pro Arg Lys Asn Val
                165                 170                 175

tcc gca gcc atc ccc agc agc atc cat ggc tct gcc aac caa cga acg     576
Ser Ala Ala Ile Pro Ser Ser Ile His Gly Ser Ala Asn Gln Arg Thr
            180                 185                 190

cac tcc acc tcc agc cct caa gtg gta gcc aaa atc ccc aaa caa agt     624
His Ser Thr Ser Ser Pro Gln Val Val Ala Lys Ile Pro Lys Gln Ser
        195                 200                 205

cca caa tca ggt atg gaa acc cat ttc gag cct ttt att tta cca ctc     672
Pro Gln Ser Gly Met Glu Thr His Phe Glu Pro Phe Ile Leu Pro Leu
    210                 215                 220

aca aac gct cca cag aaa ggt cag tcg tat aga gta gac aga ttt atg     720
Thr Asn Ala Pro Gln Lys Gly Gln Ser Tyr Arg Val Asp Arg Phe Met
225                 230                 235                 240

aat ggt gat ttt                                                     732
Asn Gly Asp Phe

<210> 4
<211> 660
<212> DNA
<213> Homo sapiens

<220>
<221> CDS
<222> (1)..(657)

<400> 4
caa gca aaa ttt acc gag ttt ccg cgg aac gtg acg gcg acc gag ggg     48
Gln Ala Lys Phe Thr Glu Phe Pro Arg Asn Val Thr Ala Thr Glu Gly
1               5                   10                  15

cag aat gtg gag atg tcc tgc gcc ttc cag agc ggc tcc gcc tcg gtg     96
Gln Asn Val Glu Met Ser Cys Ala Phe Gln Ser Gly Ser Ala Ser Val
            20                  25                  30

tat ctg gag atc caa tgg tgg ttc ctg cgg ggg ccg gag gac ctg gat     144
Tyr Leu Glu Ile Gln Trp Trp Phe Leu Arg Gly Pro Glu Asp Leu Asp
        35                  40                  45

ccc ggg gcc gag ggg gcc ggc gcg cag gtg gag ctc ttg ccc gac aga     192
Pro Gly Ala Glu Gly Ala Gly Ala Gln Val Glu Leu Leu Pro Asp Arg
    50                  55                  60

gac ccg gac agc gac ggg acc aag atc agc aca gtg aaa gtc caa ggc     240
Asp Pro Asp Ser Asp Gly Thr Lys Ile Ser Thr Val Lys Val Gln Gly
65                  70                  75                  80

aat gac atc tcc cac aag ctt cag att tcc aaa gtg agg aaa aag gat     288
Asn Asp Ile Ser His Lys Leu Gln Ile Ser Lys Val Arg Lys Lys Asp
                85                  90                  95

gaa ggc tta tat gag tgc agg gtg act gat gcc aac tac ggg gag ctt     336
Glu Gly Leu Tyr Glu Cys Arg Val Thr Asp Ala Asn Tyr Gly Glu Leu
            100                 105                 110

cag gaa cac aag gcc cag gcc tat ctg aaa gtc aat gcc aac agc cat     384
Gln Glu His Lys Ala Gln Ala Tyr Leu Lys Val Asn Ala Asn Ser His
        115                 120                 125

gcc cgc aga atg cag gcc ttc gaa gcc tcg ccc atg tgg ctg cag gat     432
Ala Arg Arg Met Gln Ala Phe Glu Ala Ser Pro Met Trp Leu Gln Asp
    130                 135                 140

atg aag ccc cgc aag aac gtc tcc gca gcc atc ccc agc agc atc cat     480
Met Lys Pro Arg Lys Asn Val Ser Ala Ala Ile Pro Ser Ser Ile His
145                 150                 155                 160

ggc tct gcc aac caa cga acg cac tcc acc tcc agc cct caa gtg gta 528
Gly Ser Ala Asn Gln Arg Thr His Ser Thr Ser Ser Pro Gln Val Val
165 170 . 175

gcc aaa atc ccc aaa caa agt cca caa tca ggt atg gaa acc cat ttc 576
Ala Lys Ile Pro Lys Gln Ser Pro Gln Ser Gly Met Glu Thr His Phe
180 185 190

gag cct ttt att tta cca ctc aca aac gct cca cag aaa ggt cag tcg 624
Glu Pro Phe Ile Leu Pro Leu Thr Asn Ala Pro Gln Lys Gly Gln Ser
195 200 205

tat aga gta gac aga ttt atg aat ggt gat ttt taa 660
Tyr Arg Val Asp Arg Phe Met Asn Gly Asp Phe
210 215

<210> 5
<211> 219
<212> PRT
<213> Homo sapiens

<400> 5
Gln Ala Lys Phe Thr Glu Phe Pro Arg Asn Val Thr Ala Thr Glu Gly
1               5                   10                  15

Gln Asn Val Glu Met Ser Cys Ala Phe Gln Ser Gly Ser Ala Ser Val
            20                  25                  30

Tyr Leu Glu Ile Gln Trp Trp Phe Leu Arg Gly Pro Glu Asp Leu Asp
        35                  40                  45

Pro Gly Ala Glu Gly Ala Gly Ala Gln Val Glu Leu Leu Pro Asp Arg
    50                  55                  60

Asp Pro Asp Ser Asp Gly Thr Lys Ile Ser Thr Val Lys Val Gln Gly
65                  70                  75                  80

Asn Asp Ile Ser His Lys Leu Gln Ile Ser Lys Val Arg Lys Lys Asp
                85                  90                  95

Glu Gly Leu Tyr Glu Cys Arg Val Thr Asp Ala Asn Tyr Gly Glu Leu
            100                 105                 110

Gln Glu His Lys Ala Gln Ala Tyr Leu Lys Val Asn Ala Asn Ser His
        115                 120                 125

Ala Arg Arg Met Gln Ala Phe Glu Ala Ser Pro Met Trp Leu Gln Asp
    130                 135                 140

Met Lys Pro Arg Lys Asn Val Ser Ala Ala Ile Pro Ser Ser Ile His
145                 150                 155                 160

Gly Ser Ala Asn Gln Arg Thr His Ser Thr Ser Ser Pro Gln Val Val
                165                 170                 175

Ala Lys Ile Pro Lys Gln Ser Pro Gln Ser Gly Met Glu Thr His Phe
            180                 185                 190

Glu Pro Phe Ile Leu Pro Leu Thr Asn Ala Pro Gln Lys Gly Gln Ser
        195                 200                 205

Tyr Arg Val Asp Arg Phe Met Asn Gly Asp Phe
    210                 215

<210> 6
<211> 3196
<212> DNA
<213> Mus musculus

<220>
<221> CDS
<222> (268)..(1020)

<400> 6
cacgcgtccg cagccagccg gcggcggaga cacttcacgg cgtggcaacc cgggtctgtg 60

ccttgaagcc tccggatcgc agccagctcg gtccatccct cactagtcgc aatcccctgt 120

gtccaagcta ctctttgcta tgagcggcag catgcgtgca gtatcgcgcc ccaggctctg 180

agagcagcct gcggacacgc ttgcctatct gtctttttag gttttggggc tctgggctac 240

acggatgtgc cccactccct tggcatg atg ggg atc ttt ttg gcg tct gtt gga 294
Met Gly Ile Phe Leu Ala Ser Val Gly
1               5

ttt atg ttc ttt tcc gtg tta tat gta caa caa ggg ctt tct tct caa 342
Phe Met Phe Phe Ser Val Leu Tyr Val Gln Gln Gly Leu Ser Ser Gln
10                  15                  20                  25

gca aaa ttt acc gag ttg ccg aga aat gtg act gct acc gaa ggg caa 390
Ala Lys Phe Thr Glu Leu Pro Arg Asn Val Thr Ala Thr Glu Gly Gln
                30                  35                  40

aat gtg gag atg tcc tgt gct ttc caa agc ggc tct gct tca gtg tac 438
Asn Val Glu Met Ser Cys Ala Phe Gln Ser Gly Ser Ala Ser Val Tyr
            45                  50                  55

ctg gag atc cag tgg tgg ttc ctt cgg ggg cca gag gac ctg gag caa 486
Leu Glu Ile Gln Trp Trp Phe Leu Arg Gly Pro Glu Asp Leu Glu Gln
        60                  65                  70

ggc acg gag gct gca ggc tcg cag gtg gag ctc tta ccc gac aga gac 534
Gly Thr Glu Ala Ala Gly Ser Gln Val Glu Leu Leu Pro Asp Arg Asp
    75                  80                  85

ccg gac aac gat ggg acc aag att agt aca gtg aaa gtc caa ggc aat 582
Pro Asp Asn Asp Gly Thr Lys Ile Ser Thr Val Lys Val Gln Gly Asn
90                  95                  100                 105

gat atc tcc cac aag ctt cag ata tcc aaa gtg aga aaa aag gat gaa 630
Asp Ile Ser His Lys Leu Gln Ile Ser Lys Val Arg Lys Lys Asp Glu
                110                 115                 120

ggt tta tac gag tgc agg gtg act gac gct aac tac ggg gag ctt cag 678
Gly Leu Tyr Glu Cys Arg Val Thr Asp Ala Asn Tyr Gly Glu Leu Gln
            125                 130                 135

gaa cac aag gcc cag gcc tat ctg aaa gtc aat gcc aac agc cat gct 726
Glu His Lys Ala Gln Ala Tyr Leu Lys Val Asn Ala Asn Ser His Ala
        140                 145                 150

cgg agg atg cag gcc ttt gaa gcc tca cct atg tgg ctg caa gac acg 774
Arg Arg Met Gln Ala Phe Glu Ala Ser Pro Met Trp Leu Gln Asp Thr
    155                 160                 165

aag cct cga aag aac gca tca tcg gtg gtt ccc agc agc gtc cac aac 822
Lys Pro Arg Lys Asn Ala Ser Ser Val Val Pro Ser Ser Val His Asn
170                 175                 180                 185

tct gcc aac caa cga atg cac tcc acc tcc agc cct caa gcg gta gcc 870
Ser Ala Asn Gln Arg Met His Ser Thr Ser Ser Pro Gln Ala Val Ala
                190                 195                 200

aaa atc ccc aag caa agt cca caa tca gca aag agc aaa tcg cct gta 918
Lys Ile Pro Lys Gln Ser Pro Gln Ser Ala Lys Ser Lys Ser Pro Val
            205                 210                 215

aaa tct acg gag cgg aca gca aag ttg acc cta tac tcc aag cac cat 966
Lys Ser Thr Glu Arg Thr Ala Lys Leu Thr Leu Tyr Ser Lys His His
        220                 225                 230

tct gca ccc ctg tac tct agt tat cta cac aag gag cat cag ctt ccg 1014
Ser Ala Pro Leu Tyr Ser Ser Tyr Leu His Lys Glu His Gln Leu Pro
    235                 240                 245

gaa gca taagtgaaga cactgtcaca cgctttattg ataatatttt ctttgggaag 1070
Glu Ala
250

ttgctgatct tttatttcaa gagaattaat gggaagagat aggacatttt ccaattacaa 1130

gaccaatttt tttcctttta tttcaacaaa taaaacctgc atttcactga ctgctcagga 1190

gttggcctga atgacatcag tatactaaat atttccatgg attccaccaa tttcctaacg 1250

agggacacct aatcttcaag aagcaaacaa agatggaaaa cctaagaacc acaaactgtc 1310

tcatacagca ccccagctga ggaacaaaac aaatagctaa atgctgacca tggcaaatca 1370

acatcagaca actttatttt acatatggaa taatcaaaga aagttttttt tttacttcct 1430

ttttgccccc tggaatttat cttggagttt cccttttttc cttgattgcc gttttcgttc 1490

aatggtagca agtgccaatt atggccaatc cttgtcaatc ctggaaggtt tatattcata 1550

tacattgagt gtggtatata tcaatgtatt ttaattcatt tggcaatttc tgtataggca 1610

aacctggcaa attctgtaaa ttgcttatag tatgtgtgat atgacttcaa ggtagatagg 1670

ctatgatgct catgcaagct gactttcttc attctatata caaatatatt catgagcata 1730

tattaggcca ccaacttctt ttcctaaaga attatttttc atttgtacct catgtatttt 1790

gtgaattttg tagtatattt ctctgttcca ctagtttgac cgctacagtt tgtctctgtt 1850

gtcctctact tccttctgga aaaatttaaa attgtgtatg tctctgataa atgaattaat 1910

tttgttgtgt gtatgctatg ttggaatttg ctgtgttctt ttaaacatgt atttattaag 1970

gtttggggat cttgagttga gtctgaagaa tgcacacctg gtttttgaca gagttcctca 2030

tgttaccaat attctatctc agagaaagaa agacaccaag tgggaaaact aagaagacat 2090

tttgacttcc caagatcctg gaagagcact tcacactctg actaaataat gttgcttttt 2150

ttgttcttca agactttttt gtagctttgt ctttctgtta gttgctgcta attatatttt 2210

aatgtctact aattaaaaat taaaatgtga ttgttggctg aatacaatat gcaaatgact 2270

gcaaagccca tactgaagaa aatagatgtt taatcttcac tcaataatta taattttaaa 2330

tagttcatca ttattttttg accttatgat attttgttta gacctgttct aattacatct 2390

ttctctggca aagaaagata gaacaatcaa tacattccct cttacagtat ggaatggttg 2450

tggcttaaga aagaatgcat ccagatggtc ttccagagag attattttat tttcattata 2510

aaaccagaaa ccatatatgt aggaatggtt cattcctaat gtaaggccat aaattgtagc 2570

ttgaaggcaa ggaatacatt tgttttttta tggtaaagga ctggcctctg acatgcactt 2630

ataagcaatg tgaatatttt cataatatgc ttgacattct cctttaacaa atattgtttt 2690

atggtaaatc tttccttgcc atttttcttc tttcatttga ttcattattt cattctaatg 2750

aagaaaataa aggtttaatt atgatacttt attaacatac aaatgtattt tctttctaag 2810

ttaaatatct gaaagttgta taaaatgatg gtagagaaat attactcatt cggtttcttt 2870

gagctttaag aatcccatac attgcagtat atattagaat actgatttaa catcaaactg 2930

ggggggaaaa tcatgtatta tacttttact caatgtctag gtaatggatt cagctaattt 2990

tacagcaagc caaatgtgta cccgtatcag taatgttcac catgcttgta ataaaagggc 3050

atatgctagt tttggaagaa tgctcattag attcattgta tcagtgtcca aaataataaa 3110

gacctgttta tcactgtgaa aaaaaaaaaa aaaaaaaaaa aaaaaaaaaa aaaaaaaaaa 3170

aaaaaaaaaa aaaaaagggc ggccgc 3196

<210> 7
<211> 251
<212> PRT
<213> Mus musculus

<400> 7
Met Gly Ile Phe Leu Ala Ser Val Gly Phe Met Phe Phe Ser Val Leu
1               5                   10                  15

Tyr Val Gln Gln Gly Leu Ser Ser Gln Ala Lys Phe Thr Glu Leu Pro
            20                  25                  30

Arg Asn Val Thr Ala Thr Glu Gly Gln Asn Val Glu Met Ser Cys Ala
        35                  40                  45

Phe Gln Ser Gly Ser Ala Ser Val Tyr Leu Glu Ile Gln Trp Trp Phe
    50                  55                  60

Leu Arg Gly Pro Glu Asp Leu Glu Gln Gly Thr Glu Ala Ala Gly Ser
65                  70                  75                  80

Gln Val Glu Leu Leu Pro Asp Arg Asp Pro Asp Asn Asp Gly Thr Lys
                85                  90                  95

Ile Ser Thr Val Lys Val Gln Gly Asn Asp Ile Ser His Lys Leu Gln
            100                 105                 110

Ile Ser Lys Val Arg Lys Lys Asp Glu Gly Leu Tyr Glu Cys Arg Val
        115                 120                 125

Thr Asp Ala Asn Tyr Gly Glu Leu Gln Glu His Lys Ala Gln Ala Tyr
    130                 135                 140

Leu Lys Val Asn Ala Asn Ser His Ala Arg Arg Met Gln Ala Phe Glu
145                 150                 155                 160

Ala Ser Pro Met Trp Leu Gln Asp Thr Lys Pro Arg Lys Asn Ala Ser
                165                 170                 175

Ser Val Val Pro Ser Ser Val His Asn Ser Ala Asn Gln Arg Met His
            180                 185                 190

Ser Thr Ser Ser Pro Gln Ala Val Ala Lys Ile Pro Lys Gln Ser Pro
        195                 200                 205

Gln Ser Ala Lys Ser Lys Ser Pro Val Lys Ser Thr Glu Arg Thr Ala
    210                 215                 220

Lys Leu Thr Leu Tyr Ser Lys His His Ser Ala Pro Leu Tyr Ser Ser
225                 230                 235                 240

Tyr Leu His Lys Glu His Gln Leu Pro Glu Ala
                245                 250


<210> 8
<211> 753
<212> DNA
<213> Mus musculus

<220>
<221> CDS
<222> (1)..(753)

<400> 8
atg ggg atc ttt ttg gcg tct gtt gga ttt atg ttc ttt tcc gtg tta     48
Met Gly Ile Phe Leu Ala Ser Val Gly Phe Met Phe Phe Ser Val Leu
1               5                   10                  15

tat gta caa caa ggg ctt tct tct caa gca aaa ttt acc gag ttg ccg     96
Tyr Val Gln Gln Gly Leu Ser Ser Gln Ala Lys Phe Thr Glu Leu Pro
            20                  25                  30

aga aat gtg act gct acc gaa ggg caa aat gtg gag atg tcc tgt gct     144
Arg Asn Val Thr Ala Thr Glu Gly Gln Asn Val Glu Met Ser Cys Ala
        35                  40                  45

ttc caa agc ggc tct gct tca gtg tac ctg gag atc cag tgg tgg ttc     192
Phe Gln Ser Gly Ser Ala Ser Val Tyr Leu Glu Ile Gln Trp Trp Phe
    50                  55                  60

ctt cgg ggg cca gag gac ctg gag caa ggc acg gag gct gca ggc tcg     240
Leu Arg Gly Pro Glu Asp Leu Glu Gln Gly Thr Glu Ala Ala Gly Ser
65                  70                  75                  80

cag gtg gag ctc tta ccc gac aga gac ccg gac aac gat ggg acc aag     288
Gln Val Glu Leu Leu Pro Asp Arg Asp Pro Asp Asn Asp Gly Thr Lys
                85                  90                  95

att agt aca gtg aaa gtc caa ggc aat gat atc tcc cac aag ctt cag     336
Ile Ser Thr Val Lys Val Gln Gly Asn Asp Ile Ser His Lys Leu Gln
            100                 105                 110

ata tcc aaa gtg aga aaa aag gat gaa ggt tta tac gag tgc agg gtg     384
Ile Ser Lys Val Arg Lys Lys Asp Glu Gly Leu Tyr Glu Cys Arg Val
        115                 120                 125

act gac gct aac tac ggg gag ctt cag gaa cac aag gcc cag gcc tat     432
Thr Asp Ala Asn Tyr Gly Glu Leu Gln Glu His Lys Ala Gln Ala Tyr
    130                 135                 140

ctg aaa gtc aat gcc aac agc cat gct cgg agg atg cag gcc ttt gaa     480
Leu Lys Val Asn Ala Asn Ser His Ala Arg Arg Met Gln Ala Phe Glu
145                 150                 155                 160

gcc tca cct atg tgg ctg caa gac acg aag cct cga aag aac gca tca     528
Ala Ser Pro Met Trp Leu Gln Asp Thr Lys Pro Arg Lys Asn Ala Ser
                165                 170                 175

tcg gtg gtt ccc agc agc gtc cac aac tct gcc aac caa cga atg cac     576
Ser Val Val Pro Ser Ser Val His Asn Ser Ala Asn Gln Arg Met His
            180                 185                 190

tcc acc tcc agc cct caa gcg gta gcc aaa atc ccc aag caa agt cca     624
Ser Thr Ser Ser Pro Gln Ala Val Ala Lys Ile Pro Lys Gln Ser Pro
        195                 200                 205

caa tca gca aag agc aaa tcg cct gta aaa tct acg gag cgg aca gca     672
Gln Ser Ala Lys Ser Lys Ser Pro Val Lys Ser Thr Glu Arg Thr Ala
    210                 215                 220

aag ttg acc cta tac tcc aag cac cat tct gca ccc ctg tac tct agt     720
Lys Leu Thr Leu Tyr Ser Lys His His Ser Ala Pro Leu Tyr Ser Ser
225                 230                 235                 240

tat cta cac aag gag cat cag ctt ccg gaa gca                         753
Tyr Leu His Lys Glu His Gln Leu Pro Glu Ala
                245                 250

<210> 9
<211> 681
<212> DNA
<213> Mus musculus

<220>
<221> CDS
<222> (1)..(681)

<400> 9
caa gca aaa ttt acc gag ttg ccg aga aat gtg act gct acc gaa ggg     48
Gln Ala Lys Phe Thr Glu Leu Pro Arg Asn Val Thr Ala Thr Glu Gly
  1               5                  10                  15

caa aat gtg gag atg tcc tgt gct ttc caa agc ggc tct gct tca gtg     96
Gln Asn Val Glu Met Ser Cys Ala Phe Gln Ser Gly Ser Ala Ser Val
             20                  25                  30

tac ctg gag atc cag tgg tgg ttc ctt cgg ggg cca gag gac ctg gag     144
Tyr Leu Glu Ile Gln Trp Trp Phe Leu Arg Gly Pro Glu Asp Leu Glu
         35                  40                  45

caa ggc acg gag gct gca ggc tcg cag gtg gag ctc tta ccc gac aga     192
Gln Gly Thr Glu Ala Ala Gly Ser Gln Val Glu Leu Leu Pro Asp Arg
     50                  55                  60

gac ccg gac aac gat ggg acc aag att agt aca gtg aaa gtc caa ggc     240
Asp Pro Asp Asn Asp Gly Thr Lys Ile Ser Thr Val Lys Val Gln Gly
 65                  70                  75                  80

aat gat atc tcc cac aag ctt cag ata tcc aaa gtg aga aaa aag gat     288
Asn Asp Ile Ser His Lys Leu Gln Ile Ser Lys Val Arg Lys Lys Asp
                 85                  90                  95

gaa ggt tta tac gag tgc agg gtg act gac gct aac tac ggg gag ctt     336
Glu Gly Leu Tyr Glu Cys Arg Val Thr Asp Ala Asn Tyr Gly Glu Leu
            100                 105                 110

cag gaa cac aag gcc cag gcc tat ctg aaa gtc aat gcc aac agc cat     384
Gln Glu His Lys Ala Gln Ala Tyr Leu Lys Val Asn Ala Asn Ser His
        115                 120                 125

gct cgg agg atg cag gcc ttt gaa gcc tca cct atg tgg ctg caa gac     432
Ala Arg Arg Met Gln Ala Phe Glu Ala Ser Pro Met Trp Leu Gln Asp
    130                 135                 140

acg aag cct cga aag aac gca tca tcg gtg gtt ccc agc agc gtc cac     480
Thr Lys Pro Arg Lys Asn Ala Ser Ser Val Val Pro Ser Ser Val His
145                 150                 155                 160

aac tct gcc aac caa cga atg cac tcc acc tcc agc cct caa gcg gta 528
Asn Ser Ala Asn Gln Arg Met His Ser Thr Ser Ser Pro Gln Ala Val
                165                 170                 175

gcc aaa atc ccc aag caa agt cca caa tca gca aag agc aaa tcg cct 576
Ala Lys Ile Pro Lys Gln Ser Pro Gln Ser Ala Lys Ser Lys Ser Pro
            180                 185                 190

gta aaa tct acg gag cgg aca gca aag ttg acc cta tac tcc aag cac 624
Val Lys Ser Thr Glu Arg Thr Ala Lys Leu Thr Leu Tyr Ser Lys His
        195                 200                 205

cat tct gca ccc ctg tac tct agt tat cta cac aag gag cat cag ctt 672
His Ser Ala Pro Leu Tyr Ser Ser Tyr Leu His Lys Glu His Gln Leu
    210                 215                 220

ccg gaa gca 681
Pro Glu Ala
225

<210> 10
<211> 227
<212> PRT
<213> Mus musculus

<400> 10
Gln Ala Lys Phe Thr Glu Leu Pro Arg Asn Val Thr Ala Thr Glu Gly
1               5                   10                  15

Gln Asn Val Glu Met Ser Cys Ala Phe Gln Ser Gly Ser Ala Ser Val
            20                  25                  30

Tyr Leu Glu Ile Gln Trp Trp Phe Leu Arg Gly Pro Glu Asp Leu Glu
        35                  40                  45

Gln Gly Thr Glu Ala Ala Gly Ser Gln Val Glu Leu Leu Pro Asp Arg
    50                  55                  60

Asp Pro Asp Asn Asp Gly Thr Lys Ile Ser Thr Val Lys Val Gln Gly
65                  70                  75                  80

Asn Asp Ile Ser His Lys Leu Gln Ile Ser Lys Val Arg Lys Lys Asp
                85                  90                  95

Glu Gly Leu Tyr Glu Cys Arg Val Thr Asp Ala Asn Tyr Gly Glu Leu
            100                 105                 110

Gln Glu His Lys Ala Gln Ala Tyr Leu Lys Val Asn Ala Asn Ser His
        115                 120                 125

Ala Arg Arg Met Gln Ala Phe Glu Ala Ser Pro Met Trp Leu Gln Asp
    130                 135                 140

Thr Lys Pro Arg Lys Asn Ala Ser Ser Val Val Pro Ser Ser Val His
145                 150                 155                 160

Asn Ser Ala Asn Gln Arg Met His Ser Thr Ser Ser Pro Gln Ala Val
                165                 170                 175

Ala Lys Ile Pro Lys Gln Ser Pro Gln Ser Ala Lys Ser Lys Ser Pro
            180                 185                 190

Val Lys Ser Thr Glu Arg Thr Ala Lys Leu Thr Leu Tyr Ser Lys His
        195                 200                 205

His Ser Ala Pro Leu Tyr Ser Ser Tyr Leu His Lys Glu His Gln Leu
    210                 215                 220

Pro Glu Ala
225

<210> 11
<211> 89
<212> PRT
<213> Homo sapiens

<400> 11
Gly Gln Asn Val Glu Met Ser Cys Ala Phe Gln Ser Gly Ser Ala Ser
  1               5                  10                  15

Val Tyr Leu Glu Ile Gln Trp Trp Phe Leu Arg Gly Pro Glu Asp Leu
             20                  25                  30

Asp Pro Gly Ala Glu Gly Ala Gly Ala Gln Val Glu Leu Leu Pro Asp
         35                  40                  45

Arg Asp Pro Asp Ser Asp Gly Thr Lys Ile Ser Thr Val Lys Val Gln
     50                  55                  60

Gly Asn Asp Ile Ser His Lys Leu Gln Ile Ser Lys Val Arg Lys Lys
 65                  70                  75                  80

Asp Glu Gly Leu Tyr Glu Cys Arg Val
                 85

<210> 12
<211> 89
<212> PRT
<213> Mus musculus

<400> 12
Gly Gln Asn Val Glu Met Ser Cys Ala Phe Gln Ser Gly Ser Ala Ser
  1               5                  10                  15

Val Tyr Leu Glu Ile Gln Trp Trp Phe Leu Arg Gly Pro Glu Asp Leu
             20                  25                  30

Glu Gln Gly Thr Glu Ala Ala Gly Ser Gln Val Glu Leu Leu Pro Asp
         35                  40                  45

Arg Asp Pro Asp Asn Asp Gly Thr Lys Ile Ser Thr Val Lys Val Gln
     50                  55                  60

Gly Asn Asp Ile Ser His Lys Leu Gln Ile Ser Lys Val Arg Lys Lys
65                  70                  75                  80

Asp Glu Gly Leu Tyr Glu Cys Arg Val
                85

<210> 13
<211> 47
<212> PRT
<213> Artificial Sequence

<220>
<223> Description of Artificial Sequence: Hidden Markov
      Model

<400> 13
Gly Gln Ser Val Thr Leu Thr Cys Met Val Ser Phe His Pro Pro Asp
  1               5                  10                  15

Tyr Thr Ile Trp Trp Tyr Arg Asn Gly Gln Pro Ile Thr Leu Thr Ile
             20                  25                  30

Asn Ser Trp Gln Tyr Glu Asp Ser Glu Thr Tyr Trp Cys Met Val
         35                  40                  45

<210> 14
<211> 2915
<212> DNA
<213> Homo sapiens

<220>
<221> CDS
<222> (404)..(1294)

<400> 14
gaattcccgg gtcgacccac gcgtccgccc acgcgtccgc ccacgcgtcc ggctgagcga 60

agcgcggcgg cggcggcggc gcctagggga gggaggggcg gcggggccga gcccacctag 120

cggagcgcgc cggccgccgg tggccgccgc cagcatgccc cggcccgcgg gccgctccgc 180

cgccagccac ccccgcggcc ctcggcggcc tgcgctcggc ccgggggcgc gggaaccgca 240

gccggagccg gaggcgggag cagcgagccg gagccccggg cgctcgaatg caggatgctc 300

gtggtcccca gcatccttga gccaccagga gtgagggctg ctgctccctg agacctggct 360

ccaaggagga tgccacagcc gcctgccagc tccggtctgc acc atg agt gat gag   415
                                                Met Ser Asp Glu
                                                  1

cgg cgg ctg cct ggc agt gca gtg ggc tgg ctg gta tgt ggg ggc ctc   463
Arg Arg Leu Pro Gly Ser Ala Val Gly Trp Leu Val Cys Gly Gly Leu
  5                  10                  15                  20

tcc ctg ctg gcc aat gcc tgg ggc atc ctc agc gtt ggc gcc aag cag 511
Ser Leu Leu Ala Asn Ala Trp Gly Ile Leu Ser Val Gly Ala Lys Gln
                25                  30                  35

aag aag tgg aag ccc ttg gag ttc ctg ctg tgt acg ctc gcg gcc acc 559
Lys Lys Trp Lys Pro Leu Glu Phe Leu Leu Cys Thr Leu Ala Ala Thr
            40                  45                  50

cac atg cta aat gtg gcc gtg ccc atc gcc acc tac tcc gtg gtg cag 607
His Met Leu Asn Val Ala Val Pro Ile Ala Thr Tyr Ser Val Val Gln
        55                  60                  65

ctg cgg cgg cag cgc ccc gac ttc gag tgg aat gag ggt ctc tgc aag 655
Leu Arg Arg Gln Arg Pro Asp Phe Glu Trp Asn Glu Gly Leu Cys Lys
    70                  75                  80

gtc ttc gtg tcc acc ttc tac acc ctc acc ctg gcc acc tgt ttc tct 703
Val Phe Val Ser Thr Phe Tyr Thr Leu Thr Leu Ala Thr Cys Phe Ser
85                  90                  95                  100

gtc acc tcc ctc tcc tac cac cgc atg tgg atg gtc tgc tgg cct gtc 751
Val Thr Ser Leu Ser Tyr His Arg Met Trp Met Val Cys Trp Pro Val
                105                 110                 115

aac tac cgg ctg agc aat gcc aag aag cag gcg gtg cac aca gtc atg 799
Asn Tyr Arg Leu Ser Asn Ala Lys Lys Gln Ala Val His Thr Val Met
            120                 125                 130

ggt atc tgg atg gtg tcc ttc atc ctg tcg gcc ctg cct gcc gtt ggc 847
Gly Ile Trp Met Val Ser Phe Ile Leu Ser Ala Leu Pro Ala Val Gly
        135                 140                 145

tgg cac gac acc agc gag cgc ttc tac acc cat ggc tgc cgc ttc atc 895
Trp His Asp Thr Ser Glu Arg Phe Tyr Thr His Gly Cys Arg Phe Ile
    150                 155                 160

gtg gct gag atc ggc ctg ggc ttt ggc gtc tgc ttc ctg ctg ctg gtg 943
Val Ala Glu Ile Gly Leu Gly Phe Gly Val Cys Phe Leu Leu Leu Val
165                 170                 175                 180

ggc ggc agc gtg gcc atg ggc gtg atc tgc aca gcc atc gcc ctc ttc 991
Gly Gly Ser Val Ala Met Gly Val Ile Cys Thr Ala Ile Ala Leu Phe
                185                 190                 195

cag acg ctg gcc gtg cag gtg ggg cgc cag gcc gac cac cgc gcc ttc 1039
Gln Thr Leu Ala Val Gln Val Gly Arg Gln Ala Asp His Arg Ala Phe
            200                 205                 210

acc gtg ccc acc atc gtg gtg gag gac gcg cag ggc aag cgg cgc tcc 1087
Thr Val Pro Thr Ile Val Val Glu Asp Ala Gln Gly Lys Arg Arg Ser
        215                 220                 225

tcc atc gat ggc tcg gag ccc gcc aaa acc tct ctg cag acc acg ggc 1135
Ser Ile Asp Gly Ser Glu Pro Ala Lys Thr Ser Leu Gln Thr Thr Gly
    230                 235                 240

ctc gtg acc acc ata gtc ttc atc tac gac tgc ctc atg ggc ttc cct 1183
Leu Val Thr Thr Ile Val Phe Ile Tyr Asp Cys Leu Met Gly Phe Pro
245                 250                 255                 260

gtg ctg gac tct acg ccc atc ccc gaa agg tct gca gtg aga cag gga 1231
Val Leu Asp Ser Thr Pro Ile Pro Glu Arg Ser Ala Val Arg Gln Gly
                265                 270                 275

gag gac tgg ggc aaa gac cag cct gag ggg ttt cat cca agc agc agg 1279
Glu Asp Trp Gly Lys Asp Gln Pro Glu Gly Phe His Pro Ser Ser Arg
            280                 285                 290

caa gac tgc ctt ccc tgagccattg caggacatga ggacatgagc tccagaatgg 1334
Gln Asp Cys Leu Pro
        295

tgccaggccg agccctgtgc ccacaggtgg tgagcttcag cagcctgcgg gccgacgcct 1394

cagcgccctg gatggcactc tgcgtgctgt ggtgctccgt ggcccaggcc ctgcttccga 1454

cctgtcctgc ctcagtttcc ccatctgtga tgagcaggtg accacgttaa ctctcagggc 1514

tgtttgaagt ctcttggttg taggccctcg ccactgagtg gcccaggtgt gagaggtagt 1574

ctagagccct ctgcggcctt tgtggaggtc cgttctcagc atgtggcctg gtgtggctcc 1634

ctaggcctga ggcctcccac tctcagtgcc ccctgccccc ttgggaaccc acactccacc 1694

ccagctaagc acaacactgt ggaccaccac caatggcacc tgagccacct tgaccaccat 1754

tagtgtcccc accctcatta ctctgccgtc gttgcctgtc cccaccacat catctccctc 1814

catggctccc accatgacgc tggcaggtgc aagcggagag cacgagggca gcagggcctg 1874

gccttccgtg tccctgcgtc atccccagcc tcactcccca cccgtgcagg cctggccagg 1934

aagggatcct ggcaggggct tccaggttct cagctcaagg cctggtcccg ggcaggcgtc 1994

caacccctgg gagcaatgta tttctttgcc ttccatcctg ggcagacccc ttacaggcct 2054

gggcattgcc atgggccctg ggtcttccca ggctaaggag aaccaggaac agctataacc 2114

ttgagctagt gaaatagggt agatgagaag gctgtctcct ccagacccct acccctacac 2174

agtggcccca caatatgaag acctggggta attccaaggt gagcatagag cctgcctgtg 2234

cccagttcct tctggccctc aggtggccaa gcccatctct tcatccttca gatagggtcc 2294

cactcccaga agaagctgct ggggtggggg tgggaggctg cctaagcctg tctgtgcttc 2354

agaggcccct ccagtccctg gctgtggggt aactgggggt atgagctgtg gccacaggtg 2414

agcaaggcag ggaactgcaa tccagccctg gccgcgggag gggccatctc tggccaatgc 2474

tgctgtgcct tcaaggactg acaagttacg taggggcaga ggtcgccagc tagccagtgt 2534

ctcctccatc tggggggcgt ctgtccactt gtcaccttag gttttcactc atttgtcacc 2594

ttggggtttt gctctgtgtg tttcatatcc aacggcaata cttgcagggg gacagagtcc 2654

tctaaatact ccaatcctgc ggtttttaca aacataaagg gggagacccc aagtggagga 2714

ccctgggcct ggagctccct cccaaacttt gtccagcatc cagcctgttc cctgggctca 2774

ctggggaggg agttgtcttc atagcacacc cagagccagg gatccctttg tagttttttg 2834

acaacggagc atttctcttc tgtacaggac ccaataaaaa cttccttatg atttgcaaaa 2894

aaaaaaaaaa agggcggccg c 2915

<210> 15
<211> 297
<212> PRT
<213> Homo sapiens

<400> 15
Met Ser Asp Glu Arg Arg Leu Pro Gly Ser Ala Val Gly Trp Leu Val
1               5                   10                  15

Cys Gly Gly Leu Ser Leu Leu Ala Asn Ala Trp Gly Ile Leu Ser Val
            20                  25                  30

Gly Ala Lys Gln Lys Lys Trp Lys Pro Leu Glu Phe Leu Leu Cys Thr
        35                  40                  45

Leu Ala Ala Thr His Met Leu Asn Val Ala Val Pro Ile Ala Thr Tyr
    50                  55                  60

Ser Val Val Gln Leu Arg Arg Gln Arg Pro Asp Phe Glu Trp Asn Glu
65                  70                  75                  80

Gly Leu Cys Lys Val Phe Val Ser Thr Phe Tyr Thr Leu Thr Leu Ala
                85                  90                  95

Thr Cys Phe Ser Val Thr Ser Leu Ser Tyr His Arg Met Trp Met Val
            100                 105                 110

Cys Trp Pro Val Asn Tyr Arg Leu Ser Asn Ala Lys Lys Gln Ala Val
        115                 120                 125

His Thr Val Met Gly Ile Trp Met Val Ser Phe Ile Leu Ser Ala Leu
    130                 135                 140

Pro Ala Val Gly Trp His Asp Thr Ser Glu Arg Phe Tyr Thr His Gly
145                 150                 155                 160

Cys Arg Phe Ile Val Ala Glu Ile Gly Leu Gly Phe Gly Val Cys Phe
                165                 170                 175

Leu Leu Leu Val Gly Gly Ser Val Ala Met Gly Val Ile Cys Thr Ala
            180                 185                 190

Ile Ala Leu Phe Gln Thr Leu Ala Val Gln Val Gly Arg Gln Ala Asp
        195                 200                 205

His Arg Ala Phe Thr Val Pro Thr Ile Val Val Glu Asp Ala Gln Gly
    210                 215                 220

Lys Arg Arg Ser Ser Ile Asp Gly Ser Glu Pro Ala Lys Thr Ser Leu
225                 230                 235                 240

Gln Thr Thr Gly Leu Val Thr Thr Ile Val Phe Ile Tyr Asp Cys Leu
                245                 250                 255

Met Gly Phe Pro Val Leu Asp Ser Thr Pro Ile Pro Glu Arg Ser Ala
            260                 265                 270

Val Arg Gln Gly Glu Asp Trp Gly Lys Asp Gln Pro Glu Gly Phe His
        275                 280                 285

Pro Ser Ser Arg Gln Asp Cys Leu Pro
    290                 295


<210> 16
<211> 891
<212> DNA
<213> Homo sapiens

<220>
<221> CDS
<222> (1)..(891)

<400> 16
atg agt gat gag cgg cgg ctg cct ggc agt gca gtg ggc tgg ctg gta     48
Met Ser Asp Glu Arg Arg Leu Pro Gly Ser Ala Val Gly Trp Leu Val
1               5                   10                  15

tgt ggg ggc ctc tcc ctg ctg gcc aat gcc tgg ggc atc ctc agc gtt     96
Cys Gly Gly Leu Ser Leu Leu Ala Asn Ala Trp Gly Ile Leu Ser Val
            20                  25                  30

ggc gcc aag cag aag aag tgg aag ccc ttg gag ttc ctg ctg tgt acg     144
Gly Ala Lys Gln Lys Lys Trp Lys Pro Leu Glu Phe Leu Leu Cys Thr
        35                  40                  45

ctc gcg gcc acc cac atg cta aat gtg gcc gtg ccc atc gcc acc tac     192
Leu Ala Ala Thr His Met Leu Asn Val Ala Val Pro Ile Ala Thr Tyr
    50                  55                  60

tcc gtg gtg cag ctg cgg cgg cag cgc ccc gac ttc gag tgg aat gag     240
Ser Val Val Gln Leu Arg Arg Gln Arg Pro Asp Phe Glu Trp Asn Glu
65                  70                  75                  80

ggt ctc tgc aag gtc ttc gtg tcc acc ttc tac acc ctc acc ctg gcc     288
Gly Leu Cys Lys Val Phe Val Ser Thr Phe Tyr Thr Leu Thr Leu Ala
                85                  90                  95

acc tgt ttc tct gtc acc tcc ctc tcc tac cac cgc atg tgg atg gtc     336
Thr Cys Phe Ser Val Thr Ser Leu Ser Tyr His Arg Met Trp Met Val
            100                 105                 110

tgc tgg cct gtc aac tac cgg ctg agc aat gcc aag aag cag gcg gtg     384
Cys Trp Pro Val Asn Tyr Arg Leu Ser Asn Ala Lys Lys Gln Ala Val
        115                 120                 125

cac aca gtc atg ggt atc tgg atg gtg tcc ttc atc ctg tcg gcc ctg     432
His Thr Val Met Gly Ile Trp Met Val Ser Phe Ile Leu Ser Ala Leu
    130                 135                 140

cct gcc gtt ggc tgg cac gac acc agc gag cgc ttc tac acc cat ggc     480
Pro Ala Val Gly Trp His Asp Thr Ser Glu Arg Phe Tyr Thr His Gly
145                 150                 155                 160

tgc cgc ttc atc gtg gct gag atc ggc ctg ggc ttt ggc gtc tgc ttc     528
Cys Arg Phe Ile Val Ala Glu Ile Gly Leu Gly Phe Gly Val Cys Phe
                165                 170                 175

ctg ctg ctg gtg ggc ggc agc gtg gcc atg ggc gtg atc tgc aca gcc     576
Leu Leu Leu Val Gly Gly Ser Val Ala Met Gly Val Ile Cys Thr Ala
            180                 185                 190

atc gcc ctc ttc cag acg ctg gcc gtg cag gtg ggg cgc cag gcc gac     624
Ile Ala Leu Phe Gln Thr Leu Ala Val Gln Val Gly Arg Gln Ala Asp
        195                 200                 205

cac cgc gcc ttc acc gtg ccc acc atc gtg gtg gag gac gcg cag ggc     672
His Arg Ala Phe Thr Val Pro Thr Ile Val Val Glu Asp Ala Gln Gly
    210                 215                 220

aag cgg cgc tcc tcc atc gat ggc tcg gag ccc gcc aaa acc tct ctg     720
Lys Arg Arg Ser Ser Ile Asp Gly Ser Glu Pro Ala Lys Thr Ser Leu
225                 230                 235                 240

cag acc acg ggc ctc gtg acc acc ata gtc ttc atc tac gac tgc ctc     768
Gln Thr Thr Gly Leu Val Thr Thr Ile Val Phe Ile Tyr Asp Cys Leu
                245                 250                 255

atg ggc ttc cct gtg ctg gac tct acg ccc atc ccc gaa agg tct gca     816
Met Gly Phe Pro Val Leu Asp Ser Thr Pro Ile Pro Glu Arg Ser Ala
            260                 265                 270

gtg aga cag gga gag gac tgg ggc aaa gac cag cct gag ggg ttt cat     864
Val Arg Gln Gly Glu Asp Trp Gly Lys Asp Gln Pro Glu Gly Phe His
        275                 280                 285

cca agc agc agg caa gac tgc ctt ccc                                 891
Pro Ser Ser Arg Gln Asp Cys Leu Pro
    290                 295


<210> 17
<211> 4166
<212> DNA
<213> Homo sapiens

<220>
<221> CDS
<222> (334)..(2160)

<400> 17
gaattcccgg gtcgacccac gcgtccggcg gcggggccga gcccacctag cggagcgcgc 60

cggccgccgg tggccgccgc cagcatgccc cggcccgcgg gccgctccgc cgccagccac 120

ccccgcggcc ctcggcggcc tgcgctcggc ccgggggcgc gggaaccgca gccggagccg 180

gaggcgggag cagcgagccg gagccccggg cgctcgaatg caggatgctc gtggtcccca 240

gcatccttga gccaccagga gtgagggctg ctgctccctg agacctggct ccaaggagga 300

tgccacagcc gcctgccagc tccggtctgc acc atg agt gat gag cgg cgg ctg 354
Met Ser Asp Glu Arg Arg Leu
1               5

cct ggc agt gca gtg ggc tgg ctg gta tgt ggg ggc ctc tcc ctg ctg 402
Pro Gly Ser Ala Val Gly Trp Leu Val Cys Gly Gly Leu Ser Leu Leu
        10                  15                  20

gcc aat gcc tgg ggc atc ctc agc gtt ggc gcc aag cag aag aag tgg 450
Ala Asn Ala Trp Gly Ile Leu Ser Val Gly Ala Lys Gln Lys Lys Trp
    25                  30                  35

aag ccc ttg gag ttc ctg ctg tgt acg ctc gcg gcc acc cac atg cta 498
Lys Pro Leu Glu Phe Leu Leu Cys Thr Leu Ala Ala Thr His Met Leu
40                  45                  50                  55

aat gtg gcc gtg ccc atc gcc acc tac tcc gtg gtg cag ctg cgg cgg 546
Asn Val Ala Val Pro Ile Ala Thr Tyr Ser Val Val Gln Leu Arg Arg
                60                  65                  70

cag cgc ccc gac ttc gag tgg aat gag ggt ctc tgc aag gtc ttc gtg 594
Gln Arg Pro Asp Phe Glu Trp Asn Glu Gly Leu Cys Lys Val Phe Val
            75                  80                  85

tcc acc ttc tac acc ctc acc ctg gcc acc tgt ttc tct gtc acc tcc 642
Ser Thr Phe Tyr Thr Leu Thr Leu Ala Thr Cys Phe Ser Val Thr Ser
        90                  95                  100

ctc tcc tac cac cgc atg tgg atg gtc tgc tgg cct gtc aac tac cgg 690
Leu Ser Tyr His Arg Met Trp Met Val Cys Trp Pro Val Asn Tyr Arg
    105                 110                 115

ctg agc aat gcc aag aag cag gcg gtg cac aca gtc atg ggt atc tgg 738
Leu Ser Asn Ala Lys Lys Gln Ala Val His Thr Val Met Gly Ile Trp
120                 125                 130                 135

atg gtg tcc ttc atc ctg tcg gcc ctg cct gcc gtt ggc tgg cac gac 786
Met Val Ser Phe Ile Leu Ser Ala Leu Pro Ala Val Gly Trp His Asp
                140                 145                 150

acc agc gag cgc ttc tac acc cat ggc tgc cgc ttc atc gtg gct gag 834
Thr Ser Glu Arg Phe Tyr Thr His Gly Cys Arg Phe Ile Val Ala Glu
            155                 160                 165

atc ggc ctg ggc ttt ggc gtc tgc ttc ctg ctg ctg gtg ggc ggc agc 882
Ile Gly Leu Gly Phe Gly Val Cys Phe Leu Leu Leu Val Gly Gly Ser
        170                 175                 180

gtg gcc atg ggc gtg atc tgc aca gcc atc gcc ctc ttc cag acg ctg     930
Val Ala Met Gly Val Ile Cys Thr Ala Ile Ala Leu Phe Gln Thr Leu
    185                 190                 195

gcc gtg cag gtg ggg cgc cag gcc gac cac cgc gcc ttc acc gtg ccc     978
Ala Val Gln Val Gly Arg Gln Ala Asp His Arg Ala Phe Thr Val Pro
200                 205                 210                 215

acc atc gtg gtg gag gac gcg cag ggc aag cgg cgc tcc tcc atc gat     1026
Thr Ile Val Val Glu Asp Ala Gln Gly Lys Arg Arg Ser Ser Ile Asp
                220                 225                 230

ggc tcg gag ccc gcc aaa acc tct ctg cag acc acg ggc ctc gtg acc     1074
Gly Ser Glu Pro Ala Lys Thr Ser Leu Gln Thr Thr Gly Leu Val Thr
            235                 240                 245

acc ata gtc ttc atc tac gac tgc ctc atg ggc ttc cct gtg ctg gtg     1122
Thr Ile Val Phe Ile Tyr Asp Cys Leu Met Gly Phe Pro Val Leu Val
        250                 255                 260

gtg agc ttc agc agc ctg cgg gcc gac gcc tca gcg ccc tgg atg gca     1170
Val Ser Phe Ser Ser Leu Arg Ala Asp Ala Ser Ala Pro Trp Met Ala
    265                 270                 275

ctc tgc gtg ctg tgg tgc tcc gtg gcc cag gcc ctg ctg ctg cct gtg     1218
Leu Cys Val Leu Trp Cys Ser Val Ala Gln Ala Leu Leu Leu Pro Val
280                 285                 290                 295

ttc ctc tgg gcc tgc gac cgc tac cgg gct gac ctc aaa gct gtc cgg     1266
Phe Leu Trp Ala Cys Asp Arg Tyr Arg Ala Asp Leu Lys Ala Val Arg
                300                 305                 310

gag aag tgc atg gcc ctc atg gcc aac gac gag gag tca gac gat gag     1314
Glu Lys Cys Met Ala Leu Met Ala Asn Asp Glu Glu Ser Asp Asp Glu
            315                 320                 325

acc agc ctg gaa ggt ggc atc tcc ccg gac ctg gtg ttg gag cgc tcc     1362
Thr Ser Leu Glu Gly Gly Ile Ser Pro Asp Leu Val Leu Glu Arg Ser
        330                 335                 340

ctg gac tat ggc tat gga ggt gat ttt gtg gcc cta gat agg atg gcc     1410
Leu Asp Tyr Gly Tyr Gly Gly Asp Phe Val Ala Leu Asp Arg Met Ala
    345                 350                 355

aag tat gag atc tcc gcc ctg gag ggg ggc ctg ccc cag ctc tac cca     1458
Lys Tyr Glu Ile Ser Ala Leu Glu Gly Gly Leu Pro Gln Leu Tyr Pro
360                 365                 370                 375

ctg cgg ccc ttg cag gag gac aag atg caa tac ctg cag gtc ccg ccc     1506
Leu Arg Pro Leu Gln Glu Asp Lys Met Gln Tyr Leu Gln Val Pro Pro
                380                 385                 390

acg cgg cgc ttc tcc cac gac gat gcg gac gtg tgg gcc gcc gtc ccg     1554
Thr Arg Arg Phe Ser His Asp Asp Ala Asp Val Trp Ala Ala Val Pro
            395                 400                 405

ctg ccc gcc ttc ctg ccg cgc tgg ggc tcc ggc gag gac ctg gcc gcc 1602
Leu Pro Ala Phe Leu Pro Arg Trp Gly Ser Gly Glu Asp Leu Ala Ala
        410                 415                 420

ctg gcg cac ctg gtg ctg cct gcc ggg ccc gag cgg cgc cgc gcc agc 1650
Leu Ala His Leu Val Leu Pro Ala Gly Pro Glu Arg Arg Arg Ala Ser
    425                 430                 435

ctc ctg gcc ttc gcg gag gac gca cca ccg tcc cgc gcg cgc cgc cgc 1698
Leu Leu Ala Phe Ala Glu Asp Ala Pro Pro Ser Arg Ala Arg Arg Arg
440                 445                 450                 455

tcg gcc gag agc ctg ctg tcg ctg cgg acc tcg gcc ctg gat agc ggc 1746
Ser Ala Glu Ser Leu Leu Ser Leu Arg Thr Ser Ala Leu Asp Ser Gly
                460                 465                 470

ccg cgg gga gcc cgc gac tcg ccc ccc ggc agc ccg cgc cgc cgc ccc 1794
Pro Arg Gly Ala Arg Asp Ser Pro Pro Gly Ser Pro Arg Arg Arg Pro
            475                 480                 485

ggg ccc ggc ccc cgc tcc gcc tcg gcc tcg ctg ctg ccc gac gcc ttc 1842
Gly Pro Gly Pro Arg Ser Ala Ser Ala Ser Leu Leu Pro Asp Ala Phe
        490                 495                 500

gcc ctg acc gcc ttc gag tgc gag cca cag gcc ctg cgc cgc ccg ccc 1890
Ala Leu Thr Ala Phe Glu Cys Glu Pro Gln Ala Leu Arg Arg Pro Pro
    505                 510                 515

ggg ccc ttc ccc gct gcg ccc gcc gcc ccc gac ggc gca gat ccc gga 1938
Gly Pro Phe Pro Ala Ala Pro Ala Ala Pro Asp Gly Ala Asp Pro Gly
520                 525                 530                 535

gag gcc ccg acg ccc cca agc agc gcc cag cgg agc cca ggg cca cgc 1986
Glu Ala Pro Thr Pro Pro Ser Ser Ala Gln Arg Ser Pro Gly Pro Arg
                540                 545                 550

ccc tct gcg cac tcg cac gcc ggc tct ctg cgc ccc ggc ctg agc gcg 2034
Pro Ser Ala His Ser His Ala Gly Ser Leu Arg Pro Gly Leu Ser Ala
            555                 560                 565

tcg tgg ggc gag ccc ggg ggg ctg cgc gcg gcg ggc ggc ggc ggc agc 2082
Ser Trp Gly Glu Pro Gly Gly Leu Arg Ala Ala Gly Gly Gly Gly Ser
        570                 575                 580

acc agc agc ttc ctg agt tcc ccc tcc gag tcc tcg ggc tac gcc acg 2130
Thr Ser Ser Phe Leu Ser Ser Pro Ser Glu Ser Ser Gly Tyr Ala Thr
    585                 590                 595

ctg cac tcg gac tcg ctg ggc tcc gcg tcc taggaccgcc ggcgcctccc 2180
Leu His Ser Asp Ser Leu Gly Ser Ala Ser
600                 605

cacggacgcc aggcaggcca ggccgctctc cggggccgca gcaccaaaga cgcccgcctc 2240

ccccccgcgc gcagacatgc gccacccctc ccaggggtga gggggcgttg gcctcagcgt 2300

ttgtcttccg gctcctccca gctggccttg tcccaggggc gacggctgcc ccggacgact 2360

gcgctgggca ccgcatgtcc cgggccgagt gaggtcgggc ctggggagct gagtgacatc 2420
ccaagcttgg gcttgggtag tgagtgacat gtgcacacgt ccagctgcgc catcaccagc 2480
cctgcgcaac aggacgtcgg gagcagggaa cctgagacag ggccactgcg ggatcggaca 2540
aagccccgct ttggagaggc tgagctggag ccattggcct ccccaggggc tttccaccca 2600
cactgcacca taaccgccac acccttcggg gggggggagg gtacagaggg tctctaagca 2660
caggggtgtt cagagcccga acaagctttg atcaggtttc cctgcttccg acctgtcctg 2720
cctcagtttc cccatctgtg atgagcaggt gaccacgtta actctcaggg ctgtttgaag 2780
tctcttggtt gtaggccctc gccactgagt ggcccaggtg tgagaggtag tctagagccc 2840
tctgcggcct ttgtggaggt ccgttctcag catgtggcct ggtgtggctc cctaggcctg 2900
aggcctccca ctctcagtgc cccctgcccc cttgggaacc cacactccac cccagctaag 2960
cacaacactg tggaccacca ccaatggcac ctgagccacc ttgaccacca ttagtgtccc 3020
caccctcatt actctgccgt cgttgcctgt ccccaccaca tcatctccct ccatggctcc 3080
caccatgacg ctggcaggtg caagcggaga gcacgagggc agcagggcct ggccttccgt 3140
gtccctgcgt catccccagc ctcactcccc acccgtgcag gcctggccag gaagggatcc 3200
tggcaggggc ttccaggttc tcagctcaag gcctggtccc gggcaggcgt ccaacccctg 3260
ggagcaatgt atttctttgc cttccatcct gggcagaccc cttacaggcc tgggcattgc 3320
catgggccct gggtcttccc aggctaagga gaaccaggaa cagctataac cttgagctag 3380
tgaaataggg tagatgagaa ggctgtctcc tccagacccc tacccctaca cagtggcccc 3440
acaatatgaa gacctggggt aattccaagg tgagcataga gcctgcctgt gcccagttcc 3500
ttctggccct caggtggcca agcccatctc ttcatccttc agatagggtc ccactcccag 3560
aagaagctgc tggggtgggg gtgggaggct gcctaagcct gtctgtgctt cagaggcccc 3620
tccagtccct ggctgtgggg taactggggg tatgagctgt ggccacaggt gagcaaggca 3680
gggaactgca atccagccct ggccgcggga ggggccatct ctggccaatg ctgctgtgcc 3740
ttcaaggact gacaagttac gtaggggcag aggtcgccag ctagccagtg tctcctccat 3800
ctggggggcg tctgtccact tgtcacctta ggttttcact catttgtcac cttggggttt 3860
tgctctgtgt gtttcatatc caacggcaat acttgcaggg ggacagagtc ctctaaatac 3920
tccaatcctg cggtttttac aaacataaag ggggagaccc caagtggagg accctgggcc 3980
tggagctccc tcccaaactt tgtccagcat ccagcctgtt ccctgggctc actggggagg 4040
gagttgtctt catagcacac ccagagccag ggatcccttt gtagtttttt gacaacggag 4100

catttctctt ctgtacagga cccaataaaa acttccttat gaaaaaaaaa aaaaaagggc 4160

ggccgc 4166

<210> 18
<211> 609
<212> PRT
<213> Homo sapiens

<400> 18
Met Ser Asp Glu Arg Arg Leu Pro Gly Ser Ala Val Gly Trp Leu Val
1               5                   10                  15

Cys Gly Gly Leu Ser Leu Leu Ala Asn Ala Trp Gly Ile Leu Ser Val
            20                  25                  30

Gly Ala Lys Gln Lys Lys Trp Lys Pro Leu Glu Phe Leu Leu Cys Thr
        35                  40                  45

Leu Ala Ala Thr His Met Leu Asn Val Ala Val Pro Ile Ala Thr Tyr
    50                  55                  60

Ser Val Val Gln Leu Arg Arg Gln Arg Pro Asp Phe Glu Trp Asn Glu
65                  70                  75                  80

Gly Leu Cys Lys Val Phe Val Ser Thr Phe Tyr Thr Leu Thr Leu Ala
                85                  90                  95

Thr Cys Phe Ser Val Thr Ser Leu Ser Tyr His Arg Met Trp Met Val
            100                 105                 110

Cys Trp Pro Val Asn Tyr Arg Leu Ser Asn Ala Lys Lys Gln Ala Val
        115                 120                 125

His Thr Val Met Gly Ile Trp Met Val Ser Phe Ile Leu Ser Ala Leu
    130                 135                 140

Pro Ala Val Gly Trp His Asp Thr Ser Glu Arg Phe Tyr Thr His Gly
145                 150                 155                 160

Cys Arg Phe Ile Val Ala Glu Ile Gly Leu Gly Phe Gly Val Cys Phe
                165                 170                 175

Leu Leu Leu Val Gly Gly Ser Val Ala Met Gly Val Ile Cys Thr Ala
            180                 185                 190

Ile Ala Leu Phe Gln Thr Leu Ala Val Gln Val Gly Arg Gln Ala Asp
        195                 200                 205

His Arg Ala Phe Thr Val Pro Thr Ile Val Val Glu Asp Ala Gln Gly
    210                 215                 220

Lys Arg Arg Ser Ser Ile Asp Gly Ser Glu Pro Ala Lys Thr Ser Leu
225                 230                 235                 240

Gln Thr Thr Gly Leu Val Thr Thr Ile Val Phe Ile Tyr Asp Cys Leu
                245                 250                 255

Met Gly Phe Pro Val Leu Val Val Ser Phe Ser Ser Leu Arg Ala Asp
            260                 265                 270

Ala Ser Ala Pro Trp Met Ala Leu Cys Val Leu Trp Cys Ser Val Ala
        275                 280                 285

Gln Ala Leu Leu Leu Pro Val Phe Leu Trp Ala Cys Asp Arg Tyr Arg
    290                 295                 300

Ala Asp Leu Lys Ala Val Arg Glu Lys Cys Met Ala Leu Met Ala Asn
305                 310                 315                 320

Asp Glu Glu Ser Asp Asp Glu Thr Ser Leu Glu Gly Gly Ile Ser Pro
                325                 330                 335

Asp Leu Val Leu Glu Arg Ser Leu Asp Tyr Gly Tyr Gly Gly Asp Phe
            340                 345                 350

Val Ala Leu Asp Arg Met Ala Lys Tyr Glu Ile Ser Ala Leu Glu Gly
        355                 360                 365

Gly Leu Pro Gln Leu Tyr Pro Leu Arg Pro Leu Gln Glu Asp Lys Met
    370                 375                 380

Gln Tyr Leu Gln Val Pro Pro Thr Arg Arg Phe Ser His Asp Asp Ala
385                 390                 395                 400

Asp Val Trp Ala Ala Val Pro Leu Pro Ala Phe Leu Pro Arg Trp Gly
                405                 410                 415

Ser Gly Glu Asp Leu Ala Ala Leu Ala His Leu Val Leu Pro Ala Gly
            420                 425                 430

Pro Glu Arg Arg Arg Ala Ser Leu Leu Ala Phe Ala Glu Asp Ala Pro
        435                 440                 445

Pro Ser Arg Ala Arg Arg Arg Ser Ala Glu Ser Leu Leu Ser Leu Arg
    450                 455                 460

Thr Ser Ala Leu Asp Ser Gly Pro Arg Gly Ala Arg Asp Ser Pro Pro
465                 470                 475                 480

Gly Ser Pro Arg Arg Arg Pro Gly Pro Gly Pro Arg Ser Ala Ser Ala
                485                 490                 495

Ser Leu Leu Pro Asp Ala Phe Ala Leu Thr Ala Phe Glu Cys Glu Pro
            500                 505                 510

Gln Ala Leu Arg Arg Pro Pro Gly Pro Phe Pro Ala Ala Pro Ala Ala
        515                 520                 525

Pro Asp Gly Ala Asp Pro Gly Glu Ala Pro Thr Pro Pro Ser Ser Ala
    530                 535                 540

Gln Arg Ser Pro Gly Pro Arg Pro Ser Ala His Ser His Ala Gly Ser
545                 550                 555                 560

Leu Arg Pro Gly Leu Ser Ala Ser Trp Gly Glu Pro Gly Gly Leu Arg
                565                 570                 575

Ala Ala Gly Gly Gly Gly Ser Thr Ser Ser Phe Leu Ser Ser Pro Ser
            580                 585                 590

Glu Ser Ser Gly Tyr Ala Thr Leu His Ser Asp Ser Leu Gly Ser Ala
        595                 600                 605

Ser

<210> 19
<211> 1827
<212> DNA
<213> Homo sapiens

<220>
<221> CDS
<222> (1)..(1827)

<400> 19
atg agt gat gag cgg cgg ctg cct ggc agt gca gtg ggc tgg ctg gta 48
Met Ser Asp Glu Arg Arg Leu Pro Gly Ser Ala Val Gly Trp Leu Val
1               5                   10                  15

tgt ggg ggc ctc tcc ctg ctg gcc aat gcc tgg ggc atc ctc agc gtt 96
Cys Gly Gly Leu Ser Leu Leu Ala Asn Ala Trp Gly Ile Leu Ser Val
            20                  25                  30

ggc gcc aag cag aag aag tgg aag ccc ttg gag ttc ctg ctg tgt acg 144
Gly Ala Lys Gln Lys Lys Trp Lys Pro Leu Glu Phe Leu Leu Cys Thr
        35                  40                  45

ctc gcg gcc acc cac atg cta aat gtg gcc gtg ccc atc gcc acc tac 192
Leu Ala Ala Thr His Met Leu Asn Val Ala Val Pro Ile Ala Thr Tyr
    50                  55                  60

tcc gtg gtg cag ctg cgg cgg cag cgc ccc gac ttc gag tgg aat gag 240
Ser Val Val Gln Leu Arg Arg Gln Arg Pro Asp Phe Glu Trp Asn Glu
65                  70                  75                  80

ggt ctc tgc aag gtc ttc gtg tcc acc ttc tac acc ctc acc ctg gcc 288
Gly Leu Cys Lys Val Phe Val Ser Thr Phe Tyr Thr Leu Thr Leu Ala
                85                  90                  95

acc tgt ttc tct gtc acc tcc ctc tcc tac cac cgc atg tgg atg gtc 336
Thr Cys Phe Ser Val Thr Ser Leu Ser Tyr His Arg Met Trp Met Val
            100                 105                 110

tgc tgg cct gtc aac tac cgg ctg agc aat gcc aag aag cag gcg gtg 384
Cys Trp Pro Val Asn Tyr Arg Leu Ser Asn Ala Lys Lys Gln Ala Val
        115                 120                 125

cac aca gtc atg ggt atc tgg atg gtg tcc ttc atc ctg tcg gcc ctg 432
His Thr Val Met Gly Ile Trp Met Val Ser Phe Ile Leu Ser Ala Leu
    130                 135                 140

cct gcc gtt ggc tgg cac gac acc agc gag cgc ttc tac acc cat ggc     480
Pro Ala Val Gly Trp His Asp Thr Ser Glu Arg Phe Tyr Thr His Gly
145                 150                 155                 160

tgc cgc ttc atc gtg gct gag atc ggc ctg ggc ttt ggc gtc tgc ttc     528
Cys Arg Phe Ile Val Ala Glu Ile Gly Leu Gly Phe Gly Val Cys Phe
                165                 170                 175

ctg ctg ctg gtg ggc ggc agc gtg gcc atg ggc gtg atc tgc aca gcc     576
Leu Leu Leu Val Gly Gly Ser Val Ala Met Gly Val Ile Cys Thr Ala
            180                 185                 190

atc gcc ctc ttc cag acg ctg gcc gtg cag gtg ggg cgc cag gcc gac     624
Ile Ala Leu Phe Gln Thr Leu Ala Val Gln Val Gly Arg Gln Ala Asp
        195                 200                 205

cac cgc gcc ttc acc gtg ccc acc atc gtg gtg gag gac gcg cag ggc     672
His Arg Ala Phe Thr Val Pro Thr Ile Val Val Glu Asp Ala Gln Gly
    210                 215                 220

aag cgg cgc tcc tcc atc gat ggc tcg gag ccc gcc aaa acc tct ctg     720
Lys Arg Arg Ser Ser Ile Asp Gly Ser Glu Pro Ala Lys Thr Ser Leu
225                 230                 235                 240

cag acc acg ggc ctc gtg acc acc ata gtc ttc atc tac gac tgc ctc     768
Gln Thr Thr Gly Leu Val Thr Thr Ile Val Phe Ile Tyr Asp Cys Leu
                245                 250                 255

atg ggc ttc cct gtg ctg gtg gtg agc ttc agc agc ctg cgg gcc gac     816
Met Gly Phe Pro Val Leu Val Val Ser Phe Ser Ser Leu Arg Ala Asp
            260                 265                 270

gcc tca gcg ccc tgg atg gca ctc tgc gtg ctg tgg tgc tcc gtg gcc     864
Ala Ser Ala Pro Trp Met Ala Leu Cys Val Leu Trp Cys Ser Val Ala
        275                 280                 285

cag gcc ctg ctg ctg cct gtg ttc ctc tgg gcc tgc gac cgc tac cgg     912
Gln Ala Leu Leu Leu Pro Val Phe Leu Trp Ala Cys Asp Arg Tyr Arg
    290                 295                 300

gct gac ctc aaa gct gtc cgg gag aag tgc atg gcc ctc atg gcc aac     960
Ala Asp Leu Lys Ala Val Arg Glu Lys Cys Met Ala Leu Met Ala Asn
305                 310                 315                 320

gac gag gag tca gac gat gag acc agc ctg gaa ggt ggc atc tcc ccg     1008
Asp Glu Glu Ser Asp Asp Glu Thr Ser Leu Glu Gly Gly Ile Ser Pro
                325                 330                 335

gac ctg gtg ttg gag cgc tcc ctg gac tat ggc tat gga ggt gat ttt     1056
Asp Leu Val Leu Glu Arg Ser Leu Asp Tyr Gly Tyr Gly Gly Asp Phe
            340                 345                 350

gtg gcc cta gat agg atg gcc aag tat gag atc tcc gcc ctg gag ggg     1104
Val Ala Leu Asp Arg Met Ala Lys Tyr Glu Ile Ser Ala Leu Glu Gly
        355                 360                 365

ggc ctg ccc cag ctc tac cca ctg cgg ccc ttg cag gag gac aag atg 1152
Gly Leu Pro Gln Leu Tyr Pro Leu Arg Pro Leu Gln Glu Asp Lys Met
    370                 375                 380

caa tac ctg cag gtc ccg ccc acg cgg cgc ttc tcc cac gac gat gcg 1200
Gln Tyr Leu Gln Val Pro Pro Thr Arg Arg Phe Ser His Asp Asp Ala
385                 390                 395                 400

gac gtg tgg gcc gcc gtc ccg ctg ccc gcc ttc ctg ccg cgc tgg ggc 1248
Asp Val Trp Ala Ala Val Pro Leu Pro Ala Phe Leu Pro Arg Trp Gly
                405                 410                 415

tcc ggc gag gac ctg gcc gcc ctg gcg cac ctg gtg ctg cct gcc ggg 1296
Ser Gly Glu Asp Leu Ala Ala Leu Ala His Leu Val Leu Pro Ala Gly
            420                 425                 430

ccc gag cgg cgc cgc gcc agc ctc ctg gcc ttc gcg gag gac gca cca 1344
Pro Glu Arg Arg Arg Ala Ser Leu Leu Ala Phe Ala Glu Asp Ala Pro
        435                 440                 445

ccg tcc cgc gcg cgc cgc cgc tcg gcc gag agc ctg ctg tcg ctg cgg 1392
Pro Ser Arg Ala Arg Arg Arg Ser Ala Glu Ser Leu Leu Ser Leu Arg
    450                 455                 460

acc tcg gcc ctg gat agc ggc ccg cgg gga gcc cgc gac tcg ccc ccc 1440
Thr Ser Ala Leu Asp Ser Gly Pro Arg Gly Ala Arg Asp Ser Pro Pro
465                 470                 475                 480

ggc agc ccg cgc cgc cgc ccc ggg ccc ggc ccc cgc tcc gcc tcg gcc 1488
Gly Ser Pro Arg Arg Arg Pro Gly Pro Gly Pro Arg Ser Ala Ser Ala
                485                 490                 495

tcg ctg ctg ccc gac gcc ttc gcc ctg acc gcc ttc gag tgc gag cca 1536
Ser Leu Leu Pro Asp Ala Phe Ala Leu Thr Ala Phe Glu Cys Glu Pro
            500                 505                 510

cag gcc ctg cgc cgc ccg ccc ggg ccc ttc ccc gct gcg ccc gcc gcc 1584
Gln Ala Leu Arg Arg Pro Pro Gly Pro Phe Pro Ala Ala Pro Ala Ala
        515                 520                 525

ccc gac ggc gca gat ccc gga gag gcc ccg acg ccc cca agc agc gcc 1632
Pro Asp Gly Ala Asp Pro Gly Glu Ala Pro Thr Pro Pro Ser Ser Ala
    530                 535                 540

cag cgg agc cca ggg cca cgc ccc tct gcg cac tcg cac gcc ggc tct 1680
Gln Arg Ser Pro Gly Pro Arg Pro Ser Ala His Ser His Ala Gly Ser
545                 550                 555                 560

ctg cgc ccc ggc ctg agc gcg tcg tgg ggc gag ccc ggg ggg ctg cgc 1728
Leu Arg Pro Gly Leu Ser Ala Ser Trp Gly Glu Pro Gly Gly Leu Arg
                565                 570                 575

gcg gcg ggc ggc ggc ggc agc acc agc agc ttc ctg agt tcc ccc tcc 1776
Ala Ala Gly Gly Gly Gly Ser Thr Ser Ser Phe Leu Ser Ser Pro Ser
            580                 585                 590

gag tcc tcg ggc tac gcc acg ctg cac tcg gac tcg ctg ggc tcc gcg     1824
Glu Ser Ser Gly Tyr Ala Thr Leu His Ser Asp Ser Leu Gly Ser Ala
        595                 600                 605

tcc                                                                 1827
Ser

<210> 20
<211> 177
<212> PRT
<213> Homo sapiens

<220>
<221> VARIANT
<222> 38, 55, 56
<223> Xaas at positions 38, 55 and 56 may be any amino acid

<400> 20
Gly Asn Ile Leu Val Ile Trp Val Ile Cys Arg Tyr Arg Arg Met Arg
  1               5                  10                  15

Thr Pro Met Asn Tyr Phe Ile Val Asn Leu Ala Val Ala Asp Leu Leu
             20                  25                  30

Phe Ser Leu Phe Thr Xaa Met Pro Phe Trp Met Val Tyr Tyr Val Met
         35                  40                  45

Gln Gly Arg Trp Pro Phe Xaa Xaa Gly Asp Phe Met Cys Arg Ile Trp
     50                  55                  60

Met Tyr Phe Asp Tyr Met Asn Met Tyr Ala Ser Ile Phe Phe Leu Thr
 65                  70                  75                  80

Cys Ile Ser Ile Asp Arg Tyr Leu Trp Ala Ile Cys His Pro Met Arg
                 85                  90                  95

Tyr Met Arg Trp Met Thr Pro Arg His Arg Ala Trp Val Met Ile Ile
            100                 105                 110

Ile Ile Trp Val Met Ser Phe Leu Ile Ser Met Pro Pro Phe Leu Met
        115                 120                 125

Phe Arg Trp Ser Thr Tyr Arg Asp Glu Asn Glu Trp Asn Met Thr Trp
    130                 135                 140

Cys Met Ile Tyr Asp Trp Pro Glu Trp Met Trp Arg Trp Tyr Val Ile
145                 150                 155                 160

Leu Met Thr Ile Ile Met Gly Phe Tyr Ile Pro Met Ile Ile Met Leu
                165                 170                 175

Phe

<210> 21
<211> 107

<212> PRT
<213> Homo sapiens

<220>
<221> VARIANT
<222> 84
<223> Xaa at position 84 may be any amino acid

<400> 21
Ile Gln Glu Arg Met Asn Glu Leu Asn Asp Arg Trp Glu Arg Leu Lys
1               5                   10                  15

Glu Leu Met Glu Gln Arg Arg Gln Met Leu Glu Asp Ser Met Arg Leu
            20                  25                  30

Gln Gln Phe Phe Arg Asp Met Asp Glu Glu Glu Ser Trp Ile Asn Glu
        35                  40                  45

Lys Glu Gln Ile Leu Asn Ser Asp Asp Tyr Gly Lys Asp Leu Thr Ser
    50                  55                  60

Val Gln Asn Leu Leu Lys Lys His Gln Ala Phe Glu Ala Asp Ile Ala
65                  70                  75                  80

Ala His Glu Xaa Asp Arg Ile Gln Ala Leu Asn Glu Phe Ala Gln Gln
                85                  90                  95

Leu Ile Gln Glu Asn His Tyr Ala Ser Glu Glu
            100                 105

<210> 22
<211> 168
<212> PRT
<213> Homo sapiens

<400> 22
Ala Asn Ala Trp Gly Ile Leu Ser Val Gly Ala Lys Gln Lys Lys Trp
1               5                   10                  15

Lys Pro Leu Glu Phe Leu Leu Cys Thr Leu Ala Ala Thr His Met Leu
            20                  25                  30

Asn Val Ala Val Pro Ile Ala Thr Tyr Ser Val Val Gln Leu Arg Arg
        35                  40                  45

Gln Arg Pro Asp Phe Glu Trp Asn Glu Gly Leu Cys Lys Val Phe Val
    50                  55                  60

Ser Thr Phe Tyr Thr Leu Thr Leu Ala Thr Cys Phe Ser Val Thr Ser
65                  70                  75                  80

Leu Ser Tyr His Arg Met Trp Met Val Cys Trp Pro Val Asn Tyr Arg
                85                  90                  95

Leu Ser Asn Ala Lys Lys Gln Ala Val His Thr Val Met Gly Ile Trp
            100                 105                 110

Met Val Ser Phe Ile Leu Ser Ala Leu Pro Ala Val Gly Trp His Asp
        115                 120                 125

Thr Ser Glu Arg Phe Tyr Thr His Gly Cys Arg Phe Ile Val Ala Glu
    130                 135                 140

Ile Gly Leu Gly Phe Gly Val Cys Phe Leu Leu Leu Val Gly Gly Ser
145                 150                 155                 160

Val Ala Met Gly Val Ile Cys Thr
                165

<210> 23
<211> 107
<212> PRT
<213> Homo sapiens

<400> 23
Phe Ser Ser Leu Arg Ala Asp Ala Ser Ala Pro Trp Met Ala Leu Cys
1               5                   10                  15

Val Leu Trp Cys Ser Val Ala Gln Ala Leu Leu Leu Pro Val Phe Leu
            20                  25                  30

Trp Ala Cys Asp Arg Tyr Arg Ala Asp Leu Lys Ala Val Arg Glu Lys
        35                  40                  45

Cys Met Ala Leu Met Ala Asn Asp Glu Glu Ser Asp Asp Glu Thr Ser
    50                  55                  60

Leu Glu Gly Gly Ile Ser Pro Asp Leu Val Leu Glu Arg Ser Leu Asp
65                  70                  75                  80

Tyr Gly Tyr Gly Gly Asp Phe Val Ala Leu Asp Arg Met Ala Lys Tyr
                85                  90                  95

Glu Ile Ser Ala Leu Glu Gly Gly Leu Pro Gln
            100                 105

<210> 24
<211> 425
<212> PRT
<213> Homo sapiens

<400> 24
Met Gly Pro Arg Arg Leu Leu Leu Val Ala Ala Cys Phe Ser Leu Cys
1               5                   10                  15

Gly Pro Leu Leu Ser Ala Arg Thr Arg Ala Arg Arg Pro Glu Ser Lys
            20                  25                  30

Ala Thr Asn Ala Thr Leu Asp Pro Arg Ser Phe Leu Leu Arg Asn Pro
        35                  40                  45

Asn Asp Lys Tyr Glu Pro Phe Trp Glu Asp Glu Glu Lys Asn Glu Ser
    50                  55                  60

Gly Leu Thr Glu Tyr Arg Leu Val Ser Ile Asn Lys Ser Ser Pro Leu
65                  70                  75                  80

Gln Lys Gln Leu Pro Ala Phe Ile Ser Glu Asp Ala Ser Gly Tyr Leu
                85                  90                  95

Thr Ser Ser Trp Leu Thr Leu Phe Val Pro Ser Val Tyr Thr Gly Val
            100                 105                 110

Phe Val Val Ser Leu Pro Leu Asn Ile Met Ala Ile Val Val Phe Ile
        115                 120                 125

Leu Lys Met Lys Val Lys Lys Pro Ala Val Val Tyr Met Leu His Leu
    130                 135                 140

Ala Thr Ala Asp Val Leu Phe Val Ser Val Leu Pro Phe Lys Ile Ser
145                 150                 155                 160

Tyr Tyr Phe Ser Gly Ser Asp Trp Gln Phe Gly Ser Glu Leu Cys Arg
                165                 170                 175

Phe Val Thr Ala Ala Phe Tyr Cys Asn Met Tyr Ala Ser Ile Leu Leu
            180                 185                 190

Met Thr Val Ile Ser Ile Asp Arg Phe Leu Ala Val Val Tyr Pro Met
        195                 200                 205

Gln Ser Leu Ser Trp Arg Thr Leu Gly Arg Ala Ser Phe Thr Cys Leu
    210                 215                 220

Ala Ile Trp Ala Leu Ala Ile Ala Gly Val Val Pro Leu Val Leu Lys
225                 230                 235                 240

Glu Gln Thr Ile Gln Val Pro Gly Leu Asn Ile Thr Thr Cys His Asp
                245                 250                 255

Val Leu Asn Glu Thr Leu Leu Glu Gly Tyr Tyr Ala Tyr Tyr Phe Ser
            260                 265                 270

Ala Phe Ser Ala Val Phe Phe Phe Val Pro Leu Ile Ile Ser Thr Val
        275                 280                 285

Cys Tyr Val Ser Ile Ile Arg Cys Leu Ser Ser Ser Ala Val Ala Asn
    290                 295                 300

Arg Ser Lys Lys Ser Arg Ala Leu Phe Leu Ser Ala Ala Val Phe Cys
305                 310                 315                 320

Ile Phe Ile Ile Cys Phe Gly Pro Thr Asn Val Leu Leu Ile Ala His
                325                 330                 335

Tyr Ser Phe Leu Ser His Thr Ser Thr Thr Glu Ala Ala Tyr Phe Ala
            340                 345                 350

Tyr Leu Leu Cys Val Cys Val Ser Ser Ile Ser Ser Cys Ile Asp Pro
        355                 360                 365

Leu Ile Tyr Tyr Tyr Ala Ser Ser Glu Cys Gln Arg Tyr Val Tyr Ser
    370                 375                 380

Ile Leu Cys Cys Lys Glu Ser Ser Asp Pro Ser Ser Tyr Asn Ser Ser
385                 390                 395                 400

Gly Gln Leu Met Ala Ser Lys Met Asp Thr Cys Ser Ser Asn Leu Asn
                405                 410                 415

Asn Ser Ile Tyr Lys Lys Leu Leu Thr
            420                 425


<210> 25
<211> 348
<212> PRT
<213> Homo sapiens

<400> 25

<210> 26
<211> 460
<212> PRT
<213> Homo sapiens

<400> 26
Met Asn Thr Ser Val Pro Pro Ala Val Ser Pro Asn Ile Thr Val Leu
  1               5                  10                  15

Ala Pro Gly Lys Gly Pro Trp Gln Val Ala Phe Ile Gly Ile Thr Thr
            20                  25                  30

Gly Leu Leu Ser Leu Ala Thr Val Thr Gly Asn Leu Leu Val Leu Ile
        35                  40                  45

Ser Phe Lys Val Asn Thr Glu Leu Lys Thr Val Asn Asn Tyr Phe Leu
    50                  55                  60

Leu Ser Leu Ala Cys Ala Asp Leu Ile Ile Gly Thr Phe Ser Met Asn
 65                  70                  75                  80

Leu Tyr Thr Thr Tyr Leu Leu Met Gly His Trp Ala Leu Gly Thr Leu
                85                  90                  95

Ala Cys Asp Leu Trp Leu Ala Leu Asp Tyr Val Ala Ser Asn Ala Ser
            100                 105                 110

Val Met Asn Leu Leu Leu Ile Ser Phe Asp Arg Tyr Phe Ser Val Thr

115                 120                 125

Arg Pro Leu Ser Tyr Arg Ala Lys Arg Thr Pro Arg Arg Ala Ala Leu
    130                 135                 140

Met Ile Gly Leu Ala Trp Leu Val Ser Phe Val Leu Trp Ala Pro Ala
145                 150                 155                 160

Ile Leu Phe Trp Gln Tyr Leu Val Gly Glu Arg Thr Val Leu Ala Gly
                165                 170                 175

Gln Cys Tyr Ile Gln Phe Leu Ser Gln Pro Ile Ile Thr Phe Gly Thr
            180                 185                 190

Ala Met Ala Ala Phe Tyr Leu Pro Val Thr Val Met Cys Thr Leu Tyr
        195                 200                 205

Trp Arg Ile Tyr Arg Glu Thr Glu Asn Arg Ala Arg Glu Leu Ala Ala
    210                 215                 220

Leu Gln Gly Ser Glu Thr Pro Gly Lys Gly Gly Gly Ser Ser Ser Ser
225                 230                 235                 240

Ser Glu Arg Ser Gln Pro Gly Ala Glu Gly Ser Pro Glu Ser Pro Pro
                245                 250                 255

Gly Arg Cys Cys Arg Cys Cys Arg Ala Pro Arg Leu Leu Gln Ala Tyr
            260                 265                 270

Ser Trp Lys Glu Glu Glu Glu Glu Asp Glu Gly Ser Met Glu Ser Leu
        275                 280                 285

Thr Ser Ser Glu Gly Glu Glu Pro Gly Ser Glu Val Val Ile Lys Met
    290                 295                 300

Pro Met Val Asp Ser Glu Ala Gln Ala Pro Thr Lys Gln Pro Pro Lys
305                 310                 315                 320

Ser Ser Pro Asn Thr Val Lys Arg Pro Thr Lys Lys Gly Arg Asp Arg
                325                 330                 335

Gly Gly Lys Gly Gln Lys Pro Arg Gly Lys Glu Gln Leu Ala Lys Arg
            340                 345                 350

Lys Thr Phe Ser Leu Val Lys Glu Lys Lys Ala Ala Arg Thr Leu Ser
        355                 360                 365

Ala Ile Leu Leu Ala Phe Ile Leu Thr Trp Thr Pro Tyr Asn Ile Met
    370                 375                 380

Val Leu Val Ser Thr Phe Cys Lys Asp Cys Val Pro Glu Thr Leu Trp
385                 390                 395                 400

Glu Leu Gly Tyr Trp Leu Cys Tyr Val Asn Ser Thr Val Asn Pro Met
                405                 410                 415

Cys Tyr Ala Leu Cys Asn Lys Ala Phe Arg Asp Thr Phe Arg Leu Leu
            420                 425                 430

Leu Leu Cys Arg Trp Asp Lys Arg Arg Trp Arg Lys Ile Pro Lys Arg
        435                 440                 445

Pro Gly Ser Val His Arg Thr Pro Ser Arg Gln Cys
    450                 455                 460

<210> 27
<211> 350
<212> PRT
<213> Rattus norvegicus

<400> 27

<210> 28
<211> 601
<212> PRT
<213> Drosophila melanogaster

<400> 28
   Met Pro Ser Ala Asp Gln Ile Leu Phe Val Asn Val Thr Thr Thr Val
     1               5                  10                  15
   Ala Ala Ala Ala Leu Thr Ala Ala Ala Ala Val Ser Thr Thr Lys Ser
                20                  25                  30
   Gly Asn Gly Asn Ala Ala Arg Gly Tyr Thr Asp Ser Asp Asp Asp Ala
            35                  40                  45
   Gly Met Gly Thr Glu Ala Val Ala Asn Ile Ser Gly Ser Leu Val Glu
        50                  55                  60
   Gly Leu Thr Thr Val Thr Ala Ala Leu Ser Thr Ala Gln Ala Asp Lys
    65                  70                  75                  80
   Asp Ser Ala Gly Glu Cys Glu Gly Ala Val Glu Glu Leu His Ala Ser
                    85                  90                  95
   Ile Leu Gly Leu Gln Leu Ala Val Pro Glu Trp Glu Ala Leu Leu Thr
               100                 105                 110
   Ala Leu Val Leu Ser Val Ile Ile Val Leu Thr Ile Ile Gly Asn Ile
           115                 120                 125
   Leu Val Ile Leu Ser Val Phe Thr Tyr Lys Pro Leu Arg Ile Val Gln
       130                 135                 140
   Asn Phe Phe Ile Val Ser Leu Ala Val Ala Asp Leu Thr Val Ala Leu
   145                 150                 155                 160
   Leu Val Leu Pro Phe Asn Val Ala Tyr Ser Ile Leu Gly Arg Trp Glu
                   165                 170                 175
   Phe Gly Ile His Leu Cys Lys Leu Trp Leu Thr Cys Asp Val Leu Cys
               180                 185                 190
   Cys Thr Ser Ser Ile Leu Asn Leu Cys Ala Ile Ala Leu Asp Arg Tyr
           195                 200                 205
   Trp Ala Ile Thr Asp Pro Ile Asn Tyr Ala Gln Lys Arg Thr Val Gly
       210                 215                 220
   Arg Val Leu Leu Leu Ile Ser Gly Val Trp Leu Leu Ser Leu Leu Ile
   225                 230                 235                 240
   Ser Ser Pro Pro Leu Ile Gly Trp Asn Asp Trp Pro Asp Glu Phe Thr
                   245                 250                 255
   Ser Ala Thr Pro Cys Glu Leu Thr Ser Gln Arg Gly Tyr Val Ile Tyr
               260                 265                 270
   Ser Ser Leu Gly Ser Phe Phe Ile Pro Leu Ala Ile Met Thr Ile Val
           275                 280                 285
   Tyr Ile Glu Ile Phe Val Ala Thr Arg Arg Arg Leu Arg Glu Arg Ala
       290                 295                 300
   Arg Ala Asn Lys Leu Asn Thr Ile Ala Leu Lys Ser Thr Glu Leu Glu
   305                 310                 315                 320
   Pro Met Ala Asn Ser Ser Pro Val Ala Ala Ser Asn Ser Gly Ser Lys
                   325                 330                 335
   Ser Arg Leu Leu Ala Ser Trp Leu Cys Cys Gly Arg Asp Arg Ala Gln
               340                 345                 350
   Phe Ala Thr Pro Met Ile Gln Asn Asp Gln Glu Ser Ile Ser Ser Glu
           355                 360                 365
   Thr His Gln Pro Gln Asp Ser Ser Lys Ala Gly Pro His Gly Asn Ser
       370                 375                 380
   Asp Pro Gln Gln Gln His Val Val Val Leu Val Lys Lys Ser Arg Arg
   385                 390                 395                 400
   Ala Lys Thr Lys Asp Ser Ile Lys His Gly Lys Thr Arg Gly Gly Arg
                   405                 410                 415
   Lys Ser Gln Ser Ser Ser Thr Cys Glu Pro His Gly Glu Gln Gln Leu
               420                 425                 430
   Leu Pro Ala Gly Gly Asp Gly Gly Ser Cys Gln Pro Gly Gly Gly His
           435                 440                 445
   Ser Gly Gly Gly Lys Ser Asp Ala Glu Ile Ser Thr Glu Ser Gly Ser
       450                 455                 460
   Asp Pro Lys Gly Cys Ile Gln Val Cys Val Thr Gln Ala Asp Glu Gln
   465                 470                 475                 480
   Thr Ser Leu Lys Leu Thr Pro Pro Gln Ser Ser Thr Gly Val Ala Ala
                   485                 490                 495
   Val Ser Val Thr Pro Leu Gln Lys Lys Thr Ser Gly Val Asn Gln Phe
               500                 505                 510
   Ile Glu Glu Lys Gln Lys Ile Ser Leu Ser Lys Glu Arg Arg Ala Ala
           515                 520                 525
   Arg Thr Leu Gly Ile Ile Met Gly Val Phe Val Ile Cys Trp Leu Pro
       530                 535                 540
   Phe Phe Leu Met Tyr Val Ile Leu Pro Phe Cys Gln Thr Cys Cys Pro
   545                 550                 555                 560
   Thr Asn Lys Phe Lys Asn Phe Ile Thr Trp Leu Gly Tyr Ile Asn Ser
                   565                 570                 575
   Gly Leu Asn Pro Val Ile Tyr Thr Ile Phe Asn Leu Asp Tyr Arg Arg
               580                 585                 590
   Ala Phe Lys Arg Leu Leu Gly Leu Asn
           595                 600


<210> 29
<211> 588
<212> PRT
<213> Homo sapiens

<400> 29
   Met Ala Arg Gly Gly Ala Gly Ala Glu Glu Ala Ser Leu Arg Ser Asn
     1               5                  10                  15
   Ala Leu Ser Trp Leu Ala Cys Gly Leu Leu Ala Leu Leu Ala Asn Ala
                20                  25                  30
   Trp Ile Ile Leu Ser Ile Ser Ala Lys Gln Gln Lys His Lys Pro Leu
            35                  40                  45
   Glu Leu Leu Leu Cys Phe Leu Ala Gly Thr His Ile Leu Met Ala Ala
        50                  55                  60
   Val Pro Leu Thr Thr Phe Ala Val Val Gln Leu Arg Arg Gln Ala Ser
    65                  70                  75                  80
   Ser Asp Tyr Asp Trp Asn Glu Ser Ile Cys Lys Val Phe Val Ser Thr
                    85                  90                  95
   Tyr Tyr Thr Leu Ala Leu Ala Thr Cys Phe Thr Val Ala Ser Leu Ser
               100                 105                 110
   Tyr His Arg Met Trp Met Val Arg Trp Pro Val Asn Tyr Arg Leu Ser
   115                 120                 125
   Asn Ala Lys Lys Gln Ala Leu His Ala Val Met Gly Ile Trp Met Val
       130                 135                 140
   Ser Phe Ile Leu Ser Thr Leu Pro Ser Ile Gly Trp His Asn Asn Gly
   145                 150                 155                 160
   Glu Arg Tyr Tyr Ala Arg Gly Cys Gln Phe Ile Val Ser Lys Ile Gly
                   165                 170                 175
   Leu Gly Phe Gly Val Cys Phe Ser Leu Leu Leu Leu Gly Gly Ile Val
               180                 185                 190
   Met Gly Leu Val Cys Val Ala Ile Thr Phe Tyr Gln Thr Leu Trp Ala
           195                 200                 205
   Arg Pro Arg Arg Ala Arg Gln Ala Arg Arg Val Gly Gly Gly Gly Gly
       210                 215                 220
   Thr Lys Ala Gly Gly Pro Gly Ala Leu Gly Thr Arg Pro Ala Phe Glu
   225                 230                 235                 240
   Val Pro Ala Ile Val Val Glu Asp Ala Arg Gly Lys Arg Arg Ser Ser
                   245                 250                 255
   Leu Asp Gly Ser Glu Ser Ala Lys Thr Ser Leu Gln Val Thr Asn Leu
               260                 265                 270
   Val Ser Ala Ile Val Phe Leu Tyr Asp Ser Leu Thr Gly Val Pro Ile
           275                 280                 285
   Leu Val Val Ser Phe Phe Ser Leu Lys Ser Asp Ser Ala Pro Pro Trp
       290                 295                 300
   Met Val Leu Ala Val Leu Trp Cys Ser Met Ala Gln Thr Leu Leu Leu
   305                 310                 315                 320
   Pro Ser Phe Ile Trp Ser Cys Glu Arg Tyr Arg Ala Asp Val Arg Thr
                   325                 330                 335
   Val Trp Glu Gln Cys Val Ala Ile Met Ser Glu Glu Asp Gly Asp Asp
               340                 345                 350
   Asp Gly Gly Cys Asp Asp Tyr Ala Glu Gly Arg Val Cys Lys Val Arg
           355                 360                 365
   Phe Asp Ala Asn Gly Ala Thr Gly Pro Gly Ser Arg Asp Pro Ala Gln
       370                 375                 380
   Val Lys Leu Leu Pro Gly Arg His Met Leu Phe Pro Pro Leu Glu Arg
   385                 390                 395                 400
   Val His Tyr Leu Gln Val Pro Leu Ser Arg Arg Leu Ser His Asp Glu
                   405                 410                 415
   Thr Asn Ile Phe Ser Thr Pro Arg Glu Pro Gly Ser Phe Leu His Lys
               420                 425                 430
   Trp Ser Ser Ser Asp Asp Ile Arg Val Leu Pro Ala Gln Ser Arg Ala
           435                 440                 445
   Leu Gly Gly Pro Pro Glu Tyr Leu Gly Gln Arg His Arg Leu Glu Asp
       450                 455                 460
   Glu Glu Asp Glu Glu Glu Ala Glu Gly Gly Gly Leu Ala Ser Leu Arg
   465                 470                 475                 480
   Gln Phe Leu Glu Ser Gly Val Leu Gly Ser Gly Gly Gly Pro Pro Arg
                   485                 490                 495
   Gly Pro Gly Phe Phe Arg Glu Glu Ile Thr Thr Phe Ile Asp Glu Thr
               500                 505                 510
   Pro Leu Pro Ser Pro Thr Ala Ser Pro Gly His Ser Pro Arg Arg Pro
           515                 520                 525
   Arg Pro Leu Gly Leu Ser Pro Arg Arg Leu Ser Leu Gly Ser Pro Glu
       530                 535                 540
   Ser Arg Ala Val Gly Leu Pro Leu Gly Leu Ser Ala Gly Arg Arg Cys
   545                 550                 555                 560
   Ser Leu Thr Gly Gly Glu Glu Ser Ala Arg Ala Trp Gly Gly Ser Trp
                   565                 570                 575
   Gly Pro Gly Asn Pro Ile Phe Pro Gln Leu Thr Leu
               580                 585


<210> 30
<211> 227
<212> PRT
<213> Homo sapiens

<400> 30
Ile Thr Phe Tyr Gln Thr Leu Trp Ala Arg Pro Arg Arg Ala Arg Gln
  1               5                  10                  15

Ala Arg Arg Val Gly Gly Gly Gly Gly Thr Lys Ala Gly Gly Pro Gly
             20                  25                  30

Ala Leu Gly Thr Arg Pro Ala Phe Glu Val Pro Ala Ile Val Val Glu
         35                  40                  45

Asp Ala Arg Gly Lys Arg Arg Ser Ser Leu Asp Gly Ser Glu Ser Ala
     50                  55                  60

Lys Thr Ser Leu Gln Val Thr Asn Leu Val Val Ser Phe Phe Ser Leu
 65                  70                  75                  80

Lys Ser Asp Ser Ala Pro Pro Trp Met Val Leu Ala Val Leu Trp Cys
                 85                  90                  95

Ser Met Ala Gln Thr Leu Leu Leu Pro Ser Phe Ile Trp Ser Cys Glu

            100                 105                 110

Arg Tyr Arg Ala Asp Val Arg Thr Val Trp Glu Gln Cys Val Ala Ile
        115                 120                 125

Met Ser Glu Glu Asp Gly Asp Asp Asp Gly Gly Cys Asp Asp Tyr Ala
    130                 135                 140

Glu Gly Arg Val Cys Lys Val Arg Phe Asp Ala Asn Gly Ala Thr Gly
145                 150                 155                 160

Pro Gly Ser Arg Asp Pro Ala Gln Val Lys Leu Leu Pro Gly Arg His
                165                 170                 175

Met Leu Phe Pro Pro Leu Glu Arg Val His Tyr Leu Gln Leu Lys Lys
            180                 185                 190

Leu Asp Leu Ala Ala Ala Ala Ala His Thr Phe Phe Val Ala Asn Pro
        195                 200                 205

Met His Leu Gln Met Arg Glu Asp Met Ala Lys Tyr Arg Arg Met Ser
    210                 215                 220

Gly Val Arg
225

SEQUENCE LISTING

<110> Millennium Biotherapeutics, Inc.

<120> NOVEL SECRETED AND MEMBRANE-ASSOCIATED PROTEINS AND
      USES THEREFOR

<130> MEI-017CPPC

<140>
<141>

<150> USSN 09/086,892
<151> 1998-05-29

<150> USSN 09/087,121
<151> 1998-05-29

<160> 30

<170> PatentIn Ver. 2.0

<210> 1
<211> 1119
<212> DNA
<213> Homo sapiens

<220>
<221> CDS
<222> (140)..(871)

<400> 1
   gtcgacccac gcgtccggca ggatgtttgc agtgtcgcgc ccagggctct gagactgagc 60
   ctgccatcca ctcgcacgcc tttctttcag ggcttttcgg ctgttggcta cactgatgtg 120
   acccccctcc cttttggga atg atg ggg atc ttt ttg gtg tat gtt gga ttt  172
                       Met Met Gly Ile Phe Leu Val Tyr Val Gly Phe
                         1               5                  10
   gtt ttc ttt tcc gtt tta tat gta caa caa ggg ctt tct tct caa gca 220
   Val Phe Phe Ser Val Leu Tyr Val Gln Gln Gly Leu Ser Ser Gln Ala
                15                  20                  25
   aaa ttt acc gag ttt ccg cgg aac gtg acg gcg acc gag ggg cag aat 268
   Lys Phe Thr Glu Phe Pro Arg Asn Val Thr Ala Thr Glu Gly Gln Asn
            30                  35                  40
   gtg gag atg tcc tgc gcc ttc cag agc ggc tcc gcc tcg gtg tat ctg 316
   Val Glu Met Ser Cys Ala Phe Gln Ser Gly Ser Ala Ser Val Tyr Leu
        45                  50                  55
   gag atc caa tgg tgg ttc ctg cgg ggg ccg gag gac ctg gat ccc ggg 364
   Glu Ile Gln Trp Trp Phe Leu Arg Gly Pro Glu Asp Leu Asp Pro Gly
    60                  65                  70                  75
   gcc gag ggg gcc ggc gcg cag gtg gag ctc ttg ccc gac aga gac ccg    412
   Ala Glu Gly Ala Gly Ala Gln Val Glu Leu Leu Pro Asp Arg Asp Pro
                   80                  85                  90
   gac agc gac ggg acc aag atc agc aca gtg aaa gtc caa ggc aat gac    460
   Asp Ser Asp Gly Thr Lys Ile Ser Thr Val Lys Val Gln Gly Asn Asp
               95                  100                 105
   atc tcc cac aag ctt cag att tcc aaa gtg agg aaa aag gat gaa ggc    508
   Ile Ser His Lys Leu Gln Ile Ser Lys Val Arg Lys Lys Asp Glu Gly
           110                 115                 120
   tta tat gag tgc agg gtg act gat gcc aac tac ggg gag ctt cag gaa    556
   Leu Tyr Glu Cys Arg Val Thr Asp Ala Asn Tyr Gly Glu Leu Gln Glu
       125                 130                 135
   cac aag gcc cag gcc tat ctg aaa gtc aat gcc aac agc cat gcc cgc    604
   His Lys Ala Gln Ala Tyr Leu Lys Val Asn Ala Asn Ser His Ala Arg
   140                 145                 150                 155
   aga atg cag gcc ttc gaa gcc tcg ccc atg tgg ctg cag gat atg aag    652
   Arg Met Gln Ala Phe Glu Ala Ser Pro Met Trp Leu Gln Asp Met Lys
                   160                 165                 170
   ccc cgc aag aac gtc tcc gca gcc atc ccc agc agc atc cat ggc tct    700
   Pro Arg Lys Asn Val Ser Ala Ala Ile Pro Ser Ser Ile His Gly Ser
               175                 180                 185
   gcc aac caa cga acg cac tcc acc tcc agc cct caa gtg gta gcc aaa    748
   Ala Asn Gln Arg Thr His Ser Thr Ser Ser Pro Gln Val Val Ala Lys
           190                 195                 200
   atc ccc aaa caa agt cca caa tca ggt atg gaa acc cat ttc gag cct    796
   Ile Pro Lys Gln Ser Pro Gln Ser Gly Met Glu Thr His Phe Glu Pro
       205                 210                 215
   ttt att tta cca ctc aca aac gct cca cag aaa ggt cag tcg tat aga    844
   Phe Ile Leu Pro Leu Thr Asn Ala Pro Gln Lys Gly Gln Ser Tyr Arg
   220                 225                 230                 235
   gta gac aga ttt atg aat ggt gat ttt taaaatcgga gacctagttc          891
   Val Asp Arg Phe Met Asn Gly Asp Phe
                   240
   agtgcaagtg attatgagag gtgagcactg agcctgcacc aattcactca gagctcaaag 951
   catgtgggtg caccccgtca gtcccctagt ggtgcttcat ttccagggca tctgagagct 1011
   ggactctggt ttttatcctt tctgtattta cacattataa gaacaataaa tcatgtaatg 1071
   ttggttacat tacaaaaaaa aaaaaaaaaa aaaaaaaagg gcggccgc              1119

<210> 2
<211> 244
<212> PRT
<213> Homo sapiens

<400> 2
Met Met Gly Ile Phe Leu Val Tyr Val Gly Phe Val Phe Phe Ser Val
1               5                   10                  15

Leu Tyr Val Gln Gln Gly Leu Ser Ser Gln Ala Lys Phe Thr Glu Phe
            20                  25                  30

Pro Arg Asn Val Thr Ala Thr Glu Gly Gln Asn Val Glu Met Ser Cys
        35                  40                  45

Ala Phe Gln Ser Gly Ser Ala Ser Val Tyr Leu Glu Ile Gln Trp Trp
    50                  55                  60

Phe Leu Arg Gly Pro Glu Asp Leu Asp Pro Gly Ala Glu Gly Ala Gly
65                  70                  75                  80

Ala Gln Val Glu Leu Leu Pro Asp Arg Asp Pro Asp Ser Asp Gly Thr
                85                  90                  95

Lys Ile Ser Thr Val Lys Val Gln Gly Asn Asp Ile Ser His Lys Leu
            100                 105                 110

Gln Ile Ser Lys Val Arg Lys Lys Asp Glu Gly Leu Tyr Glu Cys Arg
        115                 120                 125

Val Thr Asp Ala Asn Tyr Gly Glu Leu Gln Glu His Lys Ala Gln Ala
    130                 135                 140

Tyr Leu Lys Val Asn Ala Asn Ser His Ala Arg Arg Met Gln Ala Phe
145                 150                 155                 160

Glu Ala Ser Pro Met Trp Leu Gln Asp Met Lys Pro Arg Lys Asn Val
                165                 170                 175

Ser Ala Ala Ile Pro Ser Ser Ile His Gly Ser Ala Asn Gln Arg Thr
            180                 185                 190

His Ser Thr Ser Ser Pro Gln Val Val Ala Lys Ile Pro Lys Gln Ser
        195                 200                 205

Pro Gln Ser Gly Met Glu Thr His Phe Glu Pro Phe Ile Leu Pro Leu
    210                 215                 220

Thr Asn Ala Pro Gln Lys Gly Gln Ser Tyr Arg Val Asp Arg Phe Met
225                 230                 235                 240

Asn Gly Asp Phe

<210> 3
<211> 732
<212> DNA
<213> Homo sapiens

<220>
<221> CDS
<222> (1)..(732)

<400> 3
atg atg ggg atc ttt ttg gtg tat gtt gga ttt gtt ttc ttt tcc gtt     48
Met Met Gly Ile Phe Leu Val Tyr Val Gly Phe Val Phe Phe Ser Val
1               5                   10                  15

tta tat gta caa caa ggg ctt tct tct caa gca aaa ttt acc gag ttt     96
Leu Tyr Val Gln Gln Gly Leu Ser Ser Gln Ala Lys Phe Thr Glu Phe
            20                  25                  30

ccg cgg aac gtg acg gcg acc gag ggg cag aat gtg gag atg tcc tgc     144
Pro Arg Asn Val Thr Ala Thr Glu Gly Gln Asn Val Glu Met Ser Cys
        35                  40                  45

gcc ttc cag agc ggc tcc gcc tcg gtg tat ctg gag atc caa tgg tgg     192
Ala Phe Gln Ser Gly Ser Ala Ser Val Tyr Leu Glu Ile Gln Trp Trp
    50                  55                  60

ttc ctg cgg ggg ccg gag gac ctg gat ccc ggg gcc gag ggg gcc ggc     240
Phe Leu Arg Gly Pro Glu Asp Leu Asp Pro Gly Ala Glu Gly Ala Gly
65                  70                  75                  80

gcg cag gtg gag ctc ttg ccc gac aga gac ccg gac agc gac ggg acc     288
Ala Gln Val Glu Leu Leu Pro Asp Arg Asp Pro Asp Ser Asp Gly Thr
                85                  90                  95

aag atc agc aca gtg aaa gtc caa ggc aat gac atc tcc cac aag ctt     336
Lys Ile Ser Thr Val Lys Val Gln Gly Asn Asp Ile Ser His Lys Leu
            100                 105                 110

cag att tcc aaa gtg agg aaa aag gat gaa ggc tta tat gag tgc agg     384
Gln Ile Ser Lys Val Arg Lys Lys Asp Glu Gly Leu Tyr Glu Cys Arg
        115                 120                 125

gtg act gat gcc aac tac ggg gag ctt cag gaa cac aag gcc cag gcc     432
Val Thr Asp Ala Asn Tyr Gly Glu Leu Gln Glu His Lys Ala Gln Ala
    130                 135                 140

tat ctg aaa gtc aat gcc aac agc cat gcc cgc aga atg cag gcc ttc     480
Tyr Leu Lys Val Asn Ala Asn Ser His Ala Arg Arg Met Gln Ala Phe
145                 150                 155                 160

gaa gcc tcg ccc atg tgg ctg cag gat atg aag ccc cgc aag aac gtc     528
Glu Ala Ser Pro Met Trp Leu Gln Asp Met Lys Pro Arg Lys Asn Val
                165                 170                 175

tcc gca gcc atc ccc agc agc atc cat ggc tct gcc aac caa cga acg     576
Ser Ala Ala Ile Pro Ser Ser Ile His Gly Ser Ala Asn Gln Arg Thr
            180                 185                 190

cac tcc acc tcc agc cct caa gtg gta gcc aaa atc ccc aaa caa agt     624
His Ser Thr Ser Ser Pro Gln Val Val Ala Lys Ile Pro Lys Gln Ser
        195                 200                 205

cca caa tca ggt atg gaa acc cat ttc gag cct ttt att tta cca ctc     672
Pro Gln Ser Gly Met Glu Thr His Phe Glu Pro Phe Ile Leu Pro Leu
    210                 215                 220

aca aac gct cca cag aaa ggt cag tcg tat aga gta gac aga ttt atg 720
Thr Asn Ala Pro Gln Lys Gly Gln Ser Tyr Arg Val Asp Arg Phe Met
225                 230                 235                 240

aat ggt gat ttt 732
Asn Gly Asp Phe

<210> 4
<211> 660
<212> DNA
<213> Homo sapiens

<220>
<221> CDS
<222> (1)..(657)

<400> 4
caa gca aaa ttt acc gag ttt ccg cgg aac gtg acg gcg acc gag ggg 48
Gln Ala Lys Phe Thr Glu Phe Pro Arg Asn Val Thr Ala Thr Glu Gly
1               5                   10                  15

cag aat gtg gag atg tcc tgc gcc ttc cag agc ggc tcc gcc tcg gtg 96
Gln Asn Val Glu Met Ser Cys Ala Phe Gln Ser Gly Ser Ala Ser Val
            20                  25                  30

tat ctg gag atc caa tgg tgg ttc ctg cgg ggg ccg gag gac ctg gat 144
Tyr Leu Glu Ile Gln Trp Trp Phe Leu Arg Gly Pro Glu Asp Leu Asp
        35                  40                  45

ccc ggg gcc gag ggg gcc ggc gcg cag gtg gag ctc ttg ccc gac aga 192
Pro Gly Ala Glu Gly Ala Gly Ala Gln Val Glu Leu Leu Pro Asp Arg
    50                  55                  60

gac ccg gac agc gac ggg acc aag atc agc aca gtg aaa gtc caa ggc 240
Asp Pro Asp Ser Asp Gly Thr Lys Ile Ser Thr Val Lys Val Gln Gly
65                  70                  75                  80

aat gac atc tcc cac aag ctt cag att tcc aaa gtg agg aaa aag gat 288
Asn Asp Ile Ser His Lys Leu Gln Ile Ser Lys Val Arg Lys Lys Asp
                85                  90                  95

gaa ggc tta tat gag tgc agg gtg act gat gcc aac tac ggg gag ctt 336
Glu Gly Leu Tyr Glu Cys Arg Val Thr Asp Ala Asn Tyr Gly Glu Leu
            100                 105                 110

cag gaa cac aag gcc cag gcc tat ctg aaa gtc aat gcc aac agc cat 384
Gln Glu His Lys Ala Gln Ala Tyr Leu Lys Val Asn Ala Asn Ser His
        115                 120                 125

gcc cgc aga atg cag gcc ttc gaa gcc tcg ccc atg tgg ctg cag gat 432
Ala Arg Arg Met Gln Ala Phe Glu Ala Ser Pro Met Trp Leu Gln Asp
    130                 135                 140

atg aag ccc cgc aag aac gtc tcc gca gcc atc ccc agc agc atc cat 480
Met Lys Pro Arg Lys Asn Val Ser Ala Ala Ile Pro Ser Ser Ile His
145                 150                 155                 160

ggc tct gcc aac caa cga acg cac tcc acc tcc agc cct caa gtg gta    528
Gly Ser Ala Asn Gln Arg Thr His Ser Thr Ser Ser Pro Gln Val Val
                165                 170                 175

gcc aaa atc ccc aaa caa agt cca caa tca ggt atg gaa acc cat ttc    576
Ala Lys Ile Pro Lys Gln Ser Pro Gln Ser Gly Met Glu Thr His Phe
            180                 185                 190

gag cct ttt att tta cca ctc aca aac gct cca cag aaa ggt cag tcg    624
Glu Pro Phe Ile Leu Pro Leu Thr Asn Ala Pro Gln Lys Gly Gln Ser
        195                 200                 205

tat aga gta gac aga ttt atg aat ggt gat ttt taa                    660
Tyr Arg Val Asp Arg Phe Met Asn Gly Asp Phe
    210                 215


<210> 5
<211> 219
<212> PRT
<213> Homo sapiens

<400> 5
Gln Ala Lys Phe Thr Glu Phe Pro Arg Asn Val Thr Ala Thr Glu Gly
  1               5                  10                  15

Gln Asn Val Glu Met Ser Cys Ala Phe Gln Ser Gly Ser Ala Ser Val
             20                  25                  30

Tyr Leu Glu Ile Gln Trp Trp Phe Leu Arg Gly Pro Glu Asp Leu Asp
         35                  40                  45

Pro Gly Ala Glu Gly Ala Gly Ala Gln Val Glu Leu Leu Pro Asp Arg
     50                  55                  60

Asp Pro Asp Ser Asp Gly Thr Lys Ile Ser Thr Val Lys Val Gln Gly
 65                  70                  75                  80

Asn Asp Ile Ser His Lys Leu Gln Ile Ser Lys Val Arg Lys Lys Asp
                 85                  90                  95

Glu Gly Leu Tyr Glu Cys Arg Val Thr Asp Ala Asn Tyr Gly Glu Leu
            100                 105                 110

Gln Glu His Lys Ala Gln Ala Tyr Leu Lys Val Asn Ala Asn Ser His
        115                 120                 125

Ala Arg Arg Met Gln Ala Phe Glu Ala Ser Pro Met Trp Leu Gln Asp
    130                 135                 140

Met Lys Pro Arg Lys Asn Val Ser Ala Ala Ile Pro Ser Ser Ile His
145                 150                 155                 160

Gly Ser Ala Asn Gln Arg Thr His Ser Thr Ser Ser Pro Gln Val Val
                165                 170                 175

Ala Lys Ile Pro Lys Gln Ser Pro Gln Ser Gly Met Glu Thr His Phe
            180                 185                 190

Glu Pro Phe Ile Leu Pro Leu Thr Asn Ala Pro Gln Lys Gly Gln Ser
        195                 200                 205

Tyr Arg Val Asp Arg Phe Met Asn Gly Asp Phe
    210                 215

<210> 6
<211> 3196
<212> DNA
<213> Mus musculus

<220>
<221> CDS
<222> (268)..(1020)

<400> 6
   cacgcgtccg cagccagccg gcggcggaga cacttcacgg cgtggcaacc cgggtctgtg 60
   ccttgaagcc tccggatcgc agccagctcg gtccatccct cactagtcgc aatcccctgt 120
   gtccaagcta ctctttgcta tgagcggcag catgcgtgca gtatcgcgcc ccaggctctg 180
   agagcagcct gcggacacgc ttgcctatct gtctttttag gttttggggc tctgggctac 240
   acggatgtgc cccactccct tggcatg atg ggg atc ttt ttg gcg tct gtt gga 294
                                 Met Gly Ile Phe Leu Ala Ser Val Gly
                                 1               5
   ttt atg ttc ttt tcc gtg tta tat gta caa caa ggg ctt tct tct caa    342
   Phe Met Phe Phe Ser Val Leu Tyr Val Gln Gln Gly Leu Ser Ser Gln
    10                  15                  20                  25
   gca aaa ttt acc gag ttg ccg aga aat gtg act gct acc gaa ggg caa    390
   Ala Lys Phe Thr Glu Leu Pro Arg Asn Val Thr Ala Thr Glu Gly Gln
                    30                  35                  40
   aat gtg gag atg tcc tgt gct ttc caa agc ggc tct gct tca gtg tac    438
   Asn Val Glu Met Ser Cys Ala Phe Gln Ser Gly Ser Ala Ser Val Tyr
                45                  50                  55
   ctg gag atc cag tgg tgg ttc ctt cgg ggg cca gag gac ctg gag caa    486
   Leu Glu Ile Gln Trp Trp Phe Leu Arg Gly Pro Glu Asp Leu Glu Gln
            60                  65                  70
   ggc acg gag gct gca ggc tcg cag gtg gag ctc tta ccc gac aga gac    534
   Gly Thr Glu Ala Ala Gly Ser Gln Val Glu Leu Leu Pro Asp Arg Asp
        75                  80                  85
   ccg gac aac gat ggg acc aag att agt aca gtg aaa gtc caa ggc aat    582
   Pro Asp Asn Asp Gly Thr Lys Ile Ser Thr Val Lys Val Gln Gly Asn
    90                  95                  100                 105
   gat atc tcc cac aag ctt cag ata tcc aaa gtg aga aaa aag gat gaa    630
   Asp Ile Ser His Lys Leu Gln Ile Ser Lys Val Arg Lys Lys Asp Glu
                    110                 115                 120
   ggt tta tac gag tgc agg gtg act gac gct aac tac ggg gag ctt cag 678
   Gly Leu Tyr Glu Cys Arg Val Thr Asp Ala Asn Tyr Gly Glu Leu Gln
   125 130 135
   gaa cac aag gcc cag gcc tat ctg aaa gtc aat gcc aac agc cat gct 726
   Glu His Lys Ala Gln Ala Tyr Leu Lys Val Asn Ala Asn Ser His Ala
   140 145 150
   cgg agg atg cag gcc ttt gaa gcc tca cct atg tgg ctg caa gac acg 774
   Arg Arg Met Gln Ala Phe Glu Ala Ser Pro Met Trp Leu Gln Asp Thr
   155 160 165
   aag cct cga aag aac gca tca tcg gtg gtt ccc agc agc gtc cac aac 822
   Lys Pro Arg Lys Asn Ala Ser Ser Val Val Pro Ser Ser Val His Asn
   170 175 180 185
   tct gcc aac caa cga atg cac tcc acc tcc agc cct caa gcg gta gcc 870
   Ser Ala Asn Gln Arg Met His Ser Thr Ser Ser Pro Gln Ala Val Ala
   190 195 200
   aaa atc ccc aag caa agt cca caa tca gca aag agc aaa tcg cct gta 918
   Lys Ile Pro Lys Gln Ser Pro Gln Ser Ala Lys Ser Lys Ser Pro Val
   205 210 215
   aaa tct acg gag cgg aca gca aag ttg acc cta tac tcc aag cac cat 966
   Lys Ser Thr Glu Arg Thr Ala Lys Leu Thr Leu Tyr Ser Lys His His
   220 225 230
   tct gca ccc ctg tac tct agt tat cta cac aag gag cat cag ctt ccg 1014
   Ser Ala Pro Leu Tyr Ser Ser Tyr Leu His Lys Glu His Gln Leu Pro
   235 240 245
   gaa gca taagtgaaga cactgtcaca cgctttattg ataatatttt ctttgggaag 1070
   Glu Ala
   250
   ttgctgatct tttatttcaa gagaattaat gggaagagat aggacatttt ccaattacaa 1130
   gaccaatttt tttcctttta tttcaacaaa taaaacctgc atttcactga ctgctcagga 1190
   gttggcctga atgacatcag tatactaaat atttccatgg attccaccaa tttcctaacg 1250
   agggacacct aatcttcaag aagcaaacaa agatggaaaa cctaagaacc acaaactgtc 1310
   tcatacagca ccccagctga ggaacaaaac aaatagctaa atgctgacca tggcaaatca 1370
   acatcagaca actttatttt acatatggaa taatcaaaga aagttttttt tttacttcct 1430
   ttttgccccc tggaatttat cttggagttt cccttttttc cttgattgcc gttttcgttc 1490
   aatggtagca agtgccaatt atggccaatc cttgtcaatc ctggaaggtt tatattcata 1550
   tacattgagt gtggtatata tcaatgtatt ttaattcatt tggcaatttc tgtataggca 1610
   aacctggcaa attctgtaaa ttgcttatag tatgtgtgat atgacttcaa ggtagatagg 1670
   ctatgatgct catgcaagct gactttcttc attctatata caaatatatt catgagcata 1730
   tattaggcca ccaacttctt ttcctaaaga attatttttc atttgtacct catgtatttt 1790
   gtgaattttg tagtatattt ctctgttcca ctagtttgac cgctacagtt tgtctctgtt 1850
   gtcctctact tccttctgga aaaatttaaa attgtgtatg tctctgataa atgaattaat 1910
   tttgttgtgt gtatgctatg ttggaatttg ctgtgttctt ttaaacatgt atttattaag 1970
   gtttggggat cttgagttga gtctgaagaa tgcacacctg gtttttgaca gagttcctca 2030
   tgttaccaat attctatctc agagaaagaa agacaccaag tgggaaaact aagaagacat 2090
   tttgacttcc caagatcctg gaagagcact tcacactctg actaaataat gttgcttttt 2150
   ttgttcttca agactttttt gtagctttgt ctttctgtta gttgctgcta attatatttt 2210
   aatgtctact aattaaaaat taaaatgtga ttgttggctg aatacaatat gcaaatgact 2270
   gcaaagccca tactgaagaa aatagatgtt taatcttcac tcaataatta taattttaaa 2330
   tagttcatca ttattttttg accttatgat attttgttta gacctgttct aattacatct 2390
   ttctctggca aagaaagata gaacaatcaa tacattccct cttacagtat ggaatggttg 2450
   tggcttaaga aagaatgcat ccagatggtc ttccagagag attattttat tttcattata 2510
   aaaccagaaa ccatatatgt aggaatggtt cattcctaat gtaaggccat aaattgtagc 2570
   ttgaaggcaa ggaatacatt tgttttttta tggtaaagga ctggcctctg acatgcactt 2630
   ataagcaatg tgaatatttt cataatatgc ttgacattct cctttaacaa atattgtttt 2690
   atggtaaatc tttccttgcc atttttcttc tttcatttga ttcattattt cattctaatg 2750
   aagaaaataa aggtttaatt atgatacttt attaacatac aaatgtattt tctttctaag 2810
   ttaaatatct gaaagttgta taaaatgatg gtagagaaat attactcatt cggtttcttt 2870
   gagctttaag aatcccatac attgcagtat atattagaat actgatttaa catcaaactg 2930
   ggggggaaaa tcatgtatta tacttttact caatgtctag gtaatggatt cagctaattt 2990
   tacagcaagc caaatgtgta cccgtatcag taatgttcac catgcttgta ataaaagggc 3050
   atatgctagt tttggaagaa tgctcattag attcattgta tcagtgtcca aaataataaa 3110
   gacctgttta tcactgtgaa aaaaaaaaaa aaaaaaaaaa aaaaaaaaaa aaaaaaaaaa 3170
   aaaaaaaaaa aaaaaagggc ggccgc 3196

<210> 7
<211> 251
<212> PRT
<213> Mus musculus

<400> 7
Met Gly Ile Phe Leu Ala Ser Val Gly Phe Met Phe Phe Ser Val Leu
1               5                   10                  15

Tyr Val Gln Gln Gly Leu Ser Ser Gln Ala Lys Phe Thr Glu Leu Pro
            20                  25                  30

Arg Asn Val Thr Ala Thr Glu Gly Gln Asn Val Glu Met Ser Cys Ala
        35                  40                  45

Phe Gln Ser Gly Ser Ala Ser Val Tyr Leu Glu Ile Gln Trp Trp Phe
    50                  55                  60

Leu Arg Gly Pro Glu Asp Leu Glu Gln Gly Thr Glu Ala Ala Gly Ser
65                  70                  75                  80

Gln Val Glu Leu Leu Pro Asp Arg Asp Pro Asp Asn Asp Gly Thr Lys
                85                  90                  95

Ile Ser Thr Val Lys Val Gln Gly Asn Asp Ile Ser His Lys Leu Gln
            100                 105                 110

Ile Ser Lys Val Arg Lys Lys Asp Glu Gly Leu Tyr Glu Cys Arg Val
        115                 120                 125

Thr Asp Ala Asn Tyr Gly Glu Leu Gln Glu His Lys Ala Gln Ala Tyr
    130                 135                 140

Leu Lys Val Asn Ala Asn Ser His Ala Arg Arg Met Gln Ala Phe Glu
145                 150                 155                 160

Ala Ser Pro Met Trp Leu Gln Asp Thr Lys Pro Arg Lys Asn Ala Ser
                165                 170                 175

Ser Val Val Pro Ser Ser Val His Asn Ser Ala Asn Gln Arg Met His
            180                 185                 190

Ser Thr Ser Ser Pro Gln Ala Val Ala Lys Ile Pro Lys Gln Ser Pro
        195                 200                 205

Gln Ser Ala Lys Ser Lys Ser Pro Val Lys Ser Thr Glu Arg Thr Ala
    210                 215                 220

Lys Leu Thr Leu Tyr Ser Lys His His Ser Ala Pro Leu Tyr Ser Ser
225                 230                 235                 240

Tyr Leu His Lys Glu His Gln Leu Pro Glu Ala
                245                 250


<210> 8
<211> 753
<212> DNA
<213> Mus musculus

<220>
<221> CDS
<222> (1)..(753)

<400> 8
atg ggg atc ttt ttg gcg tct gtt gga ttt atg ttc ttt tcc gtg tta     48
Met Gly Ile Phe Leu Ala Ser Val Gly Phe Met Phe Phe Ser Val Leu
1               5                   10                  15

tat gta caa caa ggg ctt tct tct caa gca aaa ttt acc gag ttg ccg     96
Tyr Val Gln Gln Gly Leu Ser Ser Gln Ala Lys Phe Thr Glu Leu Pro
            20                  25                  30

aga aat gtg act gct acc gaa ggg caa aat gtg gag atg tcc tgt gct     144
Arg Asn Val Thr Ala Thr Glu Gly Gln Asn Val Glu Met Ser Cys Ala
        35                  40                  45

ttc caa agc ggc tct gct tca gtg tac ctg gag atc cag tgg tgg ttc     192
Phe Gln Ser Gly Ser Ala Ser Val Tyr Leu Glu Ile Gln Trp Trp Phe
    50                  55                  60

ctt cgg ggg cca gag gac ctg gag caa ggc acg gag gct gca ggc tcg     240
Leu Arg Gly Pro Glu Asp Leu Glu Gln Gly Thr Glu Ala Ala Gly Ser
65                  70                  75                  80

cag gtg gag ctc tta ccc gac aga gac ccg gac aac gat ggg acc aag     288
Gln Val Glu Leu Leu Pro Asp Arg Asp Pro Asp Asn Asp Gly Thr Lys
                85                  90                  95

att agt aca gtg aaa gtc caa ggc aat gat atc tcc cac aag ctt cag     336
Ile Ser Thr Val Lys Val Gln Gly Asn Asp Ile Ser His Lys Leu Gln
            100                 105                 110

ata tcc aaa gtg aga aaa aag gat gaa ggt tta tac gag tgc agg gtg     384
Ile Ser Lys Val Arg Lys Lys Asp Glu Gly Leu Tyr Glu Cys Arg Val
        115                 120                 125

act gac gct aac tac ggg gag ctt cag gaa cac aag gcc cag gcc tat     432
Thr Asp Ala Asn Tyr Gly Glu Leu Gln Glu His Lys Ala Gln Ala Tyr
    130                 135                 140

ctg aaa gtc aat gcc aac agc cat gct cgg agg atg cag gcc ttt gaa     480
Leu Lys Val Asn Ala Asn Ser His Ala Arg Arg Met Gln Ala Phe Glu
145                 150                 155                 160

gcc tca cct atg tgg ctg caa gac acg aag cct cga aag aac gca tca     528
Ala Ser Pro Met Trp Leu Gln Asp Thr Lys Pro Arg Lys Asn Ala Ser
                165                 170                 175

tcg gtg gtt ccc agc agc gtc cac aac tct gcc aac caa cga atg cac     576
Ser Val Val Pro Ser Ser Val His Asn Ser Ala Asn Gln Arg Met His
            180                 185                 190

tcc acc tcc agc cct caa gcg gta gcc aaa atc ccc aag caa agt cca     624
Ser Thr Ser Ser Pro Gln Ala Val Ala Lys Ile Pro Lys Gln Ser Pro
        195                 200                 205

caa tca gca aag agc aaa tcg cct gta aaa tct acg gag cgg aca gca     672
Gln Ser Ala Lys Ser Lys Ser Pro Val Lys Ser Thr Glu Arg Thr Ala
    210                 215                 220

aag ttg acc cta tac tcc aag cac cat tct gca ccc ctg tac tct agt     720
Lys Leu Thr Leu Tyr Ser Lys His His Ser Ala Pro Leu Tyr Ser Ser
225                 230                 235                 240

tat cta cac aag gag cat cag ctt ccg gaa gca                         753
Tyr Leu His Lys Glu His Gln Leu Pro Glu Ala
                245                 250

<210> 9
<211> 681
<212> DNA
<213> Mus musculus

<220>
<221> CDS
<222> (1)..(681)

<400> 9
caa gca aaa ttt acc gag ttg ccg aga aat gtg act gct acc gaa ggg     48
Gln Ala Lys Phe Thr Glu Leu Pro Arg Asn Val Thr Ala Thr Glu Gly
  1               5                  10                  15

caa aat gtg gag atg tcc tgt gct ttc caa agc ggc tct gct tca gtg     96
Gln Asn Val Glu Met Ser Cys Ala Phe Gln Ser Gly Ser Ala Ser Val
             20                  25                  30

tac ctg gag atc cag tgg tgg ttc ctt cgg ggg cca gag gac ctg gag     144
Tyr Leu Glu Ile Gln Trp Trp Phe Leu Arg Gly Pro Glu Asp Leu Glu
         35                  40                  45

caa ggc acg gag gct gca ggc tcg cag gtg gag ctc tta ccc gac aga     192
Gln Gly Thr Glu Ala Ala Gly Ser Gln Val Glu Leu Leu Pro Asp Arg
     50                  55                  60

gac ccg gac aac gat ggg acc aag att agt aca gtg aaa gtc caa ggc     240
Asp Pro Asp Asn Asp Gly Thr Lys Ile Ser Thr Val Lys Val Gln Gly
 65                  70                  75                  80

aat gat atc tcc cac aag ctt cag ata tcc aaa gtg aga aaa aag gat     288
Asn Asp Ile Ser His Lys Leu Gln Ile Ser Lys Val Arg Lys Lys Asp
                 85                  90                  95

gaa ggt tta tac gag tgc agg gtg act gac gct aac tac ggg gag ctt     336
Glu Gly Leu Tyr Glu Cys Arg Val Thr Asp Ala Asn Tyr Gly Glu Leu
            100                 105                 110

cag gaa cac aag gcc cag gcc tat ctg aaa gtc aat gcc aac agc cat     384
Gln Glu His Lys Ala Gln Ala Tyr Leu Lys Val Asn Ala Asn Ser His
        115                 120                 125

gct cgg agg atg cag gcc ttt gaa gcc tca cct atg tgg ctg caa gac     432
Ala Arg Arg Met Gln Ala Phe Glu Ala Ser Pro Met Trp Leu Gln Asp
    130                 135                 140

acg aag cct cga aag aac gca tca tcg gtg gtt ccc agc agc gtc cac     480
Thr Lys Pro Arg Lys Asn Ala Ser Ser Val Val Pro Ser Ser Val His
145                 150                 155                 160

aac tct gcc aac caa cga atg cac tcc acc tcc agc cct caa gcg gta     528
Asn Ser Ala Asn Gln Arg Met His Ser Thr Ser Ser Pro Gln Ala Val
                165                 170                 175

gcc aaa atc ccc aag caa agt cca caa tca gca aag agc aaa tcg cct     576
Ala Lys Ile Pro Lys Gln Ser Pro Gln Ser Ala Lys Ser Lys Ser Pro
            180                 185                 190

gta aaa tct acg gag cgg aca gca aag ttg acc cta tac tcc aag cac     624
Val Lys Ser Thr Glu Arg Thr Ala Lys Leu Thr Leu Tyr Ser Lys His
        195                 200                 205

cat tct gca ccc ctg tac tct agt tat cta cac aag gag cat cag ctt     672
His Ser Ala Pro Leu Tyr Ser Ser Tyr Leu His Lys Glu His Gln Leu
    210                 215                 220

ccg gaa gca                                                         681
Pro Glu Ala
225

<210> 10
<211> 227
<212> PRT
<213> Mus musculus

<400> 10
Gln Ala Lys Phe Thr Glu Leu Pro Arg Asn Val Thr Ala Thr Glu Gly
  1               5                  10                  15

Gln Asn Val Glu Met Ser Cys Ala Phe Gln Ser Gly Ser Ala Ser Val
             20                  25                  30

Tyr Leu Glu Ile Gln Trp Trp Phe Leu Arg Gly Pro Glu Asp Leu Glu
         35                  40                  45

Gln Gly Thr Glu Ala Ala Gly Ser Gln Val Glu Leu Leu Pro Asp Arg
     50                  55                  60

Asp Pro Asp Asn Asp Gly Thr Lys Ile Ser Thr Val Lys Val Gln Gly
 65                  70                  75                  80

Asn Asp Ile Ser His Lys Leu Gln Ile Ser Lys Val Arg Lys Lys Asp
                 85                  90                  95

Glu Gly Leu Tyr Glu Cys Arg Val Thr Asp Ala Asn Tyr Gly Glu Leu
            100                 105                 110

Gln Glu His Lys Ala Gln Ala Tyr Leu Lys Val Asn Ala Asn Ser His
        115                 120                 125

Ala Arg Arg Met Gln Ala Phe Glu Ala Ser Pro Met Trp Leu Gln Asp
    130                 135                 140

Thr Lys Pro Arg Lys Asn Ala Ser Ser Val Val Pro Ser Ser Val His
145                 150                 155                 160

Asn Ser Ala Asn Gln Arg Met His Ser Thr Ser Ser Pro Gln Ala Val
                165                 170                 175

Ala Lys Ile Pro Lys Gln Ser Pro Gln Ser Ala Lys Ser Lys Ser Pro
            180                 185                 190

Val Lys Ser Thr Glu Arg Thr Ala Lys Leu Thr Leu Tyr Ser Lys His
        195                 200                 205

His Ser Ala Pro Leu Tyr Ser Ser Tyr Leu His Lys Glu His Gln Leu
    210                 215                 220

Pro Glu Ala
225

<210> 11
<211> 89
<212> PRT
<213> Homo sapiens

<400> 11
Gly Gln Asn Val Glu Met Ser Cys Ala Phe Gln Ser Gly Ser Ala Ser
  1               5                  10                  15

Val Tyr Leu Glu Ile Gln Trp Trp Phe Leu Arg Gly Pro Glu Asp Leu
             20                  25                  30

Asp Pro Gly Ala Glu Gly Ala Gly Ala Gln Val Glu Leu Leu Pro Asp
         35                  40                  45

Arg Asp Pro Asp Ser Asp Gly Thr Lys Ile Ser Thr Val Lys Val Gln
     50                  55                  60

Gly Asn Asp Ile Ser His Lys Leu Gln Ile Ser Lys Val Arg Lys Lys
 65                  70                  75                  80

Asp Glu Gly Leu Tyr Glu Cys Arg Val
                 85

<210> 12
<211> 89
<212> PRT
<213> Mus musculus

<400> 12
Gly Gln Asn Val Glu Met Ser Cys Ala Phe Gln Ser Gly Ser Ala Ser
  1               5                  10                  15

Val Tyr Leu Glu Ile Gln Trp Trp Phe Leu Arg Gly Pro Glu Asp Leu
             20                  25                  30

Glu Gln Gly Thr Glu Ala Ala Gly Ser Gln Val Glu Leu Leu Pro Asp
         35                  40                  45

Arg Asp Pro Asp Asn Asp Gly Thr Lys Ile Ser Thr Val Lys Val Gln
     50                  55                  60

Gly Asn Asp Ile Ser His Lys Leu Gln Ile Ser Lys Val Arg Lys Lys
65                  70                  75                  80

Asp Glu Gly Leu Tyr Glu Cys Arg Val
                85

<210> 13
<211> 47
<212> PRT
<213> Artificial Sequence

<220>
<223> Description of Artificial Sequence: Hidden Markov
      Model

<400> 13
Gly Gln Ser Val Thr Leu Thr Cys Met Val Ser Phe His Pro Pro Asp
  1               5                  10                  15

Tyr Thr Ile Trp Trp Tyr Arg Asn Gly Gln Pro Ile Thr Leu Thr Ile
             20                  25                  30

Asn Ser Trp Gln Tyr Glu Asp Ser Glu Thr Tyr Trp Cys Met Val
         35                  40                  45

<210> 14
<211> 2915
<212> DNA
<213> Homo sapiens

<220>
<221> CDS
<222> (404)..(1294)

<400> 14
gaattcccgg gtcgacccac gcgtccgccc acgcgtccgc ccacgcgtcc ggctgagcga 60

agcgcggcgg cggcggcggc gcctagggga gggaggggcg gcggggccga gcccacctag 120

cggagcgcgc cggccgccgg tggccgccgc cagcatgccc cggcccgcgg gccgctccgc 180

cgccagccac ccccgcggcc ctcggcggcc tgcgctcggc ccgggggcgc gggaaccgca 240

gccggagccg gaggcgggag cagcgagccg gagccccggg cgctcgaatg caggatgctc 300

gtggtcccca gcatccttga gccaccagga gtgagggctg ctgctccctg agacctggct 360

ccaaggagga tgccacagcc gcctgccagc tccggtctgc acc atg agt gat gag   415
                                                Met Ser Asp Glu
                                                  1

cgg cgg ctg cct ggc agt gca gtg ggc tgg ctg gta tgt ggg ggc ctc   463
Arg Arg Leu Pro Gly Ser Ala Val Gly Trp Leu Val Cys Gly Gly Leu
  5                  10                  15                  20

tcc ctg ctg gcc aat gcc tgg ggc atc ctc agc gtt ggc gcc aag cag 511
Ser Leu Leu Ala Asn Ala Trp Gly Ile Leu Ser Val Gly Ala Lys Gln
                25                  30                  35

aag aag tgg aag ccc ttg gag ttc ctg ctg tgt acg ctc gcg gcc acc 559
Lys Lys Trp Lys Pro Leu Glu Phe Leu Leu Cys Thr Leu Ala Ala Thr
            40                  45                  50

cac atg cta aat gtg gcc gtg ccc atc gcc acc tac tcc gtg gtg cag 607
His Met Leu Asn Val Ala Val Pro Ile Ala Thr Tyr Ser Val Val Gln
        55                  60                  65

ctg cgg cgg cag cgc ccc gac ttc gag tgg aat gag ggt ctc tgc aag 655
Leu Arg Arg Gln Arg Pro Asp Phe Glu Trp Asn Glu Gly Leu Cys Lys
    70                  75                  80

gtc ttc gtg tcc acc ttc tac acc ctc acc ctg gcc acc tgt ttc tct 703
Val Phe Val Ser Thr Phe Tyr Thr Leu Thr Leu Ala Thr Cys Phe Ser
85                  90                  95                  100

gtc acc tcc ctc tcc tac cac cgc atg tgg atg gtc tgc tgg cct gtc 751
Val Thr Ser Leu Ser Tyr His Arg Met Trp Met Val Cys Trp Pro Val
                105                 110                 115

aac tac cgg ctg agc aat gcc aag aag cag gcg gtg cac aca gtc atg 799
Asn Tyr Arg Leu Ser Asn Ala Lys Lys Gln Ala Val His Thr Val Met
            120                 125                 130

ggt atc tgg atg gtg tcc ttc atc ctg tcg gcc ctg cct gcc gtt ggc 847
Gly Ile Trp Met Val Ser Phe Ile Leu Ser Ala Leu Pro Ala Val Gly
        135                 140                 145

tgg cac gac acc agc gag cgc ttc tac acc cat ggc tgc cgc ttc atc 895
Trp His Asp Thr Ser Glu Arg Phe Tyr Thr His Gly Cys Arg Phe Ile
    150                 155                 160

gtg gct gag atc ggc ctg ggc ttt ggc gtc tgc ttc ctg ctg ctg gtg 943
Val Ala Glu Ile Gly Leu Gly Phe Gly Val Cys Phe Leu Leu Leu Val
165                 170                 175                 180

ggc ggc agc gtg gcc atg ggc gtg atc tgc aca gcc atc gcc ctc ttc 991
Gly Gly Ser Val Ala Met Gly Val Ile Cys Thr Ala Ile Ala Leu Phe
                185                 190                 195

cag acg ctg gcc gtg cag gtg ggg cgc cag gcc gac cac cgc gcc ttc 1039
Gln Thr Leu Ala Val Gln Val Gly Arg Gln Ala Asp His Arg Ala Phe
            200                 205                 210

acc gtg ccc acc atc gtg gtg gag gac gcg cag ggc aag cgg cgc tcc 1087
Thr Val Pro Thr Ile Val Val Glu Asp Ala Gln Gly Lys Arg Arg Ser
        215                 220                 225

tcc atc gat ggc tcg gag ccc gcc aaa acc tct ctg cag acc acg ggc 1135
Ser Ile Asp Gly Ser Glu Pro Ala Lys Thr Ser Leu Gln Thr Thr Gly
    230                 235                 240

ctc gtg acc acc ata gtc ttc atc tac gac tgc ctc atg ggc ttc cct 1183
Leu Val Thr Thr Ile Val Phe Ile Tyr Asp Cys Leu Met Gly Phe Pro
245                 250                 255                 260

gtg ctg gac tct acg ccc atc ccc gaa agg tct gca gtg aga cag gga 1231
Val Leu Asp Ser Thr Pro Ile Pro Glu Arg Ser Ala Val Arg Gln Gly
                265                 270                 275

gag gac tgg ggc aaa gac cag cct gag ggg ttt cat cca agc agc agg 1279
Glu Asp Trp Gly Lys Asp Gln Pro Glu Gly Phe His Pro Ser Ser Arg
            280                 285                 290

caa gac tgc ctt ccc tgagccattg caggacatga ggacatgagc tccagaatgg 1334
Gln Asp Cys Leu Pro
        295

tgccaggccg agccctgtgc ccacaggtgg tgagcttcag cagcctgcgg gccgacgcct 1394

cagcgccctg gatggcactc tgcgtgctgt ggtgctccgt ggcccaggcc ctgcttccga 1454

cctgtcctgc ctcagtttcc ccatctgtga tgagcaggtg accacgttaa ctctcagggc 1514

tgtttgaagt ctcttggttg taggccctcg ccactgagtg gcccaggtgt gagaggtagt 1574

ctagagccct ctgcggcctt tgtggaggtc cgttctcagc atgtggcctg gtgtggctcc 1634

ctaggcctga ggcctcccac tctcagtgcc ccctgccccc ttgggaaccc acactccacc 1694

ccagctaagc acaacactgt ggaccaccac caatggcacc tgagccacct tgaccaccat 1754

tagtgtcccc accctcatta ctctgccgtc gttgcctgtc cccaccacat catctccctc 1814

catggctccc accatgacgc tggcaggtgc aagcggagag cacgagggca gcagggcctg 1874

gccttccgtg tccctgcgtc atccccagcc tcactcccca cccgtgcagg cctggccagg 1934

aagggatcct ggcaggggct tccaggttct cagctcaagg cctggtcccg ggcaggcgtc 1994

caacccctgg gagcaatgta tttctttgcc ttccatcctg ggcagacccc ttacaggcct 2054

gggcattgcc atgggccctg ggtcttccca ggctaaggag aaccaggaac agctataacc 2114

ttgagctagt gaaatagggt agatgagaag gctgtctcct ccagacccct acccctacac 2174

agtggcccca caatatgaag acctggggta attccaaggt gagcatagag cctgcctgtg 2234

cccagttcct tctggccctc aggtggccaa gcccatctct tcatccttca gatagggtcc 2294

cactcccaga agaagctgct ggggtggggg tgggaggctg cctaagcctg tctgtgcttc 2354

agaggcccct ccagtccctg gctgtggggt aactgggggt atgagctgtg gccacaggtg 2414

agcaaggcag ggaactgcaa tccagccctg gccgcgggag gggccatctc tggccaatgc 2474

tgctgtgcct tcaaggactg acaagttacg taggggcaga ggtcgccagc tagccagtgt 2534

ctcctccatc tggggggcgt ctgtccactt gtcaccttag gttttcactc atttgtcacc 2594

ttggggtttt gctctgtgtg tttcatatcc aacggcaata cttgcagggg gacagagtcc 2654

tctaaatact ccaatcctgc ggtttttaca aacataaagg gggagacccc aagtggagga 2714

ccctgggcct ggagctccct cccaaacttt gtccagcatc cagcctgttc cctgggctca 2774

ctggggaggg agttgtcttc atagcacacc cagagccagg gatccctttg tagttttttg 2834

acaacggagc atttctcttc tgtacaggac ccaataaaaa cttccttatg atttgcaaaa 2894

aaaaaaaaaa agggcggccg c 2915

<210> 15
<211> 297
<212> PRT
<213> Homo sapiens

<400> 15
Met Ser Asp Glu Arg Arg Leu Pro Gly Ser Ala Val Gly Trp Leu Val
1               5                   10                  15

Cys Gly Gly Leu Ser Leu Leu Ala Asn Ala Trp Gly Ile Leu Ser Val
            20                  25                  30

Gly Ala Lys Gln Lys Lys Trp Lys Pro Leu Glu Phe Leu Leu Cys Thr
        35                  40                  45

Leu Ala Ala Thr His Met Leu Asn Val Ala Val Pro Ile Ala Thr Tyr
    50                  55                  60

Ser Val Val Gln Leu Arg Arg Gln Arg Pro Asp Phe Glu Trp Asn Glu
65                  70                  75                  80

Gly Leu Cys Lys Val Phe Val Ser Thr Phe Tyr Thr Leu Thr Leu Ala
                85                  90                  95

Thr Cys Phe Ser Val Thr Ser Leu Ser Tyr His Arg Met Trp Met Val
            100                 105                 110

Cys Trp Pro Val Asn Tyr Arg Leu Ser Asn Ala Lys Lys Gln Ala Val
        115                 120                 125

His Thr Val Met Gly Ile Trp Met Val Ser Phe Ile Leu Ser Ala Leu
    130                 135                 140

Pro Ala Val Gly Trp His Asp Thr Ser Glu Arg Phe Tyr Thr His Gly
145                 150                 155                 160

Cys Arg Phe Ile Val Ala Glu Ile Gly Leu Gly Phe Gly Val Cys Phe
                165                 170                 175

Leu Leu Leu Val Gly Gly Ser Val Ala Met Gly Val Ile Cys Thr Ala
            180                 185                 190

Ile Ala Leu Phe Gln Thr Leu Ala Val Gln Val Gly Arg Gln Ala Asp
        195                 200                 205

His Arg Ala Phe Thr Val Pro Thr Ile Val Val Glu Asp Ala Gln Gly
    210                 215                 220

Lys Arg Arg Ser Ser Ile Asp Gly Ser Glu Pro Ala Lys Thr Ser Leu
225                 230                 235                 240

Gln Thr Thr Gly Leu Val Thr Thr Ile Val Phe Ile Tyr Asp Cys Leu
                245                 250                 255

Met Gly Phe Pro Val Leu Asp Ser Thr Pro Ile Pro Glu Arg Ser Ala
            260                 265                 270

Val Arg Gln Gly Glu Asp Trp Gly Lys Asp Gln Pro Glu Gly Phe His
        275                 280                 285

Pro Ser Ser Arg Gln Asp Cys Leu Pro
    290                 295


<210> 16
<211> 891
<212> DNA
<213> Homo sapiens

<220>
<221> CDS
<222> (1)..(891)

<400> 16
atg agt gat gag cgg cgg ctg cct ggc agt gca gtg ggc tgg ctg gta      48
Met Ser Asp Glu Arg Arg Leu Pro Gly Ser Ala Val Gly Trp Leu Val
1               5                   10                  15

tgt ggg ggc ctc tcc ctg ctg gcc aat gcc tgg ggc atc ctc agc gtt      96
Cys Gly Gly Leu Ser Leu Leu Ala Asn Ala Trp Gly Ile Leu Ser Val
            20                  25                  30

ggc gcc aag cag aag aag tgg aag ccc ttg gag ttc ctg ctg tgt acg     144
Gly Ala Lys Gln Lys Lys Trp Lys Pro Leu Glu Phe Leu Leu Cys Thr
        35                  40                  45

ctc gcg gcc acc cac atg cta aat gtg gcc gtg ccc atc gcc acc tac     192
Leu Ala Ala Thr His Met Leu Asn Val Ala Val Pro Ile Ala Thr Tyr
    50                  55                  60

tcc gtg gtg cag ctg cgg cgg cag cgc ccc gac ttc gag tgg aat gag     240
Ser Val Val Gln Leu Arg Arg Gln Arg Pro Asp Phe Glu Trp Asn Glu
65                  70                  75                  80

ggt ctc tgc aag gtc ttc gtg tcc acc ttc tac acc ctc acc ctg gcc     288
Gly Leu Cys Lys Val Phe Val Ser Thr Phe Tyr Thr Leu Thr Leu Ala
                85                  90                  95

acc tgt ttc tct gtc acc tcc ctc tcc tac cac cgc atg tgg atg gtc     336
Thr Cys Phe Ser Val Thr Ser Leu Ser Tyr His Arg Met Trp Met Val
            100                 105                 110

tgc tgg cct gtc aac tac cgg ctg agc aat gcc aag aag cag gcg gtg     384
Cys Trp Pro Val Asn Tyr Arg Leu Ser Asn Ala Lys Lys Gln Ala Val
        115                 120                 125

cac aca gtc atg ggt atc tgg atg gtg tcc ttc atc ctg tcg gcc ctg     432
His Thr Val Met Gly Ile Trp Met Val Ser Phe Ile Leu Ser Ala Leu
    130                 135                 140

cct gcc gtt ggc tgg cac gac acc agc gag cgc ttc tac acc cat ggc     480
Pro Ala Val Gly Trp His Asp Thr Ser Glu Arg Phe Tyr Thr His Gly
145                 150                 155                 160

tgc cgc ttc atc gtg gct gag atc ggc ctg ggc ttt ggc gtc tgc ttc     528
Cys Arg Phe Ile Val Ala Glu Ile Gly Leu Gly Phe Gly Val Cys Phe
                165                 170                 175

ctg ctg ctg gtg ggc ggc agc gtg gcc atg ggc gtg atc tgc aca gcc     576
Leu Leu Leu Val Gly Gly Ser Val Ala Met Gly Val Ile Cys Thr Ala
            180                 185                 190

atc gcc ctc ttc cag acg ctg gcc gtg cag gtg ggg cgc cag gcc gac     624
Ile Ala Leu Phe Gln Thr Leu Ala Val Gln Val Gly Arg Gln Ala Asp
        195                 200                 205

cac cgc gcc ttc acc gtg ccc acc atc gtg gtg gag gac gcg cag ggc     672
His Arg Ala Phe Thr Val Pro Thr Ile Val Val Glu Asp Ala Gln Gly
    210                 215                 220

aag cgg cgc tcc tcc atc gat ggc tcg gag ccc gcc aaa acc tct ctg     720
Lys Arg Arg Ser Ser Ile Asp Gly Ser Glu Pro Ala Lys Thr Ser Leu
225                 230                 235                 240

cag acc acg ggc ctc gtg acc acc ata gtc ttc atc tac gac tgc ctc     768
Gln Thr Thr Gly Leu Val Thr Thr Ile Val Phe Ile Tyr Asp Cys Leu
                245                 250                 255

atg ggc ttc cct gtg ctg gac tct acg ccc atc ccc gaa agg tct gca     816
Met Gly Phe Pro Val Leu Asp Ser Thr Pro Ile Pro Glu Arg Ser Ala
            260                 265                 270

gtg aga cag gga gag gac tgg ggc aaa gac cag cct gag ggg ttt cat     864
Val Arg Gln Gly Glu Asp Trp Gly Lys Asp Gln Pro Glu Gly Phe His
        275                 280                 285

cca agc agc agg caa gac tgc ctt ccc                                 891
Pro Ser Ser Arg Gln Asp Cys Leu Pro
    290                 295


<210> 17
<211> 4166
<212> DNA
<213> Homo sapiens

<220>
<221> CDS
<222> (334)..(2160)

<400> 17
gaattcccgg gtcgacccac gcgtccggcg gcggggccga gcccacctag cggagcgcgc 60

cggccgccgg tggccgccgc cagcatgccc cggcccgcgg gccgctccgc cgccagccac 120

ccccgcggcc ctcggcggcc tgcgctcggc ccgggggcgc gggaaccgca gccggagccg 180

gaggcgggag cagcgagccg gagccccggg cgctcgaatg caggatgctc gtggtcccca 240

gcatccttga gccaccagga gtgagggctg ctgctccctg agacctggct ccaaggagga 300

tgccacagcc gcctgccagc tccggtctgc acc atg agt gat gag cgg cgg ctg 354
Met Ser Asp Glu Arg Arg Leu
1               5

cct ggc agt gca gtg ggc tgg ctg gta tgt ggg ggc ctc tcc ctg ctg 402
Pro Gly Ser Ala Val Gly Trp Leu Val Cys Gly Gly Leu Ser Leu Leu
        10                  15                  20

gcc aat gcc tgg ggc atc ctc agc gtt ggc gcc aag cag aag aag tgg 450
Ala Asn Ala Trp Gly Ile Leu Ser Val Gly Ala Lys Gln Lys Lys Trp
    25                  30                  35

aag ccc ttg gag ttc ctg ctg tgt acg ctc gcg gcc acc cac atg cta 498
Lys Pro Leu Glu Phe Leu Leu Cys Thr Leu Ala Ala Thr His Met Leu
40                  45                  50                  55

aat gtg gcc gtg ccc atc gcc acc tac tcc gtg gtg cag ctg cgg cgg 546
Asn Val Ala Val Pro Ile Ala Thr Tyr Ser Val Val Gln Leu Arg Arg
                60                  65                  70

cag cgc ccc gac ttc gag tgg aat gag ggt ctc tgc aag gtc ttc gtg 594
Gln Arg Pro Asp Phe Glu Trp Asn Glu Gly Leu Cys Lys Val Phe Val
            75                  80                  85

tcc acc ttc tac acc ctc acc ctg gcc acc tgt ttc tct gtc acc tcc 642
Ser Thr Phe Tyr Thr Leu Thr Leu Ala Thr Cys Phe Ser Val Thr Ser
        90                  95                  100

ctc tcc tac cac cgc atg tgg atg gtc tgc tgg cct gtc aac tac cgg 690
Leu Ser Tyr His Arg Met Trp Met Val Cys Trp Pro Val Asn Tyr Arg
    105                 110                 115

ctg agc aat gcc aag aag cag gcg gtg cac aca gtc atg ggt atc tgg 738
Leu Ser Asn Ala Lys Lys Gln Ala Val His Thr Val Met Gly Ile Trp
120                 125                 130                 135

atg gtg tcc ttc atc ctg tcg gcc ctg cct gcc gtt ggc tgg cac gac 786
Met Val Ser Phe Ile Leu Ser Ala Leu Pro Ala Val Gly Trp His Asp
                140                 145                 150

acc agc gag cgc ttc tac acc cat ggc tgc cgc ttc atc gtg gct gag 834
Thr Ser Glu Arg Phe Tyr Thr His Gly Cys Arg Phe Ile Val Ala Glu
            155                 160                 165

atc ggc ctg ggc ttt ggc gtc tgc ttc ctg ctg ctg gtg ggc ggc agc 882
Ile Gly Leu Gly Phe Gly Val Cys Phe Leu Leu Leu Val Gly Gly Ser
        170                 175                 180

gtg gcc atg ggc gtg atc tgc aca gcc atc gcc ctc ttc cag acg ctg 930
Val Ala Met Gly Val Ile Cys Thr Ala Ile Ala Leu Phe Gln Thr Leu
    185                 190                 195

gcc gtg cag gtg ggg cgc cag gcc gac cac cgc gcc ttc acc gtg ccc 978
Ala Val Gln Val Gly Arg Gln Ala Asp His Arg Ala Phe Thr Val Pro
200                 205                 210                 215

acc atc gtg gtg gag gac gcg cag ggc aag cgg cgc tcc tcc atc gat 1026
Thr Ile Val Val Glu Asp Ala Gln Gly Lys Arg Arg Ser Ser Ile Asp
                220                 225                 230

ggc tcg gag ccc gcc aaa acc tct ctg cag acc acg ggc ctc gtg acc 1074
Gly Ser Glu Pro Ala Lys Thr Ser Leu Gln Thr Thr Gly Leu Val Thr
            235                 240                 245

acc ata gtc ttc atc tac gac tgc ctc atg ggc ttc cct gtg ctg gtg 1122
Thr Ile Val Phe Ile Tyr Asp Cys Leu Met Gly Phe Pro Val Leu Val
        250                 255                 260

gtg agc ttc agc agc ctg cgg gcc gac gcc tca gcg ccc tgg atg gca 1170
Val Ser Phe Ser Ser Leu Arg Ala Asp Ala Ser Ala Pro Trp Met Ala
    265                 270                 275

ctc tgc gtg ctg tgg tgc tcc gtg gcc cag gcc ctg ctg ctg cct gtg 1218
Leu Cys Val Leu Trp Cys Ser Val Ala Gln Ala Leu Leu Leu Pro Val
280                 285                 290                 295

ttc ctc tgg gcc tgc gac cgc tac cgg gct gac ctc aaa gct gtc cgg 1266
Phe Leu Trp Ala Cys Asp Arg Tyr Arg Ala Asp Leu Lys Ala Val Arg
                300                 305                 310

gag aag tgc atg gcc ctc atg gcc aac gac gag gag tca gac gat gag 1314
Glu Lys Cys Met Ala Leu Met Ala Asn Asp Glu Glu Ser Asp Asp Glu
            315                 320                 325

acc agc ctg gaa ggt ggc atc tcc ccg gac ctg gtg ttg gag cgc tcc 1362
Thr Ser Leu Glu Gly Gly Ile Ser Pro Asp Leu Val Leu Glu Arg Ser
        330                 335                 340

ctg gac tat ggc tat gga ggt gat ttt gtg gcc cta gat agg atg gcc 1410
Leu Asp Tyr Gly Tyr Gly Gly Asp Phe Val Ala Leu Asp Arg Met Ala
    345                 350                 355

aag tat gag atc tcc gcc ctg gag ggg ggc ctg ccc cag ctc tac cca 1458
Lys Tyr Glu Ile Ser Ala Leu Glu Gly Gly Leu Pro Gln Leu Tyr Pro
360                 365                 370                 375

ctg cgg ccc ttg cag gag gac aag atg caa tac ctg cag gtc ccg ccc 1506
Leu Arg Pro Leu Gln Glu Asp Lys Met Gln Tyr Leu Gln Val Pro Pro
                380                 385                 390

acg cgg cgc ttc tcc cac gac gat gcg gac gtg tgg gcc gcc gtc ccg 1554
Thr Arg Arg Phe Ser His Asp Asp Ala Asp Val Trp Ala Ala Val Pro
            395                 400                 405

ctg ccc gcc ttc ctg ccg cgc tgg ggc tcc ggc gag gac ctg gcc gcc 1602
Leu Pro Ala Phe Leu Pro Arg Trp Gly Ser Gly Glu Asp Leu Ala Ala
        410                 415                 420

ctg gcg cac ctg gtg ctg cct gcc ggg ccc gag cgg cgc cgc gcc agc 1650
Leu Ala His Leu Val Leu Pro Ala Gly Pro Glu Arg Arg Arg Ala Ser
    425                 430                 435

ctc ctg gcc ttc gcg gag gac gca cca ccg tcc cgc gcg cgc cgc cgc 1698
Leu Leu Ala Phe Ala Glu Asp Ala Pro Pro Ser Arg Ala Arg Arg Arg
440                 445                 450                 455

tcg gcc gag agc ctg ctg tcg ctg cgg acc tcg gcc ctg gat agc ggc 1746
Ser Ala Glu Ser Leu Leu Ser Leu Arg Thr Ser Ala Leu Asp Ser Gly
                460                 465                 470

ccg cgg gga gcc cgc gac tcg ccc ccc ggc agc ccg cgc cgc cgc ccc 1794
Pro Arg Gly Ala Arg Asp Ser Pro Pro Gly Ser Pro Arg Arg Arg Pro
            475                 480                 485

ggg ccc ggc ccc cgc tcc gcc tcg gcc tcg ctg ctg ccc gac gcc ttc 1842
Gly Pro Gly Pro Arg Ser Ala Ser Ala Ser Leu Leu Pro Asp Ala Phe
        490                 495                 500

gcc ctg acc gcc ttc gag tgc gag cca cag gcc ctg cgc cgc ccg ccc 1890
Ala Leu Thr Ala Phe Glu Cys Glu Pro Gln Ala Leu Arg Arg Pro Pro
    505                 510                 515

ggg ccc ttc ccc gct gcg ccc gcc gcc ccc gac ggc gca gat ccc gga 1938
Gly Pro Phe Pro Ala Ala Pro Ala Ala Pro Asp Gly Ala Asp Pro Gly
520                 525                 530                 535

gag gcc ccg acg ccc cca agc agc gcc cag cgg agc cca ggg cca cgc 1986
Glu Ala Pro Thr Pro Pro Ser Ser Ala Gln Arg Ser Pro Gly Pro Arg
                540                 545                 550

ccc tct gcg cac tcg cac gcc ggc tct ctg cgc ccc ggc ctg agc gcg 2034
Pro Ser Ala His Ser His Ala Gly Ser Leu Arg Pro Gly Leu Ser Ala
            555                 560                 565

tcg tgg ggc gag ccc ggg ggg ctg cgc gcg gcg ggc ggc ggc ggc agc 2082
Ser Trp Gly Glu Pro Gly Gly Leu Arg Ala Ala Gly Gly Gly Gly Ser
        570                 575                 580

acc agc agc ttc ctg agt tcc ccc tcc gag tcc tcg ggc tac gcc acg 2130
Thr Ser Ser Phe Leu Ser Ser Pro Ser Glu Ser Ser Gly Tyr Ala Thr
    585                 590                 595

ctg cac tcg gac tcg ctg ggc tcc gcg tcc taggaccgcc ggcgcctccc 2180
Leu His Ser Asp Ser Leu Gly Ser Ala Ser
600                 605

cacggacgcc aggcaggcca ggccgctctc cggggccgca gcaccaaaga cgcccgcctc 2240

ccccccgcgc gcagacatgc gccacccctc ccaggggtga gggggcgttg gcctcagcgt 2300

ttgtcttccg gctcctccca gctggccttg tcccaggggc gacggctgcc ccggacgact 2360

gcgctgggca ccgcatgtcc cgggccgagt gaggtcgggc ctggggagct gagtgacatc 2420
ccaagcttgg gcttgggtag tgagtgacat gtgcacacgt ccagctgcgc catcaccagc 2480
cctgcgcaac aggacgtcgg gagcagggaa cctgagacag ggccactgcg ggatcggaca 2540
aagccccgct ttggagaggc tgagctggag ccattggcct ccccaggggc tttccaccca 2600
cactgcacca taaccgccac acccttcggg gggggggagg gtacagaggg tctctaagca 2660
caggggtgtt cagagcccga acaagctttg atcaggtttc cctgcttccg acctgtcctg 2720
cctcagtttc cccatctgtg atgagcaggt gaccacgtta actctcaggg ctgtttgaag 2780
tctcttggtt gtaggccctc gccactgagt ggcccaggtg tgagaggtag tctagagccc 2840
tctgcggcct ttgtggaggt ccgttctcag catgtggcct ggtgtggctc cctaggcctg 2900
aggcctccca ctctcagtgc cccctgcccc cttgggaacc cacactccac cccagctaag 2960
cacaacactg tggaccacca ccaatggcac ctgagccacc ttgaccacca ttagtgtccc 3020
caccctcatt actctgccgt cgttgcctgt ccccaccaca tcatctccct ccatggctcc 3080
caccatgacg ctggcaggtg caagcggaga gcacgagggc agcagggcct ggccttccgt 3140
gtccctgcgt catccccagc ctcactcccc acccgtgcag gcctggccag gaagggatcc 3200
tggcaggggc ttccaggttc tcagctcaag gcctggtccc gggcaggcgt ccaacccctg 3260
ggagcaatgt atttctttgc cttccatcct gggcagaccc cttacaggcc tgggcattgc 3320
catgggccct gggtcttccc aggctaagga gaaccaggaa cagctataac cttgagctag 3380
tgaaataggg tagatgagaa ggctgtctcc tccagacccc tacccctaca cagtggcccc 3440
acaatatgaa gacctggggt aattccaagg tgagcataga gcctgcctgt gcccagttcc 3500
ttctggccct caggtggcca agcccatctc ttcatccttc agatagggtc ccactcccag 3560
aagaagctgc tggggtgggg gtgggaggct gcctaagcct gtctgtgctt cagaggcccc 3620
tccagtccct ggctgtgggg taactggggg tatgagctgt ggccacaggt gagcaaggca 3680
gggaactgca atccagccct ggccgcggga ggggccatct ctggccaatg ctgctgtgcc 3740
ttcaaggact gacaagttac gtaggggcag aggtcgccag ctagccagtg tctcctccat 3800
ctggggggcg tctgtccact tgtcacctta ggttttcact catttgtcac cttggggttt 3860
tgctctgtgt gtttcatatc caacggcaat acttgcaggg ggacagagtc ctctaaatac 3920
tccaatcctg cggtttttac aaacataaag ggggagaccc caagtggagg accctgggcc 3980
tggagctccc tcccaaactt tgtccagcat ccagcctgtt ccctgggctc actggggagg 4040
gagttgtctt catagcacac ccagagccag ggatcccttt gtagtttttt gacaacggag 4100

catttctctt ctgtacagga cccaataaaa acttccttat gaaaaaaaaa aaaaaagggc 4160

ggccgc 4166

<210> 18
<211> 609
<212> PRT
<213> Homo sapiens

<400> 18
Met Ser Asp Glu Arg Arg Leu Pro Gly Ser Ala Val Gly Trp Leu Val
1               5                   10                  15

Cys Gly Gly Leu Ser Leu Leu Ala Asn Ala Trp Gly Ile Leu Ser Val
            20                  25                  30

Gly Ala Lys Gln Lys Lys Trp Lys Pro Leu Glu Phe Leu Leu Cys Thr
        35                  40                  45

Leu Ala Ala Thr His Met Leu Asn Val Ala Val Pro Ile Ala Thr Tyr
    50                  55                  60

Ser Val Val Gln Leu Arg Arg Gln Arg Pro Asp Phe Glu Trp Asn Glu
65                  70                  75                  80

Gly Leu Cys Lys Val Phe Val Ser Thr Phe Tyr Thr Leu Thr Leu Ala
                85                  90                  95

Thr Cys Phe Ser Val Thr Ser Leu Ser Tyr His Arg Met Trp Met Val
            100                 105                 110

Cys Trp Pro Val Asn Tyr Arg Leu Ser Asn Ala Lys Lys Gln Ala Val
        115                 120                 125

His Thr Val Met Gly Ile Trp Met Val Ser Phe Ile Leu Ser Ala Leu
    130                 135                 140

Pro Ala Val Gly Trp His Asp Thr Ser Glu Arg Phe Tyr Thr His Gly
145                 150                 155                 160

Cys Arg Phe Ile Val Ala Glu Ile Gly Leu Gly Phe Gly Val Cys Phe
                165                 170                 175

Leu Leu Leu Val Gly Gly Ser Val Ala Met Gly Val Ile Cys Thr Ala
            180                 185                 190

Ile Ala Leu Phe Gln Thr Leu Ala Val Gln Val Gly Arg Gln Ala Asp
        195                 200                 205

His Arg Ala Phe Thr Val Pro Thr Ile Val Val Glu Asp Ala Gln Gly
    210                 215                 220

Lys Arg Arg Ser Ser Ile Asp Gly Ser Glu Pro Ala Lys Thr Ser Leu
225                 230                 235                 240

Gln Thr Thr Gly Leu Val Thr Thr Ile Val Phe Ile Tyr Asp Cys Leu
                245                 250                 255

Met Gly Phe Pro Val Leu Val Val Ser Phe Ser Ser Leu Arg Ala Asp
            260                 265                 270

Ala Ser Ala Pro Trp Met Ala Leu Cys Val Leu Trp Cys Ser Val Ala
        275                 280                 285

Gln Ala Leu Leu Leu Pro Val Phe Leu Trp Ala Cys Asp Arg Tyr Arg
    290                 295                 300

Ala Asp Leu Lys Ala Val Arg Glu Lys Cys Met Ala Leu Met Ala Asn
305                 310                 315                 320

Asp Glu Glu Ser Asp Asp Glu Thr Ser Leu Glu Gly Gly Ile Ser Pro
                325                 330                 335

Asp Leu Val Leu Glu Arg Ser Leu Asp Tyr Gly Tyr Gly Gly Asp Phe
            340                 345                 350

Val Ala Leu Asp Arg Met Ala Lys Tyr Glu Ile Ser Ala Leu Glu Gly
        355                 360                 365

Gly Leu Pro Gln Leu Tyr Pro Leu Arg Pro Leu Gln Glu Asp Lys Met
    370                 375                 380

Gln Tyr Leu Gln Val Pro Pro Thr Arg Arg Phe Ser His Asp Asp Ala
385                 390                 395                 400

Asp Val Trp Ala Ala Val Pro Leu Pro Ala Phe Leu Pro Arg Trp Gly
                405                 410                 415

Ser Gly Glu Asp Leu Ala Ala Leu Ala His Leu Val Leu Pro Ala Gly
            420                 425                 430

Pro Glu Arg Arg Arg Ala Ser Leu Leu Ala Phe Ala Glu Asp Ala Pro
        435                 440                 445

Pro Ser Arg Ala Arg Arg Arg Ser Ala Glu Ser Leu Leu Ser Leu Arg
    450                 455                 460

Thr Ser Ala Leu Asp Ser Gly Pro Arg Gly Ala Arg Asp Ser Pro Pro
465                 470                 475                 480

Gly Ser Pro Arg Arg Arg Pro Gly Pro Gly Pro Arg Ser Ala Ser Ala
                485                 490                 495

Ser Leu Leu Pro Asp Ala Phe Ala Leu Thr Ala Phe Glu Cys Glu Pro
            500                 505                 510

Gln Ala Leu Arg Arg Pro Pro Gly Pro Phe Pro Ala Ala Pro Ala Ala
        515                 520                 525

Pro Asp Gly Ala Asp Pro Gly Glu Ala Pro Thr Pro Pro Ser Ser Ala
    530                 535                 540

Gln Arg Ser Pro Gly Pro Arg Pro Ser Ala His Ser His Ala Gly Ser
545                 550                 555                 560

Leu Arg Pro Gly Leu Ser Ala Ser Trp Gly Glu Pro Gly Gly Leu Arg
                565                 570                 575

Ala Ala Gly Gly Gly Gly Ser Thr Ser Ser Phe Leu Ser Ser Pro Ser
            580                 585                 590

Glu Ser Ser Gly Tyr Ala Thr Leu His Ser Asp Ser Leu Gly Ser Ala
        595                 600                 605

Ser

<210> 19
<211> 1827
<212> DNA
<213> Homo sapiens

<220>
<221> CDS
<222> (1)..(1827)

<400> 19
atg agt gat gag cgg cgg ctg cct ggc agt gca gtg ggc tgg ctg gta      48
Met Ser Asp Glu Arg Arg Leu Pro Gly Ser Ala Val Gly Trp Leu Val
  1               5                  10                  15

tgt ggg ggc ctc tcc ctg ctg gcc aat gcc tgg ggc atc ctc agc gtt      96
Cys Gly Gly Leu Ser Leu Leu Ala Asn Ala Trp Gly Ile Leu Ser Val
             20                  25                  30

ggc gcc aag cag aag aag tgg aag ccc ttg gag ttc ctg ctg tgt acg     144
Gly Ala Lys Gln Lys Lys Trp Lys Pro Leu Glu Phe Leu Leu Cys Thr
         35                  40                  45

ctc gcg gcc acc cac atg cta aat gtg gcc gtg ccc atc gcc acc tac     192
Leu Ala Ala Thr His Met Leu Asn Val Ala Val Pro Ile Ala Thr Tyr
     50                  55                  60

tcc gtg gtg cag ctg cgg cgg cag cgc ccc gac ttc gag tgg aat gag     240
Ser Val Val Gln Leu Arg Arg Gln Arg Pro Asp Phe Glu Trp Asn Glu
 65                  70                  75                  80

ggt ctc tgc aag gtc ttc gtg tcc acc ttc tac acc ctc acc ctg gcc     288
Gly Leu Cys Lys Val Phe Val Ser Thr Phe Tyr Thr Leu Thr Leu Ala
                 85                  90                  95

acc tgt ttc tct gtc acc tcc ctc tcc tac cac cgc atg tgg atg gtc     336
Thr Cys Phe Ser Val Thr Ser Leu Ser Tyr His Arg Met Trp Met Val
            100                 105                 110

tgc tgg cct gtc aac tac cgg ctg agc aat gcc aag aag cag gcg gtg     384
Cys Trp Pro Val Asn Tyr Arg Leu Ser Asn Ala Lys Lys Gln Ala Val
        115                 120                 125

cac aca gtc atg ggt atc tgg atg gtg tcc ttc atc ctg tcg gcc ctg     432
His Thr Val Met Gly Ile Trp Met Val Ser Phe Ile Leu Ser Ala Leu
    130                 135                 140

cct gcc gtt ggc tgg cac gac acc agc gag cgc ttc tac acc cat ggc 480
Pro Ala Val Gly Trp His Asp Thr Ser Glu Arg Phe Tyr Thr His Gly
145                 150                 155                 160

tgc cgc ttc atc gtg gct gag atc ggc ctg ggc ttt ggc gtc tgc ttc 528
Cys Arg Phe Ile Val Ala Glu Ile Gly Leu Gly Phe Gly Val Cys Phe
                165                 170                 175

ctg ctg ctg gtg ggc ggc agc gtg gcc atg ggc gtg atc tgc aca gcc 576
Leu Leu Leu Val Gly Gly Ser Val Ala Met Gly Val Ile Cys Thr Ala
            180                 185                 190

atc gcc ctc ttc cag acg ctg gcc gtg cag gtg ggg cgc cag gcc gac 624
Ile Ala Leu Phe Gln Thr Leu Ala Val Gln Val Gly Arg Gln Ala Asp
        195                 200                 205

cac cgc gcc ttc acc gtg ccc acc atc gtg gtg gag gac gcg cag ggc 672
His Arg Ala Phe Thr Val Pro Thr Ile Val Val Glu Asp Ala Gln Gly
    210                 215                 220

aag cgg cgc tcc tcc atc gat ggc tcg gag ccc gcc aaa acc tct ctg 720
Lys Arg Arg Ser Ser Ile Asp Gly Ser Glu Pro Ala Lys Thr Ser Leu
225                 230                 235                 240

cag acc acg ggc ctc gtg acc acc ata gtc ttc atc tac gac tgc ctc 768
Gln Thr Thr Gly Leu Val Thr Thr Ile Val Phe Ile Tyr Asp Cys Leu
                245                 250                 255

atg ggc ttc cct gtg ctg gtg gtg agc ttc agc agc ctg cgg gcc gac 816
Met Gly Phe Pro Val Leu Val Val Ser Phe Ser Ser Leu Arg Ala Asp
            260                 265                 270

gcc tca gcg ccc tgg atg gca ctc tgc gtg ctg tgg tgc tcc gtg gcc 864
Ala Ser Ala Pro Trp Met Ala Leu Cys Val Leu Trp Cys Ser Val Ala
        275                 280                 285

cag gcc ctg ctg ctg cct gtg ttc ctc tgg gcc tgc gac cgc tac cgg 912
Gln Ala Leu Leu Leu Pro Val Phe Leu Trp Ala Cys Asp Arg Tyr Arg
    290                 295                 300

gct gac ctc aaa gct gtc cgg gag aag tgc atg gcc ctc atg gcc aac 960
Ala Asp Leu Lys Ala Val Arg Glu Lys Cys Met Ala Leu Met Ala Asn
305                 310                 315                 320

gac gag gag tca gac gat gag acc agc ctg gaa ggt ggc atc tcc ccg 1008
Asp Glu Glu Ser Asp Asp Glu Thr Ser Leu Glu Gly Gly Ile Ser Pro
                325                 330                 335

gac ctg gtg ttg gag cgc tcc ctg gac tat ggc tat gga ggt gat ttt 1056
Asp Leu Val Leu Glu Arg Ser Leu Asp Tyr Gly Tyr Gly Gly Asp Phe
            340                 345                 350

gtg gcc cta gat agg atg gcc aag tat gag atc tcc gcc ctg gag ggg 1104
Val Ala Leu Asp Arg Met Ala Lys Tyr Glu Ile Ser Ala Leu Glu Gly
        355                 360                 365

ggc ctg ccc cag ctc tac cca ctg cgg ccc ttg cag gag gac aag atg 1152
Gly Leu Pro Gln Leu Tyr Pro Leu Arg Pro Leu Gln Glu Asp Lys Met
    370                 375                 380

caa tac ctg cag gtc ccg ccc acg cgg cgc ttc tcc cac gac gat gcg 1200
Gln Tyr Leu Gln Val Pro Pro Thr Arg Arg Phe Ser His Asp Asp Ala
385                 390                 395                 400

gac gtg tgg gcc gcc gtc ccg ctg ccc gcc ttc ctg ccg cgc tgg ggc 1248
Asp Val Trp Ala Ala Val Pro Leu Pro Ala Phe Leu Pro Arg Trp Gly
                405                 410                 415

tcc ggc gag gac ctg gcc gcc ctg gcg cac ctg gtg ctg cct gcc ggg 1296
Ser Gly Glu Asp Leu Ala Ala Leu Ala His Leu Val Leu Pro Ala Gly
            420                 425                 430

ccc gag cgg cgc cgc gcc agc ctc ctg gcc ttc gcg gag gac gca cca 1344
Pro Glu Arg Arg Arg Ala Ser Leu Leu Ala Phe Ala Glu Asp Ala Pro
        435                 440                 445

ccg tcc cgc gcg cgc cgc cgc tcg gcc gag agc ctg ctg tcg ctg cgg 1392
Pro Ser Arg Ala Arg Arg Arg Ser Ala Glu Ser Leu Leu Ser Leu Arg
    450                 455                 460

acc tcg gcc ctg gat agc ggc ccg cgg gga gcc cgc gac tcg ccc ccc 1440
Thr Ser Ala Leu Asp Ser Gly Pro Arg Gly Ala Arg Asp Ser Pro Pro
465                 470                 475                 480

ggc agc ccg cgc cgc cgc ccc ggg ccc ggc ccc cgc tcc gcc tcg gcc 1488
Gly Ser Pro Arg Arg Arg Pro Gly Pro Gly Pro Arg Ser Ala Ser Ala
                485                 490                 495

tcg ctg ctg ccc gac gcc ttc gcc ctg acc gcc ttc gag tgc gag cca 1536
Ser Leu Leu Pro Asp Ala Phe Ala Leu Thr Ala Phe Glu Cys Glu Pro
            500                 505                 510

cag gcc ctg cgc cgc ccg ccc ggg ccc ttc ccc gct gcg ccc gcc gcc 1584
Gln Ala Leu Arg Arg Pro Pro Gly Pro Phe Pro Ala Ala Pro Ala Ala
        515                 520                 525

ccc gac ggc gca gat ccc gga gag gcc ccg acg ccc cca agc agc gcc 1632
Pro Asp Gly Ala Asp Pro Gly Glu Ala Pro Thr Pro Pro Ser Ser Ala
    530                 535                 540

cag cgg agc cca ggg cca cgc ccc tct gcg cac tcg cac gcc ggc tct 1680
Gln Arg Ser Pro Gly Pro Arg Pro Ser Ala His Ser His Ala Gly Ser
545                 550                 555                 560

ctg cgc ccc ggc ctg agc gcg tcg tgg ggc gag ccc ggg ggg ctg cgc 1728
Leu Arg Pro Gly Leu Ser Ala Ser Trp Gly Glu Pro Gly Gly Leu Arg
                565                 570                 575

gcg gcg ggc ggc ggc ggc agc acc agc agc ttc ctg agt tcc ccc tcc 1776
Ala Ala Gly Gly Gly Gly Ser Thr Ser Ser Phe Leu Ser Ser Pro Ser
            580                 585                 590

gag tcc tcg ggc tac gcc acg ctg cac tcg gac tcg ctg ggc tcc gcg    1824
Glu Ser Ser Gly Tyr Ala Thr Leu His Ser Asp Ser Leu Gly Ser Ala
        595                 600                 605

tcc                                                                1827
Ser

<210> 20
<211> 177
<212> PRT
<213> Homo sapiens

<220>
<223> Xaa's at postions 38, 55 and 56 may be any amino
      acid

<400> 20
Gly Asn Ile Leu Val Ile Trp Val Ile Cys Arg Tyr Arg Arg Met Arg
  1               5                  10                  15

Thr Pro Met Asn Tyr Phe Ile Val Asn Leu Ala Val Ala Asp Leu Leu
             20                  25                  30

Phe Ser Leu Phe Thr Xaa Met Pro Phe Trp Met Val Tyr Tyr Val Met
         35                  40                  45

Gln Gly Arg Trp Pro Phe Xaa Xaa Gly Asp Phe Met Cys Arg Ile Trp
     50                  55                  60

Met Tyr Phe Asp Tyr Met Asn Met Tyr Ala Ser Ile Phe Phe Leu Thr
 65                  70                  75                  80

Cys Ile Ser Ile Asp Arg Tyr Leu Trp Ala Ile Cys His Pro Met Arg
                 85                  90                  95

Tyr Met Arg Trp Met Thr Pro Arg His Arg Ala Trp Val Met Ile Ile
            100                 105                 110

Ile Ile Trp Val Met Ser Phe Leu Ile Ser Met Pro Pro Phe Leu Met
        115                 120                 125

Phe Arg Trp Ser Thr Tyr Arg Asp Glu Asn Glu Trp Asn Met Thr Trp
    130                 135                 140

Cys Met Ile Tyr Asp Trp Pro Glu Trp Met Trp Arg Trp Tyr Val Ile
145                 150                 155                 160

Leu Met Thr Ile Ile Met Gly Phe Tyr Ile Pro Met Ile Ile Met Leu
                165                 170                 175

Phe

<210> 21
<211> 107
<212> PRT

<213> Homo sapiens

<220>
<223> Xaa at postion 84 may be any amino
      acid

<400> 21
Ile Gln Glu Arg Met Asn Glu Leu Asn Asp Arg Trp Glu Arg Leu Lys
 1               5                  10                  15

Glu Leu Met Glu Gln Arg Arg Gln Met Leu Glu Asp Ser Met Arg Leu
            20                  25                  30

Gln Gln Phe Phe Arg Asp Met Asp Glu Glu Glu Ser Trp Ile Asn Glu
        35                  40                  45

Lys Glu Gln Ile Leu Asn Ser Asp Asp Tyr Gly Lys Asp Leu Thr Ser
    50                  55                  60

Val Gln Asn Leu Leu Lys Lys His Gln Ala Phe Glu Ala Asp Ile Ala
65                  70                  75                  80

Ala His Glu Xaa Asp Arg Ile Gln Ala Leu Asn Glu Phe Ala Gln Gln
                85                  90                  95

Leu Ile Gln Glu Asn His Tyr Ala Ser Glu Glu
            100                 105


<210> 22
<211> 168
<212> PRT
<213> Homo sapiens

<400> 22
Ala Asn Ala Trp Gly Ile Leu Ser Val Gly Ala Lys Gln Lys Lys Trp
 1               5                  10                  15

Lys Pro Leu Glu Phe Leu Leu Cys Thr Leu Ala Ala Thr His Met Leu
            20                  25                  30

Asn Val Ala Val Pro Ile Ala Thr Tyr Ser Val Val Gln Leu Arg Arg
        35                  40                  45

Gln Arg Pro Asp Phe Glu Trp Asn Glu Gly Leu Cys Lys Val Phe Val
    50                  55                  60

Ser Thr Phe Tyr Thr Leu Thr Leu Ala Thr Cys Phe Ser Val Thr Ser
65                  70                  75                  80

Leu Ser Tyr His Arg Met Trp Met Val Cys Trp Pro Val Asn Tyr Arg
                85                  90                  95

Leu Ser Asn Ala Lys Lys Gln Ala Val His Thr Val Met Gly Ile Trp
            100                 105                 110

Met Val Ser Phe Ile Leu Ser Ala Leu Pro Ala Val Gly Trp His Asp
        115                 120                 125

Thr Ser Glu Arg Phe Tyr Thr His Gly Cys Arg Phe Ile Val Ala Glu
    130                 135                 140

Ile Gly Leu Gly Phe Gly Val Cys Phe Leu Leu Leu Val Gly Gly Ser
145                 150                 155                 160

Val Ala Met Gly Val Ile Cys Thr
                165

<210> 23
<211> 107
<212> PRT
<213> Homo sapiens

<400> 23
Phe Ser Ser Leu Arg Ala Asp Ala Ser Ala Pro Trp Met Ala Leu Cys
  1               5                  10                  15

Val Leu Trp Cys Ser Val Ala Gln Ala Leu Leu Leu Pro Val Phe Leu
             20                  25                  30

Trp Ala Cys Asp Arg Tyr Arg Ala Asp Leu Lys Ala Val Arg Glu Lys
         35                  40                  45

Cys Met Ala Leu Met Ala Asn Asp Glu Glu Ser Asp Asp Glu Thr Ser
     50                  55                  60

Leu Glu Gly Gly Ile Ser Pro Asp Leu Val Leu Glu Arg Ser Leu Asp
 65                  70                  75                  80

Tyr Gly Tyr Gly Gly Asp Phe Val Ala Leu Asp Arg Met Ala Lys Tyr
                 85                  90                  95

Glu Ile Ser Ala Leu Glu Gly Gly Leu Pro Gln
            100                 105

<210> 24
<211> 425
<212> PRT
<213> Homo sapiens

<400> 24
Met Gly Pro Arg Arg Leu Leu Leu Val Ala Ala Cys Phe Ser Leu Cys
  1               5                  10                  15

Gly Pro Leu Leu Ser Ala Arg Thr Arg Ala Arg Arg Pro Glu Ser Lys
             20                  25                  30

Ala Thr Asn Ala Thr Leu Asp Pro Arg Ser Phe Leu Leu Arg Asn Pro
         35                  40                  45

Asn Asp Lys Tyr Glu Pro Phe Trp Glu Asp Glu Glu Lys Asn Glu Ser
     50                  55                  60

Gly Leu Thr Glu Tyr Arg Leu Val Ser Ile Asn Lys Ser Ser Pro Leu
65                  70                  75                  80

Gln Lys Gln Leu Pro Ala Phe Ile Ser Glu Asp Ala Ser Gly Tyr Leu
                85                  90                  95

Thr Ser Ser Trp Leu Thr Leu Phe Val Pro Ser Val Tyr Thr Gly Val
            100                 105                 110

Phe Val Val Ser Leu Pro Leu Asn Ile Met Ala Ile Val Val Phe Ile
        115                 120                 125

Leu Lys Met Lys Val Lys Lys Pro Ala Val Val Tyr Met Leu His Leu
    130                 135                 140

Ala Thr Ala Asp Val Leu Phe Val Ser Val Leu Pro Phe Lys Ile Ser
145                 150                 155                 160

Tyr Tyr Phe Ser Gly Ser Asp Trp Gln Phe Gly Ser Glu Leu Cys Arg
                165                 170                 175

Phe Val Thr Ala Ala Phe Tyr Cys Asn Met Tyr Ala Ser Ile Leu Leu
            180                 185                 190

Met Thr Val Ile Ser Ile Asp Arg Phe Leu Ala Val Val Tyr Pro Met
        195                 200                 205

Gln Ser Leu Ser Trp Arg Thr Leu Gly Arg Ala Ser Phe Thr Cys Leu
    210                 215                 220

Ala Ile Trp Ala Leu Ala Ile Ala Gly Val Val Pro Leu Val Leu Lys
225                 230                 235                 240

Glu Gln Thr Ile Gln Val Pro Gly Leu Asn Ile Thr Thr Cys His Asp
                245                 250                 255

Val Leu Asn Glu Thr Leu Leu Glu Gly Tyr Tyr Ala Tyr Tyr Phe Ser
            260                 265                 270

Ala Phe Ser Ala Val Phe Phe Phe Val Pro Leu Ile Ile Ser Thr Val
        275                 280                 285

Cys Tyr Val Ser Ile Ile Arg Cys Leu Ser Ser Ser Ala Val Ala Asn
    290                 295                 300

Arg Ser Lys Lys Ser Arg Ala Leu Phe Leu Ser Ala Ala Val Phe Cys
305                 310                 315                 320

Ile Phe Ile Ile Cys Phe Gly Pro Thr Asn Val Leu Leu Ile Ala His
                325                 330                 335

Tyr Ser Phe Leu Ser His Thr Ser Thr Thr Glu Ala Ala Tyr Phe Ala
            340                 345                 350

Tyr Leu Leu Cys Val Cys Val Ser Ser Ile Ser Ser Cys Ile Asp Pro
        355                 360                 365

Leu Ile Tyr Tyr Tyr Ala Ser Ser Glu Cys Gln Arg Tyr Val Tyr Ser
    370                 375                 380

Ile Leu Cys Cys Lys Glu Ser Ser Asp Pro Ser Ser Tyr Asn Ser Ser
385                 390                 395                 400

Gly Gln Leu Met Ala Ser Lys Met Asp Thr Cys Ser Ser Asn Leu Asn
                405                 410                 415

Asn Ser Ile Tyr Lys Lys Leu Leu Thr
            420                 425

<210> 25
<211> 348
<212> PRT
<213> Homo sapiens

<400> 25

<210> 26
<211> 460
<212> PRT
<213> Homo sapiens

<400> 26
Met Asn Thr Ser Val Pro Pro Ala Val Ser Pro Asn Ile Thr Val Leu
1               5                   10                  15

Ala Pro Gly Lys Gly Pro Trp Gln Val Ala Phe Ile Gly Ile Thr Thr
            20                  25                  30

Gly Leu Leu Ser Leu Ala Thr Val Thr Gly Asn Leu Leu Val Leu Ile
        35                  40                  45

Ser Phe Lys Val Asn Thr Glu Leu Lys Thr Val Asn Asn Tyr Phe Leu
    50                  55                  60

Leu Ser Leu Ala Cys Ala Asp Leu Ile Ile Gly Thr Phe Ser Met Asn
65                  70                  75                  80

Leu Tyr Thr Thr Tyr Leu Leu Met Gly His Trp Ala Leu Gly Thr Leu
                85                  90                  95

Ala Cys Asp Leu Trp Leu Ala Leu Asp Tyr Val Ala Ser Asn Ala Ser
            100                 105                 110

Val Met Asn Leu Leu Leu Ile Ser Phe Asp Arg Tyr Phe Ser Val Thr
        115                 120                 125

Arg Pro Leu Ser Tyr Arg Ala Lys Arg Thr Pro Arg Arg Ala Ala Leu
    130                 135                 140

Met Ile Gly Leu Ala Trp Leu Val Ser Phe Val Leu Trp Ala Pro Ala
145                 150                 155                 160

Ile Leu Phe Trp Gln Tyr Leu Val Gly Glu Arg Thr Val Leu Ala Gly
                165                 170                 175

Gln Cys Tyr Ile Gln Phe Leu Ser Gln Pro Ile Ile Thr Phe Gly Thr
            180                 185                 190

Ala Met Ala Ala Phe Tyr Leu Pro Val Thr Val Met Cys Thr Leu Tyr
        195                 200                 205

Trp Arg Ile Tyr Arg Glu Thr Glu Asn Arg Ala Arg Glu Leu Ala Ala
    210                 215                 220

Leu Gln Gly Ser Glu Thr Pro Gly Lys Gly Gly Gly Ser Ser Ser Ser
225                 230                 235                 240

Ser Glu Arg Ser Gln Pro Gly Ala Glu Gly Ser Pro Glu Ser Pro Pro
                245                 250                 255

Gly Arg Cys Cys Arg Cys Cys Arg Ala Pro Arg Leu Leu Gln Ala Tyr
            260                 265                 270

Ser Trp Lys Glu Glu Glu Glu Glu Asp Glu Gly Ser Met Glu Ser Leu
        275                 280                 285

Thr Ser Ser Glu Gly Glu Glu Pro Gly Ser Glu Val Val Ile Lys Met
    290                 295                 300

Pro Met Val Asp Ser Glu Ala Gln Ala Pro Thr Lys Gln Pro Pro Lys
305                 310                 315                 320

Ser Ser Pro Asn Thr Val Lys Arg Pro Thr Lys Lys Gly Arg Asp Arg
                325                 330                 335

Gly Gly Lys Gly Gln Lys Pro Arg Gly Lys Glu Gln Leu Ala Lys Arg
            340                 345                 350

Lys Thr Phe Ser Leu Val Lys Glu Lys Lys Ala Ala Arg Thr Leu Ser
        355                 360                 365

Ala Ile Leu Leu Ala Phe Ile Leu Thr Trp Thr Pro Tyr Asn Ile Met
    370                 375                 380

Val Leu Val Ser Thr Phe Cys Lys Asp Cys Val Pro Glu Thr Leu Trp
385                 390                 395                 400

Glu Leu Gly Tyr Trp Leu Cys Tyr Val Asn Ser Thr Val Asn Pro Met
                405                 410                 415

Cys Tyr Ala Leu Cys Asn Lys Ala Phe Arg Asp Thr Phe Arg Leu Leu
            420                 425                 430

Leu Leu Cys Arg Trp Asp Lys Arg Arg Trp Arg Lys Ile Pro Lys Arg
        435                 440                 445

Pro Gly Ser Val His Arg Thr Pro Ser Arg Gln Cys
    450                 455                 460

<210> 27
<211> 350
<212> PRT
<213> Rattus norvegicus

<400> 27

<210> 28
<211> 601
<212> PRT
<213> Drosophila melanogaster

<400> 28
Met Pro Ser Ala Asp Gln Ile Leu Phe Val Asn Val Thr Thr Thr Val
  1               5                  10                  15

Ala Ala Ala Ala Leu Thr Ala Ala Ala Ala Val Ser Thr Thr Lys Ser
             20                  25                  30

Gly Asn Gly Asn Ala Ala Arg Gly Tyr Thr Asp Ser Asp Asp Asp Ala
         35                  40                  45

Gly Met Gly Thr Glu Ala Val Ala Asn Ile Ser Gly Ser Leu Val Glu
     50                  55                  60

Gly Leu Thr Thr Val Thr Ala Ala Leu Ser Thr Ala Gln Ala Asp Lys
 65                  70                  75                  80

Asp Ser Ala Gly Glu Cys Glu Gly Ala Val Glu Glu Leu His Ala Ser
                 85                  90                  95

Ile Leu Gly Leu Gln Leu Ala Val Pro Glu Trp Glu Ala Leu Leu Thr
            100                 105                 110

Ala Leu Val Leu Ser Val Ile Ile Val Leu Thr Ile Ile Gly Asn Ile
        115                 120                 125

Leu Val Ile Leu Ser Val Phe Thr Tyr Lys Pro Leu Arg Ile Val Gln
    130                 135                 140

Asn Phe Phe Ile Val Ser Leu Ala Val Ala Asp Leu Thr Val Ala Leu
145                 150                 155                 160

Leu Val Leu Pro Phe Asn Val Ala Tyr Ser Ile Leu Gly Arg Trp Glu
                165                 170                 175

Phe Gly Ile His Leu Cys Lys Leu Trp Leu Thr Cys Asp Val Leu Cys
            180                 185                 190

Cys Thr Ser Ser Ile Leu Asn Leu Cys Ala Ile Ala Leu Asp Arg Tyr
        195                 200                 205

Trp Ala Ile Thr Asp Pro Ile Asn Tyr Ala Gln Lys Arg Thr Val Gly
    210                 215                 220

Arg Val Leu Leu Leu Ile Ser Gly Val Trp Leu Leu Ser Leu Leu Ile
225                 230                 235                 240

Ser Ser Pro Pro Leu Ile Gly Trp Asn Asp Trp Pro Asp Glu Phe Thr
                245                 250                 255

Ser Ala Thr Pro Cys Glu Leu Thr Ser Gln Arg Gly Tyr Val Ile Tyr
            260                 265                 270

Ser Ser Leu Gly Ser Phe Phe Ile Pro Leu Ala Ile Met Thr Ile Val
        275                 280                 285

Tyr Ile Glu Ile Phe Val Ala Thr Arg Arg Arg Leu Arg Glu Arg Ala
    290                 295                 300

Arg Ala Asn Lys Leu Asn Thr Ile Ala Leu Lys Ser Thr Glu Leu Glu
305                 310                 315                 320

Pro Met Ala Asn Ser Ser Pro Val Ala Ala Ser Asn Ser Gly Ser Lys
                325                 330                 335

Ser Arg Leu Leu Ala Ser Trp Leu Cys Cys Gly Arg Asp Arg Ala Gln
            340                 345                 350

Phe Ala Thr Pro Met Ile Gln Asn Asp Gln Glu Ser Ile Ser Ser Glu
        355                 360                 365

Thr His Gln Pro Gln Asp Ser Ser Lys Ala Gly Pro His Gly Asn Ser
    370                 375                 380

Asp Pro Gln Gln Gln His Val Val Val Leu Val Lys Lys Ser Arg Arg
385                 390                 395                 400

Ala Lys Thr Lys Asp Ser Ile Lys His Gly Lys Thr Arg Gly Gly Arg
                405                 410                 415

Lys Ser Gln Ser Ser Ser Thr Cys Glu Pro His Gly Glu Gln Gln Leu
            420                 425                 430

Leu Pro Ala Gly Gly Asp Gly Gly Ser Cys Gln Pro Gly Gly Gly His
        435                 440                 445

Ser Gly Gly Gly Lys Ser Asp Ala Glu Ile Ser Thr Glu Ser Gly Ser
    450                 455                 460

Asp Pro Lys Gly Cys Ile Gln Val Cys Val Thr Gln Ala Asp Glu Gln
465                 470                 475                 480

Thr Ser Leu Lys Leu Thr Pro Pro Gln Ser Ser Thr Gly Val Ala Ala
                485                 490                 495

Val Ser Val Thr Pro Leu Gln Lys Lys Thr Ser Gly Val Asn Gln Phe
            500                 505                 510

Ile Glu Glu Lys Gln Lys Ile Ser Leu Ser Lys Glu Arg Arg Ala Ala
        515                 520                 525

Arg Thr Leu Gly Ile Ile Met Gly Val Phe Val Ile Cys Trp Leu Pro
    530                 535                 540

Phe Phe Leu Met Tyr Val Ile Leu Pro Phe Cys Gln Thr Cys Cys Pro
545                 550                 555                 560

Thr Asn Lys Phe Lys Asn Phe Ile Thr Trp Leu Gly Tyr Ile Asn Ser
                565                 570                 575

Gly Leu Asn Pro Val Ile Tyr Thr Ile Phe Asn Leu Asp Tyr Arg Arg
            580                 585                 590

Ala Phe Lys Arg Leu Leu Gly Leu Asn
        595                 600


<210> 29
<211> 588
<212> PRT
<213> Homo sapiens

<400> 29
Met Ala Arg Gly Gly Ala Gly Ala Glu Glu Ala Ser Leu Arg Ser Asn
  1               5                  10                  15

Ala Leu Ser Trp Leu Ala Cys Gly Leu Leu Ala Leu Leu Ala Asn Ala
             20                  25                  30

Trp Ile Ile Leu Ser Ile Ser Ala Lys Gln Gln Lys His Lys Pro Leu
         35                  40                  45

Glu Leu Leu Leu Cys Phe Leu Ala Gly Thr His Ile Leu Met Ala Ala
     50                  55                  60

Val Pro Leu Thr Thr Phe Ala Val Val Gln Leu Arg Arg Gln Ala Ser
 65                  70                  75                  80

Ser Asp Tyr Asp Trp Asn Glu Ser Ile Cys Lys Val Phe Val Ser Thr
                 85                  90                  95

Tyr Tyr Thr Leu Ala Leu Ala Thr Cys Phe Thr Val Ala Ser Leu Ser
            100                 105                 110

Tyr His Arg Met Trp Met Val Arg Trp Pro Val Asn Tyr Arg Leu Ser
        115                 120                 125

Asn Ala Lys Lys Gln Ala Leu His Ala Val Met Gly Ile Trp Met Val
    130                 135                 140

Ser Phe Ile Leu Ser Thr Leu Pro Ser Ile Gly Trp His Asn Asn Gly
145                 150                 155                 160

Glu Arg Tyr Tyr Ala Arg Gly Cys Gln Phe Ile Val Ser Lys Ile Gly
                165                 170                 175

Leu Gly Phe Gly Val Cys Phe Ser Leu Leu Leu Leu Gly Gly Ile Val
            180                 185                 190

Met Gly Leu Val Cys Val Ala Ile Thr Phe Tyr Gln Thr Leu Trp Ala
        195                 200                 205

Arg Pro Arg Arg Ala Arg Gln Ala Arg Arg Val Gly Gly Gly Gly Gly
    210                 215                 220

Thr Lys Ala Gly Gly Pro Gly Ala Leu Gly Thr Arg Pro Ala Phe Glu
225                 230                 235                 240

Val Pro Ala Ile Val Val Glu Asp Ala Arg Gly Lys Arg Arg Ser Ser
                245                 250                 255

Leu Asp Gly Ser Glu Ser Ala Lys Thr Ser Leu Gln Val Thr Asn Leu
            260                 265                 270

Val Ser Ala Ile Val Phe Leu Tyr Asp Ser Leu Thr Gly Val Pro Ile
        275                 280                 285

Leu Val Val Ser Phe Phe Ser Leu Lys Ser Asp Ser Ala Pro Pro Trp
    290                 295                 300

Met Val Leu Ala Val Leu Trp Cys Ser Met Ala Gln Thr Leu Leu Leu
305                 310                 315                 320

Pro Ser Phe Ile Trp Ser Cys Glu Arg Tyr Arg Ala Asp Val Arg Thr
                325                 330                 335

Val Trp Glu Gln Cys Val Ala Ile Met Ser Glu Glu Asp Gly Asp Asp
            340                 345                 350

Asp Gly Gly Cys Asp Asp Tyr Ala Glu Gly Arg Val Cys Lys Val Arg
        355                 360                 365

Phe Asp Ala Asn Gly Ala Thr Gly Pro Gly Ser Arg Asp Pro Ala Gln
    370                 375                 380

Val Lys Leu Leu Pro Gly Arg His Met Leu Phe Pro Pro Leu Glu Arg
385                 390                 395                 400

Val His Tyr Leu Gln Val Pro Leu Ser Arg Arg Leu Ser His Asp Glu
                405                 410                 415

Thr Asn Ile Phe Ser Thr Pro Arg Glu Pro Gly Ser Phe Leu His Lys
            420                 425                 430

Trp Ser Ser Ser Asp Asp Ile Arg Val Leu Pro Ala Gln Ser Arg Ala
        435                 440                 445

Leu Gly Gly Pro Pro Glu Tyr Leu Gly Gln Arg His Arg Leu Glu Asp
    450                 455                 460

Glu Glu Asp Glu Glu Glu Ala Glu Gly Gly Gly Leu Ala Ser Leu Arg
465                 470                 475                 480

Gln Phe Leu Glu Ser Gly Val Leu Gly Ser Gly Gly Gly Pro Pro Arg
                485                 490                 495

Gly Pro Gly Phe Phe Arg Glu Glu Ile Thr Thr Phe Ile Asp Glu Thr
            500                 505                 510

Pro Leu Pro Ser Pro Thr Ala Ser Pro Gly His Ser Pro Arg Arg Pro
        515                 520                 525

Arg Pro Leu Gly Leu Ser Pro Arg Arg Leu Ser Leu Gly Ser Pro Glu
    530                 535                 540

Ser Arg Ala Val Gly Leu Pro Leu Gly Leu Ser Ala Gly Arg Arg Cys
545                 550                 555                 560

Ser Leu Thr Gly Gly Glu Glu Ser Ala Arg Ala Trp Gly Gly Ser Trp
                565                 570                 575

Gly Pro Gly Asn Pro Ile Phe Pro Gln Leu Thr Leu
            580                 585

<210> 30
<211> 227
<212> PRT
<213> Homo sapiens

<400> 30
Ile Thr Phe Tyr Gln Thr Leu Trp Ala Arg Pro Arg Arg Ala Arg Gln
  1               5                  10                  15

Ala Arg Arg Val Gly Gly Gly Gly Gly Thr Lys Ala Gly Gly Pro Gly
             20                  25                  30

Ala Leu Gly Thr Arg Pro Ala Phe Glu Val Pro Ala Ile Val Val Glu
         35                  40                  45

Asp Ala Arg Gly Lys Arg Arg Ser Ser Leu Asp Gly Ser Glu Ser Ala
     50                  55                  60

Lys Thr Ser Leu Gln Val Thr Asn Leu Val Val Ser Phe Phe Ser Leu
 65                  70                  75                  80

Lys Ser Asp Ser Ala Pro Pro Trp Met Val Leu Ala Val Leu Trp Cys
                 85                  90                  95

Ser Met Ala Gln Thr Leu Leu Leu Pro Ser Phe Ile Trp Ser Cys Glu
            100                 105                 110

Arg Tyr Arg Ala Asp Val Arg Thr Val Trp Glu Gln Cys Val Ala Ile
      115                 120                 125

Met Ser Glu Glu Asp Gly Asp Asp Asp Gly Gly Cys Asp Asp Tyr Ala
    130                 135                 140

Glu Gly Arg Val Cys Lys Val Arg Phe Asp Ala Asn Gly Ala Thr Gly
145                 150                 155                 160

Pro Gly Ser Arg Asp Pro Ala Gln Val Lys Leu Leu Pro Gly Arg His
                165                 170                 175

Met Leu Phe Pro Pro Leu Glu Arg Val His Tyr Leu Gln Leu Lys Lys
            180                 185                 190

Leu Asp Leu Ala Ala Ala Ala Ala His Thr Phe Phe Val Ala Asn Pro
        195                 200                 205

Met His Leu Gln Met Arg Glu Asp Met Ala Lys Tyr Arg Arg Met Ser
    210                 215                 220

Gly Val Arg
225

## Description

### Background of the Invention

There is considerable medical interest in secreted and membrane-associated mammalian proteins. Many such proteins, for example, signaling factors and/or cell-surface receptors, are important in the regulation of growth and/or differentiation of cells of for triggering one or more specific cellular responses.

Signaling factors play an important role in the development and functioning of different cell types by allowing for communication between interacting cells. Such factors provide a signal between cells which can cause cells which recognize the signal to perform specialized tasks, such as cell growth, differentiation and/or proliferation.

For example, cells of the immune system characteristically express a variety of signaling proteins which are crucial to proper functioning of the immune system. Such proteins include secreted immunoglobulins and non-immunoglobulin molecules which interact with cellular adhesion molecules, as well as other selected target molecules. Many of these proteins are members of the immunoglobulin (Ig) superfamily of proteins, characterized by the existence of at least one immunoglobulin (Ig)-like domain. Such proteins function in a variety of immune cell functions ranging from immune cell development and differentiation, antigen recognition, antibody production, cellular signal transduction, and cellular homing of immune responsive cells from the circulation to sites of increased antigen concentration.

Cell surface receptors likewise play an important role in the development and functioning of different cell types by allowing for communication between interacting cells or between a cell and a soluble ligand in the intracellular milieu. For example, the G protein-coupled receptors ("GPCRs") form one of the largest receptor superfamilies found in nature, and it is estimated that greater than 1000 different such receptors exist in mammals. Upon binding of extracellular ligands, GPCRs interact with a specific subset of heterotrimeric G-proteins that can then, in their activated forms, inhibit or activate various effector enzymes and/or ion channels. The ligands for many of these receptors are known although there exists an ever-increasing number of GPCRs which have been identified in the sequencing of the human genome for which no ligands have yet been identified. This latter subfamily of GPCRs is called the ophan family of GPCRs. In addition to both GPCRs with known ligands, as well as orphan GPCRs, there exist a family of GPCR-like molecules which share significant homology as well as many of the structural properties of the GPCR superfamily. For example, a family of GPCR-like proteins which arises from three alternatively-spliced forms of a gene occurring between the CD4 and triosephosphate isomerase genes at human chromosome 12p13, has been recently identified (including protein A-1, A-2, and A-3). Ansari-Lari et al. (1996) Genome Res. 6(4):314-326. Comparative sequence analysis of the syntenic region in mouse chromosome 6 has further revealed a murine homologue of at least the A-2 splice product. Ansari-Lari et al. (1998) Genome Res. 8(1):29-40.

The fundamental knowledge that GPCRs play a role in regulating that activity of virtually every cell in the human body has fostered an extensive search for modulators of such receptors for use as human therapeutics. In fact, the superfamily of GPCRs has proven to be among the most successful drug targets. Consequently, it has been recognized that the newly isolated orphan GPCRs, as well as the GPCR-like proteins, have great potential for drug discovery.

Given the importance of such secreted proteins *(e.g.,* signaling factors) and membrane-associated proteins (*e.g.*, G-protein coupled receptors) in the proper functioning of a variety of cellular processes, there exists a need to identify novel signaling factors and/or receptors as well as for modulators of such molecules for use in regulating a variety of cellular responses and for use in the design and development of new therapies. Moreover, with the identification of each new GPCR or GPCR-like protein, there exists a need for identifying the surrogate ligands for such molecules.

### Summary of the Invention

The present invention is based, at least in part, on the discovery of novel secreted proteins and membrane-associated proteins. In one aspect, the present invention invloves novel signaling molecules, referred to herein as Brain-Derived Signaling Factor ("BDSF") molecules, as well as the nucleic acids encoding them. The BDSF molecules of the present invention are useful as modulating agents in regulating a variety of cellular processes. Accordingly, in one aspect, this invention provides isolated nucleic acid molecules encoding BDSF proteins or biologically active portions thereof, as well as nucleic acid fragments suitable as primers or hybridization probes for the detection of BDSF-encoding nucleic acids.

In one embodiment, a BDSF nucleic acid molecule is 60% homologous to the nucleotide sequence shown in SEQ ID NO:1, SEQ ID NO:3, SEQ ID NO:4, or the nucleotide sequence of the DNA insert of the plasmid deposited with ATCC as Accession Number 98756, or complements thereof. In a preferred embodiment, the isolated nucleic acid molecule has the nucleotide sequence shown in SEQ ID NO:1, SEQ ID N0:3, SEQ ID N0:4, or a complement thereof. In yet another preferred embodiment, an isolated nucleic acid molecule has the nucleotide sequence of the DNA insert of the plasmid deposited with ATCC as Accession Number 98756, or a complement thereof. In another embodiment, the nucleic acid molecule further comprises nucleotides 244-701 of SEQ ID NO:1. In another embodiment, the nucleic acid molecule further comprises nucleotides 31-487 of SEQ ID NO:4

In yet another embodiment, a BDSF nucleic acid molecule is 60% homologous to the nucleotide sequence shown in SEQ ID NO:6, SEQ ID NO:8, SEQ ID NO:9, or a complement thereof. In a preferred embodiment, an isolated nucleic acid molecule has the nucleotide sequence shown in SEQ ID NO:6, SEQ ID NO: 8, SEQ ID NO:9, or a complement thereof.

In another embodiment, a BDSF nucleic acid molecule includes a nucleotide sequence encoding a protein having an amino acid sequence sufficiently homologous to the amino acid sequence of SEQ ID NO:2 or SEQ ID NO:5. In another embodiment, a BDSF nucleic acid molecule includes a nucleotide sequence encoding a protein having an amino acid sequence sufficiently homologous to the amino acid sequence of SEQ ID NO:7 or SEQ ID NO:10. In a preferred embodiment, a BDSF nucleic acid molecule includes a nucleotide sequence encoding a protein having an amino acid sequence at least 60% homologous to the amino acid sequence of SEQ ID NO:2, SEQ ID NO:5, SEQ ID NO:7 or SEQ ID NO:10. In another preferred embodiment, an isolated nucleic acid molecule encodes the amino acid sequence of human BDSF. In yet another preferred embodiment, the nucleic acid molecule includes a nucleotide sequence encoding a protein having the amino acid sequence of SEQ ID NO: 2 or SEQ ID NO:5. In another preferred embodiment, an isolated nucleic acid molecule encodes the amino acid sequence of murine BDSF. In yet another preferred embodiment, the nucleic acid molecule includes a nucleotide sequence encoding a protein having the amino acid sequence of SEQ ID NO: 7 or SEQ ID NO:10.

In another embodiment, an isolated nucleic acid molecule of the present invention encodes a protein, preferably a BDSF protein, which includes an immunoglobulin-like domain. In another embodiment, an isolated nucleic acid molecule of the present invention encodes a protein, preferably a BDSF protein, which includes a signal sequence, an immunoglobulin-like domain, and, preferably, is secreted. In yet another embodiment, a BDSF nucleic acid molecule encodes a BDSF protein and is a naturally occurring nucleotide sequence.

Another embodiment of the invention features nucleic acid molecules, preferably BDSF nucleic acid molecules, which specifically detect BDSF nucleic acid molecules relative to nucleic acid molecules encoding non-BDSF proteins. For example, in one embodiment, such a nucleic acid molecule is at least 450, preferably 500-700, more preferably 700-900, more preferably 900-1100, and even more preferably 1100-1120 nucleotides in length and hybridizes under stringent conditions to a nucleic acid molecule comprising the nucleotide sequence shown in SEQ ID NO:1, SEQ ID NO:6, the nucleotide sequence of the DNA insert of the plasmid deposited with ATCC as Accession Number 98756, or a complement thereof. Another embodiment of the invention provides an isolated nucleic acid molecule which is antisense to the coding strand of a BDSF nucleic acid.

Another aspect of the invention provides a vector comprising a BDSF nucleic acid molecule. In certain embodiments, the vector is a recombinant expression vector. In another embodiment, the invention provides a host cell containing a vector of the invention. The invention also provides a method for producing a protein, preferably a BDSF protein, by culturing in a suitable medium, a host cell of the invention containing a recombinant expression vector such that the protein is produced.

Another aspect of this invention features isolated or recombinant BDSF proteins and polypeptides. In one embodiment, an isolated protein, preferably a BDSF protein, includes an immunoglobulin-like domain. In another embodiment, an isolated protein, preferably a BDSF protein, includes a signal sequence, an immunoglobulin-like domain, and is, preferably, secreted. In another embodiment, an isolated protein, preferably a BDSF protein, has an amino acid sequence sufficiently homologous to the amino acid sequence of SEQ ID NO:2 or SEQ ID NO:5. In another embodiment, an isolated protein, preferably a BDSF protein, has an amino acid sequence sufficiently homologous to the amino acid sequence of SEQ ID NO:7 or SEQ ID NO:10. In a preferred embodiment, a protein, preferably a BDSF protein, has an amino acid sequence at least about 60% homologous to the amino acid sequence of SEQ ID NO:2, SEQ ID NO:5, SEQ ID NO:7, or SEQ ID NO:10. In another embodiment, the invention features fragments of the proteins having the amino acid sequence of SEQ ID NO:2 or SEQ ID NO:5, wherein the fragment comprises at least 15 contiguous amino acids of the amino acid sequence of SEQ ID NO:2, SEQ ID NO: 5, or an amino acid sequence encoded by the DNA insert of the plasmid deposited with the ATCC as Accession No. 98756. In another embodiment, the invention features fragments of the proteins having the amino acid sequence of SEQ ID NO: 7 or SEQ ID NO:10, wherein the fragment comprises at least 15 contiguous amino acids of the amino acid sequence of SEQ ID NO:7 or SEQ ID NO:10. In another embodiment, a protein, preferably a BDSF protein, has the amino acid sequence of SEQ ID NO:2, SEQ ID NO: 5, SEQ ID NO:7 or SEQ ID NO:10.

Another embodiment of the invention features an isolated protein, preferably a BDSF protein, which is encoded by a nucleic acid molecule having a nucleotide sequence at least about 60% homologous to a nucleotide sequence of SEQ ID N0:1, SEQ ID NO:3, SEQ ID NO:4, or a complement thereof. This invention further features an isolated protein, preferably a BDSF protein, which is encoded by a nucleic acid molecule having a nucleotide sequence which hybridizes under stringent hybridization conditions to a nucleic acid molecule comprising the nucleotide sequence of SEQ ID NO:1, SEQ ID NO:3, SEQ ID NO:4, or a complement thereof.

Yet another embodiment of the invention features an isolated protein, preferably a BDSF protein, which is encoded by a nucleic acid molecule having a nucleotide sequence at least about 60% homologous to a nucleotide sequence of SEQ ID NO:6, SEQ ID NO:8, SEQ ID NO:9, or a complement thereof. This invention further features an isolated protein, preferably a BDSF protein, which is encoded by a nucleic acid molecule having a nucleotide sequence which hybridizes under stringent hybridization conditions to a nucleic acid molecule comprising the nucleotide sequence of SEQ ID NO:6, SEQ ID NO:8, SEQ ID NO:9, or a complement thereof.

In another aspect, the present invention involves proteins of a novel family of G protein-coupled receptor-like proteins, referred to herein as the Seven Transmembrane Signal Transducer ("STMST" family or "STMST proteins"), as well as the nucleic acids encoding them. The STMST molecules of the present invention as well as STMST ligands and/or STMST modulators, are useful in regulating a variety of cellular processes. Accordingly, in one aspect, this invention provides isolated nucleic acid molecules encoding STMST proteins or biologically active portions thereof, as well as nucleic acid fragments suitable as primers or hybridization probes for the detection of STMST-encoding nucleic acids.

In one embodiment, an STMST nucleic acid molecule is 75% homologous to the nucleotide sequence shown in SEQ ID NO:14, the nucleotide sequence of the DNA insert of the plasmid deposited with ATCC as Accession Number , or complement thereof. In another embodiment, an STMST nucleic acid molecule is 80% homologous to the nucleotide sequence shown in SEQ ID NO:17, the nucleotide sequence of the DNA insert of the plasmid deposited with ATCC as Accession Number , or a complement thereof. In a preferred embodiment, an isolated STMST nucleic acid molecule has the nucleotide sequence shown SEQ ID N0:16, or a complement thereof. In another embodiment, an STMST nucleic acid molecule further comprises nucleotides 1-403 of SEQ ID NO: 1. In another embodiment, an STMST nucleic acid molecule further comprises nucleotides 1295-2915 of SEQ ID NO: 14. In another preferred embodiment, an isolated STMST nucleic acid molecule has the nucleotide sequence shown in SEQ ID NO:1. In yet another preferred embodiment, an isolated nucleic acid molecule has the nucleotide sequence of the DNA insert of the plasmid deposited with ATCC as Accession Number , or a complement thereof.

In another preferred embodiment, an isolated STMST nucleic acid molecule has the nucleotide sequence shown SEQ ID N0:19, or a complement thereof. In another embodiment, an STMST nucleic acid molecule further comprises nucleotides 1-333 of SEQ ID N0:17. In another embodiment, an STMST nucleic acid molecule further comprises nucleotides 2161-4166 of SEQ ID NO: 17. In another preferred embodiment, an isolated STMST nucleic acid molecule has the nucleotide sequence shown in SEQ ID N0:17. In yet another preferred embodiment, an isolated nucleic acid molecule has the nucleotide sequence of the DNA insert of the plasmid deposited with ATCC as Accession Number , or a complement thereof.

In another embodiment, an STMST nucleic acid molecule includes a nucleotide sequence encoding a protein having an amino acid sequence sufficiently homologous to the amino acid sequence of SEQ ID NO:15, the amino acid sequence of SEQ ID NO:18, an amino acid or an amino acid sequence encoded by the DNA insert of the plasmid deposited with the ATCC as Accession No. , or an amino acid or an amino acid sequence encoded by the DNA insert of the plasmid deposited with the ATCC as Accession No. . In another preferred embodiment, an STMST nucleic acid molecule includes a nucleotide sequence encoding a protein having an amino acid sequence at least 75% homologous to the amino acid sequence of SEQ ID NO:15 or an amino acid or an amino acid sequence encoded by the DNA insert of the plasmid deposited with the ATCC as Accession No. . In yet another preferred embodiment, an STMST nucleic acid molecule includes a nucleotide sequence encoding a protein having an amino acid sequence at least 60% homologous to the amino acid sequence of SEQ ID NO: 18 or an amino acid or an amino acid sequence encoded by the DNA insert of the plasmid deposited with the ATCC as Accession No. .

In another embodiment, an isolated nucleic acid molecule of the present invention encodes an STMST protein which includes at least one transmembrane domain. In another embodiment, an isolated nucleic acid molecule of the present invention encodes a protein which includes a 7 transmembrane receptor profile. In another embodiment, an isolated nucleic acid molecule of the present invention encodes a protein which includes a spectrin α-chain motif. In yet another embodiment, an STMST nucleic acid molecule encodes an STMST protein and is a naturally occurring nucleotide sequence.

Another embodiment of the invention features STMST nucleic acid molecules which specifically detect STMST nucleic acid molecules relative to nucleic acid molecules encoding non-STMST proteins. For example, in one embodiment, an STMST nucleic acid molecule is at least 350 nucleotides in length and hybridizes under stringent conditions to a nucleic acid molecule comprising the nucleotide sequence shown in SEQ ID NO: 14, SEQ ID NO:17, the nucleotide sequence of the DNA insert of the plasmid deposited with ATCC as Accession Number , the nucleotide sequence of the DNA insert of the plasmid deposited with ATCC as Accession Number , or a complement thereof.

Another embodiment of the invention provides an isolated nucleic acid molecule which is antisense to the coding strand of an STMST nucleic acid.

Another aspect of the invention provides a vector comprising an STMST nucleic acid molecule. In certain embodiments, the vector is a recombinant expression vector. In another embodiment, the invention provides a host cell containing a vector of the invention. The invention also provides a method for producing an STMST protein by culturing in a suitable medium, a host cell of the invention containing a recombinant expression vector such that an STMST protein is produced.

Another aspect of this invention features isolated or recombinant STMST proteins and polypeptides. In one embodiment, an isolated STMST protein includes at least one transmembrane domain. In another embodiment, an isolated STMST protein includes at least six transmembrane domains. In another embodiment, an isolated STMST protein includes seven transmembrane domains. In another embodiment, an isolated STMST protein includes a 7 transmembrane receptor profile. In another embodiment, an isolated STMST protein includes a spectrin α-chain profile. In another embodiment, an isolated STMST protein has an amino acid sequence sufficiently homologous to the amino acid sequence of SEQ ID NO:15 or SEQ ID NO:18. In a preferred embodiment, an STMST protein has an amino acid sequence at least about 75% homologous to the amino acid sequence of SEQ ID NO:15. In another preferred embodiment, an STMST protein has an amino acid sequence at least about 60% homologous to the amino acid sequence of SEQ ID NO:18. In another embodiment, an STMST protein has the amino acid sequence of SEQ ID NO:15 or SEQ ID NO:18.

Another embodiment of the invention features an isolated STMST protein which is encoded by a nucleic acid molecule having a nucleotide sequence at least about 75% homologous to a nucleotide sequence of SEQ ID NO: 14, or a complement thereof. Another embodiment of the invention features an isolated STMST protein which is encoded by a nucleic acid molecule having a nucleotide sequence at least about 80% homologous to a nucleotide sequence of SEQ ID NO:17, or a complement thereof. This invention further features an isolated STMST protein which is encoded by a nucleic acid molecule having a nucleotide sequence which hybridizes under stringent hybridization conditions to a nucleic acid molecule comprising the nucleotide sequence of SEQ ID N0:14, SEQ ID NO:17, or a complement thereof.

The proteins of the present invention, preferably BDSF of STMST proteins or biologically active portions thereof, can be operatively linked to a non-BDSF or non-STMST polypeptide to form fusion proteins. The invention further features antibodies that specifically bind BDSF or STMST proteins, such as monoclonal or polyclonal antibodies. In addition, the BDSF or STMST proteins or biologically active portions thereof can be incorporated into pharmaceutical compositions, which optionally include pharmaceutically acceptable carriers.

In another aspect, the present invention provides a method for detecting BDSF or STMST expression in a biological sample by contacting the biological sample with an agent capable of detecting a BDSF or STMST nucleic acid molecule, protein or polypeptide such that the presence of a BDSF or STMST nucleic acid molecule, protein or polypeptide is detected in the biological sample.

In another aspect, the present invention provides a method for detecting the presence of BDSF or STMST activity in a biological sample by contacting the biological sample with an agent capable of detecting an indicator of BDSF or STMST activity such that the presence of BDSF or STMST activity is detected in the biological sample.

In another aspect, the invention provides a method for modulating BDSF or STMST activity comprising contacting the cell with an agent that modulates BDSF or STMST activity such that BDSF or STMST activity in the cell is modulated. In one embodiment, the agent inhibits BDSF or STMST activity. In another embodiment, the agent stimulates BDSF or STMST activity. In one embodiment, the agent is an antibody that specifically binds to a BDSF or STMST protein. In another embodiment, the agent modulates expression of BDSF or STMST by modulating transcription of a BDSF or STMST gene or translation of a BDSF or STMST mRNA. In yet another embodiment, the agent is a nucleic acid molecule having a nucleotide sequence that is antisense to the coding strand of a BDSF or STMST mRNA or a BDSF or STMST gene.

In one embodiment, the methods of the present invention are used to treat a subject having a disorder characterized by aberrant BDSF or STMST protein or nucleic acid expression or activity by administering an agent which is a BDSF or STMST modulator to the subject. In one embodiment, the BDSF or STMST modulator is a BDSF or STMST protein, respectively. In another embodiment, the BDSF or STMST modulator is a BDSF or STMST nucleic acid molecule, respectively. In a preferred embodiment, the STMST modulator is an STMST ligand. In yet another embodiment, the BDSF or STMST modulator is a peptide, peptidomimetic, or other small molecule. In a preferred embodiment, the disorder characterized by aberrant BDSF protein or nucleic acid expression is a proliferative or differentiative disorder. In another preferred embodiment, the disorder characterized by aberrant STMST protein or nucleic acid expression is a developmental, differentiative, proliferative disorder, an inflammatory disorder, a respiratory disorder (*e.g.*, asthma), or cell death.

The present invention also provides a diagnostic assay for identifying the presence or absence of a genetic alteration characterized by at least one of (i) aberrant modification or mutation of a gene encoding a STMST protein; (ii) mis-regulation of said gene; and (iii) aberrant post-translational modification of a BDSF or STMST protein, wherein a wild-type form of said gene encodes an protein with a BDSF or STMST activity, respectively.

In another aspect the invention provides a method for identifying a compound that binds to or modulates the activity of a BDSF or STMST protein. In one embodiment, the invention provides a method for identifying a compound which binds to a BDSF or STMST protein which involves contacting the BDSF or STMST protein, or a cell expressing the BDSF or STMST protein with a test compound and determining whether the BDSF or STMST protein binds to the test compound. In another embodiment, the invention provides a method for identifying a compound which modulates the activity of a BDSF or STMST protein which involves contacting a BDSF or STMST protein with a test compound, and determining the effect of the test compound on the activity of the polypeptide to thereby identify a compound which modulates the activity of the BDSF or STMST protein.

Other features and advantages of the invention will be apparent from the following detailed description and claims.

### Brief Description of the Drawings

*Figure 1* depicts the cDNA sequence and predicted amino acid sequence of human BDSF-1. The nucleotide sequence corresponds to nucleic acids 1 to 1119 of SEQ ID NO:1. The amino acid sequence corresponds to amino acids 1 to 244 of SEQ ID NO:2.
*Figure 2* depicts the cDNA sequence and predicted amino acid sequence of murine BDSF-1. The nucleotide sequence corresponds to nucleic acids 1 to 3196 of SEQ ID NO:6. The amino acid sequence corresponds to amino acids 1 to 251 of SEQ ID NO:7.
*Figure 3* depicts an alignment of the amino acid sequence of human BDSF-1, corresponding to SEQ ID NO:2, with the amino acid sequence of murine BDSF-1, corresponding to SEQ ID NO:7. The immunoglobulin-like domains are underlined. The conserved cysteine residues of the immunoglobulin-like domain of human and murine BDSF are indicated with an asterix. The alignment was performed using the ALIGN program with a PAM120 weight residue table, a gap length penalty of 12, and a gap penatly of 4.
*Figure 4* depicts the cDNA sequence and predicted amino acid sequence of human STMST-1. The nucleotide sequence corresponds to nucleic acids 1 to 2915 of SEQ ID NO:14. The amino acid sequence corresponds to amino acids 1 to 297 of SEQ ID NO:15.
*Figure 5* depicts the cDNA sequence and predicted amino acid sequence of human STMST-2. The nucleotide sequence corresponds to nucleic acids 1 to 4166 of SEQ ID NO:17. The amino acid sequence corresponds to amino acids 1 to 609 of SEQ ID NO:18.

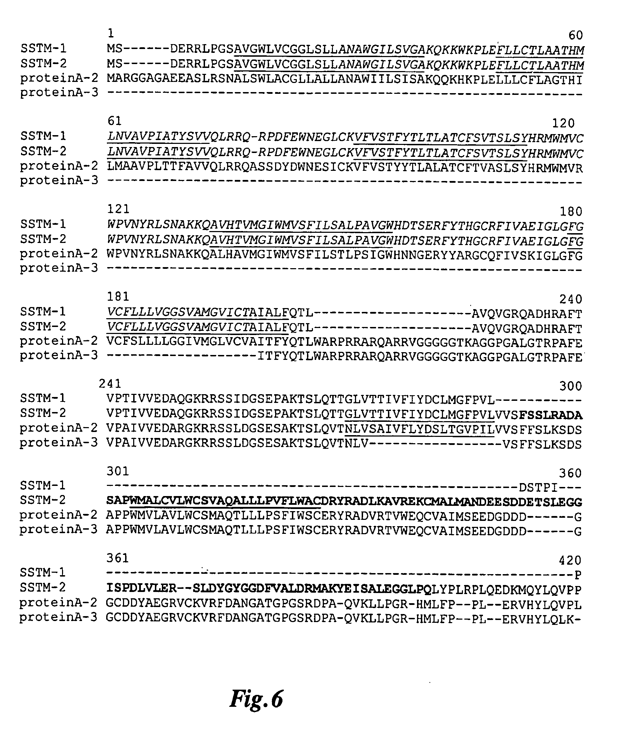

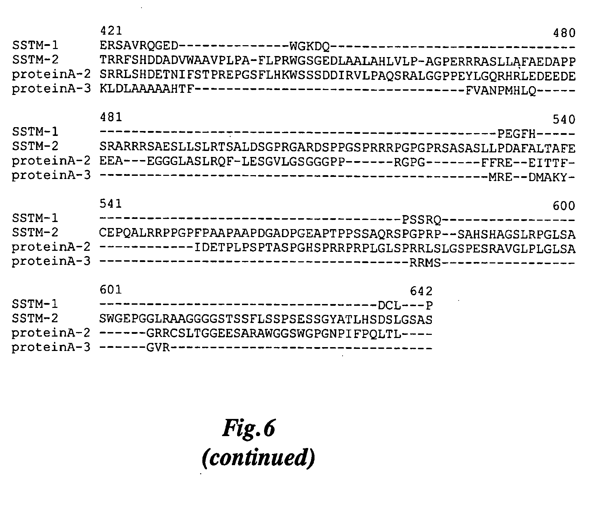

*Figure 6* depicts an alignment of the amino acid sequences of human STMST-1 (SEQ ID NO:15), human STMST-2 (SEQ ID NO:18), human protein A-2 (Accession No. U47928, SEQ ID NO:29), and human protein A-3 (Accession No. U47929, SEQ ID NO:30). The 7 transmembrane receptor profile is indicated in italics. The transmembrane domains are underlined. The spectrin α-chain profile is indicated in bold.

### Detailed Description of the Invention

The present invention is based on the discovery of novel molecules, referred to herein as BDSF protein and nucleic acid molecules, which comprise a family of molecules having certain conserved structural and functional features. The present invention is also based on the discovery of novel molecules, referred to herein as STMST protein and nucleic acid molecules, which comprise a second family of molecules having certain conserved structural and functional features.

The term "family" when referring to the protein and nucleic acid molecules of the invention is intended to mean two or more proteins or nucleic acid molecules having a common structural domain or motif and having sufficient amino acid or nucleotide sequence homology as defined herein. Such family members can be naturally occurring and can be from either the same or different species. For example, a family can contain a first protein of human origin, as well as other, distinct proteins of human origin or alternatively, can contain homologues of non-human origin. Members of a family may also have common functional characteristics.

Members of a family may also share sufficient sequence homology with other members of the same family. For example, isolated proteins of the present invention, preferably BDSF proteins, have an amino acid sequence sufficiently homologous to the amino acid sequence of SEQ ID NO:2, SEQ ID NO:5, SEQ ID NO:7 or SEQ ID NO: 10 or are encoded by a nucleotide sequence sufficiently homologous to SEQ ID NO: 1, SEQ ID NO:4, SEQ ID NO:6 or SEQ ID NO:9. In another embodiment, isolated proteins of the present invention, preferably STMST proteins, have an amino acid sequence sufficiently homologous to the amino acid sequence of SEQ ID NO:15 or SEQ ID NO: 18. As used herein, the term "sufficiently homologous" refers to a first amino acid or nucleotide sequence which contains a sufficient or minimum number of identical or equivalent (*e.g.*, an amino acid residue which has a similar side chain) amino acid residues or nucleotides to a second amino acid or nucleotide sequence such that the first and second amino acid or nucleotide sequences share common structural domains or motifs and/or a common functional activity. For example, amino acid or nucleotide sequences which share common structural domains have at least about 30-40% homology, preferably 40-50% homology, more preferably 50-60%, and even more preferably 60-70%, 70-80%, or 80-90% homology across the amino acid sequences of the domains and contain at least one and preferably two structural domains or motifs, are defined herein as sufficiently homologous. Furthermore, amino acid or nucleotide sequences which share at least 30-40%, preferably 40-50%, more preferably 50-60%, 60-70%, 70-80%, or 80-90% homology and share a common functional activity are defined herein as sufficiently homologous.

### The BDSP Protein Family

For example, the BDSF proteins of the present invention belong to a family of signaling proteins having common structural and functional characteristics. In one embodiment, the isolated proteins of the present invention, preferably BDSF proteins, are proteins having an amino acid sequence of about 150-340 amino acid residues in length, preferably about 170-320, more preferably about 190-300, more preferably about 210-280, or about 230-260 amino acid residues in length. In one embodiment, an isolated protein of the present invention, preferably a BDSF protein, includes an immunoglobulin (Ig)-like domain. As used herein, the term an "immunoglobulin-like domain" includes an amino acid sequence having about 65-115, preferably about 70-110, more preferably about 80-100 amino acid residues, and even more preferably at least about 85-95 amino acids in length and having a bit score for the alignment of the sequence to the Ig family Hidden Markov Model (HMM) of at least 10, preferably 10-15, more preferably 15-20, more preferably 20-25, even more preferably 25-35, 35-55, 55-100 or greater. The Ig family HMM has been assigned the PFAM Accession PF00047 (http://genome.wustl.edu/Pfam/WWWdata/ig.html).

To identify the presence of an Ig-like domain in a BDSF family member, the amino acid sequence of the family member is searched against a database of HMMs (*e.g.*, the Pfam database, release 2.1) using the default parameters (http://www.sanger.ac.uk/Software/Pfam/HMM_search). For example, the hmmsf program, which is available as part of the HMMER package of search programs, is a family specific default program for PF00047 having a score of 15 as the default threshold score for determining a hit. For example, a search using the amino acid sequence of SEQ ID NO:2 was performed against the HMM database resulting in the identification of an Ig-like domain in the amino acid sequence of SEQ ID NO:2 and a score of 22.43 against the Ig family HMM Accession PF00047. The results of the search are set forth below.

Score: 22.43          SEQ ID NO:2: aa41-129       HMM: aa1-47 (SEQ ID NO:13)

    GqsVTLTCmVs.fhPpdYt.IwWY.rNgqpi..................
41  GQNVEMSCAFQSGSASVYLEIQWWFLRGPEDLDPGAEGAGAQVELLPDR

    .....................tLtInsWqyEDsGtYwCmV
90  DPDSDGTKISTVKVQGNDISHKLQISKVRKKDEGLYECRV

In another example, a search was performed using the amino acid sequence of SEQ ID NO:7 against the HMM database resulting in the identification of an Ig-like domain in the amino acid sequence of SEQ ID NO:7 and a score of 22.43 against the Ig family HMM Accession PF00047. The results of the search are set forth below.

Score: 22.43 SEQ ID NO:2: aa40-128 HMM: aa1-47 (SEQ ID NO:13)

GqsVTLTCmVs.fhPpdYt.IwWY.rNgqpi..................
40 GQNVEMSCAFQSGSASVYLEIQWWFLRGPEDLEQGTEAAGSQVELLPDR

.....................tLtInsWqyEDsGtYwCmV
89 DPDNDGTKISTVKVGGNDISHKLQISKVRKKDEGLYECRV

Accordingly, in one embodiment of the invention, a BDSF protein is a human BDSF- protein having an Ig-like domain at about amino acids 41-129 of SEQ ID NO:2. Such an Ig-like domain has the amino acid sequence:

GQNVEMSCAFQSGSASVYLEIQWWFLRGPEDLDPGAEGAGAQVELLPDR
DPDSDGTKISTVKVQGNDISHKLQISKVRKKDEGLYECRV (SEQ ID
NO:11)

Accordingly, BDSF family members having at least 50-60% homology, preferably about 60-70%, more preferably about 70-80%, or about 80-90% homology with the Ig-like domain of human BDSF-1 *(e.g.,* SEQ ID NO:11) are within the scope of the invention.

In yet another embodiment of the invention, a BDSF protein is a murine BDSF-1 protein having an Ig-like domain at about amino acids 40-128 of SEQ ID NO:7. Such an Ig-like domain has the amino acid sequence:

GQNVEMSCAFQSGSASVYLEIQWWFLRGPEDLEQGTEAAGSQVELLPDR
DPDNDGTKISTVKVGGNDISHKLQISKVRKKDEGLYECRV (SEQ ID
NO:12)

Accordingly, a BDSF family member having at least 50-60% homology, preferably about 60-70%, more preferably about 70-80%, or about 80-90% homology with the Ig-like domain of murine BDSF- (*e.g*., SEQ ID NO: 12) is within the scope of the invention. Description of the Pfam database can be found in Sonhammer et al. (1997) Proteins 28(3)405-420, and description of HMMs can be found, for example, in Gribskov et al. (1990) Meth. Enzymol. 183:146-159; Gribskov et al. (1987) Proc. Natl. Acad. Sci. USA 84:4355-4358; Krogh et al.(1994) J. Mol. Biol. 235:1501-1531; and Stultz et al.(1993) Protein Sci. 2:305-314, the contents of which are incorporated by reference.

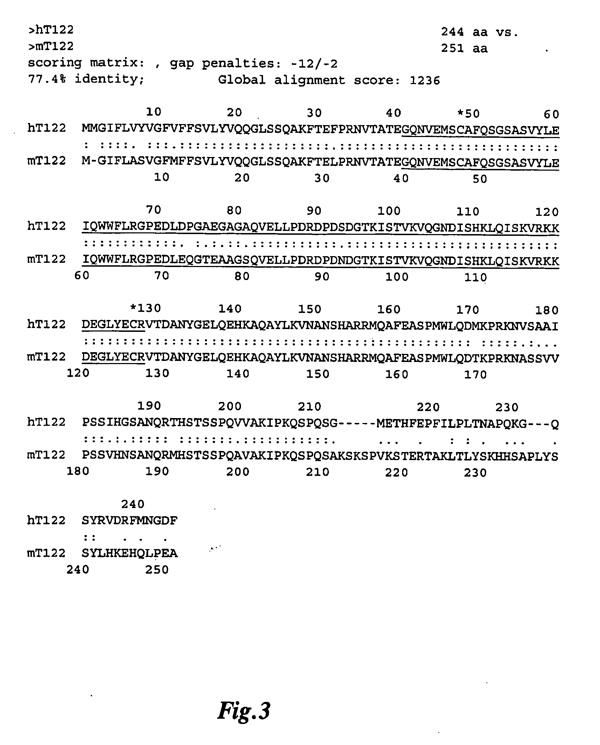

An Ig-like domain further contains at least one, preferably two, cysteine residues which are conserved between BDSF molecules. Preferably, the Ig-like domain of a protein, preferably a BDSF protein, has cysteine residues which are located in the same or similar positions as cysteine residues in other BDSF protein family members. For example, when a BDSF protein of the invention is aligned with a BDSF family member for purposes of comparison (see *e.g.,* Fig. 3) preferred cysteine residues of the invention are those in which cysteine residues in the amino acid sequence of BDSF are located in the same or similar position as the cysteine residues in other BDSF family members. As an illustrative embodiment, Fig. 3 shows cysteine residues located in the same or similar positions of the human BDSF protein (corresponding to SEQ ID NO:2) and murine BDSF protein (corresponding to SEQ ID NO:7) in the following locations: amino acid number 48 of human BDSF and amino acid number 47 of murine BDSF; and amino acid number 127 of human BDSF and amino acid number 126 of murine BDSF.

In another embodiment of the invention, a BDSF protein has an Ig-like domain and a signal sequence. As used herein, a "signal sequence" refers to a peptide of about 20-30 amino acid residues in length which occurs at the N-terminus of secretory and integral membrane proteins and which contains a majority of hydrophobic amino acid residues. For example, a signal sequence contains at least about 15-45 amino acid residues, preferably about 20-40 amino acid residues, more preferably about 20-30 amino acid residues, and more preferably about 24-28 amino acid residues, and has at least about 40-70%, preferably about 50-65%, and more preferably about 55-60% hydrophobic amino acid residues (*e.g.*, Alanine, Valine, Leucine, Isoleucine, Phenylalanine, Tyrosine, Tryptophan, or Proline). Such a "signal sequence", also referred to in the art as a "signal peptide", serves to direct a protein containing such a sequence to a lipid bilayer. For example, in one embodiment, a BDSF protein contains a signal sequence of about amino acids 1-25 of SEQ ID NO:2, or a signal sequence of about amino acids 1-24 of SEQ ID NO:7.

As used interchangeably herein, a "BDSF activity", "biological activity of BDSF" or "functional activity of BDSF", refers to an activity exerted by a BDSF protein, polypeptide or nucleic acid molecule as determined *in vivo,* or *in vitro,* according to standard techniques. In one embodiment, a BDSF activity is a direct activity, such as an association with a BDSF-target molecule. As used herein, a "target molecule" is a molecule with which a BDSF protein binds or interacts in nature (*e.g.*, a BDSF receptor), such that BDSF-mediated function is achieved. A BDSF target molecule can be a BDSF protein or polypeptide of the present invention or a non-BDSF molecule. Alternatively, a BDSF activity is an indirect activity, such as an activity mediated by interaction of the BDSF protein with a BDSF target molecule such that the target molecule modulates a downstream cellular activity (*e.g.*, interaction of an BDSF molecule with a BDSF target molecule can modulate the activity of that target molecule on an intracellular signaling pathway). In a preferred embodiment, a BDSF activity is at least one or more of the following activities: (i) interaction of a BDSF protein in the extracellular milieu with a non-BDSF protein molecule on the surface of the same cell which secreted the BDSF protein molecule; (ii) interaction of a BDSF protein in the extracellular milieu with a non-BDSF protein molecule on the surface of a different cell from that which secreted the BDSF protein molecule; (iii) complex formation between a BDSF protein and a BDSF receptor; (iv) complex formation between a BDSF protein and non-BDSF receptor; and (v) interaction of a BDSF protein with a second protein in the extracellular milieu. In yet another preferred embodiment, a BDSF activity is at least one or more of the following activities: (1) modulation of cellular signal transduction, either *in vitro* or *in vivo;* (2) modulation of protein:protein interactions, either *in vitro* or *in vivo;* (3) regulation of cellular proliferation; or (4) regulation of cellular differentiation.

Accordingly, another embodiment of the invention features isolated BDSF proteins and polypeptides having a BDSF activity. Preferred proteins are BDSF proteins having an Ig-like domain, and, preferably, a BDSF activity. In another preferred embodiment, the isolated protein further comprises a signal sequence. In still another preferred embodiment, the isolated protein is a BDSF protein having an Ig-like domain, a BDSF activity, preferably an amino acid sequence sufficiently homologous to an amino acid sequence of SEQ ID NO:2 or SEQ ID NO:7, and optionally a signal sequence and/or propeptide.

The human BDSF-1 cDNA, which is approximately 1119 nucleotides in length, encodes a protein which is approximately 244 amino acid residues in length. The human BDSF-1 protein has an Ig-like domain. An Ig-like domain includes, for example, about amino acids 41-129 of SEQ ID NO:2. The Ig-like domain further contains at least about two conserved cysteine residues. Cysteine residues can be found at least about at amino acids 48 and 127 of SEQ ID NO:2. The human BDSF-1 protein is predicted to be a secreted protein which contains a signal sequence at about amino acids 1-25 of SEQ ID NO:2. The prediction of such a signal peptide can be made, for example, utilizing the computer algorithm SIGNALP (Henrik, et al. (1997) Protein Engineering 10:1-6).

The murine BDSF-1 cDNA, which is approximately 3196 nucleotides in length, encodes a protein which is approximately 251 amino acid residues in length. The murine BDSF-1 protein has an Ig-like domain. An Ig-like domain includes, for example, about amino acids 40-128 of SEQ ID NO:7. The Ig-like domain further contains at least about two conserved cysteine residues. Cysteine residues can be found at least about at amino acids 47 and 126 of SEQ ID NO:7. The murine BDSF-1 protein is predicted to be a secreted protein which contains a signal sequence at about amino acids 1-24 of SEQ ID NO:7.

Analysis of human BDSF expression demonstrated highest levels of BDSF mRNA in adult brain tissue, in particular, in the sub-regions of the brain including amygdala, caudate nucleus, hippocampus, substania nigra, sub-thalamate nucleus and thalamus, but not in corpus callosum. Human BDSF maps to hu7p12-14 between markers WI-967 and WI-4253 close to the CMT2D (Charcot-Marie-Tooth neuropathy) locus. The syntenic chromosome in mouse, mol 1, is in close proximity with the mouse known genes: egfr (epidermal growth factor receptor), ddc (dopa decarboxylase) and cobl (cordon bleu)). BDSF also maps close to the following human genes: ADCYAP1R1 (adenylate cyclase activating polypeptide 1), AMPH (amphiphysin), BLVRA (biliverdin reductase A), OGDH (oxoglutarate dehydrogenase), OCM (oncomodulin) and EGFR (epidermal growth factor receptor).

*In situ* analysis of adult mouse brain demonstrated high punctate expression in the cortex, hypothalamus, hippocampus (including, but not restricted to, granule cells) and hind brain. Expression in the cerebellum is limited to purkinje cells. Embryonic sagital sections (day 14.5 - 15.5) showed expression in developing brain and spinal cord (*e.g.*, in the ependyma of the brain and the primordium of the lower incisor tooth).

Accordingly, it is postulated that regulation and/or modulation of BDSF (*e.g.*, using BDSF nucleic acid molecules, polypeptides, antibodies and/or BDSF modulators) can play an important role in the following: (1) regulation of neuronal proliferation and/or differentiation; (2) modulation of neuronal signaling; (3) regulation of neurodegeneration (*e.g.*, modulation of apoptotic degeneration and/or neuron atrophy); and (4) regulation of neurotoxicity. Moreover, BDSF molecules and/or modulators can provide novel therapeutic approaches for treatment of disorders and/or diseases including (1) neurodegenerative diseases (*e.g.*, Alzheimer's disease, dementias related to Alzheimer's disease (such as Pick's disease), Huntington's Disease, Parkinson's and other Lewy diffuse body diseases, multiple sclerosis, amyotrophic lateral sclerosis, progressive supranuclear palsy, epilepsy, Jakob-Creutzfieldt disease, or AIDS related dementia; (2) peripheral neuropathies and/or demylinopathies; and (3) nervous system-related disorders and/or diseases including cognitive disorders, *e.g.*, memory and learning disorders, such as amnesia, apraxia, agnosia, amnestic dysnomia, amnestic spatial disorientation, Kluver-Bucy syndrome, Alzheimer's related memory loss (Eglen R.M. (1996) Pharmacol. and Toxicol. 78(2):59-68; Perry E.K. (1995) Brain and Cognition 28(3):240-58) and learning disability; disorders affecting consciousness, *e.g.*, visual hallucinations, perceptual disturbances, or delerium associated with Lewy body dementia; schitzo-effective disorders (Dean B. (1996) Mol. Psychiatry 1(1):54-8), schizophrenia with mood swings (Bymaster F.P. (1997) J. Clin. Psychiatry 58 (suppl. 10):28-36; Yeomans J.S. (1995) Neuropharmacol. 12(1):3-16; Reimann D. (1994) J. Psychiatric Res. 28(3):195-210), depressive illness (primary or secondary); affective disorders (Janowsky D.S. (1994) Am. J. Med Genetics 54(4):335-44); sleep disorders (Kimura F. (1997) J. Neurophysiol. 77(2):709-16), *e.g.,* REM sleep abnormalities in patients suffering from, for example, depression (Riemann D. (1994) J. Psychosomatic Res. 38 Suppl. 1:15-25; Bourgin P. (1995) Neuroreport 6(3): 532-6), paradoxical sleep abnormalities (Sakai K. (1997) Eur. J. Neuroscience 9(3):415-23), sleep-wakefulness, and body temperature or respiratory depression abnormalities during sleep (Shuman S.L. (1995) Am. J. Physiol. 269(2 Pt 2):R308-17; Mallick B.N. (1997) Brain Res. 750(1-2):311-7). Other examples of nervous system related disorders include disorders affecting pain generation mechanisms, *e.g.*, pain related to irritable bowel syndrome (Mitch C.H. (1997) J. Med. Chem. 40(4):538-46; Shannon H.E. (1997) J. Pharmac. and Exp. Therapeutics 281(2):884-94; Bouaziz H. (1995) Anesthesia and Analgesia 80(6):1140-4; or Guimaraes A.P. (1994) Brain Res. 647(2):220-30) or chest pain; movement disorders (Monassi C.R. (1997) Physiol. and Behav. 62(1):53-9), *e.g.,* Parkinson's disease related movement disorders (Finn M. (1997) Pharmacol. Biochem. & Behavior 57(1-2):243-9; Mayorga A.J. (1997) Pharmacol. Biochem. & Behavior 56(2):273-9); eating disorders, *e.g.*, insulin hypersecretion related obesity (Maccario M. (1997) J. Endocrinol. Invest. 20(1):8-12; Premawardhana L.D. (1994) Clin. Endocrinol. 40(5): 617-21); or drinking disorders, *e.g.*, diabetic polydipsia (Murzi E. (1997) Brain Res. 752(1-2):184-8; Yang X. (1994) Pharmacol. Biochem. & Behavior 49(1):1-6).

### The GPCR Family

The family of G protein-coupled receptors (GPCRs), to which the STMST proteins of the present invention bear significant homology, comprise an N-terminal extracellular domain, seven transmembrane domains (also referred to as membrane-spanning domains), three extracellular domains (also referred to as extracellular loops), three cytoplasmic domains (also referred to as cytoplasmic loops), and a C-terminal cytoplasmic domain (also referred to as a cytoplasmic tail). Members of the GPCR family also share certain conserved amino acid residues, some of which have been determined to be critical to receptor function and/or G protein signaling. For example, GPCRs contain the following features: a conserved asparagine residue in the first transmembrane domain; a cysteine residue in the first extracellular loop which is believed to form a disulfide bond with a conserved cysteine residue in the second extracellular loop; a conserved leucine and aspartate residue in the second transmembrane domain; an aspartate-arginine-tyrosine motif (DRY motif) at the interface of the third transmembrane domain and the second cytoplasmic loop of which the arginine residue is almost invariant (members of the rhodopsin subfamily of GPCRs comprise a histidine-arginine-methionine motif (HRM motif) as compared to a DRY motif); a conserved tryptophan and proline residue in the fourth transmembrane domain; a conserved phenylalanine residue which is commonly found as part of the motif FXXCXXP; and a conserved leucine residue in the seventh transmembrane domain which is commonly found as part of the motif DPXXY or NPXXY. Table I depicts an alignment of the transmembrane domains of 5 GPCRs. The conserved residues described herein are indicated by asterices. An alignment of the transmembrane domains of 44 representative GPCRs can be found at http://mgdkkl.nidll.nih.gov:8000/extended.html.

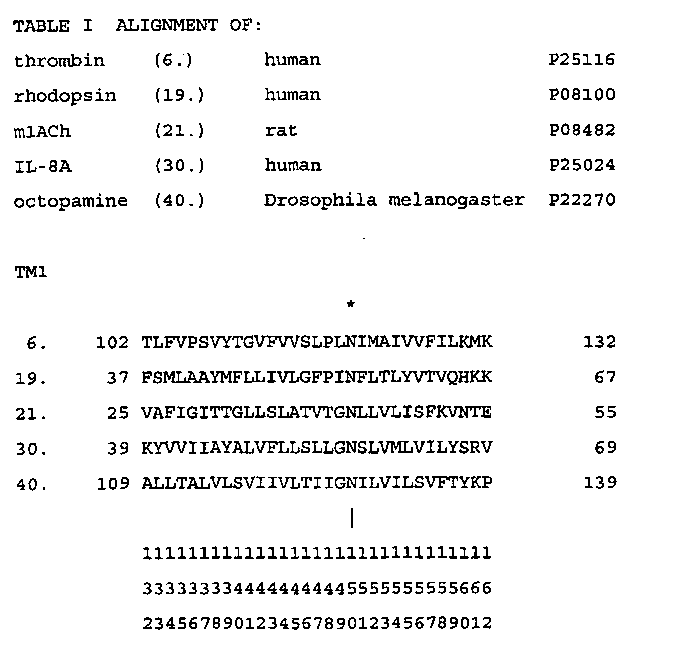

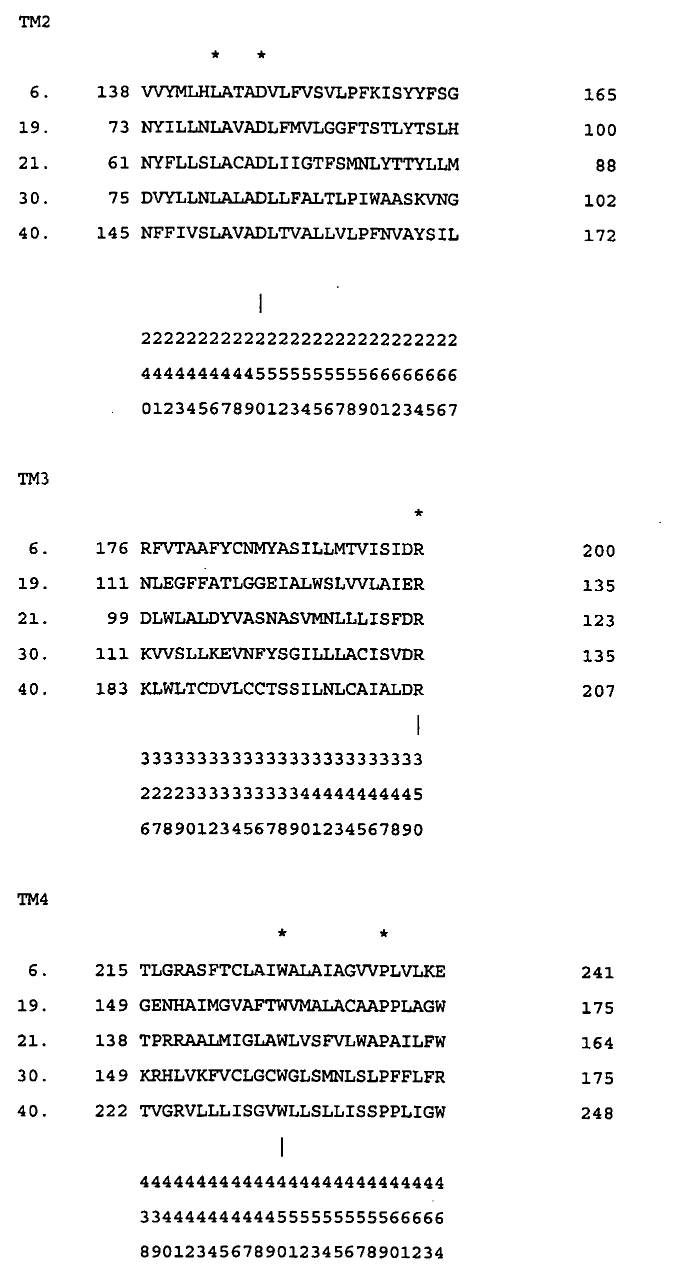

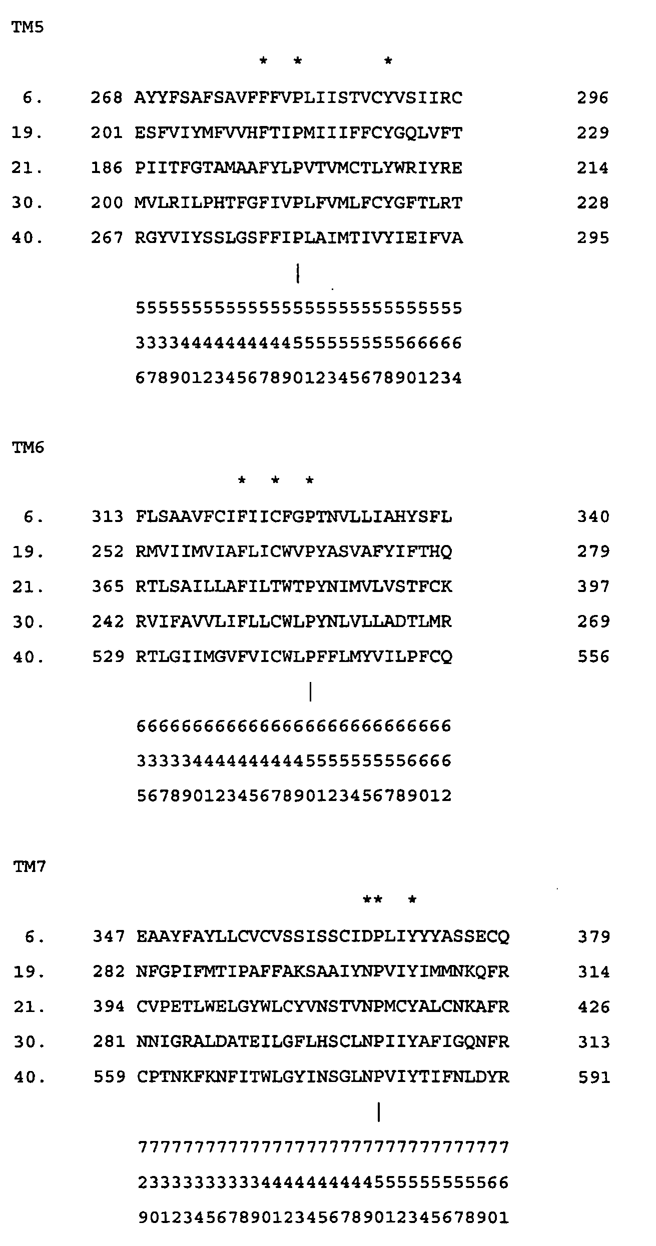

The amino acid sequences of thrombin receptor (Accession No. P25116), rhodopsin receptor (Accession No. P08100), m1ACh receptor (Accession No. P08482), IL-8A receptor (Accession No. P25024), octopamine receptor (Accession No. P22270), are set forth as SEQ ID NO:24, SEQ ID NO:25, SEQ ID NO:26, SEQ ID NO:27, and SEQ ID NO:145, respectively. Accordingly, GPCR-like proteins such as the STMST proteins of the present invention contain a siginificant number of structural characteristics of the GPCR family. For instance, the STMSTs of the present invention contain conserved cysteines found in the first 2 extracellular loops (prior to the third and fifth transmembrane domains) of most GPCRs (cys 83 and cys 161 of SEQ ID NO:15 or SEQ ID NO:18). A highly conserved asparagine residue in the first transmembrane domain is present (asn25 in SEQ ID NO:15 or SEQ ID NO:18). Transmembrane domain two of the STMST proteins contains a highly conserved leucine (leu49 of SEQ ID NO:15 or SEQ ID NO:18). The two cysteine residues are believed to form a disulfide bond that stabilizes the functional protein structure. A highly conserved tryptophan and proline in the fourth transmembrane domain of the STMST proteins is present (trp135 and pro 145 of SEQ ID NO:15 or SEQ ID NO:18). The third cytoplasmic loop contains 49 amino acid residues and is thus the longest cytoplasmic loop of the three, characteristic of G protein coupled receptors. Moreover, a highly conserved proline in the sixth transmembrane domain is present (pro260 of SEQ ID NO: 15 and SEQ ID NO:18). The proline residues in the fourth, fifth, sixth, and seventh transmembrane domains are thought to introduce kinks in the alpha-helices and may be important in the formation of the ligand binding pocket. Furthermore, the conserved (in the second cytoplasmic loop) HRM motif found in almost all Rhodopsin family GPCRs is present in the STMST proteins of the instant invention (his107, arg108, met109 of SEQ ID NO:15 or SEQ ID NO:18). (The arginine of the HRM sequence is thought to be the most important amino acid in GPCRs and is invariant). Moreover, an almost invariant proline is present in the seventh transmembrane domain of STMST-2 (pro294 of SEQ ID NO:18).

In one embodiment, the STMST proteins of the present invention are proteins having an amino acid sequence of about 150-450, preferably about 200-400, more preferably about 225-375, more preferably about 250-350, or about 275-325 amino acids in length. In another embodiment, the STMST proteins of the present invention are proteins having an amino acid sequence of about 450-750, preferably about 500-700, more preferably about 525-675, even more preferably about 550-650, and even more preferably about 575-625 amino acid residues in length. In one embodiment, the STMST proteins of the present invention contain at least one transmembrane domain. As used herein, the term "transmembrane domain" includes an amino acid sequence having at least about 10, preferably about 13, preferably about 16, more preferably about 19, 21, 23, 25, 30, 35 or 40 amino acid residues, of which at least about 50-60%, 60-70%, preferably about 70-80% more preferably about 80-90%, or about 90-95% of the amino acid residues contain non-polar side chains, for example, alanine, valine, leucine, isoleucine, proline, phenylalanine, tryptophan, and methionine. A transmembrane domain is lipophillic in nature. For example, a transmembrane domain can be found at about amino acids 11-34 of SEQ ID NO: 15 or SEQ ID NO: 18. In a preferred embodiment, an STMST protein of the present invention has more than one transmembrane domain, preferably 2, 3, 4, 5, 6, or 7 transmembrane domains. For example, transmembrane domains can be found at about amino acids 11-34, 44-67, 85-106, 127-149, 172-196, and 244-262 of SEQ ID NO: 15 as well as at 11-34, 44-67, 85-106, 127-149, 172-196, 245-269, and 277-300 of SEQ ID NO:18. In a particularly preferred embodiment, an STMST protein of the present invention has 7 transmembrane domains.

In another embodiment, an STMST family member is identified based on the presence of at least one cytoplasmic loop, also referred to herein as a cytoplasmic domain. In another embodiment, an STMST family member is identified based on the presence of at least one extracellular loop. As defined herein, the term "loop" includes an amino acid sequence having a length of at least about 4, preferably about 5-10, preferably about 10-20, and more preferably about 20-30, 30-40, 40-50, 50-60, 60-70, 70-80, 80-90, 90-100, or 100-150 amino acid residues, and has an amino acid sequence that connects two transmembrane domains within a protein or polypeptide. Accordingly, the N-terminal amino acid of a loop is adjacent to a C-terminal amino acid of a transmembrane domain in a naturally-occurring GPCR or GPCR-like molecule, and the C-terminal amino acid of a loop is adjacent to an N-terminal amino acid of a transmembrane domain in a naturally-occurring GPCR or GPCR-like molecule.

As used herein, a "cytoplasmic loop" includes an amino acid sequence located within a cell or within the cytoplasm of a cell. For example, a cytoplasmic loop is found at about amino acids 35-43, 107-126, and 197-243 of SEQ ID NO:15, or alternatively, at about amino acid residues 35-43, 107-126, and 197-244 of SEQ ID NO:18. Also as used herein, an "extracellular loop" includes an amino acid sequence located outside of a cell, or extracellularly. For example, an extracellular loop can be found at about amino acid residues 68-84 and 150-171 of SEQ ID NO:15, or alternatively, at about amino acid residues 86-84, 150-171, or 270-276 of SEQ ID NO:18.

In another embodiment of the invention, an STMST family member is identified based on the presence of a "C-terminal cytoplasmic domain", also referred to herein as a C-terminal cytoplasmic tail, in the sequence of the protein. As used herein, a "C-terminal cytoplasmic domain" includes an amino acid sequence having a length of at least about 10, preferably about 10-25, more preferably about 25-50, more preferably about 50-75, even more preferably about 75-100, 100-150, 150-200, 200-250, 250-300, 300-400, 400-500, or 500-600 amino acid resudues and is located within a cell or within the cytoplasm of a cell. Accordingly, the N-terminal amino acid residue of a "C-terminal cytoplasmic domain" is adjacent to a C-terminal amino acid residue of a transmembrane domain in a naturally-occurring GPCR or GPCR-like protein. For example, a C-terminal cytoplasmic domain is found at about amino acid residues 301-609 of SEQ ID NO:18.

In another embodiment, an STMST family member is identified based on the presence of an "N-terminal extracellular domain", also referred to herein as an N-terminal extracellular loop in the amino acid sequence of the protein. As used herein, an "N-terminal extracellular domain" includes an amino acid sequence having about 1-500, preferably about 1-400, more preferably about 1-300, more preferably about 1-200, even more preferably about 1-100, and even more preferably about 1-50, 1-25, or 1-10 amino acid residues in length and is located outside of a cell or extracellularly. The C-terminal amino acid residue of a "N-terminal extracellular domain" is adjacent to an N-terminal amino acid residue of a transmembrane domain in a naturally-occurring GPCR or GPCR-like protein. For example, an N-terminal cytoplasmic domain is found at about amino acid residues 1-10 of SEQ ID NO:15 or SEQ ID NO:18.

Accordingly in one embodiment of the invention, an STMST family member includes at least one, preferably 6 or 7, transmembrane domains and and/or at least one cytoplasmic loop, and/or at least one extracellular loop. In another embodiment, the STMST family member further includes an N-terminal extracellular domain and/or a C-terminal cytoplasmic domain. In another embodiment, the STMST family member can include six transmembrane domains, three cytoplasmic loops, and two extracellular loops, or can include six transmembrane domains, three extracellular loops, and 2 cytoplasmic loops. The former embodiment can further include an N-terminal extracellular domain. The latter embodiment can further include a C-terminal cytoplasmic domain. In another embodiment, the STMST family member can include seven transmembrane domains, three cytoplasmic loops, and three extracellular loops and can further include an N-terminal extracellular domain or a C-terminal cytoplasmic domain.

In another embodiment, an STMST family member is identified based on the presence of at least one "7 transmembrane receptor profile", also referred to as a "Rhodopsin family sequence profile", in the protein or corresponding nucleic acid molecule. As used herein, the term "7 transmembrane receptor profile" includes an amino acid sequence having at least about 100-400, preferably about 150-350, more preferably about 200-300 amino acid residues, or at least about 250-275 amino acids in length and having a bit score for the alignment of the sequence to the 7tm_1 family Hidden Markov Model (HMM) of at least 20, preferably 20-30, more preferably 30-40, more preferably 40-50, 50-75, 75-100,100-200 or greater. The 7tm_1 family HMM has been assigned the PFAM Accession PF00001 (http://genome.wustl.edu/Pfam/WWWdata/7tm_1.html).

To identify the presence of a 7 transmembrane receptor profile in an STMST family member, the amino acid sequence of the protein family member is searched against a database of HMMs (*e*.*g*., the Pfam database, release 2.1) using the default parameters (http://www.sanger.ac.uk/Software/Pfam/HMM_search). For example, the hmmsf program, which is available as part of the HMMER package of search programs, is a family specific default program for PF00001 and score of 15 is the default threshold score for determining a hit. For example, a search using the amino acid sequence of SEQ ID NO:15 was performed against the HMM database resulting in the identification of a 7 TM receptor profile in the amino acid sequence of SEQ ID NO:15. The results of the search are set forth below.

Score:44.14    SEQ ID NO:15: aa24-191    HMM: aa1-174

GNiLVIWvIcRyRRMRTPMNYFIvNLAvADLLFslft.MPFWMvYyvMq
24 ANAWGILSVGAKQKKWKPLEFLLCTLAATHMLN-VAVPIATYSVVQLRR

gRWpFGdfMCrIWmYFDYMNMYASIFfLTcISIDRYLWAICHPMrYmR
72 QRPDFEWNEGLCKVFVSTFYTLTLATCFSVTSLSYHRMWMVCWPVNYRL

WMTpRHRAWvMIiiIWvMSFlISMPPFLMFrWstyrDEneWNmTWCmIyD
121 SNAKK-QAVHTVMGIWMVSFILSALPA-VG-W-HDTSERFYTHG-CRFIV

WPewMWrWYvILmtiimgFYIPMiIMlF
166 AEIGLGFGVCFLLLV-GGSVA-MGVICT

Likewise, a search using the amino acid sequence of SEQ ID NO:18 results in an identical hit with a score of 44.14 against the 7tm_1 family HMM Accession PF00001. Accordingly, in one embodiment of the invention, an STMST protein is a human STMST-1 or a human STMST-2 protein having a 7 transmembrane receptor profile at about amino acids 24-191 of SEQ ID NO:15 or SEQ ID NO:18, respectively. Such a 7 transmembrane receptor profile has the amino acid sequence:

ANAWGILSVGAKQKKWKPLEFLLCTLAATHMLNVAVPIATYSVVQLRRQR
PDFEWNEGLCKVFVSTFYTLTLATCFSVTSLSYHRMWMVCWPVNYRLSNA
KKQAVHTVMGIWMVSFILSALPAVGWHDTSERFYTHGCRFIVAEIGLGFG
VCFLLLVGGSVAMGVICT. (SEQ ID NO:22)

Accordingly, STMST family members having at least 50-60% homology, preferably about 60-70%, more preferably about 70-80%, or about 80-90% homology with the 7 transmembrane receptor profile of human STMST-1 or STMST-2 (*e.g.*, SEQ ID NO:22) are within the scope of the invention.

In another embodiment, an STMST family member is identified based on the presence of a "spectrin α-chain profile "in the protein or corresponding nucleic acid molecule. As used herein, the term "spectrin α-chain profile" includes a protein domain having an amino acid sequence of about 50-250, preferably about 75-225, more preferably about 100-200 amino acid residues, or about 125-175 amino acids and having a bit score for the alignment of the sequence to the spectrin family (HMM) of at least 7, preferably 8-10, more preferably 10-30, more preferably 30-50, even more preferably 50-75, 75-100, 100-200 or greater. The spectrin family HMM has been assigned the PFAM Accession PF00435 (http://genome.wustl.edu/Pfam/WWWdata/spectrin.html).

To identify the presence of a spectrin alpha chain profile in a STMST family member, make the determination that a protein of interest has a particular profile, the amino acid sequence of the protein is searched against a database of HMMs (*e.g.,* the Pfam database, release 2.1) using the default parameters (http://www.sanger.ac.uk/Software/Pfam/HMM_search). For example, the hmmsf program, which is available as part of the HMMER package of search programs, is a family specific default program for PF00435 and a score of 15 is the default threshold score for determining a hit. Alternatively, the threshold score for determining a hit can be lowered (*e.g.*, to 8 bits). A description of the Pfam database can be found in Sonhammer et al. (1997) Proteins 28(3)405-420 and a detailed description of HMMs can be found, for example, in Gribskov et al.(1990) Meth. Enzymol. 183:146-159; Gribskov et al.(1987) Proc. Natl. Acad. Sci. USA 84:4355-4358; Krogh et al. (1994) J. Mol. Biol. 235:1501-1531; and Stultz et al. (1993) Protein Sci. 2:305-314, the contents of which are incorporated herein by reference. A search was performed against the HMM database resulting in the identification of a spectrin alpha chain profile in the amino acid sequence of SEQ ID NO:15. The results of the search are set forth below.

Score:8.78     SEQ ID NO:15: aa266-372  HMM: aa1-106

IqeRMnElndRWerLkelMeqRRQMLedSmrlQQFfRDmDEeEsWInEK
266 FSSLRADASAPWMALCVLWCSVAQALLLPVFLWACDRYRADLKAVREKC

EqilnSDDYGkDLtsVQnLlkKHQaFEaDIaAHE.dRIqalnefaqqLIq
315 MALMANDEESDDETSLEGGISPDLVLERSLDYGYGGDFVALDRMAKYEIS

enHYasEe
365 ALEGGLPQ

All amino acids are described using universal single letter abbreviations according to these motifs.

Accordingly, in one embodiment, an STMST protein is human STMST-2 protein which includes a spectrin a-chain profile at about amino acids 266-372 of SEQ ID NO: 18. Such a spectrin a-chain profile has the amino acid sequence: 
FSSLRADASAPWMALCVLWCSVAQALLLPVFLWACDRYRADLKAV
REKCMALMANDEESDDETSLEGGISPDLVLERSLDYGYGGDFVAL
DRMAKYEISALEGGLPQ (SEQ ID NO:23).
 Accordingly, STMST family members having at least 50-60% homology, preferably about 60-70%, more preferably about 70-80%, or about 80-90% homology with a spectrin α-chain profile of human STMST-2 (*e.g.*, SEQ ID NO:23) are within the scope of the invention.

In another embodiment, an STMST protein includes at least a spectrin α-chain profile. In another embodiment, an STMST protein includes a spectrin α-chain profile and a 7 transmembrane receptor profile. In another embodiment, an STMST protein is human STMST-2 which includes a spectrin α-chain profile having about amino acids 266-372 of SEQ ID NO:18. In yet another embodiment, an STMST protein is human STMST-2 which includes a 7 transmembrane receptor profile having about amino acids 24-191 of SEQ ID NO:18 and a spectrin α-chain profile having about amino acids 266-372 of SEQ ID N0:18.

As used interchangeably herein, an "STMST activity", "biological activity of STMST" or "functional activity of STMST", refers to an activity exerted by an STMST protein, polypeptide or nucleic acid molecule on an STMST responsive cell as determined *in vivo,* or *in vitro,* according to standard techniques. In one embodiment, an STMST activity is a direct activity, such as an association with an STMST-traget molecule. As used herein, a "target molecule" or "binding partner" is a molecule with which an STMST protein binds or interacts in nature, such that STMST-mediated function is acheived. An STMST target molecule can be a non-STMST molecule or an STMST protein or polypeptide of the present invention. In an exemplary embodiment, an STMST target molecule is an STMST ligand. Alternatively, an STMST activity is an indirect activity, such as a cellular signaling activity mediated by interaction of the STMST protein with an STMST ligand.

In a preferred embodiment, an STMST activity is at least one or more of the following activities: (i) interaction of an STMST protein with soluble STMST ligand; (ii) interaction of an STMST protein with a membrane-bound non-STMST protein; (iii) interaction of an STMST protein with an intracellular protein (e.g., an intracellular enzyme or signal transduction molecule); and (iv) indirect interaction of an STMST protein with an intracellular protein (e.g., a downstream signal transduction molecule.

In yet another preferred embodiment, an STMST activity is at least one or more of the following activities: (1) modulation of cellular signal transduction, either *in vitro* or *in vivo;* (2) regulation of gene transcription in a cell expressing an STMST protein; (3) regulation of gene transcription in a cell expressing an STMST protein, wherein said cell is involved inflammation; (4) regulation of cellular proliferation; (5) regulation of cellular differentiation; (6) regulation of develpoment; (7) regulation of cell death; (8) regulation of regulation of inflammation; (9) regulation of respiratory cell function (e.g., asthma); (10) regulation of actin binding; (11) regulation of cytoskeletal attachment; and (12) regulation of chemotaxis, trafficking and/or migration.

Accordingly, another embodiment of the invention features isolated STMST proteins and polypeptides having an STMST activity. Preferred STMST proteins have at least one transmembrane domain and an STMST activity. In a preferred embodiment, an STMST protein has a 7 transmembrane receptor profile and an STMST activity. In another preferred embodiment, an STMST protein has a spectrin α-chain profile and an STMST activity. In still another preferred embodiment, an STMST protein has a 7 transmembrane receptor profile, a spectrin α-chain profile, and STMST activity. In still another preferred embodiment, an STMST protein has a 7 transmembrane receptor profile, a spectrin α-chain profile, an STMST activity, and an amino acid sequence sufficiently homologous to an amino acid sequence of SEQ ID NO:15, SEQ ID NO:18, SEQ ID NO:21, or SEQ ID NO:24.

The human STMST-1 cDNA, which is approximately 2915 nucleotides in length, encodes a protein which is approximately 297 amino acid residues in length. The human STMST-1 protein contains 6 transmembrane domains at about amino acids 11-34, 44-67, 85-106, 127-149, 172-196, and 244-262 of SEQ ID NO: 1 The human STMST-1 protein further contains a 7 transmembrane receptor profile. The 7 transmembrane receptor profile can be found at least, for example, from about amino acids 24-191 of SEQ ID NO:15.

The human STMST-2 cDNA, which is approximately 4166 nucleotides in length, encodes approximately 609 amino acid residues of the human STMST-1 protein. The human STMST-2 protein contains 7 transmembrane domains at about amino acids 11-34, 44-67, 85-106, 127-149, 172-196, 245-269, and 277-300 of SEQ ID NO:18. The human STMST-2 protein further contains a 7 transmembrane receptor profile. The 7 transmembrane receptor profile can be found at least, for example, from about amino acids 24-191 of SEQ ID NO:18. Moreover, the human STMST protein contains a spectrin α-chain profile from about amino acids 266-372 of SEQ ID NO:5.

Various aspects of the invention are described in further detail in the following subsections:

### I. Isolated Nucleic Acid Molecules

One aspect of the invention pertains to isolated nucleic acid molecules that encode BDSF proteins or biologically active portions thereof, as well as nucleic acid fragments sufficient for use as hybridization probes to identify BDSF-encoding nucleic acids (*e.g.*, BDSF mRNA) and fragments for use as PCR primers for the amplification or mutation of BDSF nucleic acid molecules. Another aspect of the invention pertains to isolated nucleic acid molecules that encode STMST proteins or biologically active portions thereof, as well as nucleic acid fragments sufficient for use as hybridization probes to identify STMST-encoding nucleic acids (*e.g.*, STMST mRNA) and fragments for use as PCR primers for the amplification or mutation of STMST nucleic acid molecules.

As used herein, the term "nucleic acid molecule" is intended to include DNA molecules (*e.g.*, cDNA or genomic DNA) and RNA molecules (*e.g.*, mRNA) and analogs of the DNA or RNA generated using nucleotide analogs. The nucleic acid molecule can be single-stranded or double-stranded, but preferably is double-stranded DNA.

An "isolated" nucleic acid molecule is one which is separated from other nucleic acid molecules which are present in the natural source of the nucleic acid. Preferably, an "isolated" nucleic acid is free of sequences which naturally flank the nucleic acid (*i.e.*, sequences located at the 5' and 3' ends of the nucleic acid) in the genomic DNA of the organism from which the nucleic acid is derived. For example, in various embodiments, the isolated BDSF nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, preferably a BDSF or STMST nucleic acid molecule, or a portion thereof, can be isolated using standard molecular biology techniques and the sequence information provided herein. Using all or portion of the nucleic acid sequence of a BDSF or STMST nucleic acid molecule of the present invention, as a hybridization probe, BDSF or STMST nucleic acid molecules can be isolated using standard hybridization and cloning techniques (*e.g.*, as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989).

Moreover, a nucleic acid molecule encompassing all or a portion of a BDSF or STMST nucleic acid molecule, can be isolated by the polymerase chain reaction (PCR) using synthetic oligonucleotide primers designed based upon the sequence of the BDSF or STMST nucleic acid molecules described herein.

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to BDSF or STMST nucleotide sequences can be prepared by standard synthetic techniques, *e.g.*, using an automated DNA synthesizer.

### A. BDSF Nucleic Acid Molecules

In a preferred embodiment, an isolated nucleic acid molecule of the invention comprises the nucleotide sequence shown in SEQ ID NO:1. The sequence of SEQ ID NO:1 corresponds to the human BDSF cDNA. This cDNA comprises sequences encoding the human BDSF protein *(i.e.,* "the coding region", from nucleotides 140-871), as well as 5' untranslated sequences (nucleotides 1-139) and 3' untranslated sequences (nucleotides 872-1119). Alternatively, the nucleic acid molecule can comprise only the coding region of SEQ ID NO: (*e.g*., nucleotides 140-871, corresponding to SEQ ID NO:3).

In another preferred embodiment, an isolated nucleic acid molecule of the invention comprises the nucleotide sequence shown in SEQ ID NO:6. The sequence of SEQ ID NO:6 corresponds to the murine BDSF cDNA. This cDNA comprises sequences encoding the murine BDSF protein *(i.e.,* "the coding region", from nucleotides 268-1020), as well as 5' untranslated sequences (nucleotides 1-267) and 3' untranslated sequences (nucleotides 1021-3196). Alternatively, the nucleic acid molecule can comprise only the coding region of SEQ ID NO:6 (*e.g.*, nucleotides 268-1020, corresponding to SEQ ID NO:8).

In yet another preferred embodiment, an isolated nucleic acid molecule of the invention comprises a nucleic acid molecule which is a complement of the nucleotide sequence shown in SEQ ID NO:1, SEQ ID NO:3, SEQ ID NO:4, SEQ ID NO:6, SEQ ID NO:8, SEQ ID NO:9, the nucleotide sequence of the DNA insert of the plasmid deposited with ATCC as Accession Number 98756, or a portion of any of these nucleotide sequences. A nucleic acid molecule which is complementary to the nucleotide sequence shown in SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, is one which is sufficiently complementary to the nucleotide sequence shown in SEQ ID NO:1, SEQ ID NO:3, SEQ ID NO:4, SEQ ID NO:6, SEQ ID N0:8, SEQ ID NO:9, or the nucleotide sequence of the DNA insert of the plasmid deposited with ATCC as Accession Number 98756, such that it can hybridize to the nucleotide sequence shown in SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, thereby forming a stable duplex.

In still another preferred embodiment, an isolated nucleic acid molecule of the present invention comprises a nucleotide sequence which is at least about 30-35%, preferably about 35-40%, more preferably at least about 40-45%, more preferably at least about 45-50%, and even more preferably at least about 50-55%, 55-60%, 60-65%, 65-70%, 70-75%, 75-80%, 80-85%, 85-90%, or 90-95% or more homologous to the nucleotide sequences shown in SEQ ID NO:1, SEQ ID NO:3, SEQ ID NO:4, SEQ ID NO:6, SEQ ID NO:8, SEQ ID NO:9, the nucleotide sequence of the DNA insert of the plasmid deposited with ATCC as Accession Number 98756, or a portion of any of these nucleotide sequences.

Moreover, the nucleic acid molecule of the invention can comprise only a portion of the nucleic acid sequence of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, for example a fragment which can be used as a probe or primer or a fragment encoding a biologically active portion of a BDSF protein. The nucleotide sequence determined from the cloning of the BDSF genes allows for the generation of probes and primers designed for use in identifying and/or cloning other BDSF family members, as well as BDSF homologues from other species. The probe/primer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sense sequence of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, of an anti-sense sequence of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, or of a naturally occurring mutant of SEQ ID NO:1, SEQ ID NO:3, SEQ ID NO:4, SEQ ID NO:6, SEQ ID N0:8, SEQ ID NO:9, or the nucleotide sequence of the DNA insert of the plasmid deposited with ATCC as Accession Number 98756. In an exemplary embodiment, a nucleic acid molecule of the present invention comprises a nucleotide sequence which is about 450, preferably 450-750, more preferably 750-950, more preferably 950-1100, and even more preferably 1100-1150 nucleotides in length and hybridizes under stringent hybridization conditions to a nucleic acid molecule of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756.

Probes based on the BDSF nucleotide sequences can be used to detect transcripts or genomic sequences encoding the same or homologous proteins. In preferred embodiments, the probe further comprises a label group attached thereto, *e.g.*, the label group can be a radioisotope, a fluorescent compound, an enzyme, or an enzyme co-factor. Such probes can be used as a part of a diagnostic test kit for identifying cells or tissue which misexpress a BDSF protein, such as by measuring a level of a BDSF-encoding nucleic acid in a sample of cells from a subject *e.g.*, detecting BDSF mRNA levels or determining whether a genomic BDSF gene has been mutated or deleted.

A nucleic acid fragment encoding a "biologically active portion of a BDSF protein" can be prepared by isolating a portion of the nucleotide sequence of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, which encodes a polypeptide having a BDSF biological activity (the biological activities of the BDSF proteins have previously been described), expressing the encoded portion of the BDSF protein (*e.g.*, by recombinant expression *in vitro)* and assessing the activity of the encoded portion of the BDSF protein.

The invention further encompasses nucleic acid molecules that differ from the nucleotide sequence shown in SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, due to degeneracy of the genetic code and thus encode the same BDSF proteins as those encoded by the nucleotide sequence shown in SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756. In another embodiment, an isolated nucleic acid molecule of the invention has a nucleotide sequence encoding a protein having an amino acid sequence shown in SEQ ID NO:2 or SEQ ID NO:7.

In addition to the BDSF nucleotide sequences shown in SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, it will be appreciated by those skilled in the art that DNA sequence polymorphisms that lead to changes in the amino acid sequences of the BDSF proteins may exist within a population (*e.g.*, the human population). Such genetic polymorphism in the BDSF genes may exist among individuals within a population due to natural allelic variation. As used herein, the terms "gene" and "recombinant gene" refer to nucleic acid molecules comprising an open reading frame encoding a BDSF protein (or STMST protein), preferably a mammalian BDSF protein (or STMST protein). Such natural allelic variations can typically result in 1-5% variance in the nucleotide sequence of a BDSF gene (or STMST gene). Any and all such nucleotide variations and resulting amino acid polymorphisms in BDSF genes (and STMST genes) that are the result of natural allelic variation and that do not alter the functional activity of a BDSF protein (or STMST protein, respectively) are intended to be within the scope of the invention.

Moreover, nucleic acid molecules encoding other BDSF family members, and thus which have a nucleotide sequence which differs from the BDSF sequences of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, are intended to be within the scope of the invention. For example, a BDSF cDNA can be identified based on the nucleotide sequence of human BDSF or the nucleotide sequence of murine BDSF. Moreover, nucleic acid molecules encoding BDSF proteins from different species, and thus which have a nucleotide sequence which differs from the human BDSF sequences of SEQ ID NO:1, SEQ ID NO:3, SEQ ID NO:4, or the nucleotide sequence of the DNA insert of the plasmid deposited with ATCC as Accession Number 98756, or which differs from the murine BDSF sequences of SEQ ID NO:6, SEQ ID NO:8 or SEQ ID NO:9 are intended to be within the scope of the invention. For example, a rat BDSF cDNA can be identified based on the nucleotide sequence of a human or murine BDSF.

Nucleic acid molecules corresponding to natural allelic variants and homologues of the BDSF cDNAs of the invention can be isolated based on their homology to the BDSF nucleic acids disclosed herein using the cDNAs disclosed herein, or a portion thereof, as a hybridization probe according to standard hybridization techniques under stringent hybridization conditions.

Accordingly, in another embodiment, an isolated nucleic acid molecule of the invention is at least 15 nucleotides in length and hybridizes under stringent conditions to the nucleic acid molecule comprising the nucleotide sequence of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756. In other embodiment, the nucleic acid is at least 30, 50, 100, 250 or 500 nucleotides in length. As used herein, the term "hybridizes under stringent conditions" is intended to describe conditions for hybridization and washing under which nucleotide sequences at least 60% homologous to each other typically remain hybridized to each other. Preferably, the conditions are such that sequences at least about 70%, more preferably at least about 80%, even more preferably at least about 85% or 90% homologous to each other typically remain hybridized to each other. Such stringent conditions are known to those skilled in the art and can be found in Current Protocols in Molecular Biology, John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule of the invention that hybridizes under stringent conditions to the sequence of SEQ ID NO:1 or SEQ ID NO:6 corresponds to a naturally-occurring nucleic acid molecule. As used herein, a "naturally-occurring" nucleic acid molecule refers to an RNA or DNA molecule having a nucleotide sequence that occurs in nature (*e.g.*, encodes a natural protein).

In addition to naturally-occurring allelic variants of the BDSF sequences that may exist in the population, the skilled artisan will further appreciate that changes can be introduced by mutation into the nucleotide sequences of SEQ ID NO:1, SEQ ID N0:3, SEQ ID NO:4, SEQ ID NO:6, SEQ ID NO:8, SEQ ID NO:9, or the nucleotide sequence of the DNA insert of the plasmid deposited with ATCC as Accession Number 98756, thereby leading to changes in the amino acid sequence of the encoded BDSF proteins, without altering the functional ability of the BDSF proteins. For example, nucleotide substitutions leading to amino acid substitutions at "non-essential" amino acid residues can be made in the sequence of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756. A "non-essential" amino acid residue is a residue that can be altered from the wild-type sequence of BDSF (*e.g.*, the sequence of SEQ ID NO:2 or SEQ ID NO:7) without altering the biological activity, whereas an "essential" amino acid residue is required for biological activity. For example, amino acid residues that are conserved among the BDSF proteins of the present invention, are predicted to be particularly unamenable to alteration (*e.g*., the conserved cysteines set forth in Figure 3). Moreover, amino acid residues that are defined by the Ig-like domain profile sequence are particularly unamenable to alteration. Furthermore, additional amino acid residues that are conserved between the BDSF proteins of the present invention and other BDSF family members.

Accordingly, another aspect of the invention pertains to nucleic acid molecules encoding BDSF proteins that contain changes in amino acid residues that are not essential for activity. Such BDSF proteins differ in amino acid sequence from SEQ ID NO:2 or SEQ ID NO:7 yet retain biological activity. In one embodiment, the isolated nucleic acid molecule comprises a nucleotide sequence encoding a protein, wherein the protein comprises an amino acid sequence at least about 60% homologous to the amino acid sequence of SEQ ID NO:2 or SEQ ID NO:7. Preferably, the protein encoded by the nucleic acid molecule is at least about 65-70% homologous to SEQ ID NO:2 or SEQ ID NO:7, more preferably at least about 75-80% homologous to SEQ ID NO:2 or SEQ ID NO:7, even more preferably at least about 85-90% homologous to SEQ ID NO:2 or SEQ ID NO:7, and most preferably at least about 95% homologous to SEQ ID NO:2 or SEQ ID NO:7.

An isolated nucleic acid molecule encoding a BDSF protein homologous to the protein of SEQ ID NO:2 or SEQ ID NO:7 can be created by introducing one or more nucleotide substitutions, additions or deletions into the nucleotide sequence of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, such that one or more amino acid substitutions, additions or deletions are introduced into the encoded protein. Mutations can be introduced into SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, by standard techniques, such as site-directed mutagenesis and PCR-mediated mutagenesis. Preferably, conservative amino acid substitutions are made at one or more predicted non-essential amino acid residues. For example, sequence comparisons of the human BDSF-1 and murine BDSF-1 protein are 90.5% identical over the first 211 amino acids of the protein which includes the Ig-like domain. This region, therefore, suggests a highly conserved function and thus, indicates an "essential region" of human BDSF-1 and murine BDSF-1. A "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.,* threonine, valine, isoleucine) and aromatic side chains (*e.g.*, tyrosine, phenylalanine, tryptophan, histidine). Thus, a predicted nonessential amino acid residue in a BDSF protein is preferably replaced with another amino acid residue from the same side chain family. Alternatively, in another embodiment, mutations can be introduced randomly along all or part of a BDSF coding sequence, such as by saturation mutagenesis, and the resultant mutants can be screened for BDSF biological activity to identify mutants that retain activity. Following mutagenesis of SEQ ID NO:1, SEQ ID NO:3, SEQ ID NO:4, SEQ ID NO:6, SEQ ID NO:8, SEQ ID NO:9, or the nucleotide sequence of the DNA insert of the plasmid deposited with ATCC as Accession Number 98756, the encoded protein can be expressed recombinantly and the activity of the protein can be determined.

In a preferred embodiment, a mutant BDSF protein can be assayed for the ability to (1) modulate cellular signal transduction; (2) modulate protein:protein interactions; (3) regulate cellular proliferation; or (4) regulate cellular differentiation.

### B. STMST Nucleic Acid Molecules

A nucleic acid molecule of the present invention, *e.g.*, a nucleic acid molecule having the nucleotide sequence of SEQ ID NO:14, the nucleotide sequence of SEQ ID NO:17, the nucleotide sequence of the DNA insert of the plasmid deposited with ATCC as Accession Number , the nucleotide sequence of the DNA insert of the plasmid deposited with ATCC as Accession Number , or a portion thereof, can be isolated using standard molecular biology techniques and the sequence information provided herein. Using all or portion of the nucleic acid sequence of SEQ ID NO:14, the nucleotide sequence of SEQ ID NO:17, the nucleotide sequence of the DNA insert of the plasmid deposited with ATCC as Accession Number , or the nucleotide sequence of the DNA insert of the plasmid deposited with ATCC as Accession Number as a hybridization probe, STMST nucleic acid molecules can be isolated using standard hybridization and cloning techniques (*e.g.*, as described in Sambrook, J., Fritsh, E. F., and Maniatis, T. Molecular Cloning: A Laboratory Manual. 2nd, ed, Cold Spring Harbor Laboratory, Cold Spring Harbor Laboratory Press, Cold Spring Harbor, NY, 1989).

Moreover, a nucleic acid molecule encompassing all or a portion of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number can be isolated by the polymerase chain reaction (PCR) using synthetic oligonucleotide primers designed based upon the sequence of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number .

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to STMST nucleotide sequences can be prepared by standard synthetic techniques, *e.g.*, using an automated DNA synthesizer.

In a preferred embodiment, an isolated nucleic acid molecule of the invention comprises the nucleotide sequence shown in SEQ ID NO: 14. The sequence of SEQ ID NO: 14 corresponds to the human STMST-1 cDNA. This cDNA comprises sequences encoding the human STMST-1 protein (*i.e.,* "the coding region", from nucleotides 404-1294), as well as 5' untranslated sequences (nucleotides 1-403) and 3' untranslated sequences (nucleotides 1295-2915). Alternatively, the nucleic acid molecule can comprise only the coding region of SEQ ID NO: 14 (*e.g.*, nucleotides 404-1294, corresponding to SEQ ID NO:16).

In another preferred embodiment, an isolated nucleic acid molecule of the invention comprises the nucleotide sequence shown in SEQ ID NO:17. The sequence of SEQ ID NO:14 corresponds to the human STMST-2 cDNA. This cDNA comprises sequences encoding the human STMST-2 protein *(i.e.,* "the coding region", from nucleotides 334-2160), as well as 5' untranslated sequences (nucleotides 1-333) and 3' untranslated sequences (nucleotides 2161-4166). Alternatively, the nucleic acid molecule can comprise only the coding region of SEQ ID NO:14 (*e.g.*, nucleotides 334-2160, corresponding to SEQ ID NO:19).

In another preferred embodiment, an isolated nucleic acid molecule of the invention comprises a nucleic acid molecule which is a complement of the nucleotide sequence shown in SEQ ID NO:14, SEQ ID NO:17, the nucleotide sequence of the DNA insert of the plasmid deposited with ATCC as Accession Number , the nucleotide sequence of the DNA insert of the plasmid deposited with ATCC as Accession Number , or a portion of any of these nucleotide sequences. A nucleic acid molecule which is complementary to the nucleotide sequence shown in SEQ ID N0:14, SEQ ID N0:17, the nucleotide sequence of the DNA insert of the plasmid deposited with ATCC as Accession Number or the nucleotide sequence of the DNA insert of the plasmid deposited with ATCC as Accession Number is one which is sufficiently complementary to the nucleotide sequence shown in SEQ ID NO:14, SEQ ID NO:17, or such that it can hybridize to the nucleotide sequence shown in SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number , thereby forming a stable duplex.

In still another preferred embodiment, an isolated nucleic acid molecule of the present invention comprises a nucleotide sequence which is at least about 60-65%, preferably at least about 70-75%, more preferable at least about 80-85%, and even more preferably at least about 90-95% or more homologous to the nucleotide sequences shown in SEQ ID NO:14, SEQ ID N0:17, the nucleotide sequence of the DNA insert of the plasmid deposited with ATCC as Accession Number , or the nucleotide sequence of the DNA insert of the plasmid deposited with ATCC as Accession Number , or a portion of any of these nucleotide sequences.

Moreover, the nucleic acid molecule of the invention can comprise only a portion of the nucleic acid sequence of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number , for example a fragment which can be used as a probe or primer or a fragment encoding a biologically active portion of an STMST protein. The nucleotide sequence determined from the cloning of the STMST-1 genes allows for the generation of probes and primers designed for use in identifying and/or cloning other STMST family members, as well as STMST homologues from other species. The probe/primer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sense sequence of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number , of an anti-sense sequence of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number , or of a naturally occurring mutant of SEQ ID NO:14 or SEQ ID NO:17. In an exemplary embodiment, a nucleic acid molecule of the present invention comprises a nucleotide sequence which is greater that 350, 351-450, 451-550, 551-650, 651-750, or 751-850, 851-950, 951-1050, 1051-1150, or 1151-1250 nucleotides in length and hybridizes under stringent hybridization conditions to a nucleic acid molecule of SEQ ID NO:14 or SEQ ID NO:17, the nucleotide sequence of the DNA insert of the plasmid deposited with ATCC as Accession Number , or the nucleotide sequence of the DNA insert of the plasmid deposited with ATCC as Accession Number .

Probes based on the STMST nucleotide sequences can be used to detect transcripts or genomic sequences encoding the same or homologous proteins. In preferred embodiments, the probe further comprises a label group attached thereto, *e.g.*, the label group can be a radioisotope, a fluorescent compound, an enzyme, or an enzyme co-factor. Such probes can be used as a part of a diagnostic test kit for identifying cells or tissue which misexpress an STMST protein, such as by measuring a level of an STMST-encoding nucleic acid in a sample of cells from a subject *e.g.*, detecting STMST mRNA levels or determining whether a genomic STMST gene has been mutated or deleted.

A nucleic acid fragment encoding a "biologically active portion of an STMST protein" can be prepared by isolating a portion of the nucleotide sequence of SEQ ID NO:14, SEQ ID NO: 17, the nucleotide sequence of the DNA insert of the plasmid deposited with ATCC as Accession Number , or the nucleotide sequence of the DNA insert of the plasmid deposited with ATCC as Accession Number , which encodes a polypeptide having an STMST biological activity (the biological activities of the STMST proteins have previously been described), expressing the encoded portion of the STMST protein (*e.g.*, by recombinant expression *in vitro)* and assessing the activity of the encoded portion of the STMST protein.

The invention further encompasses nucleic acid molecules that differ from the nucleotide sequence shown in SEQ ID NO:14, SEQ ID NO: 17, the nucleotide sequence of the DNA insert of the plasmid deposited with ATCC as Accession Number , or the nucleotide sequence of the DNA insert of the plasmid deposited with ATCC as Accession Number , due to degeneracy of the genetic code and thus encode the same STMST proteins as those encoded by the nucleotide sequence shown in SEQ ID NO:14 or SEQ ID NO:17. In another embodiment, an isolated nucleic acid molecule of the invention has a nucleotide sequence encoding a protein having an amino acid sequence shown in SEQ ID NO:15 or SE ID NO:5.

In addition to the STMST nucleotide sequences shown in SEQ ID N0:14, SEQ ID NO:17, the nucleotide sequence of the DNA insert of the plasmid deposited with ATCC as Accession Number , or the nucleotide sequence of the DNA insert of the plasmid deposited with ATCC as Accession Number , it will be appreciated by those skilled in the art that DNA sequence polymorphisms that lead to changes in the amino acid sequences of the STMST proteins may exist within a population (*e.g.,* the human population). Such genetic polymorphism in the STMST genes may exist among individuals within a population due to natural allelic variation.

Moreover, nucleic acid molecules encoding other STMST family members and thus which have a nucleotide sequence which differs from the STMST-1 sequences of SEQ ID NO:14 or SEQ ID NO:17 are intended to be within the scope of the invention. For example, an STMST-3 cDNA can be identified based on the nucleotide sequence of human STMST-1 or STMST-2. Moreover, nucleic acid molecules encoding STMST proteins from different species, and thus which have a nucleotide sequence which differs from the STMST sequences of SEQ ID NO:14 or SEQ ID NO:17 are intended to be within the scope of the invention. For example, an mouse STMST cDNA can be identified based on the nucleotide sequence of a human STMST.

Nucleic acid molecules corresponding to natural allelic variants and homologues of the STMST cDNAs of the invention can be isolated based on their homology to the STMST nucleic acids disclosed herein using the cDNAs disclosed herein, or a portion thereof, as a hybridization probe according to standard hybridization techniques under stringent hybridization conditions.

Accordingly, in another embodiment, an isolated nucleic acid molecule of the invention is at least 15 nucleotides in length and hybridizes under stringent conditions to the nucleic acid molecule comprising the nucleotide sequence of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number . In another embodiment, the nucleic acid is at least 30, 50, 100, 250 or 500 nucleotides in length. Preferably, an isolated nucleic acid molecule of the invention that hybridizes under stringent conditions to the sequence of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number , corresponds to a naturally-occurring nucleic acid molecule. As used herein, a "naturally-occurring" nucleic acid molecule refers to an RNA or DNA molecule having a nucleotide sequence that occurs in nature (*e.g.*, encodes a natural protein).

In addition to naturally-occurring allelic variants of the STMST sequences that may exist in the population, the skilled artisan will further appreciate that changes can be introduced by mutation into the nucleotide sequences of SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number , thereby leading to changes in the amino acid sequence of the encoded STMST proteins, without altering the functional ability of the STMST proteins. For example, nucleotide substitutions leading to amino acid substitutions at "non-essential" amino acid residues can be made in the sequence of SEQ ID NO:14, SEQ ID N0:17, the nucleotide sequence of the DNA insert of the plasmid deposited with ATCC as Accession Number , or the nucleotide sequence of the DNA insert of the plasmid deposited with ATCC as Accession Number . A "non-essential" amino acid residue is a residue that can be altered from the wild-type sequence of STMST (*e.g.,* the sequence of SEQ ID NO:15) without altering the biological activity, whereas an "essential" amino acid residue is required for biological activity. For example, amino acid residues that are conserved among the STMST proteins of the present invention, are predicted to be particularly unamenable to alteration. Moreover, amino acid residues that are defined by the 7 transmembrane signature profile and the spectrin α-chain, repeated domain signature profile are particularly unamenable to alteration. Furthermore, additional amino acid residues that are conserved between the STMST proteins of the present invention and members of the G protein coupled receptor protein family are not likely to be amenable to alteration (*e.g.*, the conserved asn residue within the first TM domain, asn25 of SEQ ID NO:15 or SEQ ID NO:18; the conserved cys in the first extracellular loop, cys83 of SEQ ID NO:15 or SEQ ID NO:18; the conserved arg at the interface of the third TM domain and the first cytoplasmic loop, arg 108 of SEQ DI NO:2 or SEQ ID NO:18; the conserved trp and pro in the fourth TM domain, trp135 and pro145 pf SEQ ID NO:15 or SEQ ID NO:18; the conserved cys residue in the second extracellular domain, cys161 of SEQ ID NO:15 or SEQ ID NO:18; the conserved phe residue in the fifth TM domain, phe251 of SEQ ID NO:15 or SEQ ID NO:18; or the conserved pro in the seventh TM domain, pro294 of SEQ ID NO:18).

Accordingly, another aspect of the invention pertains to nucleic acid molecules encoding STMST proteins that contain changes in amino acid residues that are not essential for activity. Such STMST proteins differ in amino acid sequence from SEQ ID NO:15 and SEQ ID NO:18 yet retain biological activity. In one embodiment, the isolated nucleic acid molecule comprises a nucleotide sequence encoding a protein, wherein the protein comprises an amino acid sequence at least about 75% homologous to the amino acid sequence of SEQ ID NO: 15. Preferably, the protein encoded by the nucleic acid molecule is at least about 75-80% homologous to SEQ ID NO:15, more preferably at least about 80-85% homologous to SEQ ID NO:15, even more preferably at least about 85-90% homologous to SEQ ID N0:15, and even more preferably at least about 90-95% homologous to SEQ ID NO:15. In another embodiment, the isolated nucleic acid molecule comprises a nucleotide sequence encoding a protein, wherein the protein comprises an amino acid sequence at least about 65% homologous to the amino acid sequence of SEQ ID NO: 18. Preferably, the protein encoded by the nucleic acid is at least about 65-70% homologous to SEQ ID N0:15, more preferably at least about 70-75% homologous to SEQ ID NO:18, even more preferably at least about 75-80%, and even more preferably at least about 80-85%, 85-90%, or 90-95% homologous to SEQ ID NO:18.

An isolated nucleic acid molecule encoding an STMST protein homologous to the protein of SEQ ID NO:15 or SEQ ID NO:18 can be created by introducing one or more nucleotide substitutions, additions or deletions into the nucleotide sequence of SEQ ID NO:14, SEQ ID NO:17, or such that one or more amino acid substitutions, additions or deletions are introduced into the encoded protein. Mutations can be introduced into SEQ ID NO:14, SEQ ID NO:17, the nucleotide sequence of the DNA insert of the plasmid deposited with ATCC as Accession Number , or the nucleotide sequence of the DNA insert of the plasmid deposited with ATCC as Accession Number , by standard techniques, such as site-directed mutagenesis and PCR-mediated mutagenesis. Preferably, conservative amino acid substitutions are made at one or more predicted non-essential amino acid residues. Thus, a predicted nonessential amino acid residue in an STMST protein is preferably replaced with another amino acid residue from the same side chain family. Alternatively, in another embodiment, mutations can be introduced randomly along all or part of an STMST coding sequence, such as by saturation mutagenesis, and the resultant mutants can be screened for STMST biological activity to identify mutants that retain activity. Following mutagenesis of SEQ ID NO:14, SEQ ID NO:17, or the encoded protein can be expressed recombinantly and the activity of the protein can be determined.

In a preferred embodiment, a mutant STMST protein can be assayed for the ability to (1) modulation of cellular signal transduction, either *in vitro* or *in vivo*; (2) regulation of gene transcription in a cell expressing an STMST protein; (3) regulation of gene transcription in a cell expressing an STMST protein, wherein said cell is involved inflammation; (4) regulation of cellular proliferation; (5) regulation of cellular differentiation; (6) regulation of develpoment; (7) regulation of cell death; (8) regulation of inflammation; and (9) regulation of respiratory cell function.

### C. Antisense Nucleic Acid Molecules

In addition to the nucleic acid molecules encoding BDSF and STMST proteins described above, another aspect of the invention pertains to isolated nucleic acid molecules which are antisense thereto. An "antisense" nucleic acid comprises a nucleotide sequence which is complementary to a "sense" nucleic acid encoding a protein, *e.g.*, complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire BDSF or STMST coding strand, or to only a portion thereof. In one embodiment, an antisense nucleic acid molecule is antisense to a "coding region" of the coding strand of a nucleotide sequence encoding a BDSF or STMST protein. The term "coding region" refers to the region of the nucleotide sequence comprising codons which are translated into amino acid residues (*e.g.*, the coding region of human BDSF corresponds to SEQ ID NO:3, the coding region of murine BDSF corresponds to SEQ ID NO:8, the coding region of human STMST-1 corresponds to SEQ ID NO:16 and the coding region of human STMST-2 corresponds to SEQ ID NO:19). In another embodiment, the antisense nucleic acid molecule is antisense to a "noncoding region" of the coding strand of a nucleotide sequence encoding BDSF. The term "noncoding region" refers to 5' and 3' sequences which flank the coding region that are not translated into amino acids *(i.e.,* also referred to as 5' and 3' untranslated regions).

Given the coding strand sequences encoding BDSF and STMST proteins disclosed herein *(e.g.,* SEQ ID NO:3, SEQ ID NO:8, SEQ ID NO:16 and SEQ ID NO:19), antisense nucleic acids of the invention can be designed according to the rules of Watson and Crick base pairing. The antisense nucleic acid molecule can be complementary to the entire coding region of BDSF or STMST mRNA, but more preferably is an oligonucleotide which is antisense to only a portion of the coding or noncoding region of BDSF or STMST mRNA. For example, the antisense oligonucleotide can be complementary to the region surrounding the translation start site of BDSF or STMST mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (*e.g.*, an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.*, phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation *(i.e.,* RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a BDSF or STMST protein to thereby inhibit expression of the protein, *e.g.*, by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention include direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g.*, by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-0-methylribonucleotide (Inoue et al. (1987) Nucleic Acids Res. 15:6131-6148) or a chimeric RNA-DNA analogue (Inoue et al. (1987) FEBS Lett. 215:327-330).

In still another embodiment, an antisense nucleic acid of the invention is a ribozyme. Ribozymes are catalytic RNA molecules with ribonuclease activity which are capable of cleaving a single-stranded nucleic acid, such as an mRNA, to which they have a complementary region. Thus, ribozymes (*e.g.*, hammerhead ribozymes described in Haselhoff and Gerlach (1988) Nature 334:585-591) can be used to catalytically cleave BDSF or STMST mRNA transcripts to thereby inhibit translation of BDSF or STMST mRNA, respectively. A ribozyme having specificity for a BDSF-encoding nucleic or STMST-encoding nucleic acid acid can be designed based upon the nucleotide sequence of a BDSF cDNA or STMST mRNA disclosed herein. For example, a derivative of a *Tetrahymena* L-19 IVS RNA can be constructed in which the nucleotide sequence of the active site is complementary to the nucleotide sequence to be cleaved in a BDSF-encoding mRNA or STMST-encoding mRNA. See, *e.g.,* Cech et al. U.S. Patent No. 4,987,071; and Cech et al. U.S. Patent No. 5,116,742. Alternatively, BDSF mRNA can be used to select a catalytic RNA having a specific ribonuclease activity from a pool of RNA molecules. See, *e.g.*, Bartel, D. and Szostak, J.W. (1993) Science 261:1411-1418.

Alternatively, BDSF or STMST gene expression can be inhibited by targeting nucleotide sequences complementary to the regulatory region of the BDSF or STMST genes (*e.g.*, promoter and/or enhancer sequences) to form triple helical structures that prevent transcription of the BDSF or STMST genes in target cells. See generally, Helene, C. (1991) Anticancer Drug Des. 6(6):569-84; Helene, C. et al. (1992) Ann. N.Y. Acad. Sci. 660:27-36; and Maher, L.J. (1992) Bioassays 14(12):807-15.

### D. Peptide Nucleic Acids

In yet another embodiment, the nucleic acid molecules of the present invention can be modified at the base moiety, sugar moiety or phosphate backbone to improve, *e.g.*, the stability, hybridization, or solubility of the molecule. For example, the deoxyribose phosphate backbone of the nucleic acid molecules can be modified to generate peptide nucleic acids (see Hyrup B. et al. (1996) Bioorganic & Medicinal Chemistry 4 (1): 5-23). As used herein, the terms "peptide nucleic acids" or "PNAs" refer to nucleic acid mimics, *e.g.*, DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al.* (1996) *supra;* Perry-O'Keefe et al. PNAS 93: 14670-675.

PNAs of BDSF or STMST nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of BDSF or STMST nucleic acid molecules can also be used in the analysis of single base pair mutations in a gene, (*e.g.*, by PNA-directed PCR clamping); as 'artificial restriction enzymes' when used in combination with other enzymes, (*e.g.*, S1 nucleases (Hyrup B. (1996) *supra*)); or as probes or primers for DNA sequencing or hybridization (Hyrup B. *et al.* (1996) *supra;* Perry-O'Keefe *supra).*

In another embodiment, PNAs can be modified, (*e.g.*, to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras can be generated which may combine the advantageous properties of PNA and DNA. Such chimeras allow DNA recognition enzymes, (*e.g.*, RNAse H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup B. (1996) *supra*). The synthesis of PNA-DNA chimeras can be performed as described in Hyrup B. (1996) *supra* and Finn P.J. et al. (1996) Nucleic Acids Res. 24 (17): 3357-63. For example, a DNA chain can be synthesized on a solid support using standard phosphoramidite coupling chemistry and modified nucleoside analogs, *e.g.*, 5'-(4-methoxytrityl)amino-5'-deoxy-thymidine phosphoramidite, can be used as a between the PNA and the 5' end of DNA (Mag, M. et al. (1989) Nucleic Acid Res. 17: 5973-88). PNA monomers are then coupled in a stepwise manner to produce a chimeric molecule with a 5' PNA segment and a 3' DNA segment (Finn P.J. *et al.* (1996) *supra).* Alternatively, chimeric molecules can be synthesized with a 5' DNA segment and a 3' PNA segment (Peterser, K.H . et al. (1975) Bioorganic Med. Chem. Lett. 5: 1119-11124).

In other embodiments, the oligonucleotide may include other appended groups such as peptides *(e.g.,* for targeting host cell receptors *in vivo*), or agents facilitating transport across the cell membrane (see, *e.g.,* Letsinger et al. (1989) Proc. Natl. Acad. Sci. US. 86:6553-6556; Lemaitre et al. (1987) Proc. Natl. Acad. Sci. USA 84:648-652; PCT Publication No. WO88/09810, published December 15, 1988) or the blood-brain barrier (see, *e.g*., PCT Publication No. WO89/10134, published April 25, 1988). In addition, oligonucleotides can be modified with hybridization-triggered cleavage agents (See, *e.g.,* Krol et al. (1988) BioTechniques 6:958-976) or intercalating agents. (See, *e.g.,* Zon (1988) Pharm. Res. 5:539-549). To this end, the oligonucleotide may be conjugated to another molecule, (*e.g.*, a peptide, hybridization triggered cross-linking agent, transport agent, or hybridization-triggered cleavage agent).

### E. STMST Ligands and Modulators

Furthermore, given the fact that an important use for the STMST molecules of the present invention is in the screening for STMST ligands (*e.g.*, surrogate ligands) and/or STMST modulators, it is intended that the following are also within the scope of the present invention: isolated nucleic acids which encode and STMST ligands or STMST modulators, probes and/or primers useful for identifying STMST ligands or STMST modulators based on the sequences of nucleic acids which encode and STMST ligands or STMST modulators, isolated nucleic acid molecules which are complementary or antisense to the sequences of nucleic acids which encode and STMST ligands or STMST modulators, isolated nucleic acid molecules which are at least about 60-65%, preferably at least about 70-75%, more preferable at least about 80-85%, and even more preferably at least about 90-95% or more homologous to the sequences of nucleic acids which encode and STMST ligands or STMST modulators, portions of nucleic acids which encode and STMST ligands or STMST modulators (*e.g.*, biologically-active portions), naturally-occurring allelic variants of nucleic acids which encode and STMST ligands or STMST modulators, nucleic acid molecules which hybridize under stringent hybridization conditions to nucleic acids which encode and STMST ligands or STMST modulators, functionally-active mutants of nucleic acids which encode and STMST ligands or STMST modulators, PNAs of nucleic acids which encode and STMST ligands or STMST modulators, as well as vectors containing a nucleic acid encoding an STMST ligand or STMST modulator, described herein, host cells into which an expression vector encoding an STMST ligand or STMST modulator has been introduced, and homologous recombinant animal which express STMST ligands or STMST modulators.

### II. Isolated BDSF and STMST Proteins, Fragments Thereof and Antibodies Thereto

One aspect of the invention pertains to isolated BDSF proteins, and biologically active portions thereof, as well as polypeptide fragments suitable for use as immunogens to raise anti-BDSF antibodies. Another aspect of the invention pertains to isolated STMST proteins, and biologically active portions thereof, as well as polypeptide fragments suitable for use as immunogens to raise anti-STMST antibodies. In one embodiment, native proteins (*e.g*., BDSF or STMST proteins) can be isolated from cells or tissue sources by an appropriate purification scheme using standard protein purification techniques. In another embodiment, proteins (*e.g.*, BDSF or STMST proteins) are produced by recombinant DNA techniques. Alternative to recombinant expression, a BDSF or STMST protein or polypeptide can be synthesized chemically using standard peptide synthesis techniques.

An "isolated" or "purified" protein or biologically active portion thereof is substantially free of cellular material or other contaminating proteins from the cell or tissue source from which the protein is derived, or substantially free from chemical precursors or other chemicals when chemically synthesized. The language "substantially free of cellular material" includes preparations of protein (*e.g.*, BDSF or STMST proteins) in which the protein is separated from cellular components of the cells from which it is isolated or recombinantly produced. In one embodiment, the language "substantially free of cellular material" includes preparations of protein having less than about 30% (by dry weight) of non-BDSF protein or non-STMST protein (also referred to herein as a "contaminating protein"), more preferably less than about 20% of non-BDSF protein or non-STMST protein, still more preferably less than about 10% of non-BDSF protein or non-STMST protein, and most preferably less than about 5% non-BDSF protein or non-STMST protein. When the protein or biologically active portion thereof is recombinantly produced, it is also preferably substantially free of culture medium, *i.e.,* culture medium represents less than about 20%, more preferably less than about 10%, and most preferably less than about 5% of the volume of the protein preparation.

The language "substantially free of chemical precursors or other chemicals" includes preparations of protein *(e.g.,* BDSF or STMST proteins) in which the protein is separated from chemical precursors or other chemicals which are involved in the synthesis of the protein. In one embodiment, the language "substantially free of chemical precursors or other chemicals" includes preparations of protein having less than about 30% (by dry weight) of chemical precursors or non-BDSF chemicals (or non-BDSF chemicals), more preferably less than about 20% chemical precursors or non-BDSF chemicals (or non-BDSF chemicals), still more preferably less than about 10% chemical precursors or non-BDSF chemicals (or non-BDSF chemicals), and most preferably less than about 5% chemical precursors or non-BDSF chemicals (or non-BDSF chemicals).

### A. BDSF Proteins and Biologically-Active Portions Thereof

In a preferred embodiment, the BDSF protein has an amino acid sequence shown in SEQ ID NO:2 or SEQ ID NO:7. In other embodiments, the BDSF protein is substantially homologous to SEQ ID NO:2 or SEQ ID NO:7, and retains the functional activity of the protein of SEQ ID NO:2 or SEQ ID NO:7, yet differs in amino acid sequence due to natural allelic variation or mutagenesis, as described in detail in subsection I above. Accordingly, in another embodiment, the BDSF protein is a protein which comprises an amino acid sequence at least about 60% homologous to the amino acid sequence of SEQ ID NO:2 or SEQ ID NO:7 and retains the functional activity of the BDSF proteins of SEQ ID NO:2 or SEQ ID NO:7. Preferably, the protein is at least about 35-40% homologous to SEQ ID NO:2 or SEQ ID NO:7, more preferably at least about 40-45% homologous to SEQ ID NO:2 or SEQ ID NO:7, even more preferably at least about 45-50% homologous to SEQ ID NO:2 or SEQ ID NO:7, and even more preferably at least about 50-55%, 55-60%, 60-65%, 65-70%, 70-75%, 75-80%, 80-85%, 85-90%, or 90-95% or more homologous to SEQ ID NO:2 or SEQ ID NO:7.

To determine the percent homology of two amino acid sequences or of two nucleic acids, the sequences are aligned for optimal comparison purposes *(e.g.,* gaps can be introduced in the sequence of a first amino acid or nucleic acid sequence for optimal alignment with a second amino or nucleic acid sequence and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence *(e.g.,* when aligning a second sequence to the BDSF amino acid sequence of SEQ ID NO:2 or SEQ ID NO:7 having 293 amino acid residues, at least 88, preferably at least 117, more preferably at least 147, even more preferably at least 176, and even more preferably at least 205, 234 or 264 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.*, as used herein amino acid or nucleic acid "homology" is equivalent to amino acid or nucleic acid "identity"). The percent homology between the two sequences is a function of the number of identical positions shared by the sequences (*i.e.*, % homology = # of identical positions/total # of positions x 100). The comparison of sequences and determination of percent homology between two sequences can be accomplished using a mathematical algorithim. A preferred, non-limiting example of a mathematical algorithim utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to BDSF nucleic acid molecules of the invention. BLAST protein searches can be performed with the XBLAST program, score = 50, wordlength = 3 to obtain amino acid sequences homologous to BDSF protein molecules of the invention. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs *(e.g.,* XBLAST and NBLAST) can be used. See http://www.ncbi.nlm.nih.gov. Another preferred, non-limiting example of a mathematical algorithim utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

Biologically active portions of a BDSF protein include peptides comprising amino acid sequences sufficiently homologous to or derived from the amino acid sequence of the BDSF protein, *e.g.*, the amino acid sequence shown in SEQ ID NO:2 or SEQ ID NO:7, which include less amino acids than the full length BDSF proteins, and exhibit at least one activity of a BDSF protein. Typically, biologically active portions comprise a domain or motif with at least one activity of the BDSF protein. A biologically active portion of a BDSF protein can be a polypeptide which is, for example, 10, 25, 50, 100 or more amino acids in length.

In one embodiment, a biologically active portion of a BDSF protein comprises an Ig-like domain. In another embodiment, a biologically active portion of a BDSF protein comprises at least an Ig-like domain and a signal sequence.

It is to be understood that a preferred biologically active portion of a BDSF protein of the present invention may contain at least one of the above-identified structural domains. A more preferred biologically active portion of a BDSF protein may contain at least two of the above-identified structural domains. Moreover, other biologically active portions, in which other regions of the protein are deleted, can be prepared by recombinant techniques and evaluated for one or more of the functional activities of a native BDSF protein.

### B. STMST Proteins and Biologically-Active Portions Thereof

In a preferred embodiment, the STMST protein has an amino acid sequence shown in SEQ ID NO:15. In other embodiments, the STMST protein is substantially homologous to SEQ ID NO:15, and retains the functional activity of the protein of SEQ ID NO:15, yet differs in amino acid sequence due to natural allelic variation or mutagenesis, as described in detail in subsection I above. Accordingly, in another embodiment, the STMST protein is a protein which comprises an amino acid sequence at least about 75% homologous to the amino acid sequence of SEQ ID NO:15 and retains the functional activity of the STMST proteins of SEQ ID NO:15, respectively. Preferably, the protein is at least about 75-80%% homologous to SEQ ID NO:15, more preferably at least about 80-85% homologous to SEQ ID NO:15, even more preferably at least about 85-90% homologous to SEQ ID NO:15, and most preferably at least about 90-95% or more homologous to SEQ ID NO:15.

In a preferred embodiment, the STMST protein has an amino acid sequence shown in SEQ ID NO:18. In other embodiments, the STMST protein is substantially homologous to SEQ ID NO:18, and retains the functional activity of the protein of SEQ ID NO:18, yet differs in amino acid sequence due to natural allelic variation or mutagenesis, as described in detail in subsection I above. Accordingly, in another embodiment, the STMST protein is a protein which comprises an amino acid sequence at least about 65% homologous to the amino acid sequence of SEQ ID NO:15 and retains the functional activity of the STMST proteins of SEQ ID NO:15, respectively. Preferably, the protein is at least about 65-70%% homologous to SEQ ID NO:15, more preferably at least about 70-75% homologous to SEQ ID NO:15, even more preferably at least about 75-80% homologous to SEQ ID NO:15, and most preferably at least about 80-85%,85-90%, or 90-95% homologous to SEQ ID NO:15.

Biologically active portions of an STMST protein include peptides comprising amino acid sequences sufficiently homologous to or derived from the amino acid sequence of the STMST protein, *e*.*g*., the amino acid sequence shown in SEQ ID NO:15 or the amino acid sequence shown in SEQ ID NO: 18, which include less amino acids than the full length STMST proteins, and exhibit at least one activity of an STMST protein. Typically, biologically active portions comprise a domain or motif with at least one activity of the STMST protein. A biologically active portion of an STMST protein can be a polypeptide which is, for example, 10, 25, 50, 100 or more amino acids in length.

In one embodiment, a biologically active portion of an STMST protein comprises at least a transmembrane domain. In another embodiment, a biologically active portion of an STMST protein comprises at least one 7 transmembrane receptor profile. In another embodiment, a biologically active portion of an STMST protein comprises at least a spectrin a-chain, repeated domain profile. In another embodiment a biologically active portion of an STMST protein comprises at least a 7 transmembrane receptor profile and a spectrin α-chain profile.

It is to be understood that a preferred biologically active portion of an STMST protein of the present invention may contain at least one of the above-identified structural domains and/or profiles. A more preferred biologically active portion of an STMST protein may contain at least two of the above-identified structural domains and/or profiles. Moreover, other biologically active portions, in which other regions of the protein are deleted, can be prepared by recombinant techniques and evaluated for one or more of the functional activities of a native STMST protein.

### C. Chimeric/Fusion Proteins

The invention also provides BDSF and STMST chimeric or fusion proteins. As used herein, a "chimeric protein" or "fusion protein" comprises a BDSF or STMST polypeptide operatively linked to a non-BDSF polypeptide or non-STMST polypeptide, respectively. A "BDSF polypeptide" refers to a polypeptide having an amino acid sequence corresponding to BDSF, whereas a "non-BDSF polypeptide" refers to a polypeptide having an amino acid sequence corresponding to a protein which is not substantially homologous to the BDSF protein, e.g., a protein which is different from the BDSF protein and which is derived from the same or a different organism. Likewise, a "STMST polypeptide" refers to a polypeptide having an amino acid sequence corresponding to STMST, whereas a "non-STMST polypeptide" refers to a polypeptide having an amino acid sequence corresponding to a protein which is not substantially homologous to the STMST protein, *e.g.*, a protein which is different from the STMST protein and which is derived from the same or a different organism.

Within a BDSF or STMST fusion protein the BDSF or STMST polypeptide can correspond to all or a portion of a BDSF or STMST protein, respectively. In a preferred embodiment, a fusion protein comprises at least one biologically active portion of a BDSF or STMST protein. In another preferred embodiment, a fusion protein comprises at least two biologically active portions of a BDSF or STMST protein. Within the fusion protein, the term "operatively linked" is intended to indicate that the BDSF or STMST polypeptide and the non-BDSF polypeptide or non-STMST polypeptide are fused in-frame to each other. The non-BDSF polypeptide or non-STMST polypeptide can be fused to the N-terminus or C-terminus of the BDSF polypeptide or STMST polypeptide, respectively.

For example, in one embodiment, the fusion protein is a GST-BDSF fusion protein in which the BDSF sequences are fused to the C-terminus of the GST sequences. Such fusion proteins can facilitate the purification of recombinant BDSF. In another embodiment, the fusion protein is a GST-STMST fusion protein in which the STMST sequences are fused to the C-terminus of the GST sequences. Such fusion proteins can facilitate the purification of recombinant STMST.

In another embodiment, the fusion protein is a BDSF protein containing a heterologous signal sequence at its N-terminus. For example, the native BDSF signal sequence (i.e, about amino acids 1 to 25 of SEQ ID NO:2 or about amino acids 1 to 24 of SEQ ID NO:7) can be removed and replaced with a signal sequence from another protein. In another embodiment, the fusion protein is an STMST protein containing a heterologous signal sequence at its N-terminus. In certain host cells *(e.g.,* mammalian host cells), expression and/or secretion of BDSF can be increased through use of a heterologous signal sequence.

The fusion proteins of the invention can be incorporated into pharmaceutical compositions and administered to a subject *in vivo.* The fusion proteins can be used to affect the bioavailability of a BDSF or STMST target molecule. Use of BDSF fusion proteins may be useful therapeutically for the treatment of proliferative disorders (*e.g.*, cancer). Use of STMST fusion proteins may be useful therapeutically for the treatment of respiratory disorders *(e.g.,* asthma). Moreover, the fusion proteins of the invention can be used as immunogens to produce antibodies in a subject, to purify ligands and/or target molecules and in screening assays to identify molecules which inhibit the interaction of BDSF with a BDSF target molecule or the interaction of STMST with an STMST ligand.

Preferably, a chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (*e.g.*, a GST polypeptide). A BDSF-encoding nucleic acid or STMST-encoding nucleic acid can be cloned into such an expression vector such that the fusion moiety is linked in-frame to the BDSF protein or STMST protein, respectively.

### D. Agonist/Anatgonist Variant Proteins

The present invention also pertains to variants of the BDSF proteins which function as either BDSF agonists (mimetics) or as BDSF antagonists and to variants of the STMST proteins which function as either STMST agonists (mimetics) or as STMST antagonists. Variants of the BDSF and BDSF proteins can be generated by mutagenesis, *e.g.*, discrete point mutation or truncation of a BDSF or STMST protein, respectively. An agonist of the BDSF or STMST proteins can retain substantially the same, or a subset, of the biological activities of the naturally occurring form of a BDSF protein or STMST protein, respectively. An antagonist can inhibit one or more of the activities of the naturally occurring form of the protein by, for example, competitively inhibiting the activity of the BDSF or STMST protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the protein.

In one embodiment, variants of a BDSF or STMST protein which function as either agonists (mimetics) or as antagonists can be identified by screening combinatorial libraries of mutants, *e.g.*, truncation mutants, of a BDSF or STMST protein for agonist or antagonist activity. In one embodiment, a variegated library of BDSF or STMST variants is generated by combinatorial mutagenesis at the nucleic acid level and is encoded by a variegated gene library. A variegated library can be produced by, for example, enzymatically ligating a mixture of synthetic oligonucleotides into gene sequences such that a degenerate set of potential BDSF or STMST sequences is expressible as individual polypeptides, or alternatively, as a set of larger fusion proteins (*e.g.*, for phage display) containing the set of BDSF or STMST sequences therein. There are a variety of methods which can be used to produce libraries of variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential BDSF or STMST sequences. Methods for synthesizing degenerate oligonucleotides are known in the art (see, *e.g.*, Narang, S.A. (1983) Tetrahedron 39:3; Itakura et al. (1984) Annu. Rev. Biochem. 53:323; Itakura et al. (1984) Science 198:1056; Ike et al. (1983) Nucleic Acid Res. 11:477.

In addition, libraries of fragments of a BDSF or STMST protein coding sequence can be used to generate a variegated population of fragments for screening and subsequent selection of protein variants. In one embodiment, a library of coding sequence fragments can be generated by treating a double stranded PCR fragment of a coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of a BDSF or STMST protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis procedures described herein. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recrusive ensemble mutagenesis (REM), a new technique which enhances the frequency of functional mutants in the libraries, can be used in combination with the screening assays to identify BDSF or STMST variants (Arkin and Yourvan (1992) PNAS 89:7811-7815; Delgrave et al. (1993) Protein Engineering 6(3):327-331).

In one embodiment, cell based assays can be exploited to analyze a variegated library. For example, a library of expression vectors can be transfected into a cell line which ordinarily synthesizes and secretes BDSF or STMST. The transfected cells are then cultured such that BDSF and a particular mutant BDSF (or STMST and a particular mutant) are secreted and the effect of expression of the mutant on BDSF or STMST activity in cell supernatants can be detected, *e.g.*, by any of a number of enzymatic or binding assays. Plasmid DNA can then be recovered from the cells which score for inhibition, or alternatively, potentiation of BDSF or STMST activity, and the individual clones further characterized.

### E. Protein Fragments, Immunogenic Peptides and Antibodies

An isolated BDSF or STMST protein, or a portion or fragment thereof, can be used as an immunogen to generate antibodies that bind BDSF or STMST using standard techniques for polyclonal and monoclonal antibody preparation. A full-length protein can be used or, alternatively, the invention provides antigenic peptide fragments or use as immunogens. For example, an antigenic peptide of BDSF comprises at least 8 amino acid residues of the amino acid sequence shown in SEQ ID NO:2 or SEQ ID NO:7 and encompasses an epitope of BDSF such that an antibody raised against the peptide forms a specific immune complex with BDSF. Likewise, an antigenic peptide of STMST comprises at least 8 amino acid residues of the amino acid sequence shown in SEQ ID NO:15 and encompasses an epitope of STMST such that an antibody raised against the peptide forms a specific immune complex with STMST. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of BDSF that are located on the surface of the protein, *e.g.*, hydrophilic regions.

A BDSF or STMST immunogen typically is used to prepare antibodies by immunizing a suitable subject, (*e.g.*, rabbit, goat, mouse or other mammal) with the immunogen. An appropriate immunogenic preparation can contain, for example, recombinantly expressed protein or a chemically synthesized polypeptide. The preparation can further include an adjuvant, such as Freund's complete or incomplete adjuvant, or similar immunostimulatory agent. Immunization of a suitable subject with an immunogenic BDSF preparation induces a polyclonal anti-BDSF antibody response. Likewise, immunization of a suitable subject with an immunogenic STMST preparation induces a polyclonal anti-STMST antibody response.

Accordingly, another aspect of the invention pertains to anti-BDSF antibodies or anti-STMST antibodies. The term "antibody" as used herein refers to immunoglobulin molecules and immunologically active portions of immunoglobulin molecules, *i.e.,* molecules that contain an antigen binding site which specifically binds (immunoreacts with) an antigen, such as BDSF or STMST. Examples of immunologically active portions of immunoglobulin molecules include F(ab) and F(ab')₂ fragments which can be generated by treating the antibody with an enzyme such as pepsin. The invention provides polyclonal and monoclonal antibodies that bind BDSF or STMST. The term "monoclonal antibody" or "monoclonal antibody composition", as used herein, refers to a population of antibody molecules that contain only one species of an antigen binding site capable of immunoreacting with a particular epitope of BDSF or STMST. A monoclonal antibody composition thus typically displays a single binding affinity for a particular BDSF protein with which it immunoreacts.

Polyclonal antibodies can be prepared as described above by immunizing a suitable subject with a BDSF or STMST immunogen. The specific antibody titer in the immunized subject can be monitored over time by standard techniques, such as with an enzyme linked immunosorbent assay (ELISA) using immobilized BDSF or STMST. If desired, the antibody molecules can be isolated from the mammal (*e.g.*, from the blood) and further purified by well known techniques, such as protein A chromatography to obtain the IgG fraction. At an appropriate time after immunization, *e.g.*, when the specific antibody titers are highest, antibody-producing cells can be obtained from the subject and used to prepare monoclonal antibodies by standard techniques, such as the hybridoma technique originally described by Kohler and Milstein (1975) Nature 256:495-497) (see also, Brown et al. (1981) J. Immunol. 127:539-46; Brown et al. (1980) J. Biol. Chem .255:4980-83; Yeh et al. (1976) PNAS 76:2927-31; and Yeh et al. (1982) Int. J. Cancer 29:269-75), the more recent human B cell hybridoma technique (Kozbor et al. (1983) Immunol Today 4:72), the EBV-hybridoma technique (Cole et al. (1985), Monoclonal Antibodies and Cancer Therapy, Alan R. Liss, Inc., pp. 77-96) or trioma techniques. The technology for producing monoclonal antibody hybridomas is well known (see generally R. H. Kenneth, in Monoclonal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet. 3:231-36). Briefly, an immortal cell line (typically a myeloma) is fused to lymphocytes (typically splenocytes) from a mammal immunized with a BDSF or STMST immunogen as described above, and the culture supernatants of the resulting hybridoma cells are screened to identify a hybridoma producing a monoclonal antibody that binds BDSF or STMST, respectively.

Any of the many well known protocols used for fusing lymphocytes and immortalized cell lines can be applied for the purpose of generating monoclonal antibodies (see, *e.g.,* G. Galfre et al. (1977) Nature 266:55052; Gefter et al. Somatic Cell Genet.*,* cited *supra;* Lerner, Yale J. Biol. Med.*,* cited *supra;* Kenneth, Monoclonal Antibodies*,* cited *supra).* Moreover, the ordinarily skilled worker will appreciate that there are many variations of such methods which also would be useful. Typically, the immortal cell line (*e.g.*, a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g.*, the P3-NS1/1-Ag4-1, P3-x63-Ag8.653 or Sp2/O-Agl4 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind BDSF or STMST, *e.g.*, using a standard ELISA assay.

Alternative to preparing monoclonal antibody-secreting hybridomas, a monoclonal anti-BDSF or anti-STMST antibody can be identified and isolated by screening a recombinant combinatorial immunoglobulin library (*e.g.*, an antibody phage display library) with BDSF or STMST, respectively, to thereby isolate immunoglobulin library members that bind BDSF or STMST. Kits for generating and screening phage display libraries are commercially available (*e.g.*, the Pharmacia *Recombinant Phage Antibody System,* Catalog No. 27-9400-01; and the Stratagene *SurfZAP*^{™} *Phage Display Kit,* Catalog No. 240612). Additionally, examples of methods and reagents particularly amenable for use in generating and screening antibody display library can be found in, for example, Ladner et al. U.S. Patent No. 5,223,409; Kang et al. PCT International Publication No. WO 92/18619; Dower et al. PCT International Publication No. WO 91/17271; Winter et al. PCT International Publication WO 92/20791; Markland et al. PCT International Publication No. WO 92/15679; Breitling et al. PCT International Publication WO 93/01288; McCafferty et al. PCT International Publication No. WO 92/01047; Garrard et al. PCT International Publication No. WO 92/09690; Ladner et al. PCT International Publication No. WO 90/02809; Fuchs et al. (1991) Bio/Technology 9:1370-1372; Hay et al..(1992) Hum. Antibod. Hybridomas 3:81-85; Huse et al. (1989) Science 246:1275-1281; Griffiths et al. (1993) EMBO J 12:725-734; Hawkins et al. (1992) J Mol. Biol. 226:889-896; Clarkson et al. (1991) Nature 352:624-628; Gram et al. (1992) PNAS 89:3576-3580; Garrad et al. (1991) Bio/Technology 9:1373-1377; Hoogenboom et al. (1991) Nuc. Acid Res. 19:4133-4137; Barbas et al. (1991) PNAS 88:7978-7982; and McCafferty et al. Nature (1990) 348:552-554.

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson et al. International Application No. PCT/US86/02269; Akira, et al. European Patent Application 184,187; Taniguchi, M., European Patent Application 171,496; Morrison et al. European Patent Application 173,494; Neuberger et al. PCT International Publication No. WO 86/01533; Cabilly et al. U.S. Patent No. 4,816,567; Cabilly et al. European Patent Application 125,023; Better et al. (1988) Science 240:1041-1043; Liu et al. (1987) PNAS 84:3439-3443; Liu et al. (1987) J. Immunol. 139:3521-3526; Sun et al. (1987) PNAS 84:214-218; Nishimura et al. (1987) Canc. Res. 47:999-1005; Wood et al. (1985) Nature 314:446-449; and Shaw et al. (1988) J. Natl. Cancer Inst. 80:1553-1559); Morrison, S. L. (1985) Science 229:1202-1207; Oi et al. (1986) BioTechniques 4:214; Winter U.S. Patent 5,225,539; Jones et al. (1986) Nature 321:552-525; Verhoeyan et al. (1988) Science 239:1534; and Beidler et al. (1988) J. Immunol. 141:4053-4060.

An anti-BDSF or anti-STMST antibody (*e.g.*, monoclonal antibody) can be used to isolate BDSF or STMST by standard techniques, such as affinity chromatography or immunoprecipitation. An anti-BDSF or anti-STMST antibody can facilitate the purification of natural BDSF or STMST from cells and of recombinantly produced BDSF or STMST expressed in host cells. Moreover, an anti-BDSF or anti-STMST antibody can be used to detect BDSF protein or STMST (*e.g.*, in a cellular lysate or cell supernatant) in order to evaluate the abundance and pattern of expression of the BDSF or STMST protein. Anti-BDSF or anti-STMST antibodies can be used diagnostically to monitor protein levels in tissue as part of a clinical testing procedure, *e.g.*, to, for example, determine the efficacy of a given treatment regimen. Detection can be facilitated by coupling *(i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, -galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I,¹³¹I, ³⁵S or ³H.

### III. Recombinant Expression Vectors and Host Cells

Another aspect of the invention pertains to vectors, preferably expression vectors, containing a nucleic acid encoding a BDSF or STMST protein (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.,* replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence *(e.g.,* in an *in vitro* transcription/translation system or in a host cell when the vector is introduced into the host cell). The term "regulatory sequence" is intended to includes promoters, enhancers and other expression control elements (*e.g.*, polyadenylation signals). Such regulatory sequences are described, for example, in Goeddel; Gene Expression Technology: Methods in Enzymology 185, Academic Press, San Diego, CA (1990). Regulatory sequences include those which direct constitutive expression of a nucleotide sequence in many types of host cell and those which direct expression of the nucleotide sequence only in certain host cells *(e.g.,* tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e.g.*, BDSF proteins, mutant forms of BDSF proteins, STMST proteins, mutant forms of STMST proteins, fusion proteins, etc.).

The recombinant expression vectors of the invention can be designed for expression of BDSF or STMST proteins in prokaryotic or eukaryotic cells. For example, BDSF or STMST proteins can be expressed in bacterial cells such as *E. coli,* insect cells (using baculovirus expression vectors) yeast cells or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be utilized in BDSF or STMST activity assays, (*e*.*g*., direct assays or competitive assays described in detail below), or to generate antibodies specific for BDSF or STMST proteins, for example. In a preferred embodiment, a BDSF or STMST fusion protein expressed in a retroviral expression vector of the present invention can be utilized to infect bone marrow cells which are subsequently transplanted into irradiated recipients. The pathology of the subject recipient is then examined after sufficient time has passed (e.g six (6) weeks).

Examples of suitable inducible non-fusion *E. coli* expression vectors include pTrc (Amann et al., (1988) Gene 69:301-315) and pET 11d (Studier et al., Gene Expression Technology: Methods in Enzymology 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gnl). This viral polymerase is supplied by host strains BL21(DE3) or HMS174(DE3) from a resident prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, S., Gene Expression Technology: Methods in Enzymology 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli* (Wada et al., (1992) Nucleic Acids Res. 20:2111-2118). Such alteration of nucleic acid sequences of the invention can be carried out by standard DNA synthesis techniques.

In another embodiment, the BDSF or STMST expression vector is a yeast expression vector. Examples of vectors for expression in yeast *S. cerivisae* include pYepSecl (Baldari, et al., (1987) Embo J. 6:229-234), pMFa (Kurjan and Herskowitz, (1982) Cell 30:933-943), pJRY88 (Schultz et al., (1987) Gene 54:113-123), pYES2 (Invitrogen Corporation, San Diego, CA), and picZ (InVitrogen Corp, San Diego, CA).

Alternatively, BDSF or STMST proteins can be expressed in insect cells using baculovirus expression vectors. Baculovirus vectors available for expression of proteins in cultured insect cells *(e.g.,* Sf 9 cells) include the pAc series (Smith et al. (1983) Mol. Cell Biol. 3:2156-2165) and the pVL series (Lucklow and Summers (1989) Virology 170:31-39).

In yet another embodiment, a nucleic acid of the invention is expressed in mammalian cells using a mammalian expression vector. Examples of mammalian expression vectors include pCDM8 (Seed, B. (1987) Nature 329:840) and pMT2PC (Kaufman et al. (1987) EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989.

In another embodiment, the recombinant mammalian expression vector is capable of directing expression of the nucleic acid preferentially in a particular cell type (*e.g.*, tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters *(e.g.,* the neurofilament promoter; Byrne and Ruddle (1989) PNAS 86:5473-5477), pancreas-specific promoters (Edlund et al. (1985) Science 230:912-916), and mammary gland-specific promoters (*e.g.*, milk whey promoter; U.S. Patent No. 4,873,316 and European Application Publication No. 264,166). Developmentally-regulated promoters are also encompassed, for example the murine hox promoters (Kessel and Gruss (1990) Science 249:374-379) and the α-fetoprotein promoter (Campes and Tilghman (1989) Genes Dev. 3:537-546).

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to BDSF or STMST mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. *et al.,* Antisense RNA as a molecular tool for genetic analysis, Reviews - Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a BDSF or STMST protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g.*, DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker *(e.g.,* resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding a BDSF or STMST protein or can be introduced on a separate vector. Cells stably transfected with the introduced nucleic acid can be identified by drug selection (*e.g*., cells that have incorporated the selectable marker gene will survive, while the other cells die).

A host cell of the invention, such as a prokaryotic or eukaryotic host cell in culture, can be used to produce (*i.e.,* express) a BDSF or STMST protein. Accordingly, the invention further provides methods for producing a BDSF or STMST protein using the host cells of the invention. In one embodiment, the method comprises culturing the host cell of invention (into which a recombinant expression vector encoding a BDSF or STMST protein has been introduced) in a suitable medium such that a BDSF or STMST protein is produced. In another embodiment, the method further comprises isolating a BDSF or STMST protein from the medium or the host cell.

The host cells of the invention can also be used to produce nonhuman transgenic animals. For example, in one embodiment, a host cell of the invention is a fertilized oocyte or an embryonic stem cell into which BDSF-coding sequences or STMST-coding sequences have been introduced. Such host cells can then be used to create non-human transgenic animals in which exogenous BDSF or STMST sequences have been introduced into their genome or homologous recombinant animals in which endogenous BDSF or STMST sequences have been altered, respectively. Such animals are useful for studying the function and/or activity of a BDSF or STMST and for identifying and/or evaluating modulators of BDSF or STMST activity.

As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous BDSF or STMST gene has been altered by homologous recombination between the endogenous gene and an exogenous DNA molecule introduced into a cell of the animal, *e.g.,* an embryonic cell of the animal, prior to development of the animal.

A transgenic animal of the invention can be created by introducing a BDSF- or STMST-encoding nucleic acid into the male pronuclei of a fertilized oocyte, *e.g.*, by microinjection, retroviral infection, and allowing the oocyte to develop in a pseudopregnant female foster animal. The BDSF cDNA sequence of SEQ ID NO: 1 or the nucleotide sequence of the DNA insert of the plasmid deposited with ATCC as Accession Number 98756 can be introduced as a transgene into the genome of a non-human animal. Likewise, the STMST cDNA sequence of SEQ ID NO: 14, SEQ ID NO:17, the nucleotide sequence of the DNA insert of the plasmid deposited with ATCC as Accession Number or the nucleotide sequence of the DNA insert of the plasmid deposited with ATCC as Accession Number can be introduced as a transgene into the genome of a non-human animal. Alternatively, a nonhuman homologue of a human BDSF gene or STMST gene, such as a mouse BDSF or STMST gene, can be used as a transgene. Alternatively, a BDSF or STMST gene homologue can be isolated based on hybridization to a BDSF or STMST cDNA sequences and used as a transgene.

Intronic sequences and polyadenylation signals can also be included in the transgene to increase the efficiency of expression of the transgene. A tissue-specific regulatory sequence(s) can be operably linked to a BDSF or STMST transgene to direct expression of a BDSF or STMST protein to particular cells. Methods for generating transgenic animals *via* embryo manipulation and microinjection, particularly animals such as mice, have become conventional in the art and are described, for example, in U.S. Patent Nos. 4,736,866 and 4,870,009, both by Leder et al.*,* U.S. Patent No. 4,873,191 by Wagner et al. and in Hogan, B., Manipulating the Mouse Embryo, (Cold Spring Harbor Laboratory Press, Cold Spring Harbor, N.Y., 1986). Similar methods are used for production of other transgenic animals. A transgenic founder animal can be identified based upon the presence of a BDSF or STMST transgene in its genome and/or expression of BDSF or STMST mRNA in tissues or cells of the animals. A transgenic founder animal can then be used to breed additional animals carrying the transgene. Moreover, transgenic animals carrying a transgene encoding a BDSF or STMST protein can further be bred to other transgenic animals carrying other transgenes.

To create a homologous recombinant animal, a vector is prepared which contains at least a portion of a BDSF or STMST gene into which a deletion, addition or substitution has been introduced to thereby alter, *e.g.*, functionally disrupt, the BDSF or STMST gene. The BDSF or STMST gene can be a human gene *(e.g.,* the cDNA of SEQ ID NO:1 or the cDNA sequence of SEQ ID NO:14 or SEQ ID NO:17), but more preferably, is a non-human homologue of a human BDSF or STMST gene such as a murine BDSF or STMST gene. (For example, the mouse BDSF gene of SEQ ID NO:6 can be used to construct a homologous recombination vector suitable for altering an endogenous BDSF gene in the mouse genome.) In a preferred embodiment, the vector is designed such that, upon homologous recombination, the endogenous BDSF or STMST gene is functionally disrupted *(i.e.,* no longer encodes a functional protein; also referred to as a "knock out" vector). Alternatively, the vector can be designed such that, upon homologous recombination, the endogenous BDSF or STMST gene is mutated or otherwise altered but still encodes functional protein (*e.g*., the upstream regulatory region can be altered to thereby alter the expression of the endogenous BDSF or STMST protein).

In the homologous recombination vector, the altered portion of the BDSF or STMST gene is flanked at its 5' and 3' ends by additional nucleic acid sequence of the BDSF or STMST gene to allow for homologous recombination to occur between the exogenous BDSF or STMST gene carried by the vector and an endogenous BDSF or STMST gene in an embryonic stem cell. The additional flanking BDSF or STMST nucleic acid sequence is of sufficient length for successful homologous recombination with the endogenous gene. Typically, several kilobases of flanking DNA (both at the 5' and 3' ends) are included in the vector (see *e.g.*, Thomas, K.R. and Capecchi, M. R. (1987) Cell 51:503 for a description of homologous recombination vectors). The vector is introduced into an embryonic stem cell line (*e.g.*, by electroporation) and cells in which the introduced BDSF or STMST gene has homologously recombined with the endogenous BDSF or STMST gene are selected (see *e.g.,* Li, E. et al. (1992) Cell 69:915). The selected cells are then injected into a blastocyst of an animal (*e.g.*, a mouse) to form aggregation chimeras (see e.g., Bradley, A. in Teratocarcinomas and Embryonic Stem Cells: A Practical Approach, E.J. Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley, A. (1991) Current Opinion in Biotechnology 2:823-829 and in PCT International Publication Nos.: WO 90/11354 by Le Mouellec et al.*;* WO 91/01140 by Smithies et al.*;* WO 92/0968 by Zijlstra et al.*;* and WO 93/04169 by Berns et al.

In another embodiment, transgenic non-humans animals can be produced which contain selected systems which allow for regulated expression of the transgene. One example of such a system is the *crelloxP* recombinase system of bacteriophage P1. For a description of the *crelloxP* recombinase system, see, *e.g.*, Lakso et al. (1992) PNAS 89:6232-6236. Another example of a recombinase system is the FLP recombinase system of *Saccharomyces cerevisiae* (O'Gorman et al. (1991) Science 251:1351-1355. If a *cre*/*loxP* recombinase system is used to regulate expression of the transgene, animals containing transgenes encoding both the *Cre* recombinase and a selected protein are required. Such animals can be provided through the construction of "double" transgenic animals, *e.g.*, by mating two transgenic animals, one containing a transgene encoding a selected protein and the other containing a transgene encoding a recombinase.

Clones of the non-human transgenic animals described herein can also be produced according to the methods described in Wilmut, I. et al. (1997) Nature 385:810-813. In brief, a cell, *e.g.*, a somatic cell, from the transgenic animal can be isolated and induced to exit the growth cycle and enter Gₒ phase. The quiescent cell can then be fused, *e.g.*, through the use of electrical pulses, to an enucleated oocyte from an animal of the same species from which the quiescent cell is isolated. The recontructed oocyte is then cultured such that it develops to morula or blastocyte and then transferred to pseudopregnant female foster animal. The offspring borne of this female foster animal will be a clone of the animal from which the cell, *e.g.*, the somatic cell, is isolated.

### IV. Pharmaceutical Compositions

The BDSF nucleic acid molecules, BDSF proteins, anti-BDSF antibodies, STMST nucleic acid molecules, STMST proteins and anti-STMST antibodies (also referred to herein as "active compounds") of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g.*, intravenous, intradermal, subcutaneous, oral (*e.g.*, inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g.*, a BDSF protein, anti-BDSF antibody, STMST protein or anti-STMST antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.*, a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, *e.g.*, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see *e.g.*, Chen et al. (1994) PNAS 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.*, retroviral vectors, the pharmaceutical preparation can include one or more cells which produce the gene delivery system.

The pharmaceutical compositions can be included in a container, pack, or dispenser together with instructions for administration.

### V. Uses and Methods of the Invention

The nucleic acid molecules, proteins, protein homologues, and antibodies described herein can be used in one or more of the following methods: a) screening assays; b) predictive medicine (*e.g.*, diagnostic assays, prognostic assays, monitoring clinical trials, and pharmacogenetics); and c) methods of treatment (*e.g.*, therapeutic and prophylactic).

As described herein, a BDSF protein of the invention has one or more of the following activities: (i) interaction of a BDSF protein in the extracellular milieu with a non-BDSF protein molecule on the surface of the same cell which secreted the BDSF protein molecule; (ii) interaction of a BDSF protein in the extracellular milieu with a non-BDSF protein molecule on the surface of a different cell from that which secreted the BDSF protein molecule; (iii) complex formation between a BDSF protein and a BDSF receptor; (iv) complex formation between a BDSF protein and non-BDSF receptor; and (v) interaction of a BDSF protein with a second protein in the extracellular milieu, and can thus be used in, for example, (1) modulation of cellular signal transduction, either *in vitro* or *in vivo;* (2) modulation of protein:protein interaction, either *in vitro* or *in vivo;* (3) regulation of cellular proliferation; or (4) regulation of cellular differentiation.

As described herein, an STMST protein of the invention has one or more of the following activities: (i) interaction of an STMST protein with soluble STMST ligand; (ii) interaction of an STMST protein with a membrane-bound non-STMST protein; (iii) interaction of an STMST protein with an intracellular protein (*e.g.*, an intracellular enzyme or signal transduction molecule); and (iv) indirect interaction of an STMST protein with an intracellular protein (*e.g.*, a downstream signal transduction molecule, and can can thus be used in, for example, (1) modulation of cellular signal transduction, either *in vitro* or *in vivo;* (2) regulation of gene transcription in a cell expressing an STMST protein; (3) regulation of gene transcription in a cell expressing an STMST protein, wherein said cell is involved inflammation; (4) regulation of cellular proliferation; (5) regulation of cellular differentiation; (6) regulation of develpoment; (7) regulation of cell death; (8) regulation of inflammation; and (9) regulation of respiratory cell function.

Accordingly, the isolated nucleic acid molecules of the invention can be used, for example, to express BDSF or STMST protein (*e.g., via* a recombinant expression vector in a host cell in gene therapy applications), to detect BDSF or STMST mRNA (*e.g.*, in a biological sample) or a genetic alteration in a BDSF or STMST gene, and to modulate BDSF or STMST activity, as described further below. The BDSF or STMST proteins can be used to treat disorders characterized by insufficient or excessive production of a BDSF, STMST, BDSF target molecule, or STMST target molecule. In addition, the BDSF or STMST proteins can be used to screen for naturally occurring BDSF or STMST target molecules, to screen for drugs or compounds which modulate BDSF or STMST activity, as well as to treat disorders characterized by insufficient or excessive production of BDSF or STMST protein or production of BDSF or STMST protein forms which have decreased or aberrant activity compared to BDSF or STMST wild type protein. Moreover, the anti-BDSF antibodies or anti-STMST antibodies of the invention can be used to detect and isolate BDSF or STMST proteins, regulate the bioavailability of BDSF or STMST proteins, and modulate BDSF or STMST activity.

Accordingly one embodiment of the present invention involves a method of use (*e.g.*, a diagnostic assay, prognostic assay, or a prophylactic/therapeutic method of treatment) wherein a molecule of the present invention (*e.g.*, a BDSF protein, BDSF nucleic acid, BDSF modulator, STMST protein, STMST nucleic acid, STMST ligand or STMST modulator) is used, for example, to diagnose, prognose and/or treat a disease and/or condition in which any of the aforementioned activities is indicated. In another embodiment, the present invention involves a method of use (*e.g.*, a diagnostic assay, prognostic assay, or a prophylactic/therapeutic method of treatment) wherein a molecule of the present invention (*e.g.*, a BDSF protein, BDSF nucleic acid, BDSF modulator, STMST protein, STMST nucleic acid, STMST ligand or STMST modulator) is used, for example, for the diagnosis, prognosis, and/or treatment of subjects, preferably a human subject, in which any of the aforementioned activities is pathologically perturbed. In a preferred embodiment, the methods of use (*e.g*., diagnostic assays, prognostic assays, or prophylactic/therapeutic methods of treatment) involve administering to a subject, preferably a human subject, a molecule of the present invention (*e.g.*, a BDSF protein, BDSF nucleic acid, a BDSF modulator, STMST protein, STMST nucleic acid, STMST ligand or STMST modulator) for the diagnosis, prognosis, and/or therapeutic treatment. In another embodiment, the methods of use (*e.g*., diagnostic assays, prognostic assays, or prophylactic/therapeutic methods of treatment) involve administering to a human subject a molecule of the present invention (*e.g.*, a BDSF protein, BDSF nucleic acid, BDSF modulator, STMST protein, STMST nucleic acid, STMST ligand or STMST modulator).

### A. Screening Assays:

The invention provides a method (also referred to herein as a "screening assay") for identifying modulators, *i.e.,* candidate or test compounds or agents (*e.g.*, peptides, peptidomimetics, small molecules or other drugs) which bind to BDSF or STMST proteins, have a stimulatory or inhibitory effect on, for example, BDSF or STMST expression or BDSF or STMST activity, or have a stimulatory or inhibitory effect on, for example, the activity of an BDSF or STMST target molecule.

In one embodiment, the invention provides assays for screening candidate or test compounds which are target molecules of a BDSF or STMST protein or polypeptide or biologically active portion thereof. In another embodiment, the invention provides assays for screening candidate or test compounds which bind to or modulate the activity of a BDSF or STMST protein or polypeptide or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, K.S. (1997) Anticancer Drug Des. 12:145).

Examples of methods for the synthesis of molecular libraries can be found in the art, for example in: DeWitt et al. (1993) Proc. Natl. Acad Sci. U.S.A. 90:6909; Erb et al. (1994) Proc. Natl. Acad Sci. USA 91:11422; Zuckermann et al. (1994). J. Med Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (*e*.*g*., Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner *supra.*)*.*

### 1. Cell-Based Assays

In one embodiment, an assay is a cell-based assay in which a cell which expresses a BDSF protein or biologically active portion thereof is contacted with a test compound and the ability of the test compound to modulate BDSF activity determined. Determining the ability of the test compound to modulate BDSF activity can be accomplished by monitoring the bioactivity of the BDSF protein or biologically active portion thereof. The cell, for example, can be of mammalian origin or a yeast cell. Determining the ability of the test compound to modulate BDSF activity can be accomplished, for example, by coupling the BDSF protein or biologically active portion thereof with a radioisotope or enzymatic label such that binding of the BDSF protein or biologically active portion thereof to its cognate target molecule can be determined by detecting the labeled BDSF protein or biologically active portion thereof in a complex. For example, compounds (*e.g.*, BDSF protein or biologically active portion thereof) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a compound (*e.g.*, BDSF protein or biologically active portion thereof) to interact with its cognate target molecule without the labeling of any of the interactants. For example, a microphysiometer can be used to detect the interaction of a compound with its cognate target molecule without the labeling of either the compound or the receptor. McConnell, H. M. et al. (1992) Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between compound and receptor.

In a preferred embodiment, the assay comprises contacting a cell which expresses a BDSF protein or biologically active portion thereof, with a target molecule to form an assay mixture, contacting the assay mixture with a test compound, and determining the ability of the test compound to modulate the activity of the BDSF protein or biologically active portion thereof, wherein determining the ability of the test compound to modulate the activity of the BDSF protein or biologically active portion thereof, comprises determining the ability of the test compound to modulate a biological activity of the BDSF expressing cell (*e.g.*, determining the ability of the test compound to modulate signal transduction or protein:protein interactions).

In another preferred embodiment, the assay comprises contacting a cell which is responsive to a BDSF protein or biologically active portion thereof, with a BDSF protein or biologically-active portion thereof, to form an assay mixture, contacting the assay mixture with a test compound, and determining the ability of the test compound to modulate the activity of the BDSF protein or biologically active portion thereof, wherein determining the ability of the test compound to modulate the activity of the BDSF protein or biologically active portion thereof comprises determining the ability of the test compound to modulate a biological activity of the BDSF-responsive cell *(e.g.,* determining the ability of the test compound to modulate signal transduction or protein:protein interactions).

In another embodiment, an assay is a cell-based assay comprising contacting a cell expressing a BDSF target molecule with a test compound and determining the ability of the test compound to modulate (*e.g.* stimulate or inhibit) the activity of the BDSF target molecule. Determining the ability of the test compound to modulate the activity of a BDSF target molecule can be accomplished, for example, by determining the ability of the BDSF protein to bind to or interact with the BDSF target molecule.

Determining the ability of the BDSF protein to bind to or interact with a BDSF target molecule can be accomplished by one of the methods described above for determining direct binding. In a preferred embodiment, determining the ability of the BDSF protein to bind to or interact with a BDSF target molecule can be accomplished by determining the activity of the target molecule. For example, the activity of the target molecule can be determined by detecting induction of a cellular second messenger of the target (*i.e.* intracellular Ca²⁺, diacylglycerol, IP₃, etc.), detecting catalytic/enzymatic activity of the target an appropriate substrate, detecting the induction of a reporter gene (comprising a target-responsive regulatory element operatively linked to a nucleic acid encoding a detectable marker, *e.g.*, luciferase), or detecting a target-regulated cellular response, for example, signal transduction or protein:protein interactions.

In another embodiment, an assay is a cell-based assay in which a cell which expresses an STMST protein on the cell surface is contacted with a test compound and the ability of the test compound to bind to the STMST protein determined. The cell, for example, can be of mammalian origin or a yeast cell. Determining the ability of the test compound to bind to an STMST protein can be accomplished, for example, by coupling the test compound with a radioisotope or enzymatic label such that binding of the test compound to the STMST protein can be determined by detecting the labeled compound in a complex. For example, test compounds can be labeled with ¹²⁵I, ³⁵S, ¹⁴C, or ³H, either directly or indirectly, and the radioisotope detected by direct counting of radioemmission or by scintillation counting. Alternatively, test compounds can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a test compound to interact with an STMST protein without the labeling of any of the interactants. For example, a microphysiometer can be used to detect the interaction of a test compound with an STMST protein without the labeling of either the test compound or the receptor, as described above.

In a preferred embodiment, the assay comprises contacting a cell which expresses an STMST protein or biologically active portion thereof, on the cell surface with an STMST ligand, to form an assay mixture, contacting the assay mixture with a test compound, and determining the ability of the test compound to interact with the STMST protein or biologically active portion thereof, wherein determining the ability of the test compound to interact with the STMST protein or biologically active portion thereof, comprises determining the ability of the test compound to preferentially bind to the STMST protein or biologically active portion thereof, as compared to the ability of the STMST ligand to bind to the STMST protein or biologically active portion thereof.

Determining the ability of the STMST ligand or STMST modulator to bind to or interact with an STMST protein or biologically active portion thereof, can be accomplished by one of the methods described above for determining direct binding. In a preferred embodiment, determining the ability of the STMST ligand or modulator to bind to or interact with an STMST protein or biologically active portion thereof, can be accomplished by determining the activity of an STMST protein or of a downstream STMST target molecule. For example, the target molecule can be a cellular second messenger, and the activity of the target molecule can be determined by detecting induction of of the target *(i.e.* intracellular Ca²⁺, diacylglycerol, IP₃, etc.), detecting catalytic/enzymatic activity of the target on an appropriate substrate, detecting the induction of a reporter gene (comprising an STMST-responsive regulatory element operatively linked to a nucleic acid encoding a detectable marker, *e.g.,* luciferase), or detecting a cellular response, for example, a proliferative response or an inflammatory response. Accordingly, in one embodiment the present invention involves a method of identifying a compound which modulates the activity of an STMST protein, comprising contacting a cell which expresses an STMST protein with a test compound, determining the ability of the test compound to modulate the activity the STMST protein, and identifying the compound as a modulator of STMST activity. In another embodiment, the present invention involves a method of identifying a compound which modulates the activity of an STMST protein, comprising contacting a cell which expresses an STMST protein with a test compound, determining the ability of the test compound to modulate the activity of a downstream STMST target molecule, and identifying the compound as a modulator of STMST activity.

### 2. Cell-Free Assays

In yet another embodiment, an assay of the present invention is a cell-free assay in which a BDSF or STMST protein or biologically active portion thereof is contacted with a test compound and the ability of the test compound to bind to the BDSF or STMST protein or biologically active portion thereof is determined. Binding of the test compound to the BDSF or STMST protein can be determined either directly or indirectly as described above. In a preferred embodiment, the assay includes contacting the BDSF or STMST protein or biologically active portion thereof with a known compound which binds BDSF or STMST (*e.g.*, a BDSF or STMST target molecule) to form an assay mixture, contacting the assay mixture with a test compound, and determining the ability of the test compound to interact with a BDSF or STMST protein, wherein determining the ability of the test compound to interact with a BDSF or STMST protein comprises determining the ability of the test compound to preferentially bind to BDSF or STMST or biologically active portion thereof as compared to the known compound.

In another embodiment, the assay is a cell-free assay in which a BDSF protein or biologically active portion thereof is contacted with a test compound and the ability of the test compound to modulate (*e.g.*, stimulate or inhibit) the activity of the BDSF protein or biologically active portion thereof is determined. Determining the ability of the test compound to modulate the activity of a BDSF protein can be accomplished, for example, by determining the ability of the BDSF protein to bind to a BDSF target molecule by one of the methods described above for determining direct binding. Determining the ability of the BDSF protein to bind to a BDSF target molecule can also be accomplished using a technology such as real-time Biomolecular Interaction Analysis (BIA). Sjolander, S. and Urbaniczky, C. (1991) Anal. Chem. 63:2338-2345 and Szabo et al. (1995) Curr. Opin. Struct. Biol. 5:699-705. As used herein, "BIA" is a technology for studying biospecific interactions in real time, without labeling any of the interactants (*e.g.*, BIAcore). Changes in the optical phenomenon of surface plasmon resonance (SPR) can be used as an indication of real-time reactions between biological molecules.

In an alternative embodiment, determining the ability of the test compound to modulate the activity of a BDSF protein can be accomplished by determining the ability of the BDSF protein to further modulate the activity of a downstream effector (*e.g.*, a growth factor mediated signal transduction pathway component) of a BDSF target molecule. For example, the activity of the effector molecule on an appropriate target can be determined or the binding of the effector to an appropriate target can be determined as previously described.

In yet another embodiment, the cell-free assay involves contacting a BDSF protein or biologically active portion thereof with a known compound which binds the BDSF protein to form an assay mixture, contacting the assay mixture with a test compound, and determining the ability of the test compound to interact with the BDSF protein, wherein determining the ability of the test compound to interact with the BDSF protein comprises determining the ability of the BDSF protein to preferentially bind to or modulate the activity of a BDSF target molecule.

In yet another embodiment, an assay of the present invention is a cell-free assay in which an STMST protein or biologically active portion thereof is contacted with a test compound and the ability of the test compound to bind to the STMST protein or biologically active portion thereof is determined. Binding of the test compound to the STMST protein can be determined either directly or indirectly or using BIA as described above.

In a preferred embodiment, the assay includes contacting the STMST protein or biologically active portion thereof with a known ligand which binds STMST to form an assay mixture, contacting the assay mixture with a test compound, and determining the ability of the test compound to interact with an STMST protein, wherein determining the ability of the test compound to interact with an STMST protein comprises determining the ability of the test compound to preferentially bind to STMST or biologically active portion thereof as compared to the known ligand.

In another embodiment, the assay is a cell-free assay in which an STMST protein or biologically active portion thereof is contacted with a test compound and the ability of the test compound to modulate (*e.g.*, stimulate or inhibit) the activity of the STMST protein or biologically active portion thereof is determined. Determining the ability of the test compound to modulate the activity of an STMST protein can be accomplished, for example, by determining the ability of the STMST protein to modulate the activity of a downstream STMST target molecule by one of the methods described above for cell-based assays. For example, the catalytic/enzymatic activity of the target molecule on an appropriate substrate can be determined as previously described.

In yet another embodiment, the cell-free assay involves contacting an STMST protein or biologically active portion thereof with a known ligand which binds the STMST protein to form an assay mixture, contacting the assay mixture with a test compound, and determining the ability of the test compound to interact with the STMST protein, wherein determining the ability of the test compound to interact with the STMST protein comprises determining the ability of the test compound to preferentially bind to or modulate the activity of an STMST target molecule, as compared to the known ligand.

The cell-free assays of the present invention are amenable to use of both soluble and/or membrane-bound forms of isolated proteins (*e.g.* BDSF or STMST proteins or biologically active portions thereof or receptors to which BDSF targets bind or STMST proteins or biologically active portions thereof or STMST proteins). In the case of cell-free assays in which a membrane-bound form an isolated protein is used (*e.g.*, a cell surface receptor) it may be desirable to utilize a solubilizing agent such that the membrane-bound form of the isolated protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In more than one embodiment of the above assay methods of the present invention, it may be desirable to immobilize either BDSF or STMST or their target molecule to facilitate separation of complexed from uncomplexed forms of one or both of the proteins, as well as to accommodate automation of the assay. Binding of a test compound to a BDSF or STMST protein, or interaction of a BDSF or STMST protein with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, GST/ BDSF fusion proteins, GST/STMST fusion proteins or GST/target fusion proteins can be adsorbed onto glutathione sepharose beads (Sigma Chemical, St. Louis, MO) or glutathione derivatized microtitre plates, which are then combined with the test compound or the test compound and either the non-adsorbed target protein, BDSF or STMST protein, and the mixture incubated under conditions conducive to complex formation (*e.g.*, at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of BDSF or STMST binding or activity determined using standard techniques.

Other techniques for immobilizing proteins on matrices can also be used in the screening assays of the invention. For example, a BDSF protein. STMST fusion protein, a BDSF target molecule or STMST target molecule can be immobilized utilizing conjugation of biotin and streptavidin. Biotinylated BDSF or STMST protein or target molecules can be prepared from biotin-NHS (N-hydroxy-succinimide) using techniques well known in the art (*e.g.*, biotinylation kit, Pierce Chemicals, Rockford, IL), and immobilized in the wells of streptavidin-coated 96 well plates (Pierce Chemical). Alternatively, antibodies reactive with BDSF protein, STMST proteins or target molecules but which do not interfere with binding of the BDSF or STMST protein to its target molecule can be derivatized to the wells of the plate, and unbound target or BDSF or STMST protein trapped in the wells by antibody conjugation. Methods for detecting such complexes, in addition to those described above for the GST-immobilized complexes, include immunodetection of complexes using antibodies reactive with the BDSF protein, STMST protein or target molecules, as well as enzyme-linked assays which rely on detecting an enzymatic activity associated with the BDSF protein, STMST protein or respective target molecule.

In another embodiment, modulators of BDSF or STMST expression are identified in a method wherein a cell is contacted with a candidate compound and the expression of BDSF or STMST mRNA or protein in the cell is determined. The level of expression of BDSF or STMST mRNA or protein in the presence of the candidate compound is compared to the level of expression of BDSF or STMST mRNA or protein in the absence of the candidate compound. The candidate compound can then be identified as a modulator of BDSF or STMST expression based on this comparison. For example, when expression of BDSF or STMST mRNA or protein is greater (statistically significantly greater) in the presence of the candidate compound than in its absence, the candidate compound is identified as a stimulator of BDSF or STMST mRNA or protein expression. Alternatively, when expression of BDSF or STMST mRNA or protein is less (statistically significantly less) in the presence of the candidate compound than in its absence, the candidate compound is identified as an inhibitor of BDSF or STMST mRNA or protein expression. The level of BDSF or STMST mRNA or protein expression in the cells can be determined by methods described herein for detecting BDSF or STMST mRNA or protein.

In yet another aspect of the invention, the BDSF or STMST proteins can be used as "bait proteins" in a two-hybrid assay or three-hybrid assay (see, e.g., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with BDSF or STMST ("BDSF-binding proteins", "BDSF-bp", "STMST-binding proteins" or "STMST-bp") and are involved in BDSF or STMST activity. Such binding proteins are also likely to be involved in the propagation of signals by the BDSF proteins, STMST proteins or respective targets as, for example, downstream elements of a BDSF- or STMST-mediated signaling pathway. Alternatively, such BDSF-binding proteins or STMST-binding proteins are likely to be BDSF or STMST inhibitors.

The two-hybrid system is based on the modular nature of most transcription factors, which consist of separable DNA-binding and activation domains. Briefly, the assay utilizes two different DNA constructs. In one construct, the gene that codes for a BDSF or STMST protein is fused to a gene encoding the DNA binding domain of a known transcription factor (*e.g.*, GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a BDSF-dependent complex or STMST-dependent complex, the DNA-binding and activation domains of the transcription factor are brought into close proximity. This proximity allows transcription of a reporter gene (*e.g.*, LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the BDSF or STMST protein.

This invention further pertains to novel agents identified by the above-described screening assays and to processes for producing such agents by use of these assays. Accordingly, in one embodiment, the present invention includes a compound or agent obtainable by a method comprising the steps of any one of the aformentioned screening assays (*e.g.*, cell-based assays or cell-free assays). For example, in one embodiment, the invention includes a compound or agent obtainable by a method comprising contacting a cell which expresses a BDSF or STMST target molecule with a test compound and the determining the ability of the test compound to bind to, or modulate the activity of, the BDSF or STMST target molecule. In another embodiment, the invention includes a compound or agent obtainable by a method comprising contacting a cell which expresses a BDSF or STMST target molecule with a BDSF or STMST protein or biologically-active portion thereof, to form an assay mixture, contacting the assay mixture with a test compound, and determining the ability of the test compound to interact with, or modulate the activity of, the BDSF or STMST target molecule. In another embodiment, the invention includes a compound or agent obtainable by a method comprising contacting a BDSF or STMST protein or biologically active portion thereof with a test compound and determining the ability of the test compound to bind to, or modulate (*e.g.*, stimulate or inhibit) the activity of, the BDSF or STMST protein or biologically active portion thereof. In yet another embodiment, the present invention included a compound or agent obtainable by a method comprising contacting a BDSF or STMST protein or biologically active portion thereof with a known compound which binds the BDSF or STMST protein to form an assay mixture, contacting the assay mixture with a test compound, and determining the ability of the test compound to interact with, or modulate the activity of the BDSF or STMST protein.

Accordingly, it is within the scope of this invention to further use an agent identified as described herein in an appropriate animal model. For example, an agent identified as described herein (*e.g.*, a BDSF or STMST modulating agent, an antisense BDSF or STMST nucleic acid molecule, a BDSF-specific or STMST-specific antibody, or a BDSF- or STMST-binding partner) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

The present inventon also pertains to uses of novel agents identified by the above-described screening assays for diagnoses, prognoses, and treatments as described herein. Accordingly, it is within the scope of the present invention to use such agents in the design, formulation, synthesis, manufacture, and/or production of a drug or pharmaceutical composition for use in diagnosis, prognosis, or treatment, as described herein. For example, in one embodiment, the present invention includes a method of synthesizing or producing a drug or pharmaceutical composition by reference to the structure and/or properties of a compound obtainable by one of the above-described screening assays. For example, a drug or pharmaceutical composition can be synthesized based on the structure and/or properties of a compound obtained by a method in which a cell which expresses a BDSF or STMST target molecule is contacted with a test compound and the ability of the test compound to bind to, or modulate the activity of, the BDSF or STMST target molecule is determined. In another exemplary embodiment, the present invention includes a method of synthesizing or producing a drug or pharmaceutical composition based on the structure and/or properties of a compound obtainable by a method in which a BDSF or STMST protein or biologically active portion thereof is contacted with a test compound and the ability of the test compound to bind to, or modulate (*e.g.*, stimulate or inhibit) the activity of, the BDSF or STMST protein or biologically active portion thereof is determined.

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. These applications are described in the subsections below.

### 1. Chromosome Mapping

Once the sequence (or a portion of the sequence) of a gene has been isolated, this sequence can be used to map the location of the gene on a chromosome. This process is called chromosome mapping. Accordingly, portions or fragments of the BDSF or STMST nucleotide sequences, described herein, can be used to map the location of the BDSF or STMST genes on a chromosome. The mapping of the BDSF or STMST sequences to chromosomes is an important first step in correlating these sequences with genes associated with disease.

Briefly, BDSF or STMST genes can be mapped to chromosomes by preparing PCR primers (preferably 15-25 bp in length) from the BDSF or STMST nucleotide sequences. Computer analysis of the BDSF or STMST sequences can be used to predict primers that do not span more than one exon in the genomic DNA, thus complicating the amplification process. These primers can then be used for PCR screening of somatic cell hybrids containing individual human chromosomes. Only those hybrids containing the human gene corresponding to the BDSF or STMST sequences will yield an amplified fragment.

Somatic cell hybrids are prepared by fusing somatic cells from different mammals (*e.g.*, human and mouse cells). As hybrids of human and mouse cells grow and divide, they gradually lose human chromosomes in random order, but retain the mouse chromosomes. By using media in which mouse cells cannot grow, because they lack a particular enzyme, but human cells can, the one human chromosome that contains the gene encoding the needed enzyme, will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. (D'Eustachio P. et al. (1983) Science 220:919-924). Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the BDSF or STMST nucleotide sequences to design oligonucleotide primers, sublocalization can be achieved with panels of fragments from specific chromosomes. Other mapping strategies which can similarly be used to map a 9o, 1p, or 1v sequence to its chromosome include *in situ* hybridization (described in Fan, Y. et al. (1990) PNAS, 87:6223-27), pre-screening with labeled flow-sorted chromosomes, and pre-selection by hybridization to chromosome specific cDNA libraries.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical such as colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma et al., Human Chromosomes: A Manual of Basic Techniques (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. et al. (1987) Nature, 325:783-787.

Moreover, differences in the DNA sequences between individuals affected and unaffected with a disease associated with the BDSF or STMST gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### 2. Tissue Typing

The BDSF or STMST sequences of the present invention can also be used to identify individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identification. This method does not suffer from the current limitations of "Dog Tags" which can be lost, switched, or stolen, making positive identification difficult. The sequences of the present invention are useful as additional DNA markers for RFLP (described in U.S. Patent 5,272,057).

Furthermore, the sequences of the present invention can be used to provide an alternative technique which determines the actual base-by-base DNA sequence of selected portions of an individual's genome. Thus, the BDSF or STMST nucleotide sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The BDSF or STMST nucleotide sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1, SEQ ID NO:6, SEQ ID NO:14 or SEQ ID NO:17, can comfortably provide positive individual identification with a panel of perhaps 10 to 1,000 primers which each yield a noncoding amplified sequence of 100 bases. If predicted coding sequences, such as those in SEQ ID NO:3, SEQ ID NO:8, SEQ ID NO: 16 or SEQ ID NO: 19 are used, a more appropriate number of primers for positive individual identification would be 500-2,000.

If a panel of reagents from BDSF or STMST nucleotide sequences described herein is used to generate a unique identification database for an individual, those same reagents can later be used to identify tissue from that individual. Using the unique identification database, positive identification of the individual, living or dead, can be made from extremely small tissue samples.

### 3. Use of Partial BDSF or STMST Sequences in Forensic Biology

DNA-based identification techniques can also be used in forensic biology. Forensic biology is a scientific field employing genetic typing of biological evidence found at a crime scene as a means for positively identifying, for example, a perpetrator of a crime. To make such an identification, PCR technology can be used to amplify DNA sequences taken from very small biological samples such as tissues, e.g., hair or skin, or body fluids, *e.g.*, blood, saliva, or semen found at a crime scene. The amplified sequence can then be compared to a standard, thereby allowing identification of the origin of the biological sample.

The sequences of the present invention can be used to provide polynucleotide reagents, *e.g.*, PCR primers, targeted to specific loci in the human genome, which can enhance the reliability of DNA-based forensic identifications by, for example, providing another "identification marker" (*i.e.* another DNA sequence that is unique to a particular individual). As mentioned above, actual base sequence information can be used for identification as an accurate alternative to patterns formed by restriction enzyme generated fragments. Sequences targeted to noncoding regions of SEQ ID NO: 1 are particularly appropriate for this use as greater numbers of polymorphisms occur in the noncoding regions, making it easier to differentiate individuals using this technique. Examples of polynucleotide reagents include the BDSF or STMST nucleotide sequences or portions thereof, *e.g.*, fragments derived from the noncoding regions of SEQ ID NO:1, SEQ ID NO:6, SEQ ID NO:14 or SEQ ID NO:17 having a length of at least 20 bases, preferably at least 30 bases.

The BDSF or STMST nucleotide sequences described herein can further be used to provide polynucleotide reagents, *e.g.,* labeled or labelable probes which can be used in, for example, an *in situ* hybridization technique, to identify a specific tissue, *e.g.,* brain tissue. This can be very useful in cases where a forensic pathologist is presented with a tissue of unknown origin. Panels of such BDSF or STMST probes can be used to identify tissue by species and/or by organ type.

In a similar fashion, these reagents, *e.g.*, BDSF or STMST primers or probes can be used to screen tissue culture for contamination *(i.e.* screen for the presence of a mixture of different types of cells in a culture).

### C. Predictive Medicine:

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining BDSF or STMST protein and/or nucleic acid expression as well as BDSF or STMST activity, in the context of a biological sample (*e.g.*, blood, serum, cells, tissue) to thereby determine whether an individual is afflicted with a disease or disorder, or is at risk of developing a disorder, associated with aberrant BDSF or STMST expression or activity. The invention also provides for prognostic (or predictive) assays for determining whether an individual is at risk of developing a disorder associated with BDSF or STMST protein, nucleic acid expression or activity. For example, mutations in a BDSF or STMST gene can be assayed in a biological sample. Such assays can be used for prognostic or predictive purpose to thereby phophylactically treat an individual prior to the onset of a disorder characterized by or associated with BDSF or STMST protein, nucleic acid expression or activity.

Another aspect of the invention pertains to monitoring the influence of agents (*e.g.*, drugs, compounds) on the expression or activity of BDSF in clinical trials.

These and other agents are described in further detail in the following sections.

### 1. Diagnostic Assays

An exemplary method for detecting the presence or absence of BDSF or STMST protein or nucleic acid in a biological sample involves obtaining a biological sample from a test subject and contacting the biological sample with a compound or an agent capable of detecting BDSF or STMST protein or nucleic acid (*e.g.*, mRNA, genomic DNA) that encodes BDSF or STMST protein such that the presence of BDSF or STMST protein or nucleic acid is detected in the biological sample. A preferred agent for detecting BDSF or STMST mRNA or genomic DNA is a labeled nucleic acid probe capable of hybridizing to BDSF or STMST mRNA or genomic DNA. The nucleic acid probe can be, for example, a full-length BDSF nucleic acid, or a portion thereof, such as an oligonucleotide of at least 15, 30, 50, 100, 250 or 500 nucleotides in length and sufficient to specifically hybridize under stringent conditions to BDSF or STMST mRNA or genomic DNA. Other suitable probes for use in the diagnostic assays of the invention are described herein.

A preferred agent for detecting BDSF or STMST protein is an antibody capable of binding to BDSF or STMST protein, preferably an antibody with a detectable label. Antibodies can be polyclonal, or more preferably, monoclonal. An intact antibody, or a fragment thereof (*e.g.*, Fab or F(ab')₂) can be used. The term "labeled", with regard to the probe or antibody, is intended to encompass direct labeling of the probe or antibody by coupling *(i.e.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect BDSF or STMST mRNA, protein, or genomic DNA in a biological sample *in vitro* as well as *in vivo.* For example, *in vitro* techniques for detection of BDSF or STMST mRNA include Northern hybridizations and *in situ* hybridizations. *In vitro* techniques for detection of BDSF or STMST protein include enzyme linked immunosorbent assays (ELISAs), Western blots, immunoprecipitations and immunofluorescence. *In vitro* techniques for detection of BDSF or STMST genomic DNA include Southern hybridizations. Furthermore, *in vivo* techniques for detection of BDSF or STMST protein include introducing into a subject a labeled anti-BDSF or anti-STMST antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a serum sample isolated by conventional means from a subject.

In another embodiment, the methods further involve obtaining a control biological sample from a control subject, contacting the control sample with a compound or agent capable of detecting BDSF or STMST protein, mRNA, or genomic DNA, such that the presence of BDSF or STMST protein, mRNA or genomic DNA is detected in the biological sample, and comparing the presence of BDSF or STMST protein, mRNA or genomic DNA in the control sample with the presence of BDSF or STMST protein, mRNA or genomic DNA in the test sample.

The invention also encompasses kits for detecting the presence of BDSF or STMST in a biological sample. For example, the kit can comprise a labeled compound or agent capable of detecting BDSF or STMST protein or mRNA in a biological sample; means for determining the amount of BDSF or STMST in the sample; and means for comparing the amount of BDSF or STMST in the sample with a standard. The compound or agent can be packaged in a suitable container. The kit can further comprise instructions for using the kit to detect BDSF or STMST protein or nucleic acid.

### 2. Prognostic Assays

The diagnostic methods described herein can furthermore be utilized to identify subjects having or at risk of developing a disease or disorder associated with aberrant BDSF or STMST expression or activity. For example, the assays described herein, such as the preceding diagnostic assays or the following assays, can be utilized to identify a subject having or at risk of developing a disorder associated with BDSF protein, nucleic acid expression or activity such as a proliferative disorder (*e.g.*, cancer). Alternatively, the assays described herein, such as the preceding diagnostic assays or the following assays, can be utilized to identify a subject having or at risk of developing a disorder associated with BDSF protein, nucleic acid expression or activity such as a proliferative disorder. Alternatively, the prognostic assays can be utilized to identify a subject having or at risk for developing a differentiative disorder. Thus, the present invention provides a method for identifying a disease or disorder associated with aberrant BDSF or STMST expression or activity in which a test sample is obtained from a subject and BDSF or STMST protein or nucleic acid (e.g, mRNA, genomic DNA) is detected, wherein the presence of BDSF or STMST protein or nucleic acid is diagnostic for a subject having or at risk of developing a disease or disorder associated with aberrant BDSF or STMST expression or activity. As used herein, a "test sample" refers to a biological sample obtained from a subject of interest. For example, a test sample can be a biological fluid (*e.g.*, serum), cell sample, or tissue.

Furthermore, the prognostic assays described herein can be used to determine whether a subject can be administered an agent (*e.g.*, an agonist, antagonist, peptidomimetic, protein, peptide, nucleic acid, small molecule, or other drug candidate) to treat a disease or disorder associated with aberrant BDSF or STMST expression or activity. For example, such methods can be used to determine whether a subject can be effectively treated with an agent for a proliferative disorder (*e.g.*, cancer), a differentiative disorder or an immune disorder. Thus, the present invention provides methods for determining whether a subject can be effectively treated with an agent for a disorder associated with aberrant BDSF or STMST expression or activity in which a test sample is obtained and BDSF or STMST protein or nucleic acid expression or activity is detected (*e.g.*, wherein the abundance of BDSF or STMST protein or nucleic acid expression or activity is diagnostic for a subject that can be administered the agent to treat a disorder associated with aberrant BDSF or STMST expression or activity.)

The methods of the invention can also be used to detect genetic alterations in a BDSF or STMST gene, thereby determining if a subject with the altered gene is at risk for a disorder characterized by an aberrant proliferative response. In preferred embodiments, the methods include detecting, in a sample of cells from the subject, the presence or absence of a genetic alteration characterized by at least one of an alteration affecting the integrity of a gene encoding a BDSF or STMST-protein, or the mis- expression of the BDSF or STMST gene. For example, such genetic alterations can be detected by ascertaining the existence of at least one of 1) a deletion of one or more nucleotides from a BDSF or STMST gene; 2) an addition of one or more nucleotides to a BDSF or STMST gene; 3) a substitution of one or more nucleotides of a BDSF or STMST gene, 4) a chromosomal rearrangement of a BDSF or STMST gene; 5) an alteration in the level of a messenger RNA transcript of a BDSF or STMST gene, 6) aberrant modification of a BDSF or STMST gene, such as of the methylation pattern of the genomic DNA, 7) the presence of a non-wild type splicing pattern of a messenger RNA transcript of a BDSF or STMST gene, 8) a non-wild type level of a BDSF or STMST-protein, 9) allelic loss of a BDSF or STMST gene, and 10) inappropriate post-translational modification of a BDSF or STMST-protein. As described herein, there are a large number of assay techniques known in the art which can be used for detecting alterations in a BDSF or STMST gene. A preferred biological sample is a tissue or serum sample isolated by conventional means from a subject.

In certain embodiments, detection of the alteration involves the use of a probe/primer in a polymerase chain reaction (PCR) (see, *e.g.*, U.S. Patent Nos. 4,683,195 and 4,683,202), such as anchor PCR or RACE PCR, or, alternatively, in a ligation chain reaction (LCR) (see, *e.g.*, Landegran et al. (1988) Science 241:1077-1080; and Nakazawa et al. (1994) PNAS 91:360-364), the latter of which can be particularly useful for detecting point mutations in the BDSF or STMST-gene (see Abravaya et al. (1995) Nucleic Acids Res .23:675-682). This method can include the steps of collecting a sample of cells from a patient, isolating nucleic acid (*e.g.*, genomic, mRNA or both) from the cells of the sample, contacting the nucleic acid sample with one or more primers which specifically hybridize to a BDSF or STMST gene under conditions such that hybridization and amplification of the BDSF or STMST-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al.., 1990, Proc. Natl. Acad. Sci. USA 87:1874-1878), transcriptional amplification system (Kwoh, D.Y. et al., 1989, Proc. Natl. Acad. Sci. USA 86:1173-1177), Q-Beta Replicase (Lizardi, P.M. et all, 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a BDSF or STMST gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in BDSF can be identified by hybridizing a sample and control nucleic acids, *e.g.*, DNA or RNA, to high density arrays containing hundreds or thousands of oligonucleotides probes (Cronin, M.T. et al. (1996) Human Mutation 7: 244-255; Kozal, M.J. et al. (1996) Nature Medicine 2: 753-759). For example, genetic mutations in BDSF or STMST can be identified in two dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential ovelapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the BDSF or STMST gene and detect mutations by comparing the sequence of the sample BDSF or STMST with the corresponding wild-type (control) sequence. Examples of sequencing reactions include those based on techniques developed by Maxim and Gilbert ((1977) PNAS 74:560) or Sanger ((1977) PNAS 74:5463). It is also contemplated that any of a variety of automated sequencing procedures can be utilized when performing the diagnostic assays ((1995) Biotechniques 19:448), including sequencing by mass spectrometry (see, *e.g.,* PCT International Publication No. WO 94/16101; Cohen et al. (1996) Adv. Chromatogr. 36:127-162; and Griffin et al. (1993) Appl. Biochem. Biotechnol. 38:147-159).

Other methods for detecting mutations in the BDSF or STMST gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes of formed by hybridizing (labeled) RNA or DNA containing the wild-type BDSF or STMST sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S 1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl Acad Sci USA 85:4397; Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in BDSF or STMST cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on a BDSF sequence, *e.g.*, a wild-type BDSF or STMST sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in BDSF or STMST genes. For example, single strand conformation polymorphism (SSCP) may be used to detect differences in electrophoretic mobility between mutant and wild type nucleic acids (Orita et al. (1989) Proc Natl. Acad. Sci USA: 86:2766, see also Cotton (1993) Mutat Res 285:125-144; and Hayashi (1992) Genet Anal Tech Appl 9:73-79). Single-stranded DNA fragments of sample and control BDSF or STMST nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) Proc. Natl Acad. Sci USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, *e.g.,* in clinical settings to diagnose patients exhibiting symptoms or family history of a disease or illness involving a BDSF or STMST gene.

Furthermore, any cell type or tissue in which BDSF is expressed may be utilized in the prognostic assays described herein.

### 3.Monitoring of Effects During Clinical Trials

Monitoring the influence of agents (*e.g.*, drugs, compounds) on the expression or activity of a BDSF or STMST protein (*e.g.,* modulation of angiogenesis or of an inflammatory response) an be applied not only in basic drug screening, but also in clinical trials. For example, the effectiveness of an agent determined by a screening assay as described herein to increase BDSF or STMST gene expression, protein levels, or upregulate BDSF or STMST activity, can be monitored in clinical trials of subjects exhibiting decreased BDSF or STMST gene expression, protein levels, or downregulated BDSF or STMST activity. Alternatively, the effectiveness of an agent determined by a screening assay to decrease BDSF or STMST gene expression, protein levels, or downregulate BDSF or STMST activity, can be monitored in clinical trials of subjects exhibiting increased BDSF or STMST gene expression, protein levels, or upregulated BDSF or STMST activity. In such clinical trials, the expression or activity of a BDSF or STMST gene, and preferably, other genes that have been implicated in, for example, a proliferative disorder can be used as a "read out" or markers of the phenotype of a particular cell.

For example, and not by way of limitation, genes, including BDSF or STMST, that are modulated in cells by treatment with an agent (*e.g.*, compound, drug or small molecule) which modulates BDSF or STMST activity (*e.g.*, identified in a screening assay as described herein) can be identified. Thus, to study the effect of agents on proliferative disorders, for example, in a clinical trial, cells can be isolated and RNA prepared and analyzed for the levels of expression of BDSF or STMST and other genes implicated in the proliferative disorder, respectively. The levels of gene expression *(i.e.,* a gene expression pattern) can be quantified by Northern blot analysis or RT-PCR, as described herein, or alternatively by measuring the amount of protein produced, by one of the methods as described herein, or by measuring the levels of activity of BDSF or STMST or other genes. In this way, the gene expression pattern can serve as a marker, indicative of the physiological response of the cells to the agent. Accordingly, this response state may be determined before, and at various points during treatment of the individual with the agent.

In a preferred embodiment, the present invention provides a method for monitoring the effectiveness of treatment of a subject with an agent (*e.g.*, an agonist, antagonist, peptidomimetic, protein, peptide, nucleic acid, small molecule, or other drug candidate identified by the screening assays described herein) comprising the steps of (i) obtaining a pre-administration sample from a subject prior to administration of the agent; (ii) detecting the level of expression of a BDSF or STMST protein, mRNA, or genomic DNA in the preadministration sample; (iii) obtaining one or more post-administration samples from the subject; (iv) detecting the level of expression or activity of the BDSF or STMST protein, mRNA, or genomic DNA in the post-administration samples; (v) comparing the level of expression or activity of the BDSF or STMST protein, mRNA, or genomic DNA in the pre-administration sample with the BDSF or STMST protein, mRNA, or genomic DNA in the post administration sample or samples; and (vi) altering the administration of the agent to the subject accordingly. For example, increased administration of the agent may be desirable to increase the expression or activity of BDSF or STMST to higher levels than detected, *i.e.,* to increase the effectiveness of the agent. Alternatively, decreased administration of the agent may be desirable to decrease expression or activity of BDSF or STMST to lower levels than detected, *i.e.* to decrease the effectiveness of the agent. According to such an embodiment, BDSF expression or activity may be used as an indicator of the effectiveness of an agent, even in the absence of an observable phenotypic response.

### C. Methods of Treatment:

The present invention provides for both prophylactic and therapeutic methods of treating a subject at risk of (or susceptible to) a disorder or having a disorder associated with aberrant BDSF or STMST expression or activity. With regards to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (*e.g.,* a patient's "drug response phenotype", or "drug response genotype".) Thus, another aspect of the invention provides methods for tailoring an individual's prophylactic or therapeutic treatment with either the BDSF or STMST molecules of the present invention or BDSF or STMST modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

### 1. Prophylactic Methods

In one aspect, the invention provides a method for preventing in a subject, a disease or condition associated with an aberrant BDSF or STMST expression or activity, by administering to the subject a BDSF or STMST or an agent which modulates BDSF or STMST expression or at least one BDSF or STMST activity. Subjects at risk for a disease which is caused or contributed to by aberrant BDSF or STMST expression or activity can be identified by, for example, any or a combination of diagnostic or prognostic assays as described herein. Administration of a prophylactic agent can occur prior to the manifestation of symptoms characteristic of the BDSF or STMST aberrancy, such that a disease or disorder is prevented or, alternatively, delayed in its progression. Depending on the type of BDSF or STMST aberrancy, for example, a BDSF or STMST, BDSF or STMST agonist or BDSF or STMST antagonist agent can be used for treating the subject. The appropriate agent can be determined based on screening assays described herein. The prophylactic methods of the present invention are further discussed in the following subsections.

### 2. Therapeutic Methods

Another aspect of the invention pertains to methods of modulating BDSF or STMST expression or activity for therapeutic purposes. Accordingly, in an exemplary embodiment, the modulatory method of the invention involves contacting a cell with a BDSF or STMST or agent that modulates one or more of the activities of BDSF or STMST protein activity associated with the cell. An agent that modulates BDSF or STMST protein activity can be an agent as described herein, such as a nucleic acid or a protein, a naturally-occurring target molecule of a BDSF or STMST protein, a BDSF or STMST antibody, a BDSF or STMST agonist or antagonist, a peptidomimetic of a BDSF or STMST agonist or antagonist, or other small molecule. In one embodiment, the agent stimulates one or more BDSF or STMST activities. Examples of such stimulatory agents include active BDSF or STMST protein and a nucleic acid molecule encoding BDSF or STMST that has been introduced into the cell. In another embodiment, the agent inhibits one or more BDSF or STMST activites. Examples of such inhibitory agents include antisense BDSF or STMST nucleic acid molecules, anti-BDSF or STMST antibodies, and BDSF or STMST inhibitors. These modulatory methods can be performed *in vitro* (*e.g.,* by culturing the cell with the agent) or, alternatively, *in vivo* (e.g, by administering the agent to a subject). As such, the present invention provides methods of treating an individual afflicted with a disease or disorder characterized by aberrant expression or activity of a BDSF or STMST protein or nucleic acid molecule. In one embodiment, the method involves administering an agent (*e.g.*, an agent identified by a screening assay described herein), or combination of agents that modulates (*e.g.*, upregulates or downregulates) BDSF or STMST expression or activity. In another embodiment, the method involves administering a BDSF or STMST protein or nucleic acid molecule as therapy to compensate for reduced or aberrant BDSF or STMSTexpression or activity.

Stimulation of BDSF activity is desirable in situations in which BDSF or STMST is abnormally downregulated and/or in which increased BDSF or STMST activity is likely to have a beneficial effect. For example, stimulation of BDSF or STMST activity is desirable in situations in which a BDSF or STMST is downregulated and/or in which increased BDSF or STMST activity is likely to have a beneficial effect. Likewise, inhibition of BDSF or STMST activity is desirable in situations in which BDSF or STMST is abnormally upregulated and/or in which decreased BDSF or STMST activity is likely to have a beneficial effect.

### 3. Pharmacogenomics

The BDSF or STMST molecules of the present invention, as well as agents, or modulators which have a stimulatory or inhibitory effect on BDSF or STMST activity (*e.g.*, BDSF or STMST gene expression) as identified by a screening assay described herein can be administered to individuals to treat (prophylactically or therapeutically) disorders (e.g, cancer) associated with aberrant BDSF or STMST activity. In conjunction with such treatment, pharmacogenomics *(i.e.,* the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a BDSF or STMST molecule or BDSF or STMST modulator as well as tailoring the dosage and/or therapeutic regimen of treatment with a BDSF or STMST molecule or BDSF or STMST modulator.

Pharmacogenomics deals with clinically significant hereditary variations in the response to drugs due to altered drug disposition and abnormal action in affected persons. See *e.g.*, Eichelbaum, M., Clin Exp Pharmacol Physiol, 1996, 23(10-11) :983-985 and Linder, M.W., Clin Chem, 1997,43(2):254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (*e.g.*, a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach", can be utilized to identify genes that predict drug response. According to this method, if a gene that encodes a drugs target is known (*e.g.*, a BDSF or STMST protein or a BDSF receptor of the present invention), all common variants of that gene can be fairly easily identified in the population and it can be determined if having one version of the gene versus another is associated with a particular drug response.

As an illustrative embodiment, the activity of drug metabolizing enzymes is a major determinant of both the intensity and duration of drug action. The discovery of genetic polymorphisms of drug metabolizing enzymes (*e.g.*, N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Alternatively, a method termed the "gene expression profiling", can be utilized to identify genes that predict drug response. For example, the gene expression of an animal dosed with a drug (*e.g.,* a BDSF or STMST molecule or BDSF or STMST modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment an individual. This knowledge, when applied to dosing or drug selection, can avoid adverse reactions or therapeutic failure and thus enhance therapeutic or prophylactic efficiency when treating a subject with a BDSF or STMST molecule or BDSF modulator, such as a modulator identified by one of the exemplary screening assays described herein.

This invention is further illustrated by the following examples which should not be construed as limiting. The contents of all references, patents and published patent applications cited throughout this application are incorporated herein by reference. References throughout the instant specification to websites maintained as part of the World Wide Web are referred to herein by the prefix http://. The information contained in such websites is publically-avialable and can be accessed elctronically by contacting the cited address.

### EXAMPLES

The novel genes exemplified herein were isolated utilizing a methodology which takes advantage of the fact that a majority of secreted and membrane-associated proteins posess at their amino termini a signal sequence, as defined herein. The methodology identifies such secreted and/or membrane-associated proteins by virtue of their ability to direct export of a reporter protein, alkaline phosphatase (AP), from mammalian cells. The above described methology, referred to herein as "signal trapping" or "signal sequence trapping" is described in detail in PCT/US97/20201 (WO98/22491 published May 28, 1998), the content of which is incorporated herein in its entirety. The present methodology is further combined with an improved method for cDNA library construction in which directional random promed cDNA libraries are prepared.

### Example 1: Identification and Characterization of Human and Murine BDSF cDNA

In this example, the identification and characterization of the genes encoding human BDSF-1 (also referred to as "TANGO 122" and "hT122") and murine BDSF-1 (also referred to as "mT122") are described.

### Isolation of the human BDSF cDNA

The invention is based, at least in part, on the discovery of a human gene encoding a novel brain-derived signaling factor protein, referred to herein as BDSF.

The methodology used to isolate the human BDSF-1 gene takes advantage of the fact that molecules such as BDSF-1 have an amino terminal signal sequence which directs certain secreted and membrane-bound proteins through the cellular secretory apparatus.

The human BDSF-1 mRNA was identified by screening of a human fetal brain cDNA library. This library was prepared using mRNA purchased from Clontech, Palo Alto (Cat. no, 6573-1). A signal trap cDNA library was prepared by ligating random primed double stranded cDNA into the expression vector, ptrAP1, resulting in fusions of cDNAs to the reporter, alkaline phosphatase (AP). DNAs from individual clones from this library were prepared by standard techniques and transfected into human embryonic kidney fibroblasts (293T cells). After 48 hours, cell supernatants were collected and assayed for AP activity. Clones giving rise to detectable AP activity in the supernatants of transfected cells were analyzed further by DNA sequencing and the novel clones subjected to further DNA sequencing.

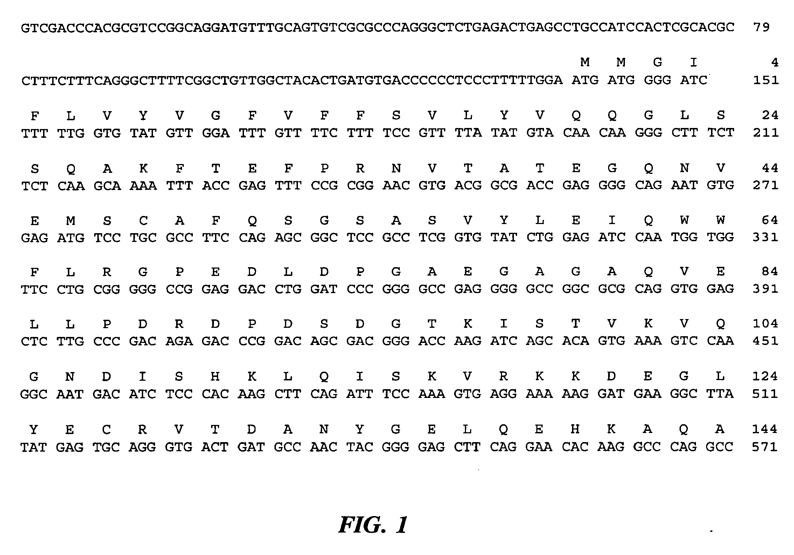

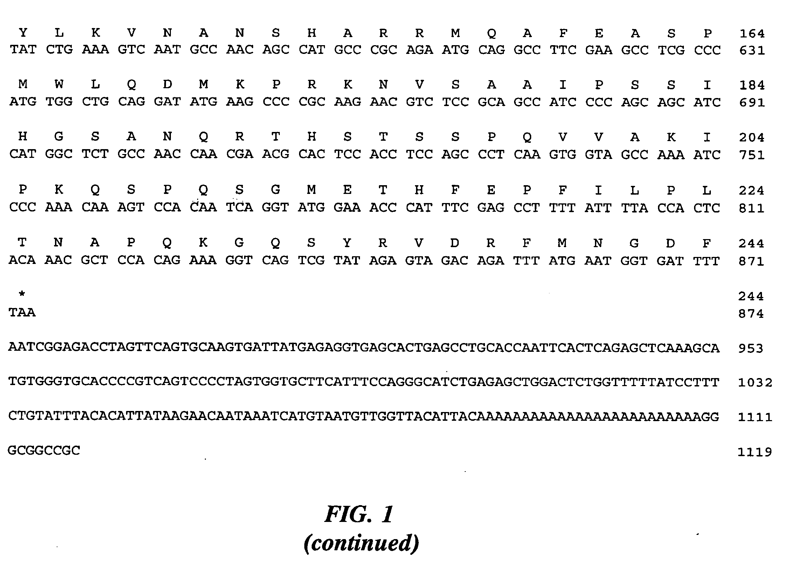

The nucleotide sequence encoding the human BDSF-1 protein is shown in Figure 1 and is set forth as SEQ ID NO: 1. The full length protein encoded by this nucleic acid is comprised of about 244 amino acids and has the amino acid sequence shown in Figure 1 and set forth as SEQ ID NO:2. The coding portion (open reading frame) of SEQ ID NO:1 is set forth as SEQ ID NO:3. Notable features of the human BDSF-1 protein include a signal peptide (about amino acids 1-25 of SEQ ID NO:2), an Ig-like domain (about amino acids 41-129 of SEQ ID NO:2) and two conserved cysteine residues (about amino acids 48 and 127 of SEQ ID NO:2). A clone, comprising the entire coding region of human BDSF-1 has been deposited with the American Type Culture Collection (ATCC), Manassas, Virginia, on May 15, 1998 and assigned Accession Number 98756.

### Isolation of the murine BDSF cDNA

The murine BDSF-1 gene was identified from a murine choroid plexus cDNA library. More specifically, a murine choroid plexus cDNA library was plated out and colonies picked into 96 well plates. The colonies were cultured, plasmids were prepared from each well, and several of the inserts were sequenced. The nucleotide sequences were compared against the human BDSF-1 nucleotide sequence. Upon review of the results from this sequence comparison, a murine BDSF-1 gene was obtained.

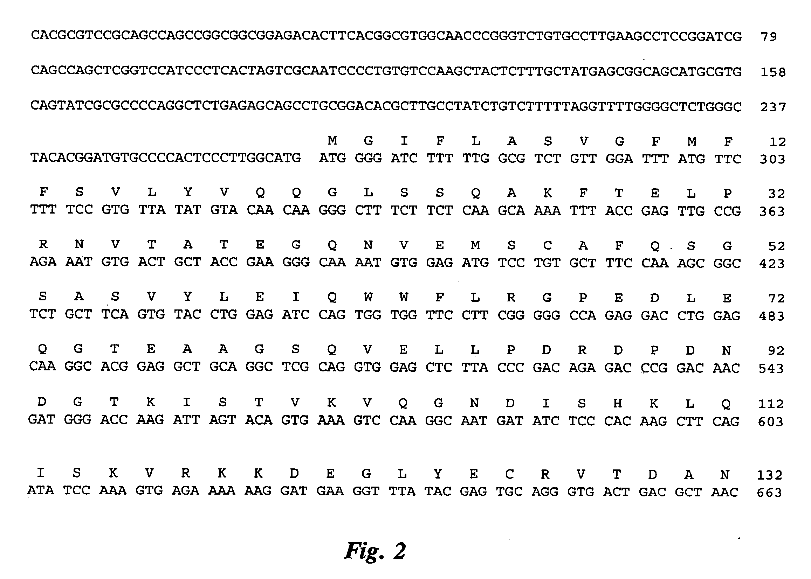

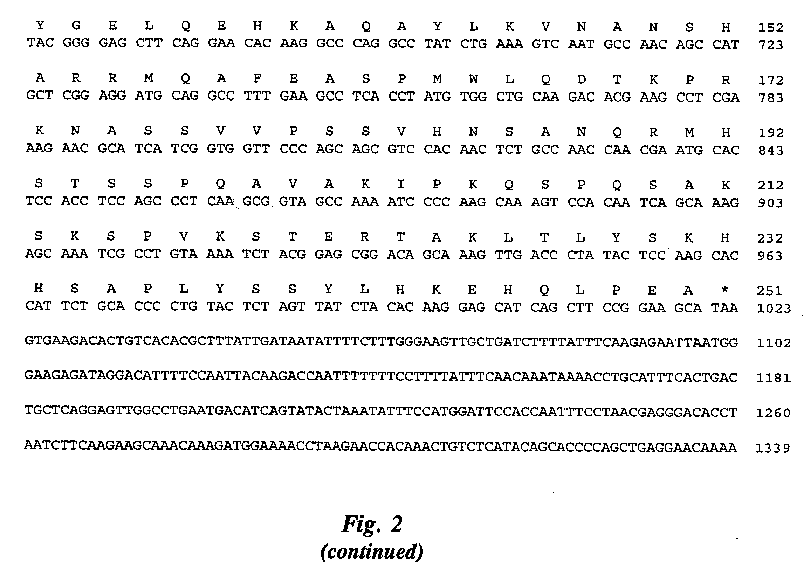

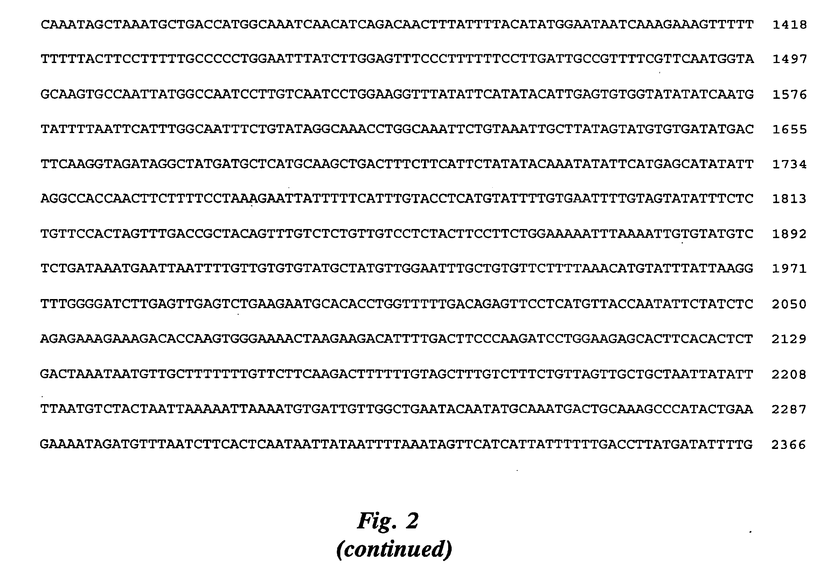

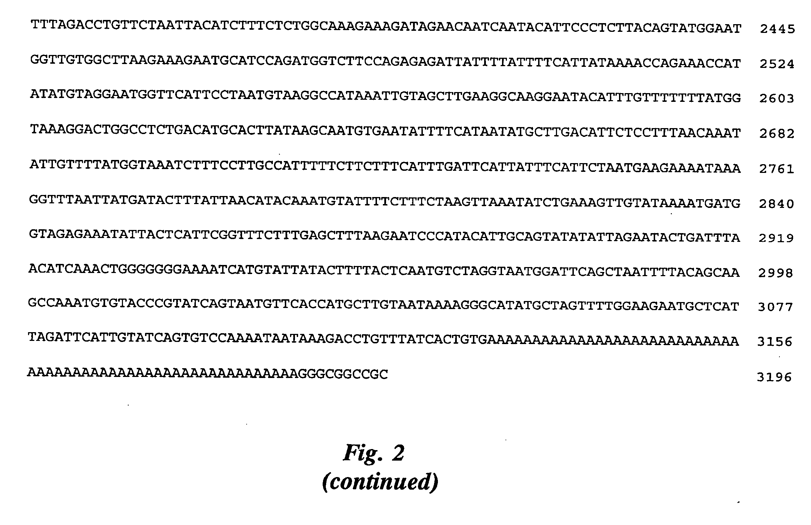

The nucleotide sequence encoding the murine BDSF-1 protein is shown in Figure 2 and is set forth as SEQ ID NO: 6. The full length protein encoded by this nucleic acid is comprised of about 251 amino acids and has the amino acid sequence shown in Figure 2 and set forth as SEQ ID NO:7. The coding portion (open reading frame) of SEQ ID NO:6 is set forth as SEQ ID NO:8. Notable features of the murine BDSF-1 protein include a signal peptide (about amino acids 1-24 of SEQ ID NO:7), an Ig-like domain (about amino acids 40-128 of SEQ ID NO:7) and two conserved cysteine residues (about amino acids 47 and 126 of SEQ ID NO:7).

### Analysis of Human and Murine BDSF

A BLAST search (Altschul et al. (1990) J. Mol. Biol. 215:403) of the nucleotide and protein sequences of human BDSF and murine BDSF has revealed that BDSF does not display significant homology to other proteins except for distant similarity to other Ig-like domain containing proteins. An alignment of human BDSF and murine BDSF, however, indicated that these proteins are 77.4% identical overall and 90.5% identical over the first 211 amino acids. The alignment of human BDSF-1 and murine BDSF-1 is presented in Figure 3. The alignment of human BDSF-1 and murine BDSF-1 was performed using the ALIGN program. When utilizing the ALIGN program for comparing amino acid sequences, a PAM120 weight residue table, a gap length penalty of 12, and a gap penalty of 4 was used.

### Expression of human BDSF

The expression of human BDSF was analyzed using Northern blot hybridization and a probe specific for human BDSF. The DNA was radioactively labeled with ³²P-dCTP using the Prime-It-kit (Stratagene, La Jolla, CA) according to the instructions of the supplier. Filters containing human mRNA (Multi-Tissue Northern I, Multi-Tissue Northern II and Multi-Tissue Northern III from Clontech, Palo Alto, CA) were probed in ExpressHyb hybridization solution (Clontech) and washed at high stringency according to manufacturer's recommendations. In addition, filters containing human brain mRNA (Brain-Subregion Blot from Clontech) were also probed for human BDSF-1 expression.

Results of Northern blot hybridization indicate that human BDSF is expressed as an approximately 5.0 kilobase transcript in all tissues (spleen, lymph node, thymus, peripheral blood leukocytes, bone marrow, stomach, thyroid, spinal cord, trachea, adrenal, testis, small intestine, heart, placenta, lung, liver, kidney and pancreas). BDSF mRNA expression was also observed in human fetal liver.

The highest level of human BDSF expression was found in the adult brain where the pattern of transcripts was also different than for the other tissues. In the brain, major transcripts of 2.6 kb, 3.2 kb and 6.5 kb were observed. This pattern of BDSF mRNA transcripts in the brain is found in the sub-regions of the brain including amygdala, caudate nucleus, hippocampus, substania nigra, sub-thalamate nucleus and thalamus, but not in corpus callosum.

### Example 2: Expression of Recombinant BDSF Protein in Bacterial Cells

BDSF is expressed as a recombinant glutathione-S-transferase (GST) fusion polypeptide in *E. coli* and the fusion polypeptide isolated and characterized. Specifically, human or murine BDSF is fused to GST and this fusion polypeptide is expressed in *E. coli, e.g.,* strain PEB199. Expression of the GST-BDSF fusion protein in PEB199 is induced with IPTG. The recombinant fusion polypeptide is purified from crude bacterial lysates of the induced PEB199 strain by affinity chromatography on glutathione beads. Using polyacrylamide gel electrophoretic analysis of the polypeptide purified from the bacterial lysates, the molecular weight of the resultant fusion polypeptide is determined.

### Example 3: Expression of Recombinant BDSF Protein in COS Cells

To express the human or murine BDSF gene in COS cells, the pcDNA/Amp vector by Invitrogen Corporation (San Diego, CA) is used. This vector contains an SV40 origin of replication, an ampicillin resistance gene, an *E. coli* replication origin, a CMV promoter followed by a polylinker region, and an SV40 intron and polyadenylation site. A DNA fragment encoding the entire BDSF protein and a HA tag (Wilson et al. (1984) Cell 37:767) fused in-frame to its 3' end of the fragment is cloned into the polylinker region of the vector, thereby placing the expression of the recombinant protein under the control of the CMV promoter.

To construct the plasmid, the BDSF DNA sequence is amplified by PCR using two primers. The 5' primer contains the restriction site of interest followed by approximately twenty nucleotides of the BDSF coding sequence starting from the initiation codon; the 3' end sequence contains complementary sequences to the other restriction site of interest, a translation stop codon, the HA tag and the last 20 nucleotides of the BDSF coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the BDSF gene is inserted in the correct orientation. The ligation mixture is transformed into *E. coli* cells (strains HB 101, DH5a, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the BDSF-pcDNA/Amp plasmid DNA using the calcium phosphate or calcium chloride co-precipitation methods, DEAE-dextran-mediated transfection, lipofection, or electroporation. Other suitable methods for transfecting host cells can be found in Sambrook, J., Fritsh, E. F., and Maniatis, T. Molecular Cloning: A Laboratory Manual. 2nd, ed, Cold Spring Harbor Laboratory, Cold Spring Harbor Laboratory Press, Cold Spring Harbor, NY, 1989. The expression of the BDSF protein is detected by radiolabelling (³⁵S-methionine or ³⁵S-cysteine available from NEN, Boston, MA, can be used) and immunoprecipitation (Harlow, E. and Lane, D. Antibodies: A Laboratory Manual, Cold Spring Harbor Laboratory Press, Cold Spring Harbor, NY, 1988) using an HA specific monoclonal antibody. Briefly, the cells are labeled for 8 hours with ³⁵S-methionine (or ³⁵S-cysteine). The culture media are then collected and the cells are lysed using detergents (RIPA buffer, 150 mM NaCl, 1% NP-40, 0.1% SDS, 0.5% DOC, 50 mM Tris, pH 7.5). Both the cell lysate and the culture media are precipitated with an HA specific monoclonal antibody. Precipitated proteins are then analyzed by SDS-PAGE.

Alternatively, DNA containing the BDSF coding sequence is cloned directly into the polylinker of the pCDNA/Amp vector using the appropriate restriction sites. The resulting plasmid is transfected into COS cells in the manner described above, and the expression of the BDSF protein is detected by radiolabelling and immunoprecipitation using a BDSF specific monoclonal antibody

### Example 4: Retroviral Delivery of BDSF

The entire open reading frame of BDSF is subcloned into the retroviral vector MSCVneo, described in Hawley et al. (1994) Gene Therapy 1:136-138. Cells (293Ebna, Invitrogen) are then transiently transfected with the BDSF construct and with constructs containing viral regulatory elements, to produce high titre retrovirus containing the BDSF gene. The virus is then used to transfect mice. These mice are then tested for any gross pathology and for changes in biological response, *e.g.*, cell proliferation and/or differentiation, using standard assays.

### Example 5: Chromosomal Location of BDSF

Human BDSF maps to hu7p12-14 between markers WI-967 and WI-4253 close to the CMT2D (Charcot-Marie-Tooth neuropathy) locus. The syntenic chromosome in mouse, mo11, is in close priximity with the mouse known genes: egfr (epidermal growth factor receptor), ddc (dopa decarboxylase) and cob 1(cordon bleu)). BDSF also mapped close to the following human genes: ADCYAP1R1 (adenylate cyclase activating polypeptide 1), AMPH (amphiphysin), BLVRA (biliverdin reductase A), OGDH (oxoglutarate dehydrogenase), OCM (oncomodulin) and EGFR (epidermal growth factor receptor).

### Example 6: In situ Expression of BDSF

Mouse tissues were analysed *in situ* for expression of BDSF. The *in situ* data (a 6 day film exposure of coronal brain sections from adult mouse brain) demonstrated high punctate expression in the cortex, hypothalamus, hippocampus, and hind brain, indicating that a subset of cells within these regions is positive. Expression in the hippocampus includes, but is not restricted to granule cells. Expression in the cerebellum is limited to purkinje cells. Embryonic sagital sections (day 14.5 - 15.5) had expression in developing brain and spinal cord. For embryonic mouse expression (embryonic sagital sections, day 14.5 and 15.5), signal was detected in the ependyma of the brain and the primordium of the lower incisor tooth.

### Example 7: Identification And Characterization of STMST-1 cDNAs

In this example, the identification and characterization of the genes encoding human STMST-1 and STMST-2 (also referred to as "TANGO123a" (clone Fmhu123a) and "TANGO 123c" (clone Fmhul23c), respectively) is described.

### Isolation of the human STMST cDNAs

The 'signal sequence trapping' method described above was utilized to analyse the sequences of several cDNAs of a cDNA library derived from bronchial epithelial cells which had been stimulated with the cytokine, TNFα. This analysis identified a partial human clone having an insert of approximately 231 kb containing a protein-encoding sequence of approximately 225 nucleotides capable of encoding approximately 75 amino acids of STMST (*e.g.*, the start met through residue 74 of, for example, SEQ ID NO:15). This cDNA was used to re-screen the library. Two full-length cDNA clones were isolated. Sequencing of these clones revealed the nucleotide sequences of human STMST-1 and STMST-2.

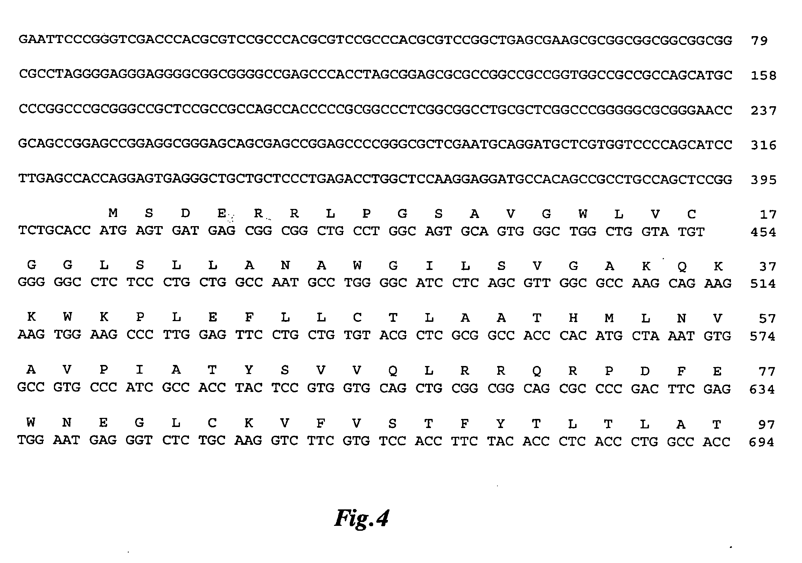

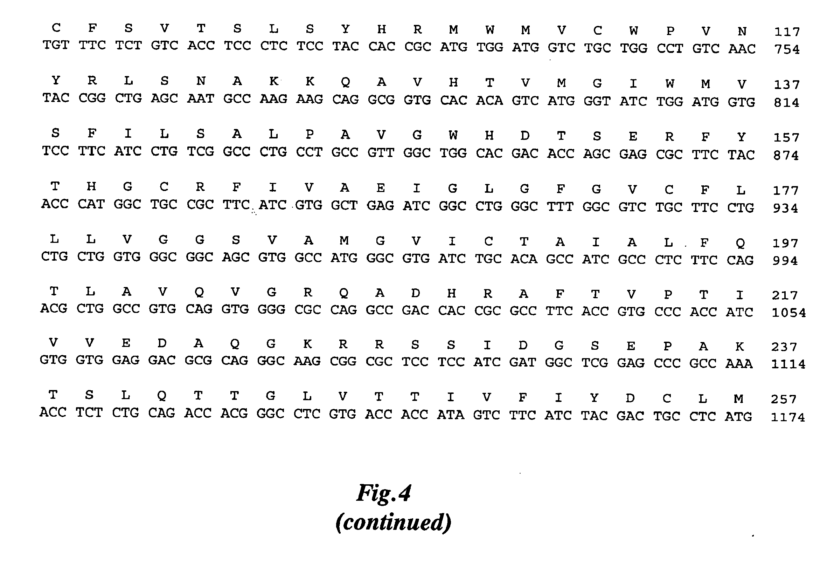

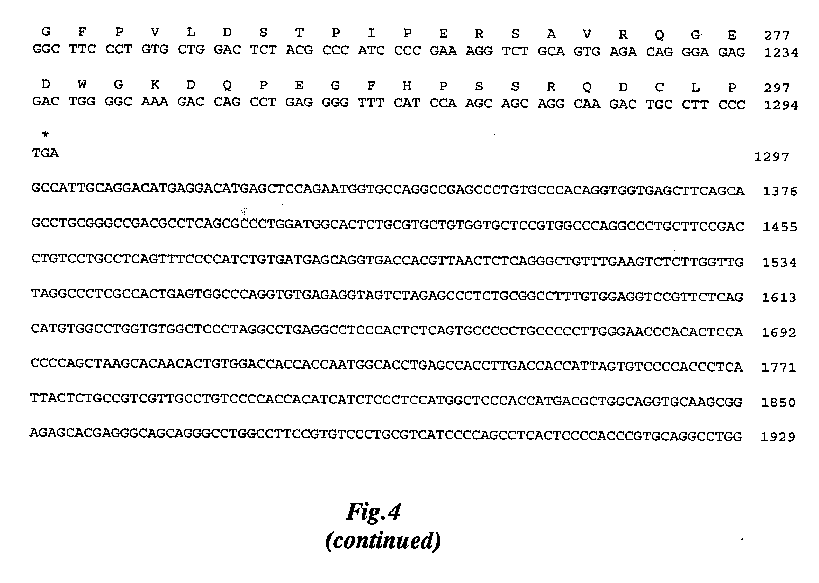

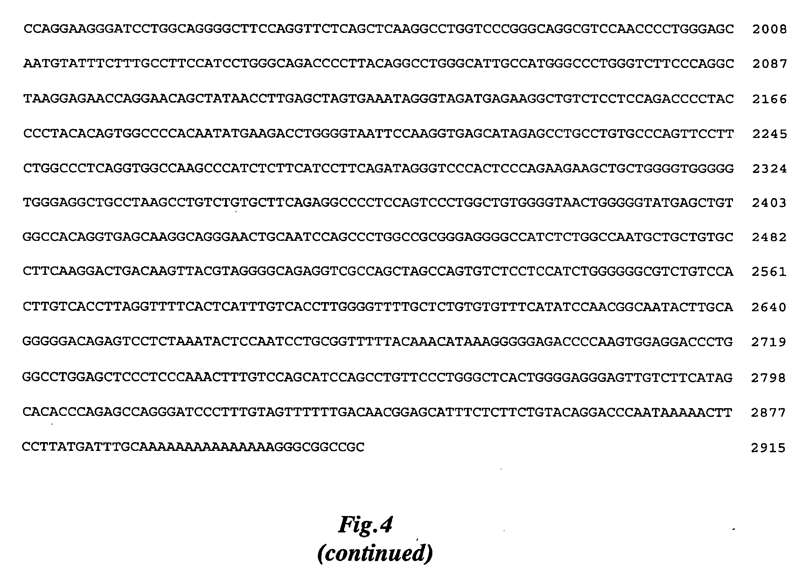

The nucleotide sequence encoding the human STMST-1 protein is shown in Figure 4 and is set forth as SEQ ID N0:14. The full length protein encoded by this nucleic acid is comprised of about 297 amino acids and has the amino acid sequence shown in Figure 4 and set forth as SEQ ID NO:15. The coding portion (open reading frame) of SEQ ID NO:14 is set forth as SEQ ID NO:16.

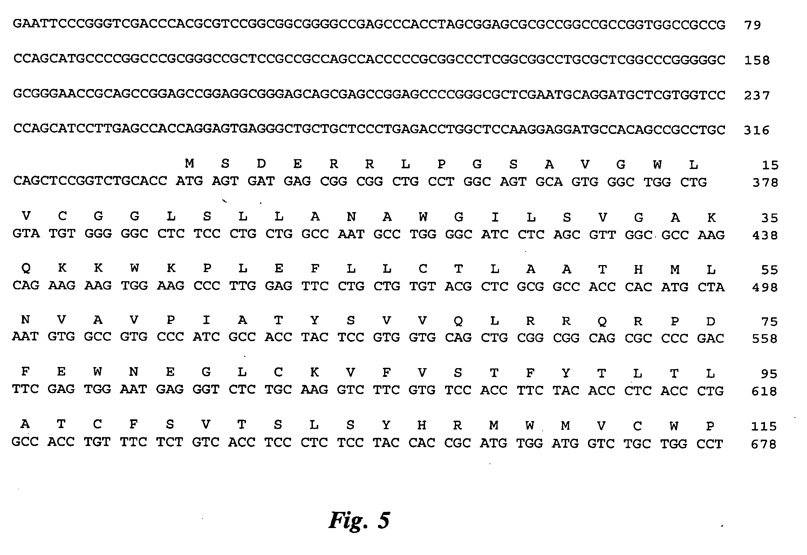

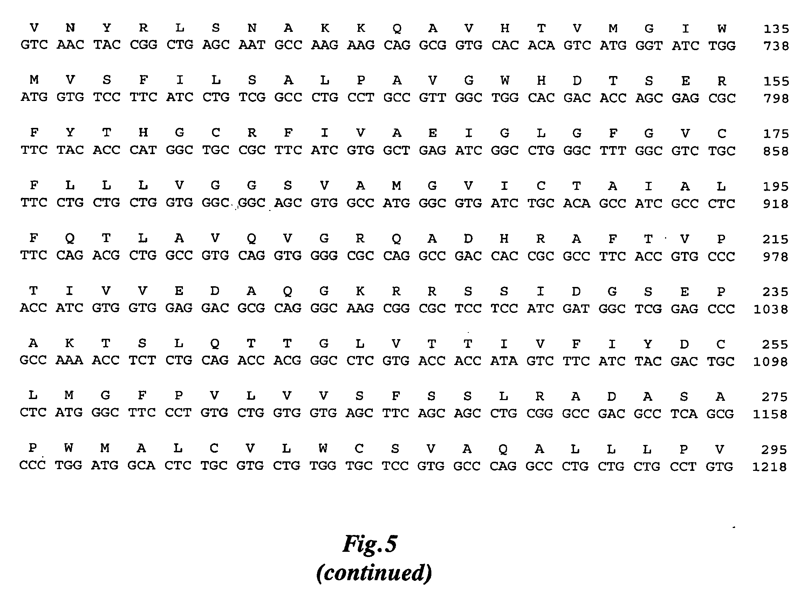

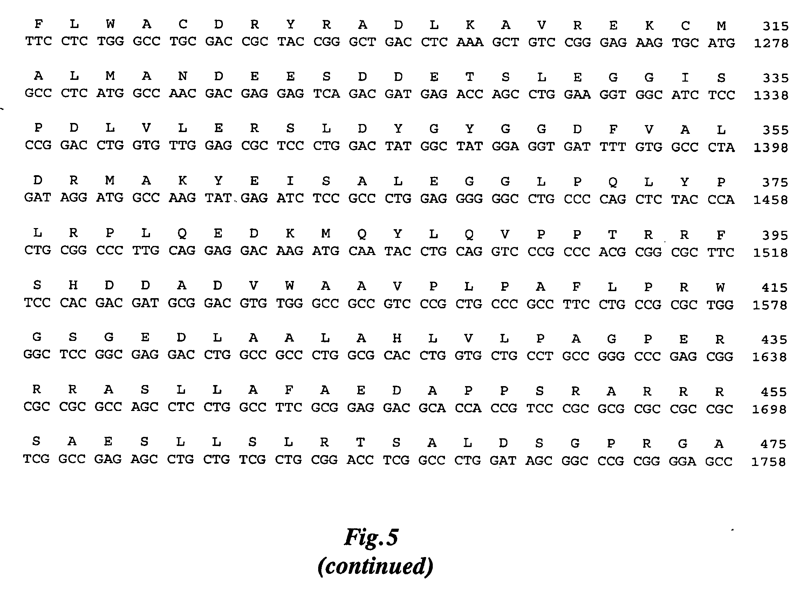

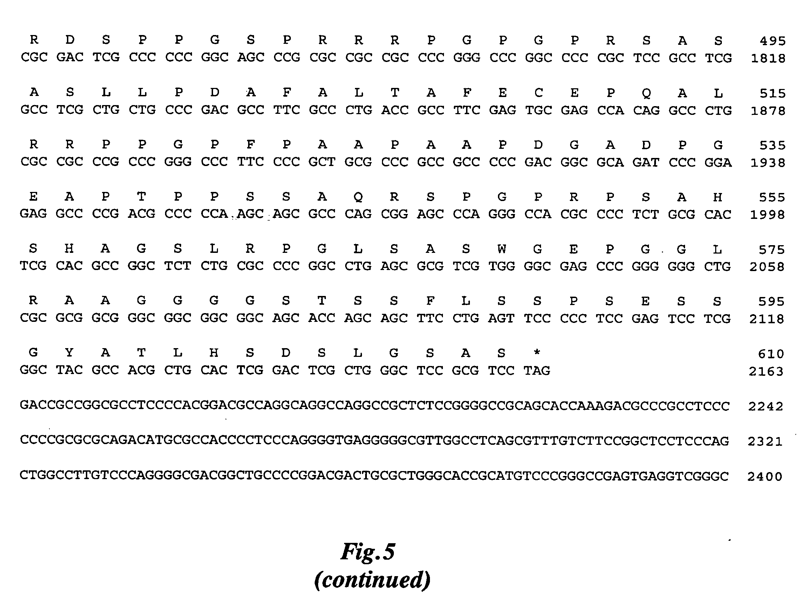

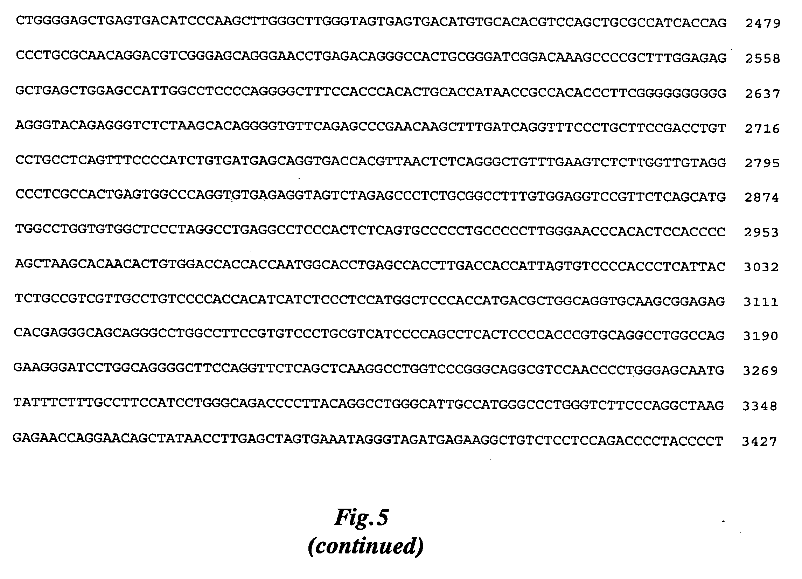

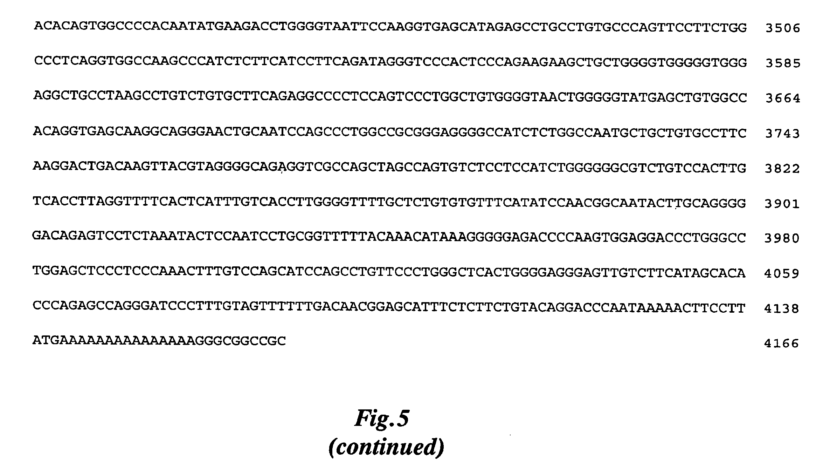

The nucleotide sequence encoding the human STMST-2 protein is shown in Figure 5 and is set forth as SEQ ID NO:17. The full length protein encoded by this nucleic acid is comprised of about 609 amino acids and has the amino acid sequence shown in Figure 5 and set forth as SEQ ID NO: 18. The coding portion (open reading frame) of SEQ ID NO:17 is set forth as SEQ ID NO:19.

### Analysis of Human STMST-1 and STMST-2

Examination of the cDNA sequences depicted in Figures 3 and 4 showed that they were likely encoded by alternatively spliced mRNAs derived from the same gene. Thus, the amino acid sequence of STMST-1 diverges from that of STMST-2 at about amino acid residue 263 of SEQ ID NO:15 or SEQ ID NO:18. The amino acid sequence of STMST-1 lacks the extensive cytoplasmic domain of STMST-2.

A BLAST search (Altschul et al. (1990) J. Mol. Biol. 215:403) of the nucleotide sequence of human STMST-2 has revealed that STMST-2 is significantly similar to a protein identified as protein A-2 (human A-2, Accession No. U47928; murine A-2, Acession No. AC002393) which were sequenced as part of the sequencing of human chromosome 12p13 and mouse chromosome 6, respectively. The human A-2 protein appears to be one of a family of alternatively-spliced gene products which further includes protein A-1 (Acession No. U47925) as well as A-3 (Acession No. U47929). The A-2 proteins, like the STMST proteins of the present invention, include many features indicative of the G protein-coupled receptor family of proteins.

For instance, the STMSTs of the present invention contain conserved cysteines found in the first 2 extracellular loops (prior to the third and fifth transmembrane domains) of most GPCRs (cys 83 and cys 161 of SEQ ID NO:15 or SEQ ID NO:18). A highly conserved asparagine residue in the first transmembrane domain is present (asn25 in SEQ ID NO:15 or SEQ ID NO:18). Transmembrane domain two of the STMST proteins contains a highly conserved leucine (leu49 of SEQ ID N0:15 or SEQ ID NO: 18). The two cysteine residues are believed to form a disulfide bond that stabilizes the functional protein structure. A highly conserved tryptophan and proline in the fourth transmembrane domain of the STMST proteins is present (trp135 and pro 145 of SEQ ID NO:15 or SEQ ID NO: 18). The third cytoplasmic loop contains 49 amino acid residues and is thus the longest cytoplasmic loop of the three, characteristic of G protein coupled receptors. Moreover, a highly conserved proline in the sixth transmembrane domain is present (pro260 of SEQ ID NO:15 and SEQ ID NO:18). The proline residues in the fourth, fifth, sixth, and seventh transmembrane domains are thought to introduce kinks in the alpha-helices and may be important in the formation of the ligand binding pocket. Furthermore, the conserved (in the second cytoplasmic loop) HRM motif found in almost all Rhodopsin family GPCRs is present in the STMST proteins of the instant invention (his107, arg108, met109 of SEQ ID NO:15 or SEQ ID NO:18). (The arginine of the HRM sequence is thought to be the most important amino acid in GPCRs and is invariant). Moreover, an almost invariant proline is present in the seventh transmembrane domain of STMST-2 (pro294 of SEQ ID N0:18).

As such, the STMST family of proteins, like the A-2 family of proteins, are refered to herein as G protein-coupled receptor-like proteins.

STMST-1 is also predicted to contain the following sites: cAMP and cGMP-dependent protein kinase phosphorylation site at aa 225-228 (KRRS); Protein kinase C phosphorylation sites at aa 153-155 (SER) and at aa 290-292 (SSR); Casein kinase II phosphorylation sites at aa 228-231 (SSID) and at aa 291-294 (SRQD); N-myristoylation sites at aa 9-14 (GSAVGW), aa 169-174 (GLGFGV), aa 181-186 (GGSVAM), aa 187-192 (GVICTA), aa 232-237 (GSEPAK), and at aa 244-249 (GLVTTI); Amidation site at aa 223-226 (QGKR).

Likewise, STMST is predicted to contain the following sites: cAMP- and cGMP-dependent protein kinase phosphorylation sites at aa 225-228 (KRRS), aa 393-396 (RRFS), aa 436-439 (RRAS), and at aa 453-456 (RRRS); Protein kinase C phosphorylation sites at aa 253-255 (SER), aa 268-270 (SLR), aa 392-394 (TRR), aa 462-464 (SLR), aa 482-484 (SPR), and at aa 560-562 (SLR); Casein kinase II phosphorylation sites at aa 228-231 (SSID), aa 324-327 (SDDE), aa 328-331 (TSLE), aa 364-367 (SALE), aa 396-399 (SHDD), aa 417-420 (SGED), aa 466-469 (SALD), aa 506-509 (TAFE), aa 568-571 (SWGE), and at aa 590-593 (SPSE); Tyrosine kinase phosphorylation site at aa 342-348 (RSLDYGY); N-myristoylation sites at aa 9-14 (GSAVGW), aa 169-174 (GLGFGV), aa 181-186 (GGSVAM), aa 187-192 (GVICTA), aa 232-237 (GSEPAK), aa 244-249 (GLVTTI), aa 531-536 (GADPGE), aa 564-569 (GLSASW), aa 573-578 (GGLRAA), and at aa 579-584 (GGGGST); Amidation site at aa 223-226 (QGKR).

### Tissue Distribution of STMST-1 mRNA

This Example describes the tissue distribution of STMST mRNA, as determined by Northern blot hybridization.

Northern blot hybridizations with the various RNA samples were performed (Clontech Multi-tissue Northern I and human fetal tissue northern) under standard conditions and washed under stringent conditions. A 4.5 Kb mRNA transcript was detected in heart, brain, placenta, lung, liver, skeletal muscle, fetal brain, fetal lung, and fetal kidney. Expression was highest in fetal brain.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
(a) a nucleic acid molecule comprising a nucleotide sequence which is at least 75% homologous to the nucleotide sequence of SEQ ID NO: 14 or a complement thereof;
(b) a nucleic acid molecule comprising a nucleotide sequence which is at least 80% homologous to the nucleotide sequence of SEQ ID NO:17, or a complement thereof.

2. The isolated nucleic acid molecule of claim 1 which is selected from the group consisting of:
(a) a nucleic acid molecule having the nucleotide sequence of SEQ ID NO: 16 or SEQ ID NO: 19, or a complement thereof;
(b) a nucleic acid molecule comprising the nucleotide sequence of nucleotides 1-403 of SEQ ID NO:1, nucleotides 1295-2915 of SEQ ID NO:14, nucleotides 1-333 of SEQ ID NO:17, or nucleotides 2161-4166 of SEQ ID NO:17;
(c) a nucleic acid molecule having the nucleotide sequence shown in SEQ ID NO: 1 or SEQ ID NO: 17;
(d) a nucleic acid molecule including a nucleotide sequence encoding a protein having an amino acid sequence sufficiently homologous to the amino acid sequence of SEQID NO: 15, or the amino acid sequence of SEQ ID NO: 18;
(e) a nucleic acid molecule including a nucleotide sequence encoding a protein which includes a 7 transmembrane receptor profile, or a protein which includes a spectrin a-chain motif; and
(f) a nucleic acid molecule including a nucleotide sequence at least 350 nucleotides in length and hybridises under stringent conditions to a nucleic acid molecule comprising the nucleotide sequence of SEQ ID NO: 14 or SEQ ID NO: 17.

3. A vector comprising the nucleic acid molecule of claim 1 or 2.

4. The vector of claim 3 which is a recombinant expression vector.

5. A host cell which contains the vector of claim 3.

6. An isolated polypeptide selected from the group consisting of:
(a) a polypeptide encoded by a nucleic acid molecule which hybridizes to a nucleic acid molecule comprising the nucleotide sequence of SEQ ID NO: 14 or SEQ ID NO: 17, or a complement thereof under stringent conditions;
(b) a polypeptide which is encoded by a nucleic acid molecule comprising a nucleotide sequence which is at least 75% homologous to a nucleic acid comprising the nucleotide sequence of SEQ ID NO: 14, or a complement thereof;
(c) a polypeptide which is encoded by a nucleic acid molecule comprising a nucleotide sequence which is at least 80% homologous to a nucleic acid comprising the nucleotide sequence of SEQ ID NO: 17, or a complement thereof;
(d) a polypeptide comprising an amino acid sequence which is at least 60% homologous to the amino acid sequence of SEQ ID NO: 18; and
(e) a polypeptide comprising an amino acid sequence which is at least 75% homologous to the amino acid sequence of SEQ ID NO: 15.

7. The isolated polypeptide of claim 6 having the amino acid sequence of SEQ ID NO: 15 or SEQ ID NO: 18.

8. The polypeptide of claim 6 further comprising heterologous amino acid sequences.

9. An antibody which selectively binds to a polypeptide of claim 6.

10. A method for producing a polypeptide of claim 6 comprising culturing the host cell of claim 5 in suitable medium.

11. A method for detecting STMST expression in a biological sample comprising:
contacting the sample with an agent capable of detecting an STMST nucleic acid molecule, protein or polypeptide; such that
the presence of an STMST nucleic acid molecule protein or polypeptide is detected in the sample.

12. A method for detecting the presence of STMST activity in a biological sample comprising:
contacting the biological sample with an agent capable of detecting an indicator of STMST activity; such that
the presence eof STMST activity is detected in the biological sample.

13. A method of modulating STMST activity comprising contacting the cell with an agent that modulates STMST activity such that STMST activity in the cell is modulated.
